(19) Europäisches Patentamt
European Patent Office
Office européen des brevets

(11) EP 3 035 937 B1

(12) **EUROPEAN PATENT SPECIFICATION**

(45) Date of publication and mention of the grant of the patent:
**31.12.2025 Bulletin 2026/01**

(21) Application number: **14789437.2**

(22) Date of filing: **22.08.2014**

(51) International Patent Classification (IPC):
***A61K 31/52*** (2006.01) ***A61P 9/00*** (2006.01)

(52) Cooperative Patent Classification (CPC):
**A61K 31/277; A61P 9/10; A61P 9/12; A61P 11/00; A61P 13/12;** Y02A 50/30

(86) International application number:
**PCT/US2014/052382**

(87) International publication number:
**WO 2015/027206 (26.02.2015 Gazette 2015/08)**

(54) **METHODS OF TREATING AND PREVENTING ENDOTHELIAL DYSFUNCTION USING BARDOXOLONE METHYL OR ANALOGS THEREOF**

VERFAHREN ZUR BEHANDLUNG UND VORBEUGUNG EINER ENDOTHELFEHLFUNKTION UNTER VERWENDUNG VON BARDOXOLONMETHYL ODER ANALOGEN DAVON

PROCÉDÉS DE TRAITEMENT ET DE PRÉVENTION D'UN DYSFONCTIONNEMENT ENDOTHÉLIAL AU MOYEN DE BARDOXOLONE MÉTHYLE OU D'ANALOGUES DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.08.2013 US 201361869527 P**

(43) Date of publication of application:
**29.06.2016 Bulletin 2016/26**

(73) Proprietor: **Reata Pharmaceuticals Holdings, LLC**
**Plano, TX 75024 (US)**

(72) Inventors:
- **CHIN, Melanie Pei-Heng**
  **Irving, TX 75063-2648 (US)**
- **MEYER, Colin J.**
  **Irving, TX 75063-2648 (US)**

(74) Representative: **Potter Clarkson**
**Chapel Quarter**
**Mount Street**
**Nottingham NG1 6HQ (GB)**

(56) References cited:
**WO-A2-2009/146216**

- **Q. Q. WU ET AL: "Bardoxolone methyl (BARD) ameliorates ischemic AKI and increases expression of protective genes Nrf2, PPAR , and HO-1", AMERICAN JOURNAL OF PHYSIOLOGY. RENAL PHYSIOLOGY, vol. 300, no. 5, 2 February 2011 (2011-02-02), pages F1180 - F1192, XP055043940, ISSN: 1931-857X, DOI: 10.1152/ajprenal.00353.2010**
- **MCCULLOUGH ET AL: "Cardiac and renal function in patients with type 2 diabetes who have chronic kidney disease; potential effects or bardoxolone methyl.", DRUG DESIGN DEVELOPMENT AND THERAPY, vol. 6, 2012 - 2012, pages 141 - 149, XP002733792**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 035 937 B1

## Description

The present application claims the priority benefit of United States provisional application number 61/869,527, filed August 23, 2013.

## BACKGROUND OF THE INVENTION

### 1. Field of the Invention

The present invention relates generally to the fields of biology and medicine. More particularly, it concerns, in some aspects, methods for treating and/or preventing cardiovascular disease (including patients diagnosed with or at risk for pulmonary arterial hypertension, other forms of pulmonary hypertension, atherosclerosis, restenosis, hyperlipidemia, hypercholesterolemia, metabolic syndrome, or obesity) using bardoxolone methyl.

### 2. Description of Related Art

Diseases of the cardiovascular system frequently involve oxidative stress and inflammation in the affected tissues. Oxidative stress arises in cells when the production of antioxidant proteins, such as glutathione, catalase, and superoxide dismutase, is inadequate to cope with intracellular or local levels of reactive oxygen or nitrogen species, such as superoxide, hydrogen peroxide, and peroxynitrite. Although nitric oxide is an important signaling molecule, its excessive production can also contribute to oxidative stress. Inflammation is a biological process that provides resistance to infectious or parasitic organisms and the repair of damaged tissue. Inflammation is commonly characterized by localized vasodilation, redness, swelling, and pain, the recruitment of leukocytes to a site of infection or injury, production of inflammatory cytokines, such as TNF-$\alpha$ and IL-1, and production of reactive oxygen or nitrogen species. In the later stages of inflammation, tissue remodeling, angiogenesis, and scar formation (fibrosis) may occur as part of the wound healing process. Under normal circumstances, the inflammatory response is regulated and temporary, and resolves in an orchestrated fashion once the infection or injury has been dealt with adequately. However, acute inflammation can become excessive and life-threatening if regulatory mechanisms fail. Alternatively, inflammation can become chronic and cause cumulative tissue damage or systemic complications. Specialized cells activated by pro-inflammatory signaling pathways, such as macrophages, can be a significant source of reactive oxygen and nitrogen species, creating or perpetuating oxidative stress in surrounding tissues. Inflammatory cytokines, such as TNF$\alpha$, IL-6, and gamma-interferon, can also stimulate the production of reactive oxygen/nitrogen species in a variety of cells and thereby promote oxidative stress.

Endothelial dysfunction, the failure of vascular endothelial cells to perform their normal functions, is a common early feature of many cardiovascular diseases and related disorders, including atherosclerosis, hypertension, coronary artery disease, chronic kidney disease, pulmonary hypertension, vascular complications of diabetes, and cardiovascular complications of many chronic diseases. *See, e.g.,* Pepine, 1998. Under normal circumstances, the endothelium (a single layer of cells lining essentially the entire vascular system) regulates the balance between vasoconstriction and vasodilation. It also exerts anticoagulant and antiplatelet properties and provides a physical barrier between the bloodstream and the rest of the body, regulating both cellular trafficking and the passage of fluid into tissue. Known risk factors for cardiovascular disease, including hyperlipidemia, cigarette smoking, and diabetes, are associated with endothelial dysfunction. Damage to the endothelium is believed to be a critical early step in the development of atherosclerotic plaques. Endothelial dysfunction can be detected clinically by elevations in the number of circulating endothelial cells (CECs). *See, e.g.,* Burger (2012).

A hallmark of endothelial dysfunction is impaired endothelium-dependent vasodilation, which is mediated by nitric oxide (NO) produced by endothelial nitric oxide synthase (eNOS), a constitutive form of NOS that is principally expressed in endothelial cells (*e.g.*, Davignon, 2004). In healthy vasculature, NO produced by the endothelium diffuses to vascular smooth muscle cells (VSMC), where it activates guanylate cyclase and stimulates production of cyclic guanosine monophosphate (cGMP), thereby promoting relaxation of the VSMC and, consequently, vasodilation. Other functions of the endothelium (*e.g.*, inhibition of platelet aggregation, inhibition of leukocyte adherence, and inhibition of VSMC proliferation) are also mediated by NO. In dysfunctional endothelium, NO production is impaired. Oxidative stress is a major underlying factor in the development of endothelial dysfunction. Many risk factors associated with cardiovascular disease (*e.g.*, hypertension, activation of the renin/angiotensin system, hypercholesterolemia, cigarette smoking, and diabetes) can activate NADPH oxidases (NOX) in endothelial cells, VSMC, and other cells of the vascular wall. Activation of NOX increases local concentrations of superoxide. This excess superoxide is a direct source of oxidative stress and also can activate other enzymes that produce reactive oxygen species (*e.g.*, xanthine oxidase; Forstermann, 2006). Excess superoxide can also react with NO to form peroxynitrite, which in turn can oxidize (and deplete) tetrahydrobiopterin (BH4), an essential cofactor for the production of NO by eNOS. When BH4 is depleted, eNOS becomes "uncoupled" and

produces superoxide instead of NO, adding to the overall state of oxidative stress (*e.g.*, Forstermann, 2006).

The clinical implications of endothelial dysfunction are significant. Endothelial dysfunction leads to damage of the arterial wall, and is recognized as an early marker for atherosclerosis, occurring before the presence of detectable atherosclerotic plaques (*e.g.*, Davignon, 2004). As noted above, endothelial dysfunction leads to contraction of VSMC, leading to vasoconstriction and hypertension. More generally, endothelial dysfunction is implicated in disorders involving proliferation of VSMC, including restenosis following vascular surgery and pulmonary arterial hypertension (PAH).

Atherosclerosis, the underlying defect leading to many forms of cardiovascular disease, occurs when a physical defect or injury to the lining (endothelium) of an artery triggers endothelial dysfunction and an inflammatory response involving the proliferation of vascular smooth muscle cells and the infiltration of leukocytes into the affected area. Ultimately, a complicated lesion known as an atherosclerotic plaque may form. Such a plaque comprises the above-mentioned cells combined with deposits of cholesterol-bearing lipoproteins and other materials. These plaques can directly interfere with adequate blood circulation or can rupture creating a thrombus (blood clot) that precipitates a heart attack, stroke, or other ischemic event (*e.g.,* Hansson *et al.,* 2006).

Pharmaceutical treatments for cardiovascular disease include preventive treatments, such as the use of drugs intended to lower blood pressure or circulating levels of cholesterol and lipoproteins, as well as treatments designed to reduce the adherent tendencies of platelets and other blood cells (thereby reducing the rate of plaque progression and the risk of thrombus formation). More recently, drugs, such as streptokinase and tissue plasminogen activator, have been introduced and are used to dissolve the thrombus and restore blood flow. Surgical treatments include coronary artery bypass grafting to create an alternative blood supply, balloon angioplasty to compress plaque tissue and increase the diameter of the arterial lumen, and carotid endarterectomy to remove plaque tissue in the carotid artery. Such treatments, especially balloon angioplasty, may be accompanied by the use of stents, expandable mesh tubes designed to support the artery walls in the affected area and keep the vessel open. Recently, the use of drug-eluting stents has become common in order to prevent post-surgical restenosis (renarrowing of the artery) in the affected area. Restenosis is primarily driven by proliferation of VSMC triggered by injury-driven inflammatory signaling and endothelial dysfunction. These devices are wire stents coated with a biocompatible polymer matrix containing a drug that inhibits cell proliferation (*e.g.*, paclitaxel or rapamycin). The polymer allows a slow, localized release of the drug in the affected area with minimal exposure of non-target tissues. Despite the significant benefits offered by such treatments, mortality from cardiovascular disease remains high and significant unmet needs in the treatment of cardiovascular disease remain. MCCULLOUGH ET AL: "Cardiac and renal function in patients with type 2 diabetes who have chronic kidney disease; potential effects or bardoxolone methyl.",DRUG DESIGN DEVELOPMENT AND THERAPY, vol. 6, 2012, - 2012, pages 141-149 discloses a clinical study evaluating bardoxolone methyl's effects on cardiac and renal function in patients with type 2 diabetes who have chronic kidney disease.

Pulmonary hypertension (PH) is a condition in which elevated pressure is found in the pulmonary artery. Pulmonary hypertension (PH) is defined as a resting mean pulmonary artery pressure greater than 25 mmHg. It can lead to right ventricular hypertrophy and right-sided heart failure if it is not successfully treated. Endothelial dysfunction is commonly implicated in the pathogenesis of PH (*e.g.,* Gologanu *et al.,* 2012; Bolignano *et al.,* 2013; Dumitrascu *et al.,* 2013; Kosmadakis *et al.,* 2013; Guazzi and Galie, 2012). Pulmonary hypertension may arise in relation to a variety of conditions. The World Health Organization recognizes five classes of PH (Bolignano *et al.,* 2013): (I) Idiopathic, familial, and associated pulmonary arterial hypertension or PAH; (II) PH associated with left-sided heart disease; (III) PH associated with lung diseases, such as COPD and/or hypoxia (*e.g.*, from sleep apnea); (IV) Chronic thromboembolic PH arising from obstruction of pulmonary arterial vessels; and (V) PH with unclear or multifactorial causes (*e.g.,* dialysis-dependent chronic kidney disease).

Pulmonary arterial hypertension (PAH), a particularly serious subtype of pulmonary hypertension (Class 1 in the WHO classification of PH), may in its origin be idiopathic, familial, secondary to congenital heart disease, secondary to connective tissue disease, secondary to portal hypertension and pulmonary veno-occlusive disease, or related to drug or toxin exposure (*e.g.,* Bolignano *et al.,* 2013). PAH is a disease of the small pulmonary arteries that is characterized by excessive vasoconstriction, fibrosis, thrombosis, pulmonary vascular remodeling, and right ventricular hypertrophy (RVH). Endothelial dysfunction is believed to play a key role in the pathogenesis of this disease (*e.g.*, Humbert, 2004). PAH results in a progressive increase in pulmonary vascular resistance, which ultimately leads to right ventricular failure and death. Although PAH does not metastasize or disrupt tissue boundaries, it shares some common features with cancer, including hyperproliferation and resistance to apoptosis of certain cells (*e.g.,* VSMC) as well as glycolytic metabolism of these proliferating cells (analogous to the well-known Warburg effect in cancer). Activation of transcription factors implicated in cancer (*e.g.*, NF-kappa B and STAT3) has been reported in PAH (*e.g.,* Paulin *et al.,* 2012; Hosokawa, 2013).

Approximately 15,000 - 20,000 patients in the United States are living with PAH. Despite treatment with existing PAH therapies, the 1-year mortality rate for PAH is 15% and the 5-year survival rate is only 22%-38% (Thenappan, 2007). Clearly, improved therapies for PAH are needed.

## SUMMARY OF THE INVENTION

In one aspect, the present invention provides a compound of the formula

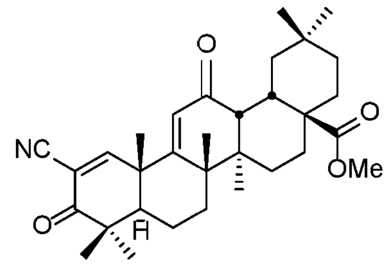

for use in treating or preventing a cardiovascular disease in a patient in need thereof, wherein the patient has been identified as not having at least one of the following characteristics:

(a) a history of left-sided myocardial disease;
(b) an elevated B-type natriuretic peptide (BNP) level;
(c) an elevated albumin/creatinine ratio (ACR); and
(d) chronic kidney disease (CKD).

Described herein, but not falling within the literal scope of the claims, are methods for treating and/or preventing endothelial dysfunction in patients who are diagnosed with or at risk for cardiovascular disease (including patients diagnosed with or at risk for pulmonary arterial hypertension, other forms of pulmonary hypertension, atherosclerosis, restenosis, hyperlipidemia, hypercholesterolemia, metabolic syndrome, or obesity) and other diseases or conditions using bardoxolone methyl and analogs thereof.

Described herein, but not falling within the literal scope of the claims, are methods of treating or preventing endothelial dysfunction in a patient in need thereof, comprising administering to the patient a pharmaceutically effective amount of a compound of the formula:

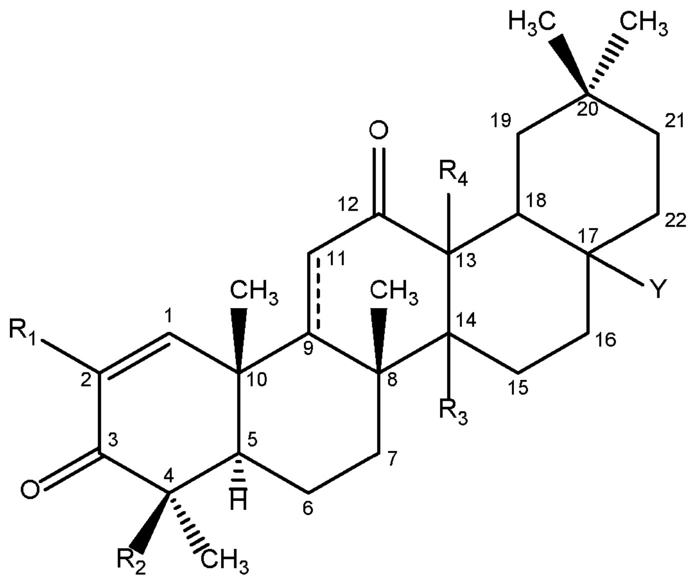

(I),

wherein:

$R_1$ is -CN, halo, $-CF_3$, or $-C(O)R_a$, wherein $R_a$ is -OH, alkoxy$_{(C1-4)}$, $-NH_2$, alkylamino$_{(C1-4)}$, or $-NH-S(O)_2$-alkyl$_{(C1-4)}$;
$R_2$ is hydrogen or methyl;
$R_3$ and $R_4$ are each independently hydrogen, hydroxy, methyl or as defined below when either of these groups is taken together with group $R_c$; and
Y is:

-H, -OH, -SH, -CN, -F, $-CF_3$, $-NH_2$ or -NCO;
alkyl$_{(C\leq8)}$, alkenyl$_{(C\leq8)}$, alkynyl$_{(C\leq8)}$, aryl$_{(C\leq12)}$, aralkyl$_{(C\leq12)}$, heteroaryl$_{(C\leq8)}$, heterocycloalkyl$_{(C\leq12)}$, alkoxy$_{(C\leq8)}$, aryloxy$_{(C\leq12)}$, acyloxy$_{(C\leq8)}$, alkylamino$_{(C\leq8)}$, dialkylamino$_{(C\leq8)}$, alkenylamino$_{(C\leq8)}$, arylamino$_{(C\leq8)}$, aralkylamino$_{(C\leq8)}$, alkylthio$_{(C\leq8)}$, acylthio$_{(C\leq8)}$, alkylsulfonylamino$_{(C\leq8)}$, or substituted versions of any of these groups;
-alkanediyl$_{(C\leq8)}$-$R_b$, -alkenediyl$_{(C\leq8)}$-$R_b$, or a substituted version of any of these groups, wherein $R_b$ is:

hydrogen, hydroxy, halo, amino or thio; or
heteroaryl$_{(C\leq8)}$, alkoxy$_{(C\leq8)}$, alkenyloxy$_{(C\leq8)}$, aryloxy$_{(C\leq8)}$, aralkoxy$_{(C\leq8)}$, heteroaryloxy$_{(C\leq8)}$, acyloxy$_{(C\leq8)}$, alkylamino$_{(C\leq8)}$, dialkylamino$_{(C\leq8)}$, alkenylamino$_{(C\leq8)}$, arylamino$_{(C\leq8)}$, aralkylamino$_{(C\leq8)}$, heteroarylamino$_{(C\leq8)}$, alkylsulfonylamino$_{(C\leq8)}$, amido$_{(C\leq8)}$, -OC(O)NH-alkyl$_{(C\leq8)}$, $-OC(O)CH_2NHC(O)O$-*t*-butyl, $-OCH_2$-alkylthio$_{(C\leq8)}$, or a substituted version of any of these groups;

$-(CH_2)_mC(O)R_c$, wherein m is 0-6 and $R_c$ is:

hydrogen, hydroxy, halo, amino, -NHOH,

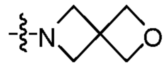,

or thio; or alkyl$_{(C\leq8)}$, alkenyl$_{(C\leq8)}$, alkynyl$_{(C\leq8)}$, aryl$_{(C\leq8)}$, aralkyl$_{(C\leq8)}$, heteroaryl$_{(C\leq8)}$, heterocycloalkyl$_{(C\leq8)}$, alkoxy$_{(C\leq8)}$, alkenyloxy$_{(C\leq8)}$, aryloxy$_{(C\leq8)}$, aralkoxy$_{(C\leq8)}$, heteroaryloxy$_{(C\leq8)}$, acyloxy$_{(C\leq8)}$, alkylamino$_{(C\leq8)}$, dialkylamino$_{(C\leq8)}$, arylamino$_{(C\leq8)}$,alkylsulfonylamino$_{(C\leq8)}$, amido$_{(C\leq8)}$, -NH-alkoxy$_{(C\leq8)}$, -NH-heterocycloalkyl$_{(C\leq8)}$, -NHC(NOH)-alkyl$_{(C\leq8)}$, -NH-amido$_{(C\leq8)}$, or a substituted version of any of these groups;
$R_c$ and $R_3$, taken together, are -O- or $-NR_d-$, wherein $R_d$ is hydrogen or alkyl$_{(C\leq4)}$; or
$R_c$ and $R_4$, taken together, are -O- or $-NR_d-$, wherein $R_d$ is hydrogen or alkyl$_{(C\leq4)}$; or

$-NHC(O)R_e$, wherein $R_e$ is:

hydrogen, hydroxy, amino; or
alkyl$_{(C\leq8)}$, alkenyl$_{(C\leq8)}$, alkynyl$_{(C\leq8)}$, aryl$_{(C\leq8)}$, aralkyl$_{(C\leq8)}$, heteroaryl$_{(C\leq8)}$, heterocycloalkyl$_{(C\leq8)}$, alkoxy$_{(C\leq8)}$, aryloxy$_{(C\leq8)}$, aralkoxy$_{(C\leq8)}$, heteroaryloxy$_{(C\leq8)}$, acyloxy$_{(C\leq8)}$, alkylamino$_{(C\leq8)}$, dialkylamino$_{(C\leq8)}$, arylamino$_{(C\leq8)}$,or a substituted version of any of these groups;

or a pharmaceutically acceptable salt or tautomer thereof,

wherein the patient has been identified as not having at least one of the following characteristics:

(a) a history of left-sided myocardial disease;
(b) an elevated B-type natriuretic peptide (BNP) level;
(c) an elevated albumin/creatinine ratio (ACR); and
(d) chronic kidney disease (CKD).

In some embodiments, the patient has pulmonary arterial hypertension or exhibits one or more symptoms of pulmonary arterial hypertension. In some embodiments, the patient has been identified as not having at least two of the characteristics. In some embodiments, the patient has been identified as not having at least three of the characteristics. In some embodiments, the patient has been identified as not having all four of the characteristics.

The compound is CDDO-Me. And in some embodiments, at least a portion of the CDDO-Me is present as a polymorphic form, wherein the polymorphic form is a crystalline form having an X-ray diffraction pattern (CuK$\alpha$) comprising significant diffraction peaks at about 8.8, 12.9, 13.4, 14.2 and 17.4 °2$\theta$. In non-limiting examples, the X-ray diffraction pattern (CuK$\alpha$) is substantially as shown in FIG. 1A or FIG. 1B. In other variations, at least a portion of the CDDO-Me is present as a polymorphic form, wherein the polymorphic form is an amorphous form having an X-ray diffraction pattern (CuK$\alpha$) with a halo peak at approximately 13.5 °2$\theta$, substantially as shown in FIG. 1C, and a $T_g$. In some variations, the compound is an amorphous form. In some variations, the compound is a glassy solid form of CDDO-Me, having an X-ray powder diffraction pattern with a halo peak at about 13.5 °2$\theta$, as shown in FIG. 1C, and a $T_g$. In some variations, the $T_g$ value falls within a range of about 120 °C to about 135 °C. In some variations, the $T_g$ value is from about 125 °C to about 130 °C.

In some embodiments, the compound is administered locally. In some embodiments, the compound is administered systemically. In some embodiments, the compound is administered orally, intraadiposally, intraarterially,

intraarticularly, intracranially, intradermally, intralesionally, intramuscularly, intranasally, intraocularly, intrapericardially, intraperitoneally, intrapleurally, intraprostatically, intrarectally, intrathecally, intratracheally, intratumorally, intraumbilically, intravaginally, intravenously, intravesicularlly, intravitreally, liposomally, locally, mucosally, orally, parenterally, rectally, subconjunctivally, subcutaneously, sublingually, topically, transbuccally, transdermally, vaginally, in crèmes, in lipid compositions, via a catheter, via a lavage, via continuous infusion, via infusion, via inhalation, via injection, via local delivery, via localized perfusion, bathing target cells directly, or any combination thereof. For example, in some variations, the compound is administered intravenously, intra-arterially or orally. For example, in some variations, the compound is administered orally.

In some embodiments, the compound is formulated as a hard or soft capsule, a tablet, a syrup, a suspension, a solid dispersion, a wafer, or an elixir. In some variations, the soft capsule is a gelatin capsule. In variations, the compound is formulated as a solid dispersion. In some variations the hard capsule, soft capsule, tablet or wafer further comprises a protective coating. In some variations, the formulated compound comprises an agent that delays absorption. In some variations, the formulated compound further comprises an agent that enhances solubility or dispersibility. In some variations, the compound is dispersed in a liposome, an oil-in-water emulsion or a water-in-oil emulsion.

In some embodiments, the pharmaceutically effective amount is a daily dose from about 0.1 mg to about 500 mg of the compound. In some variations, the daily dose is from about 1 mg to about 300 mg of the compound. In some variations, the daily dose is from about 10 mg to about 200 mg of the compound. In some variations, the daily dose is about 25 mg of the compound. In other variations, the daily dose is about 75 mg of the compound. In still other variations, the daily dose is about 150 mg of the compound. In further variations, the daily dose is from about 0.1 mg to about 30 mg of the compound. In some variations, the daily dose is from about 0.5 mg to about 20 mg of the compound. In some variations, the daily dose is from about 1 mg to about 15 mg of the compound. In some variations, the daily dose is from about 1 mg to about 10 mg of the compound. In some variations, the daily dose is from about 1 mg to about 5 mg of the compound. In some variations, the daily dose is from about 2.5 mg to about 30 mg of the compound. In some variations, the daily dose is about 2.5 mg of the compound. In other variations, the daily dose is about 5 mg of the compound. In other variations, the daily dose is about 10 mg of the compound. In other variations, the daily dose is about 20 mg of the compound. In still other variations, the daily dose is about 30 mg of the compound.

In some embodiments, the pharmaceutically effective amount is a daily dose is 0.01 - 25 mg of compound per kg of body weight. In some variations, the daily dose is 0.05 - 20 mg of compound per kg of body weight. In some variations, the daily dose is 0.1 - 10 mg of compound per kg of body weight. In some variations, the daily dose is 0.1 - 5 mg of compound per kg of body weight. In some variations, the daily dose is 0.1 - 2.5 mg of compound per kg of body weight.

In some embodiments, the pharmaceutically effective amount is administered in a single dose per day. In some embodiments, the pharmaceutically effective amount is administered in two or more doses per day.

In some embodiments, the subject is a primate. In some variations, the primate is a human. In other variations, the subject is a cow, horse, dog, cat, pig, mouse, rat or guinea pig.

In some variations of the above methods, the compound is substantially free from optical isomers thereof. In some variations of the above methods, the compound is in the form of a pharmaceutically acceptable salt. In other variations of the above methods, the compound is not a salt.

In some embodiments, the compound is formulated as a pharmaceutical composition comprising (i) a therapeutically effective amount of the compound and (ii) an excipient selected from the group consisting of (A) a carbohydrate, carbohydrate derivative, or carbohydrate polymer, (B) a synthetic organic polymer, (C) an organic acid salt, (D) a protein, polypeptide, or peptide, and (E) a high molecular weight polysaccharide. In some variations, the excipient is a synthetic organic polymer. In some variations, the excipient is selected from the group consisting of a hydroxypropyl methyl cellulose, a poly[1-(2-oxo-1-pyrrolidinyl)ethylene or copolymer thereof, and a methacrylic acid - methylmethacrylate copolymer. In some variations, the excipient is hydroxypropyl methyl cellulose phthalate ester. In some variations, the excipient is PVP/VA. In some variations, the excipient is a methacrylic acid - ethyl acrylate copolymer. In some variations, the methacrylic acid and ethyl acrylate may be present at a ratio of about 1:1. In some variations, the excipient is copovidone.

Any embodiment discussed herein with respect to one aspect of the invention applies to other aspects of the invention as well, unless specifically noted.

Further aspects and embodiments of this invention are elaborated in greater detail, for example, in the claims section.

Other objects, features and advantages of the present disclosure will become apparent from the following detailed description. It should be understood, however, that the detailed description and the specific examples, while indicating specific embodiments of the invention, are given by way of illustration only, since various changes and modifications within the scope of the invention will become apparent to those skilled in the art from this detailed description. Note that simply because a particular compound is ascribed to one particular generic formula does not mean that it cannot also belong to another generic formula.

## BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

**FIGS. 1A-C** - X-ray Powder Diffraction (XRPD) Spectra of Forms A and B of RTA 402. FIG. 1A shows unmicronized Form A; FIG. 1B shows micronized Form A; FIG. 1C shows Form B.

**FIG. 2** - Effect of Bardoxolone Methyl on Circulating Endothelial Cells in Diabetic CKD Patients. Circulating endothelial cells (CECs) and inducible nitric oxide synthase (iNOS) were measured in diabetic CKD patients treated with bardoxolone methyl for 28 (Stratum 1; dose = 25, 75, or 150 mg/day) or 56 days (Stratum 2; dose = 25 mg/day for 28 days and 75 mg/day for days 29-56). Values represent mean change on Day 28 (Stratum 1) or 56 (Stratum 2) compared to baseline + SEM. (402-C-0801). †$p < 0.05$; * $p < 0.01$ *vs.* baseline. Not all patients in either stratum had both baseline and post-treatment samples available.

**FIGS. 3A-D** - Reactive Oxygen Species (ROS) and Nitric Oxide (NO) Levels in Human Endothelial Cells after Treatment with Bardoxolone Methyl (RTA 402) and RTA 403 (CDDO-Im). Confluent human umbilical vein endothelial (HUVEC) cells were treated with the indicated concentrations of bardoxolone methyl or RTA 403 for 48 hours. Toxicity was not observed at the tested concentrations. ROS levels reflect assessment of mitochondrial superoxide using mitoSOX reagent. NO levels were measured using the DAF2-DA assay. AFU = arbitrary fluorescence units. FIG. 3A shows ROS levels after treatment with RTA 402. FIG. 3B shows ROS levels after treatment with RTA 403. FIG. 3C shows NO levels after treatment with RTA 402. FIG. 3D shows NO levels after treatment with RTA 403.

**FIG. 4** - Effect of Bardoxolone Methyl Analog on $ET_A$ and $ET_B$ Receptors in a Rat 5/6 Nephrectomy Model of Pressure-Mediated Chronic Renal Failure (CRF). Bardoxolone methyl analog RTA dh404 suppresses $ET_A$ and induces $ET_B$ receptors in the kidney of the 5/6 nephrectomy model of pressure-mediated chronic renal failure (CRF) in rats. The bardoxolone methyl analog restores normal $ET_A$ levels and partially restores ETB expression, promoting vasodilation. Sprague-Dawley rats were subjected to a sham operation (control) or 5/6 nephrectomy to induce chronic renal failure (CRF). CRF rats were treated with RTA dh404 (2 mg/kg) or vehicle once daily for 12 weeks (N = 9/group). ** $p < 0.01$, *** $p < 0.001$ vs. control; † $p < 0.05$, †† $p < 0.01$ *vs.* CRF.

**FIGS. 5A-B** - Effect of Bardoxolone Methyl on $ET_A$ Expression in Normal Healthy Non-human Primates. Bardoxolone methyl downregulates $ET_A$ receptor expression (~-65%) in normal monkeys; $ET_A$ receptor levels returned to vehicle levels after a 14 day recovery period. No differences were observed on $ET_B$ receptor expression in monkey kidney after bardoxolone administration. BARD animals were dosed orally for 28 days with 30/mg/kg/day BARD in sesame oil. A subgroup of animals was treated with BARD for 28 days and then allowed to recover for 14 days with no further treatment. ** $p < 0.01$ *vs.* vehicle control. FIG. 5A shows $ET_A$ immunohistochemistry. FIG. 5B shows $ET_A$ expression densitometry.

**FIG. 6** - Mean eGFR Over Time in BEACON (Safety Population). Mean observed eGFR over time by treatment week in placebo versus bardoxolone methyl patients. Only includes assessments of eGFR collected on or before a patient's last dose of study drug. Visits are derived relative to a patient's first dose of study drug. Data are means $\pm$ SE.

**FIGS. 7A-B** - Percentage of eGFR Decliners in Bardoxolone Methyl vs. Placebo Patients in BEACON (Safety Population). Percentage of patients with changes in eGFR from baseline of < -3, < -5, or < -7.5 mL/min/1.73 $m^2$ by treatment week in placebo (FIG. 7A) versus bardoxolone methyl (FIG. 7B) patients. Only includes assessments of eGFR collected on or before a patient's last dose of study drug. Visits are derived relative to a patient's first dose of study drug. Percentages calculated relative to number of patients with available eGFR data at each visit.

**FIG. 8** - Time to Composite Primary Outcome Event in BEACON (ITT Population). Results from a randomized, double-blind, placebo-controlled phase 3 study in T2D patients with Stage 4 CKD (BEACON, RTA402-C-0903). Patients were administered placebo or 20 mg of bardoxolone methyl once daily. Analysis includes only ESRD or cardiovascular (CV) death events occurring on or prior to study drug termination date (October 18, 2012) that were positively adjudicated by an independent Event Adjudication Committee, as outlined in the BEACON EAC Charter.

**FIG. 9** - Time to First Hospitalization for Heart Failure or Death Due to Heart Failure Event in BEACON (ITT Population). Analysis includes only heart failure events occurring on or prior to study drug termination date (October 18, 2012) that were positively adjudicated by an independent Event Adjudication Committee, as outlined in the BEACON EAC Charter. Top line is BARD; bottom line is placebo.

**FIG. 10** - Overall Survival of Bardoxolone Methyl vs. Placebo Patients in BEACON. Results from a randomized, double-blind, placebo-controlled phase 3 study in T2D patients with Stage 4 CKD (BEACON, RTA402-C-0903). Patients were administered placebo or 20 mg of bardoxolone methyl once daily. Analysis includes all deaths occurring prior to database lock (March 4, 2013). Top line is BARD; bottom line is placebo.

**FIG. 11** - Mean Serum Magnesium Levels in Bardoxolone Methyl vs. Placebo Patients in BEACON. Mean observed serum magnesium levels over time by treatment week in placebo vs. bardoxolone methyl patients. Only includes

assessments of serum magnesium collected on or before a patient's last dose of study drug. Visits are derived relative to a patient's first dose of study drug. Data are means ± SE. Top line is placebo; bottom line is bardoxolone methyl.

**FIGS. 12A-B** - Changes from Baseline over Time in Systolic (FIG. 12A) and Diastolic (FIG. 12B) Blood Pressure in Bardoxolone Methyl vs. Placebo Patients in BEACON (Safety Population). Data includes only vital assessments collected on or before a patient's last dose of study drug. Visits are derived relative to a patient's first dose of study drug.

**FIGS. 13A-B** - 24-h Ambulatory Blood Pressure Monitoring (ABPM) Sub-Study: Week 4 Changes from Baseline to Week 4 in Systolic (FIG. 13A) and Diastolic (FIG. 13B) Blood Pressure in Bardoxolone Methyl vs. Placebo Patients. Data includes only patients with baseline and WK4 24-h ABPM values. Changes in systolic blood pressure are calculated using the averages of all valid measurements taken from a patient's ambulatory blood pressure monitoring device during the entire 24-h period, daytime (6 A.M. to 10 P.M.), or nighttime (10 P.M to 6 A.M. the next day).

**FIGS. 14A-D** - Placebo-Corrected Changes from Baseline in Systolic Blood Pressure on Study Days 1 and 6 in Healthy Volunteers Administered Bardoxolone Methyl. Results from a multiple-dose, randomized, double-blind, placebo-controlled thorough QT study in healthy volunteers (RTA402-C-1006). Patients were treated with placebo, 20 mg or 80 mg of bardoxolone methyl, or 400 mg of moxifloxacin (active comparator) once daily for 6 consecutive days. Data are mean changes (± SD) from baseline 0-24 hours post-dose on Study Day 1 and Study Day 6. FIG. 14A shows dosing with 20 mg BARD on Study Day 1. FIG. 14B shows dosing with 20 mg BARD on Study Day 6. FIG. 14C shows dosing with 80 mg BARD on Study Day 1. FIG. 14D shows dosing with 80 mg BARD on Study Day 6.

**FIGS. 15A-B** - Placebo-Corrected Changes from Baseline in QTcF in Healthy Volunteers Administered Bardoxolone Methyl. Results from a multiple-dose, randomized, double-blind, placebo-controlled thorough QT study in healthy volunteers (RTA402-C-1006). QTcF interval changes in subjects administered bardoxolone methyl (20 mg or 80 mg - FIG. 15A and FIG. 15B, respectively) are shown relative to changes in patients receiving placebo treatment for 6 consecutive days. Data are mean values ± 90% CI, assessed 0-24 hours post-dose on Study Day 6, where the upper limit of the 90% CI is equivalent to the 1-sided, upper 95% confidence limit. The 10 ms threshold reference line is relevant to the upper confidence limits.

**FIGS. 16A-B** - Kaplan-Meier Plots for Fluid Overload Events in ASCEND (FIG. 16A) and Heart Failure Events in BEACON (ITT Population; FIG. 16B). Time-to-first event analysis for fluid overload events in ASCEND and heart failure in BEACON. Fluid overload events in ASCEND were taken from the adverse event reports of the local investigators. Individual signs and symptoms on the adverse event forms indicating fluid overload included: heart failure, edema, fluid overload, fluid retention, hypervolemia, dyspnea, pleural and pericardial effusions, ascites, weight increase, pulmonary rales, and pulmonary edema. Analysis includes only heart failure events occurring on or prior to study drug termination date (October 18, 2012) that were positively adjudicated by an independent Event Adjudication Committee, as outlined in the BEACON EAC Charter.

**FIGS. 17A-B** - Relationship between Plasma and Urinary Endothelin and eGFR. Scatter plots for eGFR and plasma ET-1 (FIG. 17A) and fractional urinary excretion of ET-1 (FIG. 17B). Blood and urine samples from subjects with CKD (N = 115) and without CKD (N = 27) were collected and assessed for ET-1. Estimated GFR was calculated using the Cockcroft and Gault equation.

**FIG. 18** - Effect of RTA dh404 on Lung Histology in Rat Model of Monocrotaline-Induced Pulmonary Arterial Hypertension. Mean lung histology scores after evaluation by a board-certified veterinary pathologist, based on an increasing severity scale from 0 to 5. Histology scores were analyzed non-parametrically for statistical differences using a one-way ANOVA on ranks followed by a Dunn's post-hoc test with significance set at $p < 0.05$ with Sigmaplot v12.5 (Systat, San Jose, CA).

**FIG. 19** - Effect of RTA dh404 on mRNA Expression of Nrf2 Target Genes in Rat Lung. Data are normalized to the housekeeping gene Rpl19 and presented as mean fold the vehicle control ± S.E.M. **p* < 0.05, ***p* < 0.01, and ****p* < 0.001 vs. vehicle control.

**FIG. 20** - Effect of RTA dh404 on mRNA Expression of NF-κB Target Genes in Rat Lung. Data are normalized to the average of housekeeping genes Ppib and Hprt and presented as mean fold the vehicle control ± S.E.M. **p* < 0.05 and ***p* < 0.01 vs. vehicle control. All values under asterisked lines are significant.

## DESCRIPTION OF ILLUSTRATIVE EMBODIMENTS

In one aspect, the present invention provides a compound of the formula

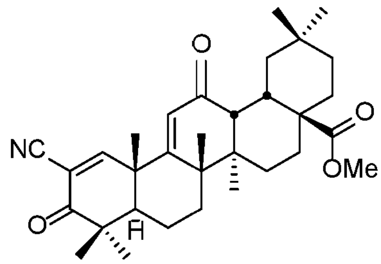

for use in treating or preventing a cardiovascular disease in a patient in need thereof, wherein the patient has been identified as not having at least one of the following characteristics:

(a) a history of left-sided myocardial disease;
(b) an elevated B-type natriuretic peptide (BNP) level;
(c) an elevated albumin/creatinine ratio (ACR); and
(d) chronic kidney disease (CKD).

These and other aspects of the invention are described in greater detail below.

Any references to methods of treatment by therapy in this description are to be interpreted as references to the compound of the present invention for use in those methods.

**I. Characteristics of Patients Who Should Be Excluded from Treatment with Bardoxolone Methyl**

Several clinical studies have shown that treatment with bardoxolone methyl improved markers of renal function (including estimated glomerular filtration rate, or eGFR), insulin resistance, and endothelial dysfunction (Pergola *et al.,* 2011). These observations led to the initiation of a large Phase 3 trial (BEACON) of bardoxolone methyl in patients with stage 4 CKD and type 2 diabetes. The primary endpoint in the BEACON trial was a composite of progression to end-stage renal disease (ESRD) and all-cause mortality. This trial was terminated due to excess severe adverse events and mortality in the group of patients treated with bardoxolone methyl.

As discussed below, subsequent analysis of the data from the BEACON trial showed that most of the severe adverse events and mortality involved heart failure and were highly correlated with the presence of one or more risk factors including: (a) elevated baseline levels of B-type natriuretic peptide (BNP; *e.g.,* >200 pg/mL); (b) baseline eGFR <20; (c) history of left-sided heart disease; (d) high baseline albumin-to-creatinine ratio (ACR; *e.g.,* >300 mg/g as defined by dipstick proteinuria of 3+); and (e) advanced age (*e.g.,* >75 years). The analysis indicated that heart failure events were likely related to the development of acute fluid overload in the first three to four weeks of BARD treatment and that this was potentially due to inhibition of endothelin-1 signaling in the kidney. A previous trial of an endothelin receptor antagonist in stage 4 CKD patients was terminated due to a pattern of adverse events and mortality very similar to that found in the BEACON trial. Subsequent non-clinical studies confirmed that BARD, at physiologically relevant concentrations, inhibits endothelin-1 expression in renal proximal tubule epithelial cells and inhibits endothelin receptor expression in human mesangial and endothelial cells. Accordingly, patients at risk of adverse events from inhibition of endothelin signaling should be excluded from future clinical use of BARD.

The present invention concerns new methods of treating disorders that include endothelial dysfunction as a significant contributing factor. It also concerns the preparation of pharmaceutical compositions for the treatment of such disorders. In the present invention, patients for treatment are selected on the basis of several criteria: (1) diagnosis of a disorder that involves endothelial dysfunction as a significant contributing factor; (2) lack of elevated levels of B-type natriuretic peptide (BNP; *e.g.,* BNP titers must be <200 pg/mL); (3) lack of chronic kidney disease (*e.g.,* eGFR > 60) or lack of advanced chronic kidney disease (*e.g.,* eGFR > 45); (4) lack of a history of left-sided myocardial disease; and (5) lack of a high ACR (*e.g.,* ACR must be <300 mg/g). In some embodiments of the invention, patients with a diagnosis of type 2 diabetes are excluded. In some embodiments of the invention, patients with a diagnosis of cancer are excluded. In some embodiments, patients of advanced age (*e.g.,* >75 years) are excluded. In some embodiments, patients are closely monitored for rapid weight gain suggestive of fluid overload. For example, patients may be instructed to weigh themselves daily for the first four weeks of treatment and contact the prescribing physician if increases of greater than five pounds are observed.

Non-dialysis-dependent CKD-related pulmonary hypertension falls under WHO Class II and dialysis-dependent CKD-related pulmonary hypertension falls under WHO Class V (Bolignano *et al.,* 2013). Only a small percentage of stage 4-5 CKD patients present with WHO Class I pulmonary hypertension (*i.e.* pulmonary arterial hypertension), and, of note, these patients will be excluded according to the criteria above.

### A. BEACON Study

#### 1. Design of Study

Study 402-C-0903, titled "Bardoxolone Methyl Evaluation in Patients with Chronic Kidney Disease and Type 2 Diabetes: The Occurrence of Renal Events" (BEACON) was a phase 3, randomized, double-blind, placebo-controlled, parallel-group, multinational, multicenter study designed to compare the efficacy and safety of bardoxolone methyl (BARD) to placebo (PBO) in patients with stage 4 chronic kidney disease and type 2 diabetes. A total of 2,185 patients were randomized 1:1 to once-daily administration of bardoxolone methyl (20 mg) or placebo. The primary efficacy endpoint of the study was the time-to-first event in the composite endpoint defined as end-stage renal disease (ESRD; need for chronic dialysis, renal transplantation, or renal death) or cardiovascular (CV) death. The study had three secondary efficacy endpoints: (1) change in estimated glomerular filtration rate (eGFR); (2) time-to-first hospitalization for heart failure or death due to heart failure; and (3) time-to-first event of the composite endpoint consisting of non-fatal myocardial infarction, non-fatal stroke, hospitalization for heart failure, or cardiovascular death.

A subset of the BEACON patients consented to additional 24-hour assessments including ambulatory blood pressure monitoring (ABPM) and 24-hour urine collections. An independent Events Adjudication Committee (EAC), blinded to study treatment assignment, evaluated whether renal events, cardiovascular events, and neurological events met the pre-specified definitions of the primary and secondary endpoints. An IDMC, consisting of external clinical experts supported by an independent statistical group, reviewed unblinded safety data throughout the study and made recommendations as appropriate.

#### 2. Demographics and Baseline Characteristics of the Population

Table 1 presents summary statistics on select demographic and baseline characteristics of patients enrolled in BEACON. Demographic characteristics were comparable across the two treatment groups. In all treatment groups combined, the average age was 68.5 years and 57% of the patients were male. The bardoxolone methyl arm had slightly more patients in the age subgroup ≥75 years than the placebo arm (27% in bardoxolone methyl arm versus 24% in the placebo arm). Mean weight and BMI across both treatment groups was 95.2 kg and 33.8 kg/m$^2$, respectively. Baseline kidney function was generally similar in the two treatment groups; mean baseline eGFR, as measured by the 4-variable Modified Diet in Renal Disease (MDRD) equation, was 22.5 mL/min/1.73 m$^2$ and the geometric mean albumin/creatinine ratio (ACR) was 215.5 mg/g for the combined treatment groups.

**Table 1. Select Demographics and Baseline Characteristics of Bardoxolone Methyl (BARD) versus Placebo (PBO) Patients in BEACON (ITT Population)**

| | BARD N = 1088 | PBO N = 1097 | Total N = 2185 |
|---|---|---|---|
| **Sex, n (%)** | | | |
| Male | 626 (58) | 625 (57) | 1251 (57) |
| Female | 462 (42) | 472 (43) | 934 (43) |
| **Age at informed consent (years)** | | | |
| n | 1088 | 1097 | 2185 |
| Mean (SD) | 68.9 (9.7) | 68.2 (9.4) | 68.5 (9.6) |
| Range (min, max) | 32, 92 | 29, 93 | 29, 93 |
| **Age subgroup, n (%)** | | | |
| <75 | 786 (72) | 829 (76) | 1615 (74) |
| ≥75 | 302 (27) | 268 (24) | 570 (26) |
| **Weight (kg)** | | | |
| n | 1087 | 1097 | 2184 |
| Mean (SD) | 95.1 (22.0) | 95.3 (21.1) | 95.2 (21.5) |
| Range (min, max) | 46, 194 | 45, 186 | 45, 194 |

(continued)

| | | | |
|---|---|---|---|
| **BMI (kg/m$^2$)** | | | |
| n | 1087 | 1097 | 2184 |
| Mean (SD) | 33.7 (7.1) | 33.9 (7.2) | 33.8 (7.1) |
| Range (min, max) | 19, 93 | 19, 64 | 19, 93 |
| **eGFR (mL/min/1.73 m$^2$) mean (SD)** | | | |
| n | 1088 | 1097 | 2185 |
| Mean (SD) | 22.4 (4.3) | 22.5 (4.6) | 22.5 (4.5) |
| Range (min, max) | 13, 34 | 13, 58 | 13, 58 |
| **eGFR MDRD subgroup, n (%)** | | | |
| 15 - <20 | 325 (30) | 347 (32) | 672 (31) |
| 20 - <25 | 399 (37) | 366 (33) | 765 (35) |
| 25 - <30 | 311 (29) | 318 (29) | 629 (29) |
| | **BARD N = 1088** | **PBO N = 1097** | **Total N = 2185** |
| **ACR (mg/g) geometric mean** | | | |
| n | 1088 | 1097 | 2185 |
| Geometric mean | 210.4 | 220.7 | 215.5 |
| (95% CI) | (188, 236) | (196, 249) | (198, 234) |
| Range (min, max) | <1, 4581 | <1, 79466 | <1, 79466 |
| **ACR subgroup, n (%)** | | | |
| <30 | 200 (18) | 211 (19) | 411 (19) |
| 30-300 | 348 (32) | 308 (28) | 656 (30) |
| >300 | 540 (50) | 578 (53) | 1118 (51) |
| Patients were administered placebo or 20 mg of bardoxolone methyl once daily. | | | |

**B. BEACON Results**

**1. Effect of Bardoxolone Methyl on eGFR**

The mean eGFR values for bardoxolone methyl-treated and placebo-treated patients are shown in FIG. 6. On average, bardoxolone methyl patients had expected increases in eGFR that occurred by Week 4 of treatment and remained above baseline through Week 48. In contrast, placebo-treated patients on average had unchanged or slight decreases from baseline. The proportion of patients with eGFR declines was markedly reduced in bardoxolone methyl- versus placebo-treated patients (FIG. 7). The eGFR trajectories and the proportions of decliners observed in BEACON after one year of treatment were consistent with modeled expectations and results from the BEAM study (RTA402-C-0804). As shown in Table 2, the number of patients who experienced a renal and urinary disorder serious adverse event (SAE) was lower in the bardoxolone methyl group than in the placebo group (52 *vs.* 71, respectively). Additionally, and as discussed in the following section, slightly fewer ESRD events were observed in the bardoxolone methyl group than in the placebo group. Collectively, these data suggest that bardoxolone methyl treatment did not worsen renal status acutely or over time.

**Table 2. Incidence of Treatment-Emergent Serious Adverse Events in BEACON within Each Primary System Organ Class (Safety Population)**

| MedDRA System Organ Class | Placebo N = 1093 n (%) | | Bardoxolone methyl N = 1092 n (%) | |
|---|---|---|---|---|
| Patients with any serious adverse event | 295 | (27) | 363 | (33) |

(continued)

| MedDRA System Organ Class | Placebo N = 1093 n (%) | | Bardoxolone methyl N = 1092 n (%) | |
|---|---|---|---|---|
| Number of serious adverse events | 557 | | 717 | |
| | | | | |
| Cardiac disorders | 84 | (8) | 124 | (11) |
| Infections and infestations | 63 | (6) | 79 | (7) |
| Renal and urinary disorders | 71 | (6) | 52 | (5) |
| Metabolism and nutrition disorders | 42 | (4) | 51 | (5) |
| Gastrointestinal disorders | 39 | (4) | 46 | (4) |
| Respiratory, thoracic and mediastinal disorders | 32 | (3) | 43 | (4) |
| Nervous system disorders | 35 | (3) | 37 | (3) |
| General disorders and administration site conditions | 20 | (2) | 29 | (3) |
| Vascular disorders | 18 | (2) | 20 | (2) |
| Injury, poisoning and procedural complications | 17 | (2) | 19 | (2) |
| Musculoskeletal and connective tissue disorders | 13 | (1) | 21 | (2) |
| Blood and lymphatic system disorders | 11 | (1) | 20 | (2) |
| Neoplasms benign, malignant and unspecified (incl. cysts and polyps) | 10 | (1) | 11 | (1) |
| Hepatobiliary disorders | 8 | (1) | 4 | (<1) |
| Psychiatric disorders | 3 | (<1) | 3 | (<1) |
| Eye disorders | 2 | (<1) | 3 | (<1) |
| Investigations | 2 | (<1) | 3 | (<1) |
| Reproductive system and breast disorders | 3 | (<1) | 2 | (<1) |
| Skin and subcutaneous tissue disorders | 1 | (<1) | 4 | (<1) |
| Ear and labyrinth disorders | 1 | (<1) | 3 | (<1) |
| Endocrine disorders | 1 | (<1) | 1 | (<1) |
| Immune system disorders | 0 | | 2 | (<1) |
| Surgical and medical procedures | 0 | | 2 | (<1) |

Table includes only serious adverse events with onset more than 30 days after a patient's last dose of study drug. Column header counts and denominators are the number of patients in the safety population. Each patient is counted at most once in each System Organ Class and Preferred Term.

## 2. Primary Composite Outcome in BEACON

Table 3 provides a summary of adjudicated primary endpoints that occurred on or before the date of study termination (October 18, 2012). Despite the slight reduction in the number of ESRD events in the bardoxolone methyl vs. placebo treatment groups, the number of composite primary endpoints was equal in the two treatment groups (HR = 0.98) due to a slight increase in cardiovascular death events, as depicted in plots of time-to-first composite primary event analysis (FIG. 8).

**Table 3. Adjudicated Primary Endpoints in Bardoxolone Methyl (BARD)** vs. **Placebo (PBO) Patients in BEACON (ITT Population)**

| | PBO N = 1097 n (%) | | BARD N = 1088 n (%) | | Hazard ratio (95% CI)[a] | p-value[b] |
|---|---|---|---|---|---|---|
| **Composite primary efficacy outcome** | 69 | (6) | 69 | (6) | 0.98 (0.70, 1.37) | 0.92 |

(continued)

| Patient's first event | | | | | | |
|---|---|---|---|---|---|---|
| End stage renal disease (ESRD) | 51 | (5) | 43 | (4) | | |
| Chronic dialysis | 47 | (4) | 40 | (4) | | |
| Renal transplant | 3 | (<1) | 1 | (<1) | | |
| Renal death | 1 | (<1) | 2 | (<1) | | |
| CV death | 18 | (2) | 26 | (2) | | |

[a] Hazard ratio (bardoxolone methyl/placebo) and 95% confidence interval (CI) were estimated using a Cox proportional hazards model with treatment group, continuous baseline eGFR, and continuous baseline log ACR as covariates. Breslow's method of handling ties in event time was used.
[b] Treatment group comparisons used SAS's Type 3 chi-square test and two-sided p-value associated with the treatment group variable in the Cox proportional hazards model.

### C. Effects of Bardoxolone Methyl on Heart Failure and Blood Pressure

#### 1. Adjudicated Heart Failure in BEACON

The data in Table 4 present a post-hoc analysis of demographic and select laboratory parameters of BEACON patients stratified by treatment group and occurrence of an adjudicated heart failure event. The number of patients with heart failure includes all events through last date of contact (ITT Population).

Comparison of baseline characteristics of patients with adjudicated heart failure events revealed that both bardoxolone methyl-treated and placebo-treated patients with heart failure were more likely to have had a prior history of cardiovascular disease and heart failure and had higher baseline values for B-type natriuretic peptide (BNP) and QTc interval with Fredericia correction (QTcF). Even though the risk for heart failure was higher in the bardoxolone methyl-treated patients, these data suggest that development of heart failure in both groups appeared to be associated with traditional risk factors for heart failure. Baseline ACR was significantly higher in bardoxolone methyl-treated patients with heart failure events than those without. Also of note, the mean baseline level of BNP in patients who experienced heart failure in both treatment groups was meaningfully elevated and suggested that these patients were likely retaining fluid and in sub-clinical heart failure prior to randomization.

**Table 4. Select Demographic and Baseline Characteristics for Bardoxolone Methyl *vs*. Placebo Patients Stratified by Heart Failure Status**

| **Patients** | **With Heart Failure** | | **Without Heart Failure** | | **Total** | |
|---|---|---|---|---|---|---|
| | **BARD** (N = 103) | **PBO** (N = 57) | **BARD** (N = 985) | **PBO** 1040) | **BARD** 1088) | **PBO** 1097) |
| **Age (years),** Mean ± SD | 70.3 ± 9 | 69.2 ± 8.2 | 68.7 ± 9.8 | 68.1 ± 9.5 | 68.9 ± 9.7 | 68.2 ± 9.4 |
| **History of CVD,** N (%) | 80 (78)[a] | 47 (82)[b] | 529 (54) | 572 (55) | 609 (56) | 619 (56) |
| **History of HF,** N (%) | 36 (35)[a] | 21 (37)[b] | 130 (13) | 133 (13) | 166 (15) | 154 (14) |
| **History of MI,** N (%) | 33 (32)[a] | 22 (39)[b] | 185 (19) | 188 (18) | 218 (20) | 210 (19) |
| **History of A-FIB,** N (%) | 4 (4) | 3 (5) | 46 (5) | 40 (4) | 50 (5) | 43 (4) |
| **Concomitant Med Use,** N (%) | | | | | | |
| ACEi/ARB | 35 (34)[a] | 16 (28)[b] | 659 (67) | 701 (67) | 694 (64) | 717 (65) |
| Diuretic | 39 (38)[a] | 15 (26)[b] | 528 (54) | 586 (56) | 567 (52) | 601 (55) |
| Beta-Blocker | 38 (37)[a] | 23 (40) | 482 (49) | 506 (49) | 520 (48) | 529 (48) |
| Statin | 57 (55) | 26 (46)[b] | 640 (65) | 721 (69) | 697 (64) | 747 (68) |
| Calcium Channel Blocker | 25 (24)[a] | 17 (30)[b] | 406 (41) | 467 (45) | 431 (40) | 484 (44) |

(continued)

| | | | | | | |
|---|---|---|---|---|---|---|
| **Concomitant Med Use,** N (%) | | | | | | |
| **eGFR (ml/min/1.73 m$^2$),** Mean ± SD | 21.7 ± 4.6 | 22.2 ± 4.7 | 22.5 ± 4.2 | 22.5 ± 4.6 | 22.4 ± 4.3 | 22.5 ± 4.6 |
| **ACR (mg/g),** Geo Mean | 353.9[a] | 302.0 | 199.3 | 216.9 | 210.4 | 220.7 |
| **SBP (mmHg),** Mean ± SD | 139.5 ± 13.3 | 142.3 ± 11.2 | 139.5 ± 11.6 | 139.6 ± 11.8 | 139.5 ± 11.7 | 139.8 ± 11.8 |
| **DBP (mmHg),** Mean ± SD | 66.4 ± 9.1[a] | 69.1 ± 8.8 | 70.4 ± 8.7 | 70.8 ± 8.6 | 70.1 ± 8.8 | 70.7 ± 8.7 |
| **BNP (pg/mL)** | | | | | | |
| Mean ± SD | 526.0 ± 549.4[a] | 429.8 ± 434.3[b] | 223.1 ± 257.5 | 232.3 ± 347.1 | 251.2 ± 309.1 | 242.7 ± 354.7 |
| >100, N (%) | 78 (76)[a] | 43 (75)[b] | 547 (56) | 544 (52) | 625 (57) | 587 (54) |
| **QTcF (ms)** | | | | | | |
| Mean ± SD | 447.9 ± 31.2[a,c] | 432.5 ± 27.6[b] | 425.3 ± 27.8 | 424.7 ± 27.9 | 427.4 ± 28.9 | 425.1 ± 28 |
| >450, N (%) | 40 (39)[a] | 14 (25) | 170 (17) | 167 (16) | 210 (19) | 181 (16) |

a $p < 0.05$ for BARD patients with HF *vs.* BARD patients without HF
b $p < 0.05$ for PBO patients with HF *vs.* PBO patients without HF
c $p < 0.05$ for BARD *vs*. PBO patients with HF

**2. Assessment of Clinical Parameters Associated with BNP Increases**

As a surrogate of fluid retention, a post-hoc analysis was performed on a subset of patients for whom BNP data were available at baseline and Week 24. Patients in the bardoxolone methyl arm experienced a significantly greater increase in BNP than patients in the placebo arm (Mean ± SD: 225 ± 598 *vs.* 34 ± 209 pg/mL, $p < 0.01$). Also noted was a higher proportion of bardoxolone methyl- vs. placebo-treated patients with increases in BNP at Week 24 (Table 5).

BNP increases at Week 24 did not appear to be related to baseline BNP, baseline eGFR, changes in eGFR, or changes in ACR. However, in bardoxolone methyl-treated patients only, baseline ACR was significantly correlated with Week 24 changes from baseline in BNP, suggesting that the propensity for fluid retention may be associated with baseline severity of renal dysfunction, as defined by albuminuria status, and not with the general changes in renal function, as assessed by eGFR (Table 6).

Further, these data suggest that increases in eGFR, which are glomerular in origin, are distinct anatomically, as sodium and water regulation occurs in the renal tubules.

**Table 5. Analysis of BNP and eGFR Values of Bardoxolone Methyl *vs.* Placebo Patients Stratified by Changes from Baseline in BNP at Week 24**

| WK24 BNP Change | Treatment | N | Median BL BNP | Mean BL eGFR | Mean WK24 ΔeGFR |
|---|---|---|---|---|---|
| **<25% Increase** | PBO | 131 | 119.0 | 23.5 | -0.6 |
| | BARD | 84 | 187.0 | 22.3 | 6.1 |
| **25% to 100% Increase** | PBO | 48 | 102.5 | 22.0 | 0.4 |
| | BARD | 45 | 119.0 | 22.7 | 5.5 |
| **≥100% Increase** | PBO | 37 | 143.5 | 23.1 | 0.1 |
| | BARD | 82 | 155.0 | 21.9 | 7.6 |
| Post-hoc analysis of changes in BNP in BEACON at Week 24. | | | | | |

**Table 6. Correlations between Changes from Baseline in BNP at Week 24 and Baseline ACR in Bardoxolone Methyl vs. Placebo Patients in BEACON**

| Treatment | N | Correlation Coefficient | P-value |
|---|---|---|---|
| PBO | 216 | 0.05 | 0.5 |
| BARD | 211 | 0.20 | <0.01 |
| Post-hoc analysis of changes in BNP in BEACON at Week 24. Only patients with baseline and Week 24 BNP values included in analysis. | | | |

### 3. Serum Electrolytes

No clinically meaningful changes were noted in serum potassium or serum sodium for the subset of patients with 24-hr urine collections (Table 7). The change in serum magnesium levels in bardoxolone methyl-treated patients was consistent with changes observed in prior studies (FIG. 11).

**Table 7. Week 4 Changes from Baseline in Serum Electrolytes in Bardoxolone Methyl vs. Placebo 24-hour ABPM Sub-Study Patients**

| | | Serum Potassium (mmol/L) | | | Serum Sodium (mmol/L) | | | Serum Magnesium (mEq/L) | | |
|---|---|---|---|---|---|---|---|---|---|---|
| | | BL | WK4 | WK4 Δ | BL | WK4 | WK4 Δ | BL | WK4 | WK4 Δ |
| PBO | n | 88 | 87 | 87 | 88 | 87 | 87 | 88 | 87 | 87 |
| | Mean ± SE | 4.8 ± 0.1 | 4.7 ± 0.1 | -0.10 ± 0.04* | 140.2 ± 0.2 | 139.7 ± 0.3 | -0.3 ± 0.2 | 1.72 ± 0.03 | 1.69 ± 0.03 | -0.03 ± 0.02 |
| BARD | n | 83 | 77 | 77 | 83 | 77 | 77 | 83 | 77 | 77 |
| | Mean ± SE | 4.7 ± 0.1 | 4.8 ± 0.1 | 0.10 ± 0.05*† | 140.1 ± 0.3 | 140.3 ± 0.3 | 0.2 ± 0.3 | 1.74 ± 0.02 | 1.53 ± 0.03 | -0.21 0.02*† |
| Data include only BEACON patients enrolled in the 24-hour ABPM sub-study. Changes in serum electrolyte values only calculated for patients with baseline and Week 4 data. * $p$ < 0.05 for Week 4 versus baseline values within each treatment group; † $p$ < 0.05 for Week 4 changes in BARD *vs*. PBO patients. | | | | | | | | | | |

### 4. 24-hour Urine Collections

A subset of patients consented to additional 24-hr assessments (sub-study) of ambulatory blood pressure monitoring (ABPM) and 24-hr urine collection at selected visits. Urinary sodium excretion data from BEACON sub-study patients revealed a clinically meaningful reduction in urine volume and excretion of sodium at Week 4 relative to baseline in the bardoxolone methyl-treated patients (Table 8). These decreases were significantly different from Week 4 changes in urine volume and urinary sodium observed in placebo-treated patients. Also of note, reductions in serum magnesium were not associated with renal loss of magnesium.

Additionally, in a pharmacokinetic study in patients with type 2 diabetes and stage 3b/4 CKD administered bardoxolone methyl for eight weeks (402-C-1102), patients with stage 4 CKD had significantly greater reductions of urinary sodium and water excretion than stage 3b CKD patients (Table 9).

**Table 8. Week 4 Changes from Baseline in 24-hour Urine Volume, Urinary Sodium, and Urinary Potassium in Bardoxolone Methyl vs. Placebo 24-hour ABPM Sub-Study Patients**

| | | Urine Volume (mL) | | | Urinary Sodium (mmol/24 h) | | | Urinary Potassium (mmol/24 h) | | | Urinary Magnesium (mmol/24 h) | | |
|---|---|---|---|---|---|---|---|---|---|---|---|---|---|
| | | BL | WK4 | WK4 Δ | BL | WK4 | WK4 Δ | BL | WK4 | WK4 Δ | BL | WK4 | WK4 Δ |
| **PBO** | n | 87 | 72 | 71 | 81 | 68 | 62 | 81 | 68 | 62 | 59 | 53 | 46 |
| | Mean ± SE | 2053 ± 82 | 1928 ± 89 | -110 ± 71 | 160 ± 8 | 145 ± 8 | -11 ± 9 | 55 ± 3 | 52 ± 3 | -3 ± 3 | 7.5 ± 0.5 | 6.0 ± 0.5 | -0.6 ± 0.4 |
| **BARD** | n | 82 | 64 | 63 | 77 | 61 | 57 | 77 | 61 | 57 | 56 | 43 | 40 |
| | Mean ± SE | 2024 ± 83 | 1792 ± 84 | -247 ± 71* | 164 ± 9 | 140 ± 9 | -27 ± 9* | 60 ± 3 | 52 ± 2 | -7 ± 3* | 7.0 ± 0.4 | 6.0 ± 0.4 | -0.9 ± 0.5 |
| Data include only BEACON patients enrolled in the 24-hour ABPM sub-study. Changes at Week 4 only calculated for patients with baseline and Week 4 data. * $p < 0.05$ for Week 4 versus baseline values within each treatment group; † $p < 0.05$ for Week 4 changes in BARD versus PBO patients. | | | | | | | | | | | | | |

**Table 9. Week 8 Changes from Baseline in 24-h Urine Volume and 24-h Urinary Sodium Bardoxolone Methyl-treated Patients Grouped by CKD Severity (from a Patient Pharmacokinetic Study)**

| CKD Stage | | Urine Volume (mL) | | Urinary Sodium (mmol/24 h) | |
|---|---|---|---|---|---|
| | N | WK8 Δ | p-value | WK8 Δ | p-value |
| Stage 3b | 9 | 355 | 0.04 | -12 | 0.02 |
| Stage 4 | 6 | -610 | | -89 | |
| Patients were treated with 20 mg bardoxolone methyl once daily for 56 consecutive days; post-treatment follow-up visit occurred on Study Day 84. Data are means. Data include patients with baseline and Week 8 data. | | | | | |

**5. Hospital Records from EAC Adjudication Packets**

The first scheduled post-baseline assessment in BEACON was at Week 4. Since many of the heart failure events occurred prior to Week 4, the clinical database provides limited information to characterize these patients. Post-hoc review of the EAC case packets for heart failure cases that occurred prior to Week 4 was performed to assess clinical, vitals, laboratory, and imaging data collected at the time of the first heart failure event (Tables 10 and 11).

Examination of these records revealed common reports of rapid weight gain immediately after randomization, dyspnea and orthopnea, peripheral edema, central/pulmonary edema on imaging, elevated blood pressure and heart rate, and preserved ejection fraction. The data suggest that heart failure was caused by rapid fluid retention concurrent with preserved ejection fraction and elevated blood pressure. The preserved ejection fraction is consistent with clinical characteristics of heart failure caused by diastolic dysfunction stemming from ventricular stiffening and impaired diastolic relaxation. This collection of signs and symptoms differs in clinical characteristics from heart failure with reduced ejection fraction, which occurs because of weakened cardiac pump function or contractile impairment (Vasan *et al.,* 1999). Therefore, rapid fluid accumulation in patients with stuff ventricles and minimal renal reserve likely resulted in increased fluid back-up into the lungs and the noted clinical presentation.

Baseline central laboratory values from the clinical database were compared to local laboratory values obtained on admission for heart failure that were included in the EAC packets. Unchanged serum creatinine, sodium, and potassium concentrations in bardoxolone methyl-treated patients with heart failure events that occurred within the first 4 weeks after randomization (Table 11) suggest that heart failure was not associated with acute renal function decline or acute kidney injury. Overall, the clinical data suggest that the etiology of heart failure is not caused by a direct renal or cardiotoxic effect, but is more likely to be due to sodium and fluid retention.

**Table 10. Post-Hoc Analysis of Cardiovascular Parameters of Bardoxolone Methyl vs. Placebo Patients with Heart Failure Events Occurring Within First 4 Weeks of Treatment**

| | | LVEF | SBP (mmHg) | | | DBP (mmHg) | | | Heart Rate (bpm) | | |
|---|---|---|---|---|---|---|---|---|---|---|---|
| | | HF | BL | HF | Δ | BL | HF | Δ | BL | HF | Δ |
| PBO | n | 4 | 8 | 6 | 6 | 8 | 6 | 6 | 8 | 5 | 5 |
| | Mean ± SE | 49% ± 6% | 141 ± 5 | 148 ± 11 | 4.7 ± 7.2 | 65 ± 3 | 65 ± 5 | 1.2 ± 3.6 | 70 ± 3 | 65 ± 3 | -3.6 ± 2.9 |
| BARD | n | 23 | 42 | 33 | 33 | 42 | 34 | 34 | 42 | 32 | 32 |
| | Mean ± SE | 52% ± 2% | 142 ± 2 | 154 ± 4 | 10.5 ± 3.1 | 67 ± 2 | 75 ± 2 | 7.9 ± 2.1 | 67 ± 1 | 81 ± 3 | 14.5 ± 2.7 |

Post-hoc analyses of heart failure cases in BEACON. Vital signs at baseline calculated from the average of three standard cuff measurements. Vital signs from HF hospitalization gathered from admission notes included in EAC Adjudication packets and represent singular assessments using different BP monitoring equipment. LVEF only assessed during HF hospitalization. Timing of HF admission calculated from event start date and treatment start date and varied from Weeks 0-4 for each patient.

**Table 11. Post-Hoc Analysis of Serum Electrolytes of Bardoxolone Methyl vs. Placebo Patients with Heart Failure Events Occurring Within First 4 Weeks of Treatment**

| | | Serum Creatinine (mg/dL) | | | Serum Sodium (mmol/L) | | | Serum Potassium (mmol/L) | | |
|---|---|---|---|---|---|---|---|---|---|---|
| | | BL | HF | Δ | BL | HF | Δ | BL | HF | Δ |
| PBO | n | 8 | 8 | 8 | 8 | 8 | 8 | 8 | 8 | 8 |
| | Mean ± SE | 3.4 ± 0.2 | 3.3 ± 0.2 | -0.1 ± 0.2 | 140.0 ± 1.0 | 137.0 ± 1.0 | -2.5 ± 0.6 | 4.5 ± 0.2 | 4.4 ± 0.1 | -0.1 ± 0.2 |
| BARD | n | 42 | 38 | 38 | 42 | 30 | 30 | 42 | 34 | 34 |
| | Mean ± SE | 2.8 ± 0.1 | 2.7 ± 0.1 | -0.1 ± 0.1 | 140.0 ± 0.0 | 139.0 ± 1.0 | -1.0 ± 0.5 | 4.7 ± 0.1 | 4.8 ± 0.1 | 0.1 ± 0.1 |

Post-hoc analyses of heart failure cases in BEACON. Baseline clinical chemistries assessed at central laboratory. Clinical chemistries from HF hospitalization gathered from hospital notes included in EAC Adjudication packets and represent assessments made at different local laboratories.

### 6. Blood Pressure in BEACON

Mean changes from baseline in systolic and diastolic blood pressures for bardoxolone methyl-treated and placebo-treated patients, based on the average of triplicate standardized blood pressure cuff measurements collected at each visit, are shown in FIG. 12. Blood pressure was increased in the bardoxolone methyl group relative to the placebo group, with mean increases of 1.9 mmHg in systolic and 1.4 mmHg in diastolic blood pressures noted in the bardoxolone methyl group by Week 4 (the first post-randomization assessment). The increases in systolic blood pressure (SBP) appeared to diminish by Week 32, while diastolic blood pressure (DBP) increases were sustained.

The Week 4 SBP and DBP increases in bardoxolone methyl-treated patients relative to placebo-treated patients were more apparent in the ABPM measurements (FIG. 13). This difference in magnitude could be due to the different techniques that were used or to differences in baseline characteristics in the ABPM sub-study patients. Patients in the ABPM sub-study had a higher baseline ACR than the entire population. Regardless, the data demonstrate that bardoxolone methyl increased blood pressure in the BEACON patient population.

### 7. Blood Pressure Changes in Prior CKD Studies

In an open label, dose-ranging study in type 2 diabetic patients with stage 3b-4 CKD (402-C-0902), no dose-related trend in blood pressure changes or change at any individual dose level was noted following 85 consecutive days of treatment at doses ranging from 2.5 to 30 mg of bardoxolone methyl (amorphous dispersion formulation, as used in BEACON). Post-hoc analysis of blood pressure data stratified by CKD stage suggests that bardoxolone methyl-treated

patients with stage 4 CKD tended to have increases in blood pressure relative to baseline levels, with the effect most appreciable in the three highest dose groups, whereas bardoxolone methyl-treated patients with stage 3b CKD had no apparent change (Table 12). Although sample sizes in the dose groups stratified by CKD stage are small, these data suggest that the effect of bardoxolone methyl treatment on blood pressure may be related to CKD stage.

Blood pressure values from a phase 2b study with bardoxolone methyl (BEAM, 402-C-0804), which used an earlier crystalline formulation of the drug and employed a titration design, were highly variable and despite noted increases in some bardoxolone methyl treatment groups, no clear dose-related trend was observed in blood pressure.

**Table 12. Changes from Baseline in Systolic and Diastolic Blood Pressure in Patients with Type 2 Diabetes and Stage 3b-4 CKD Stratified by Baseline CKD Stage Dosed with Bardoxolone Methyl**

| Dose (mg) | CKD Stage | N | ΔSBP | ΔDBP |
|---|---|---|---|---|
| 2.5 | 3b/4 | 14 | 0.1 ± 4.2 | 0.2 ± 1.8 |
| | 3b | 10 | 0 ± 4.4 | 1 ± 2 |
| | 4 | 4 | 0.3 ± 11 | -1.5 ± 3.9 |
| 5 | 3b/4 | 24 | -1.5 ± 2.3 | -1.4 ± 1.5 |
| | 3b | 19 | -2.1 ± 2 | -1.3 ± 1.4 |
| | 4 | 5 | 0.5 ± 9.1 | -1.4 ± 5.6 |
| 10 | 3b/4 | 24 | -2.4 ± 3.1 | 0.3 ± 1.3 |
| | 3b | 20 | -4.2± 3.4 | -0.3 ± 1.3 |
| | 4 | 4 | 6.1 ± 6.7 | 3.6 ± 4.5 |
| 15 | 3b/4 | 48 | 1.1 ± 2.3 | -1 ± 1.2 |
| | 3b | 26 | -2.2 ± 3.3 | -1.3 ± 1.5 |
| | 4 | 22 | 5 ± 2.8 | -0.6 ± 1.9 |
| 30 | 3b/4 | 12 | 7.2 ± 6.2 | 3.2 ± 2.2 |
| | 3b | 3 | -0.4 ± 13.8 | -1.8 ± 3.9 |
| | 4 | 9 | 9.7 ± 7.3 | 4.7 ± 2.5 |
| Patients were administered 2.5, 5, 10, 15, or 30 mg doses of bardoxolone methyl once daily for 85 days. | | | | |

### 8. Blood Pressure and QTcF in Healthy Volunteers

Intensive blood pressure monitoring was employed in a separate Thorough QT Study, which was conducted in healthy volunteers. In both bardoxolone methyl-treated groups, one given the therapeutic dose, 20 mg, which was also studied in BEACON, and one given the supratherapeutic dose of 80 mg, the change in blood pressure did not differ from changes observed in placebo-treated patients (FIG. 14) after 6 days of once daily administration. Bardoxolone methyl did not increase QTcF as assessed by placebo-corrected QTcF changes (ΔΔQTcF) after 6 days of treatment at 20 or 80 mg (FIG. 15).

Bardoxolone methyl has also been tested in non-CKD disease settings. In early clinical studies of bardoxolone methyl in oncology patients (RTA 402-C-0501, RTA 402-C-0702), after 21 consecutive days of treatment at doses that ranged from 5 to 1300 mg/day (crystalline formulation), no mean change in blood pressure was observed across all treatment groups. Similarly, in a randomized, placebo-controlled study in patients with hepatic dysfunction (RTA 402-C-0701), 14 consecutive days of bardoxolone methyl treatment at doses of 5 and 25 mg/day (crystalline formulation) resulted in mean decreases in systolic and diastolic blood pressure (Table 13).

Collectively, these data suggest that bardoxolone methyl does not prolong the QT interval and does not cause blood pressure increases in patients who do not have baseline cardiovascular morbidity or stage 4 CKD.

**Table 13. Changes from Baseline in Blood Pressure in Patients with Hepatic Dysfunction Treated with Bardoxolone Methyl**

| | | Mean ΔSBP ± SE (mmHg) | | Mean ΔDBP ± SE (mmHg) | |
|---|---|---|---|---|---|
| **Dose** | N | D7 | D14 | D7 | D14 |

(continued)

| | | Mean ΔSBP ± SE (mmHg) | | Mean ΔDBP ± SE (mmHg) | |
|---|---|---|---|---|---|
| **PBO** | 4 | -10 ± 8.5 | -1.3 ± 5.5 | -4.0 ± 2.0 | 0.0 ± 3.1 |
| **5 mg** | 6 | -12.8 ± 5.2 | -8.8 ± 5.1 | -2.0 ± 2.3 | -1.7 ± 3.2 |
| **25 mg** | 6 | -11.5 ± 5.2 | -1.2 ± 3.6 | -4.0 ± 2.8 | -1.5 ± 4.1 |

**9. Summary and Analysis of Heart Failure**

Comparison of baseline characteristics of patients with heart failure events revealed that while the risk for heart failure was higher in the bardoxolone methyl-treated patients, both bardoxolone methyl-treated and placebo-treated patients with heart failure were more likely to have had a prior history of cardiovascular disease and heart failure and on average, had higher baseline ACR, BNP, and QTcF. Thus, development of heart failure in these patients was likely associated with traditional risk factors for heart failure. Additionally, many of the patients with heart failure were in subclinical heart failure prior to randomization, as indicated by their high baseline BNP levels.

As a surrogate of fluid retention after randomization, post-hoc analysis was performed on a subset of patients for whom BNP data were available, and increases were significantly greater in bardoxolone methyl-treated patients *vs*. placebo-treated patients at Week 24, with the BNP increases in bardoxolone methyl-treated patients directly correlated with baseline ACR. Urinary sodium excretion data from BEACON ABPM sub-study patients revealed a clinically meaningful reduction in urine volume and excretion of sodium at Week 4 relative to baseline in the bardoxolone methyl-treated patients only. In another study, urinary sodium levels and water excretion were reduced in stage 4 CKD patients but not in stage 3b CKD patients. Together, these data suggest that bardoxolone methyl differentially affects sodium and water handling, with retention of these more pronounced in patients with stage 4 CKD.

Consistent with this phenotype for fluid retention, post-hoc review of the narrative descriptions for heart failure events provided in hospital admission notes, together with anecdotal reports from investigators, indicates that heart failure events in bardoxolone methyl-treated patients were often preceded by rapid fluid weight gain and were not associated with acute decompensation of the kidneys or heart.

Blood pressure changes, indicative of overall volume status, were also increased in the bardoxolone methyl group relative to the placebo group as measured by standardized blood pressure cuff monitoring in BEACON. Pre-specified blood pressure analysis in healthy volunteer studies demonstrated no changes in either systolic or diastolic blood pressure. While the intent-to-treat (ITT) analyses of phase 2 CKD studies conducted with bardoxolone methyl showed no clear changes in blood pressure, post-hoc analyses of these studies suggest that increases in both systolic and diastolic blood pressure are dependent on CKD stage. Taken together, these data suggest that the effects of bardoxolone methyl treatment on blood pressure may be associated with CKD disease severity.

Thus, the urinary electrolyte, BNP, and blood pressure data collectively support that bardoxolone methyl treatment can differentially affect volume status, having no clinically detectable effect in healthy volunteers or early-stage CKD patients, while likely promoting fluid retention in patients with more advanced renal dysfunction and with traditional risk factors associated with heart failure at baseline. The increases in eGFR are likely due to glomerular effects whereas effects on sodium and water regulation are tubular in origin. As eGFR change was not correlated with heart failure, the data suggest that effects on eGFR and sodium and water regulation are anatomically and pharmacologically distinct.

The increased risk for heart failure and related adverse events with bardoxolone methyl treatment was not observed in prior studies (Table 14). However, since prior studies of bardoxolone methyl enrolled 10-fold fewer patients, the increased risk, if present, may have been undetectable. Moreover, BEACON limited enrollment to patients with stage 4 CKD, a population known to be at higher risk for cardiovascular events relative to patients with stage 3b CKD. Thus, the advanced nature of renal disease and significant cardiovascular risk burden of the BEACON population (manifested, among other markers, by low baseline eGFR, high baseline ACR, and high baseline BNP levels) were likely important factors in the observed pattern of cardiovascular events.

To examine further the relationship between key endpoints in BEACON and clinically meaningful thresholds of traditional risk factors of fluid overload, an additional post-hoc analysis was performed. Various eligibility criteria related to these risk factors were applied to exclude patients at most risk and explore the resulting outcomes from BEACON. Combinations of select criteria, including exclusion of patients with eGFR of 20 mL/min/1.73 $m^2$ or less, markedly elevated levels of proteinuria, and either age over 75 or BNP greater than 200 pg/mL abrogate the observed imbalances (Table 15). Applying these same criteria to SAEs also markedly improves or abrogates the noted imbalances (Table 16). Taken together, these findings suggest utility of these and other renal and cardiovascular risk markers in future selection criteria for clinical studies with bardoxolone methyl.

**Table 14. Frequency of Treatment-Emergent Adverse Events Related to Heart Failure[1] by Primary System Organ Class (SOC) Observed in Prior Chronic Kidney Disease Studies with Bardoxolone Methyl**

| | Study | | 0804 (BEAM) | | | | 0902 | | | | |
|---|---|---|---|---|---|---|---|---|---|---|---|
| | | | BARD (Crystalline) | | | | BARD (SDD) | | | | |
| | SOC | Preferred Term | PBO (N = 57) | 25 mg (N = 57) | 75 mg (N = 57) | 150 mg (N = 56) | 2.5 mg (N = 14) | 5 mg (N = 25) | 10 mg (N = 28) | 15 mg (N = 50) | 30 mg (N = 14) |
| AEs | Metab | Oedema peripheral | 3 (5) | 3 (5) | 1 (2) | 3 (5) | 0 | 0 | 0 | 0 | 1 (7) |
| | | Fluid overload | 0 | 3 (5) | 2 (4) | 0 | - | - | - | - | - |
| | Genrl | Oedema peripheral | 11 (19) | 11 (19) | 10 (18) | 11 (20) | 0 | 3 (12) | 5 (18) | 3 (6) | 3 (21) |
| | | Generalised oedema | 0 | 2 (4) | 0 | 0 | - | - | - | - | - |
| | Resp | Dyspnoea | 5 (9) | 2 (4) | 6 (11) | 4 (7) | 0 | 0 | 0 | 0 | 1 (7) |
| | | Dyspnoea exertional | 0 | 1 (2) | 0 | 3 (5) | 1 (7) | 0 | 0 | 0 | 0 |
| | | Orthopnoea | 1 (2) | 0 | 0 | 0 | - | - | - | - | - |
| | | Pulmonary oedema | 0 | 0 | 1 (2) | 0 | - | - | - | - | - |
| | Inv | Ejection fraction decreased | 0 | 1 (2) | 0 | 0 | - | - | - | - | - |
| | Card | Oedema peripheral | 1 (2) | 4 (7) | 3 (5) | 4 (7) | 0 | 0 | 1 (4) | 1 (2) | 0 |
| | | Cardiac failure congestive | 3 (5) | 2 (4) | 3 (5) | 3 (5) | 0 | 0 | 1 (4) | 0 | 1 (7) |
| | | Dyspnoea paroxysmal nocturnal | 0 | 0 | 1 (2) | 0 | - | - | - | - | - |
| SAEs | Card | Cardiac failure congestive | 3 (5) | 2 (4) | 2 (4) | 2 (4) | 0 | 0 | 1 (4) | 0 | 1 (7) |
| | Genrl | Oedema peripheral | 0 | 0 | 0 | 1 (2) | - | - | - | - | - |
| | Metab | Fluid overload | 0 | 1 (2) | 1 (2) | 0 | - | - | - | - | - |
| | Resp | Dyspnoea | 1 (2) | 0 | 0 | 0 | - | - | - | - | - |
| | | Pulmonary oedema | 0 | 0 | 1 (2) | 0 | - | - | - | - | - |

In 402-C-0804, patients were administered 25, 75, 150 mg of bardoxolone methyl (crystalline formulation) or placebo once daily for 52 weeks. In RTA402-C-0903, patients were administered 2.5, 5, 10, 15, or 30 mg doses of bardoxolone methyl (SDD formulation) once daily for 85 days. 1 Adverse events with preferred terms matching Standardized MedDRA Queries for cardiac failure outlined in the BEACON EAC Charter (Submission Serial 133, dated February 2, 2012).

**Table 15. Effect of Excluding Patients with Select Baseline Characteristics on Primary Endpoints, Heart Failure, and All-Cause Mortality in BEACON**

| Event | Observed | | Eligibility Criteria (N) | | | | | | |
|---|---|---|---|---|---|---|---|---|---|
| | | N | BL BNP ≤ 200 | No h/o HF | BL ACR ≤ 1000 | BL eGFR > 20 | Age ≤ 75 | BL ACR ≤ 1000, eGFR > 20, Age ≤ 75 | BL ACR ≤ 300, eGFR > 20, BNP ≤ 200 |
| **Heart Failure** | BARD | 103 | 22 | 67 | 63 | 56 | 75 | 19 | 5 |
| | PBO | 57 | 16 | 36 | 40 | 37 | 45 | 20 | 3 |
| **All-Cause Death** | BARD | 44 | 14 | 35 | 32 | 27 | 20 | 11 | 5 |
| | PBO | 31 | 8 | 24 | 21 | 18 | 23 | 11 | 4 |
| **ESRD** | BARD | 47 | 12 | 35 | 21 | 18 | 38 | 9 | 1 |
| | PBO | 55 | 22 | 44 | 27 | 14 | 46 | 6 | 1 |
| **Randomized Patients** | BARD | 1088 | 559 | 922 | 798 | 735 | 786 | 368 | 209 |
| | PBO | 1097 | 593 | 943 | 792 | 718 | 829 | 400 | 217 |
| Post-hoc analysis of outcomes in BEACON. Observed totals for number of patients with heart failure, all-cause and cardiovascular deaths, and ESRD includes all events through last date of contact (ITT Population). | | | | | | | | | |

**Table 16. Effect of Excluding Patients with Select Baseline Characteristics on Treatment-Emergent Serious Adverse Events by Primary SOC in BEACON (ITT Population)**

| Primary SOC | All Patients | | BL ACR ≤ 1000, eGFR > 20, Age ≤ 75 | | BL ACR ≤ 300, eGFR > 20, BNP ≤ 200 | |
|---|---|---|---|---|---|---|
| **Treatment** | **PBO (N = 1097)** | **BARD (N = 1088)** | **PBO (N = 400)** | **BARD (N = 368)** | **PBO (N = 217)** | **BARD (N = 209)** |
| Blood and lymphatic system disorders | 11 (1) | 20 (2) | 3 (<1) | 4 (<1) | 2 (<1) | 0 |
| Cardiac disorders | 84 (8) | 124 (11) | 32 (3) | 35 (3) | 10 (1) | 16 (1) |
| Ear and labyrinth disorders | 1 (<1) | 3 (<1) | 1 (<1) | 1 (<1) | 0 | 1 (<1) |
| Endocrine disorders | 1 (<1) | 1 (<1) | 1 (<1) | 1 (<1) | 1 (<1) | 1 (<1) |
| Eye disorders | 2 (<1) | 3 (<1) | 1 (<1) | 1 (<1) | 1 (<1) | 0 |
| Gastrointestinal disorders | 39 (4) | 46 (4) | 13 (1) | 10 (1) | 8 (1) | 7 (1) |
| General disorders and administration site conditions | 20 (2) | 29 (3) | 3 (<1) | 2 (<1) | 2 (<1) | 3 (<1) |
| Hepatobiliary disorders | 8 (1) | 4 (<1) | 2 (<1) | 1 (<1) | 0 | 1 (<1) |
| Immune system disorders | 0 | 2 (<1) | 0 | 0 | 0 | 0 |
| Infections and infestations | 63 (6) | 79 (7) | 20 (2) | 20 (2) | 12 (1) | 9 (1) |
| Injury, poisoning and procedural complications | 17 (2) | 19 (2) | 3 (<1) | 4 (<1) | 0 | 2 (<1) |
| Investigations | 2 (<1) | 3 (<1) | 1 (<1) | 2 (<1) | 0 | 0 |
| Metabolism and nutrition disorders | 42 (4) | 51 (5) | 11 (1) | 14 (1) | 9 (1) | 5 (<1) |
| Musculoskeletal and connective tissue disorders | 13 (1) | 21 (2) | 6 (1) | 9 (1) | 3 (<1) | 6 (1) |
| Neoplasms benign, malignant and unspecified | 10 (1) | 11 (1) | 6 (1) | 3 (<1) | 2 (<1) | 1 (<1) |
| Nervous system disorders | 35 (3) | 37 (3) | 13 (1) | 6 (1) | 9 (1) | 4 (<1) |

(continued)

| Primary SOC | All Patients | | BL ACR ≤ 1000, eGFR > 20, Age ≤ 75 | | BL ACR ≤ 300, eGFR > 20, BNP ≤ 200 | |
|---|---|---|---|---|---|---|
| **Treatment** | **PBO (N = 1097)** | **BARD (N = 1088)** | **PBO (N = 400)** | **BARD (N = 368)** | **PBO (N = 217)** | **BARD (N = 209)** |
| Psychiatric disorders | 3 (<1) | 3 (<1) | 1 (<1) | 2 (<1) | 1 (<1) | 1 (<1) |
| Renal and urinary disorders | 71 (6) | 52 (5) | 14 (1) | 9 (1) | 2 (<1) | 4 (<1) |
| Reproductive system and breast disorders | 3 (<1) | 2 (<1) | 0 | 0 | 0 | 0 |
| Respiratory, thoracic and mediastinal disorders | 32 (3) | 43 (4) | 11 (1) | 15 (1) | 7 (1) | 6 (1) |
| Skin and subcutaneous tissue disorders | 1 (<1) | 4 (<1) | 1 (<1) | 1 (<1) | 1 (<1) | 1 (<1) |
| Surgical and medical procedures | 0 | 2 (<1) | 0 | 1 (<1) | 0 | 1 (<1) |
| Vascular disorders | 18 (2) | 20 (2) | 5 (<1) | 4 (<1) | 2 (<1) | 2 (<1) |
| Post-hoc analyses of treatment-emergent serious adverse events in BEACON. Event totals include only SAEs with onset no more than 30 days after a patient's last dose of study drug. | | | | | | |

**D. Potential Mechanisms of Fluid Overload in BEACON**

Data presented in prior sections suggest that bardoxolone methyl promotes fluid retention in a subset of patients who are at most risk of developing heart failure independent of drug administration. The data suggest that the effects are not associated with acute or chronic renal or cardiac toxicity. Therefore, a comprehensive list of well-established renal mechanisms that affect volume status (Table 17) was explored to determine if any of the etiologies matched the clinical phenotype observed with bardoxolone methyl.

Initial investigation focused on possible activation of the renin-angiotensin-aldosterone system. Activation of this pathway reduces serum potassium due to increased renal excretion. However, bardoxolone methyl did not affect serum potassium and slightly reduced urinary potassium in the BEACON sub-study (Table 7).

Another potential mechanism that was investigated was whether transtubular ion gradient changes may have resulted in sodium and consequent water resorption, since bardoxolone methyl affects serum magnesium and other electrolytes. However, this mechanism also involves potassium regulation, and baseline serum magnesium did not appear to be associated with fluid retention or heart failure hospitalizations.

After other etiologies were excluded for reasons listed in Table 16, suppression of endothelin signaling was the primary remaining potential mechanism of volume regulation that was consistent with the bardoxolone methyl treatment effect in BEACON. Therefore, an extensive investigation of modulation of the endothelin pathway as a potential explanation for fluid retention observed in the BEACON study was conducted.

**Table 17. Established Renal Mechanisms Affecting Volume Status**

| Mechanism | $Na^+$ Retention | $K^+$ Retention | Effect on GFR | Comments |
|---|---|---|---|---|
| **Bardoxolone Methyl** | ↑ | None | ↑ | -↑ $Na^+$ retention independent of $K^+$ in Stage 4 CKD patients, ↑ GFR |
| **Endothelin** | ↓ | None | ↓ | -Suppression of endothelin fits BARD pattern |
| **Endothelial Nitric Oxide (NO)** | ↓ | None | ↑ | -NO ↓ $Na^+$ reabsorption and ↑ GFR<br>-BARD ↑ both $Na^+$ and GFR<br>-BARD has been shown *in vitro* and *in vivo* to increase bioavailable endothelial NO, but $Na^+$ effect is likely independent of NO and GFR changes |

(continued)

| Mechanism | $Na^+$ Retention | $K^+$ Retention | Effect on GFR | Comments |
|---|---|---|---|---|
| **Antidiuretic Hormone (ADH)** | ↑ | ↑ | ↓ at ↑ levels of ADH | -ADH ↑$Na^+$ and $K^+$while ↓GFR<br>-BARD does not affect $K^+$ and ↑ GFR |
| **Transtubular ion gradients** | ↑ with ↑ GFR | ↑ | No direct effect | -Ion gradients have dual effect on $Na^+$ and $K^+$; Cl$^-$ $HCO_3^-$ gradient often generated as $HCO_3^-$ absorption dependent on $Na^+$ absorption<br>-BARD does not affect $K^+$ or $HCO_3^-$ |
| **Renin-Angiotensin-Aldosterone (RAAS)** | ↑ | ↓ | ↑ | -RAAS signaling ↑ K urinary excretion and ↓ serum levels<br>-BARD does not affect $K^+$ levels and has been shown to ↓ AII levels in CKD patients and suppress AII signaling *in vitro* and *in vivo* |
| **Pressure Natriuresis** | ↓ | ↓ | Slight ↑ | -Volume expansion leads to ↑ medullary plasma flow and ↓ hypertonicity; ↓ water absorption in the loop of Henle with ↓ of $Na^+$ and $K^+$<br>-BARD-mediated magnitude of volume expansion unlikely sufficient to promote this effect; BARD ↑ $Na^+$ and does not affect $K^+$ |
| **Prostaglandins ($PGE_2$, $PGI_2$)** | ↓ | Slight ↓ | ↑ | -PGs ↑GFR and ↑$Na^+$ urine excretion<br>-BARD ↑ $Na^+$ retention, not excretion |
| **Natriuretic peptides** | ↓ | Slight ↓ | ↑ | -Natriuretic peptides have divergent effects on $Na^+$ and GFR with slight effect on $K^+$<br>-BNP and other natriuretic peptides ↑ $Na^+$ urine excretion<br>-BARD ↑ $Na^+$retention, not excretion<br>-BARD does no interfere with natriuretic peptides, as GFR would likely ↓ |
| **Peritubular factors** | ↑ with<br>↑ GFR | ↑ with<br>↑ GFR | None | -$Na^+$ and $K^+$ move with GFR<br>-BARD does not affect $K^+$ |
| Mechanisms and characteristics of fluid retention. | | | | |

**1. Modulation of the Endothelin System**

The most directly analogous clinical data for comparison of the effects of known endothelin pathway modulators to the BEACON study are those with the endothelin receptor antagonist (ERA) avosentan. Avosentan was studied in stage 3-4 CKD patients with diabetic nephropathy in the ASCEND study, a large outcomes study to assess the time to first doubling of serum creatinine, ESRD, or death (Mann *et al.,* 2010). While the baseline eGFR in this study was slightly above the mean baseline eGFR in BEACON, patients in the ASCEND study had a mean ACR that was approximately seven-fold higher than BEACON (Table 18). Therefore, the overall cardiovascular risk profile was likely similar between the two studies.

As in BEACON, the ASCEND study was terminated prematurely due to an early imbalance in heart failure hospitalization and fluid overload events. Importantly, avosentan-induced fluid overload-related adverse events, including serious and non-serious, were increased only within the first month of treatment (FIG. 16).

Examination of key endpoints in the ASCEND study reveals an approximate threefold increase in risk of congestive heart failure (CHF) with a modest, non-significant increase in death. Additionally, a small, numerical reduction in ESRD events was also observed. The BEACON study demonstrated similar findings, albeit with a lower incidence of heart failure events. Nonetheless, the two studies showed striking similarities in clinical presentation and timing of heart failure, as well as influences on other key endpoints (Table 19).

**Table 18. Select Demographic and Baseline Characteristics for Patients in ASCEND* and BEACON (ITT Population)**

| BL Characteristic | ASCEND | | | BEACON | |
|---|---|---|---|---|---|
| | PBO (N = 459) | Avosentan 25 mg (N = 455) | Avosentan 50 mg (N = 478) | PBO (N = 1097) | BARD 20 mg (N = 1088) |
| Age | 61 ± 9 | 61 ± 9 | 61 ± 9 | 68 ± 9 | 69 ± 10 |
| History of CHF (% of patients) | 13.5% | 14.5% | 14.4% | 15% | 14% |
| Systolic Blood Pressure (mmHg) | 135 ± 15 | 137 ± 14 | 137 ± 14 | 140 ± 12 | 140 ± 12 |
| BMI ($kg/m^2$) | 30 ± 6 | 30 ± 6 | 30 ± 7 | 34 ± 7 | 34 ± 7 |
| eGFR (mL/min/1.73 $m^2$) | 33 ± 11 | 34 ± 11 | 33 ± 11 | 22 ± 5 | 22 ± 4 |
| Median ACR (mg/g) | 1540 | 1416 | 1474 | 221 | 210 |
| ACEi / ARB (% of patients) | 100% | 100% | 100% | 84% | 85% |
| Diuretics (% of patients) | 65% | 64% | 65% | 64% | 64% |
| *Results from a randomized, double-blind, placebo-controlled trial of 1392 patients with type 2 diabetes and overt nephropathy receiving avosentan (25 or 50 mg) or placebo in addition to continued angiotensin-converting enzyme inhibition and/or angiotensin receptor blockade (ASCEND). | | | | | |

**Table 19. Occurrence of Death, End Stage Renal Disease, or Heart Failure in ASCEND and BEACON (ITT Population)**

| | ASCEND | | | BEACON | |
|---|---|---|---|---|---|
| Event | **PBO** (N = 459) | **Avosentan 25 mg** (N = 455) | **Avosentan 50 mg** (N = 478) | **PBO** (N = 1097) | **BARD 20 mg** (N = 1088) |
| CHF | 2.2% | 5.9%* | 6.1%* | 5.0% | 8.8%* |
| Death | 2.6% | 3.6% | 4.6% | 2.8% | 4.0% |
| ESRD | 6.5% | 4.4% | 5.0% | 4.6% | 4.0% |
| Occurrence of adjudicated CHF, death, and ESRD events in ASCEND and BEACON. In ASCEND, for an event to be qualified as CHF, the patient had to have typical signs and/or symptoms of heart failure and receive new therapy for CHF and be admitted to the hospital for at least 24 hours; ESRD was defined as need for dialysis or renal transplantation or an eGFR < 15 mL/min/1.73 $m^2$. Percentages for BEACON include all CHF and ESRD events through last date of contact and total number of deaths at the time of database lock (March 21, 2013). ESRD in BEACON was defined as need for chronic dialysis, renal transplantation, or renal death; additional details and definitions for heart failure are outlined in the BEACON EAC Charter. * $p < 0.05$ *vs.* placebo. | | | | | |

**2. Mechanism of Endothelin Receptor Antagonist-Induced Fluid Overload**

The role of endothelin in fluid overload has been extensively investigated. Through the use of knock-out models in mice, investigators have demonstrated that acute disruption of the endothelin pathway followed by a salt challenge promotes fluid overload. Specific knock-out of endothelin-1 (ET-1), endothelin receptor type A (ETA), endothelin receptor type B (ETB), or the combination of ETA and ETB have all been shown to promote fluid overload in animals with a clinical phenotype consistent with ERA-mediated fluid overload in patients. These effects are caused by acute activation of the epithelial sodium channel (ENaC), which is expressed in the collecting ducts of the kidney, where it reabsorbs sodium and promotes fluid retention (Vachiery and Davenport, 2009).

**3. Relationship between Plasma and Urinary Endothelin-1 in Humans**

An assessment of plasma and urinary levels of endothelin-1 (ET-1) in humans with eGFR values ranging from stage 5 CKD to supra-normal (8 to 131 mL/min/1.73 $m^2$) has been previously reported (Dhaun *et al.,* 2009). Plasma levels significantly and inversely correlated with eGFR, but due to the modest slope of the curve, meaningful differences of ET-1

were not readily apparent across the large eGFR range assessed. As a surrogate for kidney production of ET-1, the organ where the most ET-1 is produced, fractional excretion of ET-1 was calculated by assessing the plasma and urinary levels of ET-1. From eGFRs >100 to approximately 30 mL/min/1.73 $m^2$, urinary levels were relatively unchanged (FIG. 17). However, ET-1 levels appear to increase exponentially with decreasing eGFR in patients with stage 4 and 5 CKD. These data suggest that renal ET-1 is primarily dysregulated in patients with advanced (stage 4 and 5) CKD. Based on these published data, the inventors hypothesized that the differential effects on fluid handling by bardoxolone methyl, if due to endothelin modulation, could be due to the disparate endogenous production of ET-1 in the kidney, which is meaningfully increased in stage 4 and 5 CKD patients.

### 4. Bardoxolone Methyl Modulates Endothelin Signaling

As described above, bardoxolone methyl reduces ET-1 expression in human cell lines, including mesangial cells found in the kidney as well as endothelial cell. Furthermore, *in vitro* and *in vivo* data suggest that bardoxolone methyl and analogs modulate the endothelin pathway to promote a vasodilatory phenotype by suppressing the vasoconstrictive $ET_A$ receptor and restoring normal levels of the vasodilatory $ET_B$ receptor. Thus, the potent activation of Nrf2-related genes with bardoxolone methyl is associated with suppression of pathological endothelin signaling and facilitates vasodilation by modulating expression of ET receptors.

## E. Rationale for BEACON Termination

### 1. Adjudicated Heart Failure

Hospitalizations for heart failure or death due to heart failure were among the cardiovascular events adjudicated by the EAC. An imbalance in adjudicated heart failure and related events was the major finding that contributed to the early termination of BEACON. Additionally, heart failure-related AEs, such as edema, contributed to a higher discontinuation rate than expected. The overall imbalance in time-to-first adjudicated heart failure appeared to result from the large contribution of events occurring within the first three to four weeks after initiation of treatment. The Kaplan-Meyer analysis shows that after this initial period the event rates between the treatment arms appear to maintain parallel trajectories. The pattern reflected in FIG. 9 suggests an acute, physiologic effect that precipitated hospitalization for heart failure versus a cumulative toxic effect.

### 2. Mortality

At the time of the termination of the study, more deaths had occurred in the bardoxolone methyl group than in the placebo group, and the relationship between mortality and heart failure was unclear. A majority of the fatal outcomes (49 of the 75 deaths) occurring prior to clinical database lock (March 4, 2013) were confirmed as being cardiovascular in nature (29 bardoxolone methyl patients vs. 20 placebo patients). Most of the cardiovascular deaths were classified as "cardiac death - not otherwise specified," based on pre-specified definitions outlined in the BEACON EAC charter. On final analysis, the Kaplan-Meier analysis for overall survival showed no apparent separation until approximately Week 24 (FIG. 10). There were three fatal heart failure events, all in bardoxolone methyl-treated patients. In addition, as reflected in Table 16, the percentage of deaths occurring in patients that were over 75 years old was higher in bardoxolone methyl-treated patients compared to placebo-treated patients. Notably, if patients over 75 years old are excluded, the numbers of fatal events in the bardoxolone methyl arm compared to the placebo arm are 20 and 23, respectively.

### 3. Summary of Other Safety Data from BEACON

In addition to the effects of bardoxolone methyl treatment on eGFR and renal SAEs, the number of hepatobiliary SAEs was reduced in the bardoxolone methyl group relative to the placebo group (4 versus 8, respectively; Table 2), and no Hy's Law cases were observed. The number of neoplasm-related SAEs was also balanced across both groups. Lastly, bardoxolone methyl treatment was not associated with QTc prolongation, as assessed by ECG assessments at Week 24 (Table 20).

**Table 20. Change from Baseline in QTcF at Week 24 in Bardoxolone Methyl versus Placebo Patients in BEACON (Safety Population)**

| | **Observed** | | **Change from baseline** | |
|---|---|---|---|---|
| **Timepoint/ QTcF interval (msec)** | **Placebo N = 1093** | **Bardoxolone methyl N = 1092** | **Placebo N = 1093** | **Bardoxolone methyl N = 1092** |
| n | 719 | 639 | 719 | 637 |
| Mean (SD) | 428.8 (29.2) | 425.8 (26.5) | 3.6 (16.4) | -0.9 (19.2) |
| Range (min, max) | 362, 559 | 355, 518 | -59, 82 | -88, 69 |
| Quartiles (25th, median, 75th) | 408, 426, 445 | 407, 425, 443 | -7, 3, 13 | -13, -1, 10 |
| Data includes only ECG assessments collected on or before a patient's last dose of study drug. Visits are derived relative to a patient's first dose of study drug. | | | | |

### F. BEACON Conclusions

In summary, interrogation of data from studies conducted with bardoxolone methyl revealed that the drug can differentially regulate fluid retention, with no clinically detectable effect in healthy volunteers or early-stage CKD patients, while likely pharmacologically promoting fluid retention in patients with advanced renal dysfunction. Since the development of heart failure in both bardoxolone methyl- and placebo-treated patients was associated with traditional risk factors for heart failure, this pharmacological effect in patients with baseline cardiac dysfunction may explain the increased risk for heart failure with bardoxolone methyl treatment in BEACON. These data suggest that decreasing the overall risk for heart failure in future clinical studies by selecting a patient population with lower baseline risk for heart failure should avoid increases in heart failure associated with bardoxolone methyl treatment. Importantly, the available data show that fluid overload in BEACON was not caused by a direct renal or cardiac toxicity. The clinical phenotype of fluid overload is similar to that observed with ERAs in advanced CKD patients, and preclinical data demonstrate that bardoxolone methyl modulates the endothelin pathway. As acute disruption of the endothelin pathway in advanced CKD patients is known to activate a specific sodium channel (ENaC) that can promote acute sodium and volume retention (Schneider, 2007), these mechanistic data, along with the clinical profile of bardoxolone methyl patients with heart failure, provide a reasonable hypothesis to the mechanism of fluid retention in BEACON. Because compromised renal function may be an important factor that contributes to a patient's inability to compensate for short-term fluid overload, and because relatively limited numbers of patients with earlier stages of CKD have been treated to date, exclusion of patients with CKD (*e.g.,* patients with an eGFR < 60) from treatment with BARD and other AIMs may be prudent and is an element of the present invention.

## II. Compounds for the Treatment or Prevention of Pulmonary Arterial Hypertension, Cardiovascular Disease and Related Disorders.

In one aspect of the present disclosure, there is provided a compound of the formula

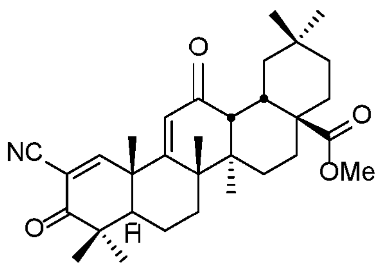

for use in treating or preventing a cardiovascular disease in a patient in need thereof, wherein the patient has been identified as not having at least one of the following characteristics:

(a) a history of left-sided myocardial disease;
(b) an elevated B-type natriuretic peptide (BNP) level;
(c) an elevated albumin/creatinine ratio (ACR); and
(d) chronic kidney disease (CKD).

Described herein, but not falling within the literal scope of the claims, are methods of reducing pulmonary artery pressure in a patient in need thereof comprising administering to the patient bardoxolone methyl or an analog thereof in an amount sufficient to reduce the patient's pulmonary artery pressure. Analogs of bardoxolone methyl include compounds of the formula:

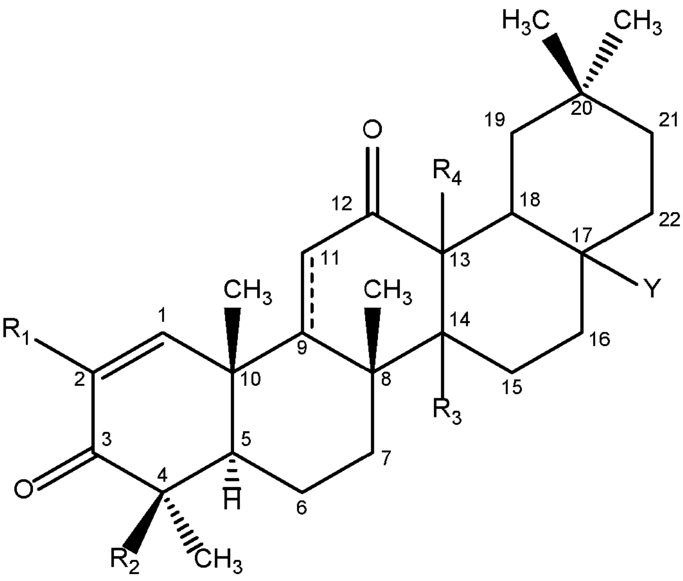

(I),

wherein:

$R_1$ is -CN, halo, $-CF_3$, or $-C(O)R_a$, wherein $R_a$ is -OH, $alkoxy_{(C1-4)}$, $-NH_2$, $alkylamino_{(C1-4)}$, or $-NH-S(O)_2-alkyl_{(C1-4)}$;
$R_2$ is hydrogen or methyl;
$R_3$ and $R_4$ are each independently hydrogen, hydroxy, methyl or as defined below when either of these groups is taken together with group $R_c$; and
Y is:

- H, -OH, -SH, -CN, -F, $-CF_3$, $-NH_2$ or -NCO;
- $alkyl_{(C\leq8)}$, $alkenyl_{(C\leq8)}$, $alkynyl_{(C\leq8)}$, $aryl_{(C\leq12)}$, $aralkyl_{(C\leq12)}$, $heteroaryl_{(C\leq8)}$, $heterocycloalkyl_{(C\leq12)}$, $alkoxy_{(C\leq8)}$, $aryloxy_{(C\leq12)}$, $acyloxy_{(C\leq8)}$, $alkylamino_{(C\leq8)}$, $dialkylamino_{(C\leq8)}$, $alkenylamino_{(C\leq8)}$, $arylamino_{(C\leq8)}$, $aralkylamino_{(C\leq8)}$, $alkylthio_{(C\leq8)}$, $acylthio_{(C\leq8)}$, $alkylsulfonylamino_{(C\leq8)}$, or substituted versions of any of these groups;
- $alkanediyl_{(C\leq8)}-R_b$, $-alkenediyl_{(C\leq8)}-R_b$, or a substituted version of any of these groups, wherein $R_b$ is:

  hydrogen, hydroxy, halo, amino or thio; or
  $heteroaryl_{(C\leq8)}$, $alkoxy_{(C\leq8)}$, $alkenyloxy_{(C\leq8)}$, $aryloxy_{(C\leq8)}$, $aralkoxy_{(C\leq8)}$, $heteroaryloxy_{(C\leq8)}$, $acyloxy_{(C\leq8)}$, $alkylamino_{(C\leq8)}$, $dialkylamino_{(C\leq8)}$, $alkenylamino_{(C\leq8)}$, $arylamino_{(C\leq8)}$, $aralkylamino_{(C\leq8)}$, $heteroarylamino_{(C\leq8)}$, $alkylsulfonylamino_{(C\leq8)}$, $amido_{(C\leq8)}$,

  - $OC(O)NH-alkyl_{(C\leq8)}$, $-OC(O)CH_2NHC(O)O$-*t*-butyl, $-OCH_2-alkylthio_{(C\leq8)}$, or a substituted version of any of these groups;

- $(CH_2)_mC(O)R_c$, wherein m is 0-6 and $R_c$ is:

  hydrogen, hydroxy, halo, amino, -NHOH,

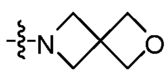

,

  or thio; or
  $alkyl_{(C\leq8)}$, $alkenyl_{(C\leq8)}$, $alkynyl_{(C\leq8)}$, $aryl_{(C\leq8)}$, $aralkyl_{(C\leq8)}$, $heteroaryl_{(C\leq8)}$, $heterocycloalkyl_{(C\leq8)}$, $alkoxy_{(C\leq8)}$, $alkenyloxy_{(C\leq8)}$, $aryloxy_{(C\leq8)}$, $aralkoxy_{(C\leq8)}$, $heteroaryloxy_{(C\leq8)}$, $acyloxy_{(C\leq8)}$, $alkylamino_{(C\leq8)}$, dialkylami-

no$_{(C\leq8)}$, arylamino$_{(C\leq8)}$, alkylsulfonylamino$_{(C\leq8)}$, amido$_{(C\leq8)}$, -NH-alkoxy$_{(C\leq8)}$, -NH-heterocyc1oalkyl$_{(C\leq8)}$, -NHC(NOH)-alkyl$_{(C\leq8)}$, -NH-amido$_{(C\leq8)}$, or a substituted version of any of these groups;
$R_c$ and $R_3$, taken together, are -O- or -$NR_d$-, wherein $R_d$ is hydrogen or alkyl$_{(C\leq4)}$; or
$R_c$ and $R_4$, taken together, are -O- or -$NR_d$-, wherein $R_d$ is hydrogen or alkyl$_{(C\leq4)}$; or

- $NHC(O)R_e$, wherein $R_e$ is:

hydrogen, hydroxy, amino; or
alkyl$_{(C\leq8)}$, alkenyl$_{(C\leq4)}$, alkynyl$_{(C\leq8)}$, aryl$_{(C\leq8)}$, aralkyl$_{(C\leq8)}$, heteroaryl$_{(C\leq8)}$, heterocycloalkyl$_{(C\leq8)}$, alkoxy$_{(C\leq8)}$, aryloxy$_{(C\leq8)}$, aralkoxy$_{(C\leq8)}$, heteroaryloxy$_{(C\leq8)}$, acyloxy$_{(C\leq8)}$, alkylamino$_{(C\leq8)}$, dialkylamino$_{(C\leq8)}$, arylamino$_{(C\leq8)}$, or a substituted version of any of these groups;

or a pharmaceutically acceptable salt or tautomer thereof,

These compounds are known as antioxidant inflammation modulators. These compounds have shown the ability to activate Nrf2, as measured by elevated expression of one or more Nrf2 target genes (*e.g.,* NQO1 or HO-1; Dinkova-Kostova *et al.,* 2005). Further, these compounds are capable of indirect and direct inhibition of pro-inflammatory transcription factors including NF-kappa B and STAT3 (Ahmad *et al.,* 2006; Ahmad *et al.,* 2008). In some aspects, there are provided methods of preventing pulmonary arterial hypertension in a subject in need thereof comprising administering to the subject bardoxolone methyl in an amount sufficient to prevent pulmonary arterial hypertension in the subject. In some aspects, there are provided methods of preventing progression of pulmonary arterial hypertension in a subject in need thereof comprising administering to the subject bardoxolone methyl in an amount sufficient to prevent progression of pulmonary arterial hypertension in the subject.

Triterpenoids, biosynthesized in plants by the cyclization of squalene, are used for medicinal purposes in many Asian countries; and some, such as ursolic and oleanolic acid, are known to be anti-inflammatory and anti-carcinogenic (Huang *et al.,* 1994; Nishino *et al.,* 1988). However, the biological activity of these naturally-occurring molecules is relatively weak, and therefore the synthesis of new analogs to enhance their potency was undertaken (Honda *et al.,* 1997; Honda *et al.,* 1998). An ongoing effort for the improvement of anti-inflammatory and antiproliferative activity of oleanolic and ursolic acid analogs led to the discovery of 2-cyano-3,12-dioxooleane-1,9(11)-dien-28-oic acid (CDDO) and related compounds (Honda *et al.,* 1997, 1998, 1999, 2000a, 2000b, 2002; Suh *et al.,* 1998; 1999; 2003; Place *et al.,* 2003; Liby *et al.,* 2005). Several potent derivatives of oleanolic acid were identified, including methyl-2-cyano-3,12-dioxooleana-1,9-dien-28-oic acid (CDDO-Me; RTA 402; bardoxolone methyl). RTA 402, an antioxidant inflammation modulator (AIM), suppresses the induction of several important inflammatory mediators, such as iNOS, COX-2, TNFα, and IFNγ, in activated macrophages, thereby restoring redox homeostasis in inflamed tissues. RTA 402 has also been reported to activate the Keap1/Nrf2/ARE signaling pathway resulting in the production of several anti-inflammatory and antioxidant proteins, such as heme oxygenase-1 (HO-1). It induces the cytoprotective transcription factor Nrf2 and suppresses the activities of the pro-oxidant and pro-inflammatory transcription factors NF-κB and STAT3. *In vivo,* RTA 402 has demonstrated significant single agent anti-inflammatory activity in several animal models of inflammation such as renal damage in the cisplatin model and acute renal injury in the ischemia-reperfusion model. In addition, significant reductions in serum creatinine have been observed in patients treated with RTA 402.

Accordingly, in pathologies involving oxidative stress alone or oxidative stress exacerbated by inflammation, treatment may comprise administering to a subject a therapeutically effective amount of a compound of this invention, such as those described above or throughout this specification. Treatment may be administered preventively in advance of a predictable state of oxidative stress (*e.g.,* organ transplantation or the administration of therapy to a cancer patient), or it may be administered therapeutically in settings involving established oxidative stress and inflammation.

The triterpenoid for use in accordance with the methods of this invention is shown here.

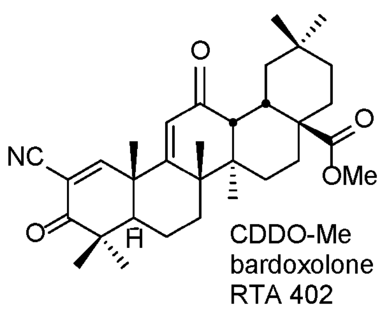

CDDO-Me
bardoxolone methyl
RTA 402

Examples of triterpenoids described herein, but not falling within the scope of the claims, that may be used in accordance with the methods described herein are shown here.

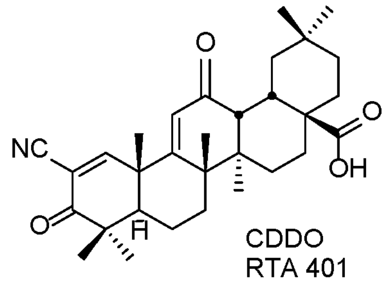

CDDO
RTA 401

, ,

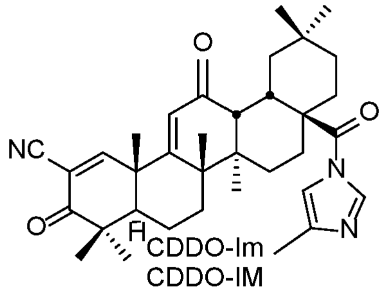

CDDO-Im
CDDO-IM
RTA 403

,

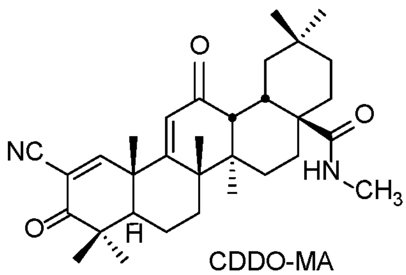

CDDO-MA

,

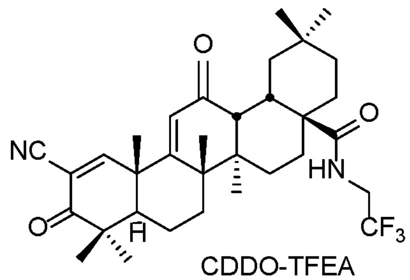

CDDO-TFEA
RTA 404

,

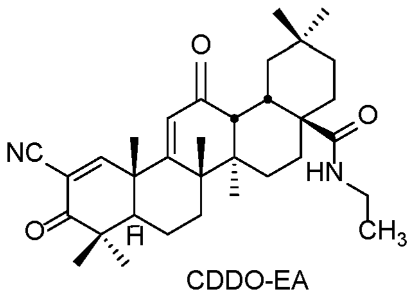

CDDO-EA
RTA 405

,

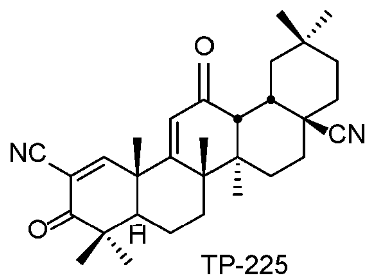

TP-225

,

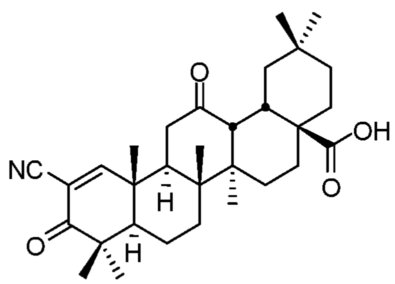

,

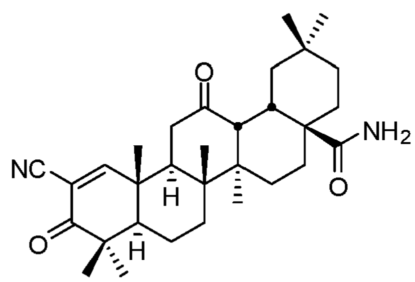

,

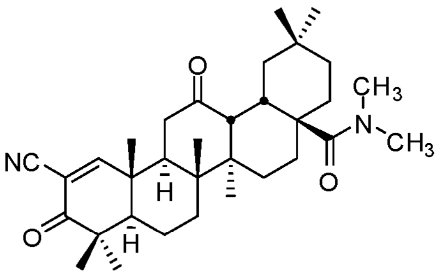
,
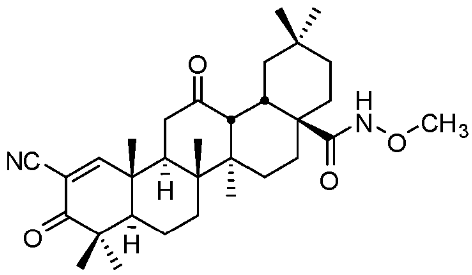
,

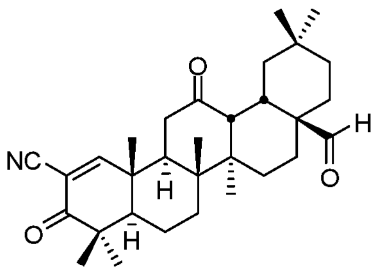
,
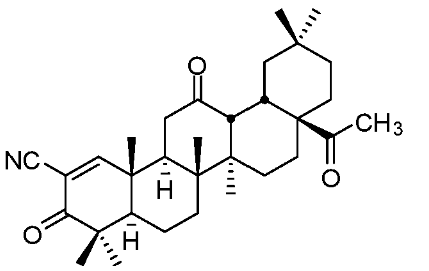
,

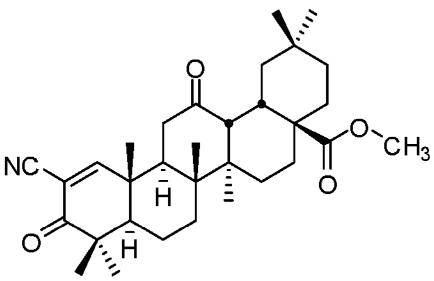
,
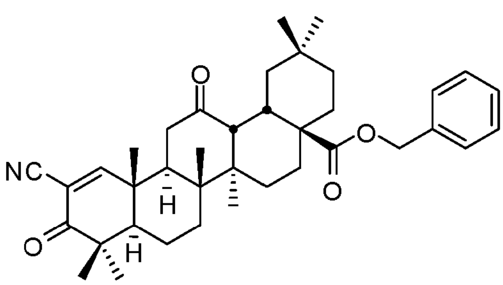
,

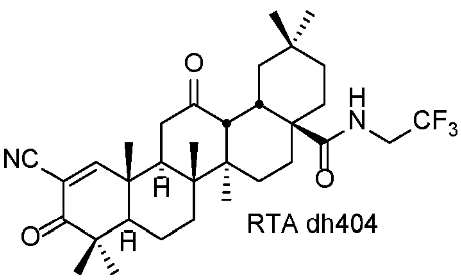
RTA dh404
,
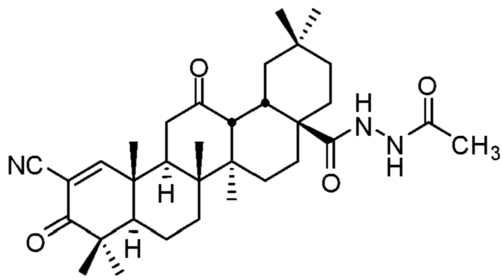
,

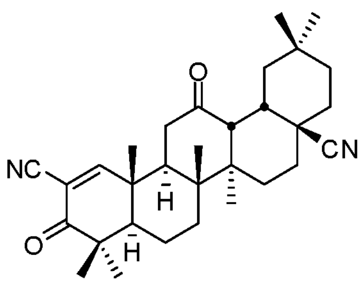
,
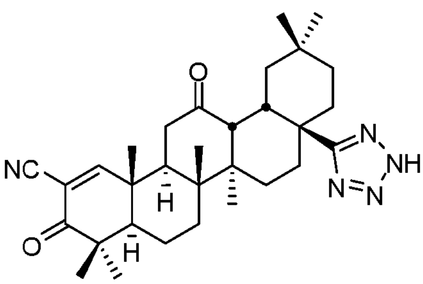
,

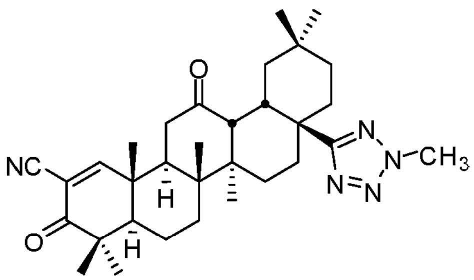
,
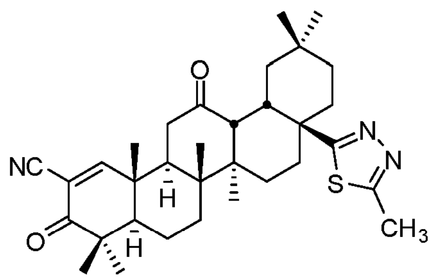
,
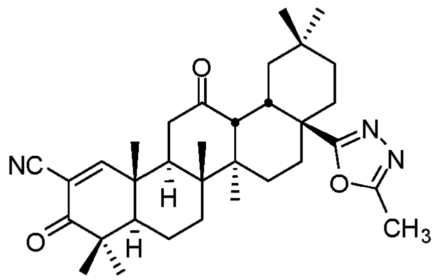
,
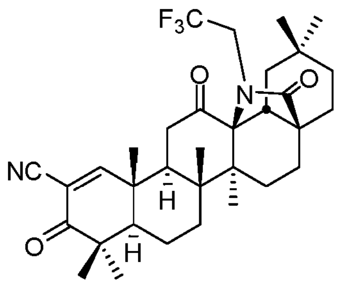
,
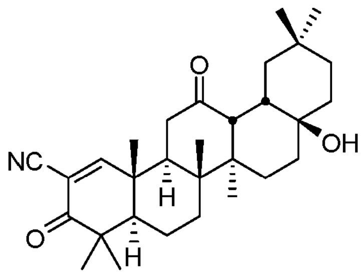
,
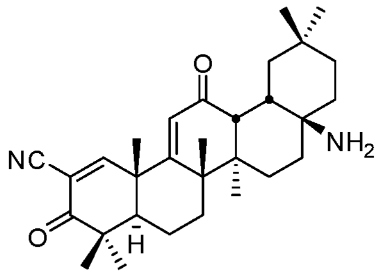
,
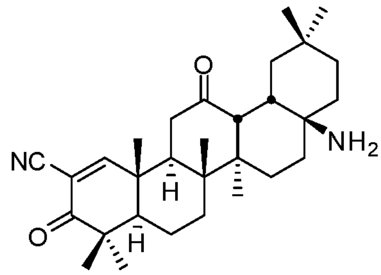
,
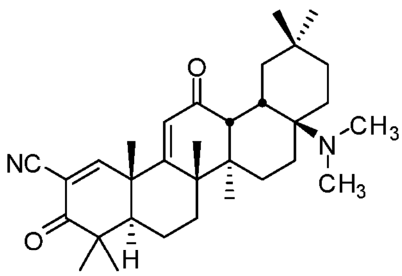
,
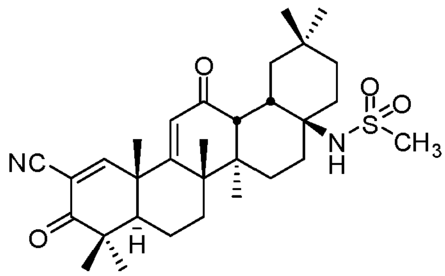
,
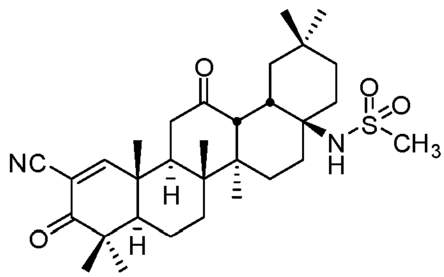
,
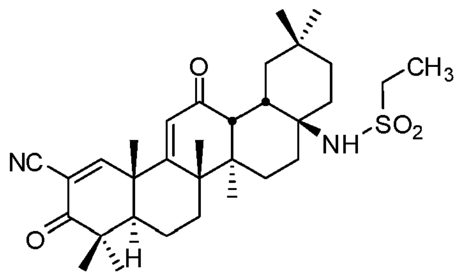
,

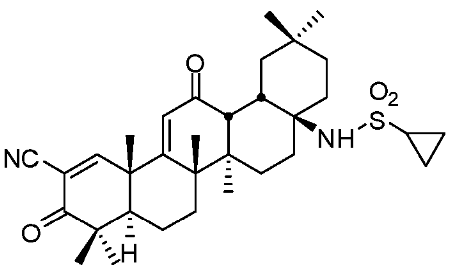
,
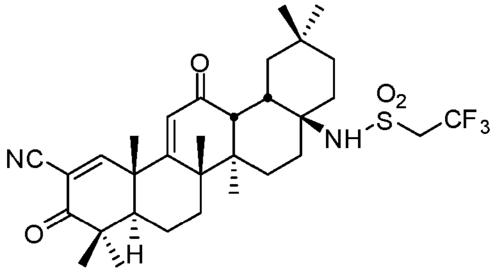
,
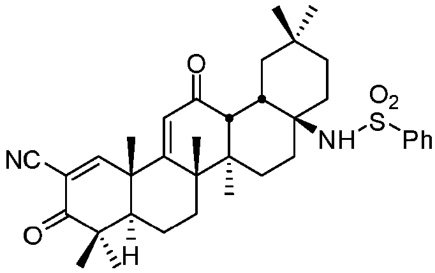
,
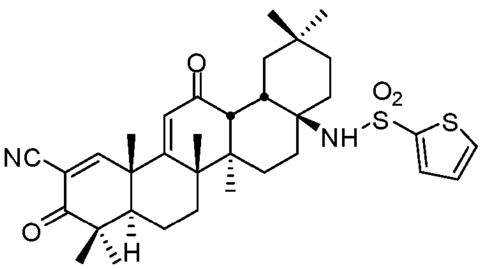
,
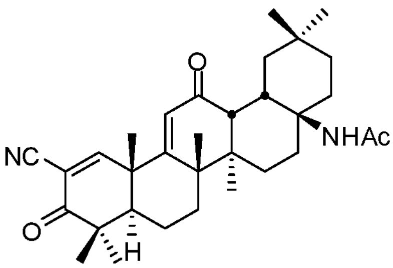
,
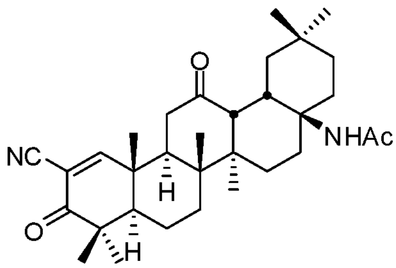
,
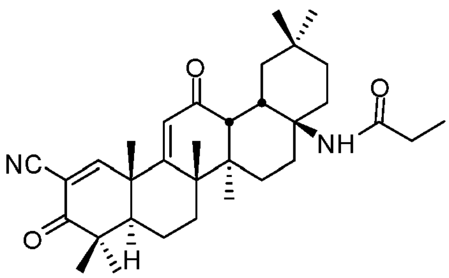
,
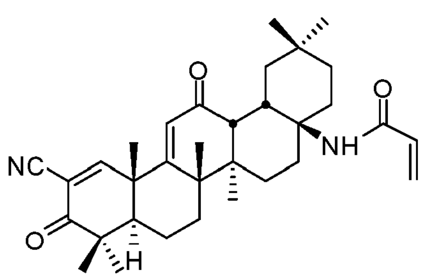
,
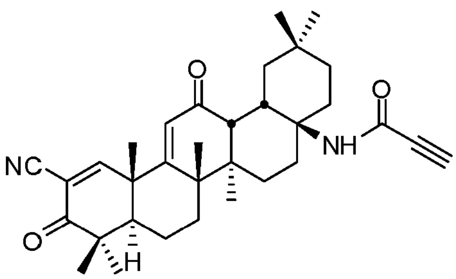
,
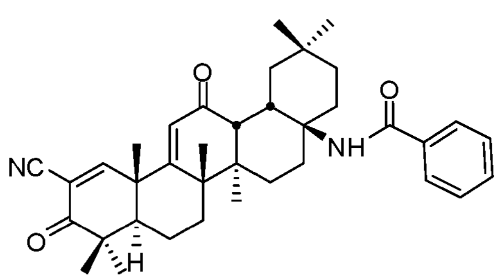
,

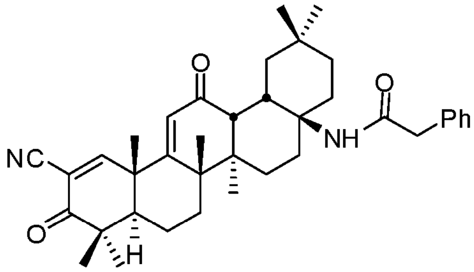
,
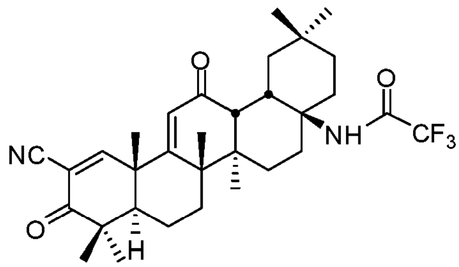
,
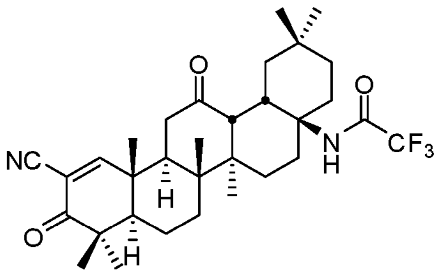
,
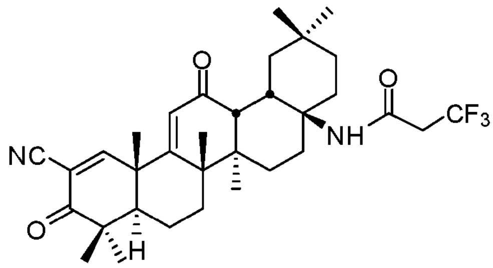
,
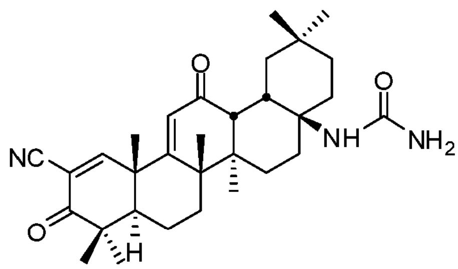
,
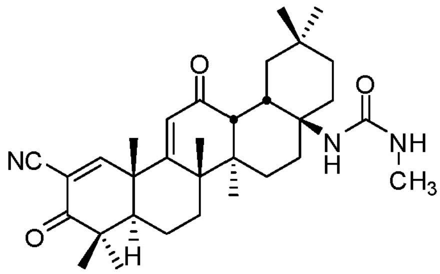
,
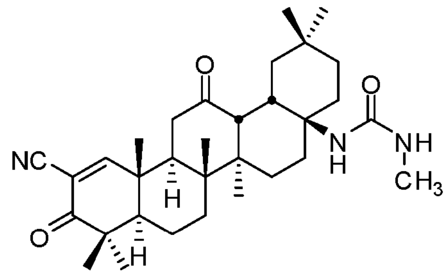
,
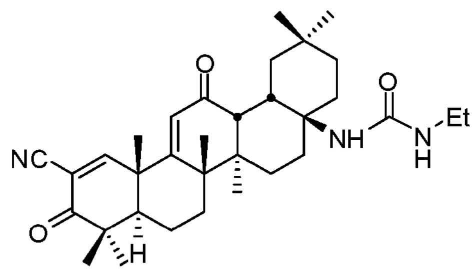
,
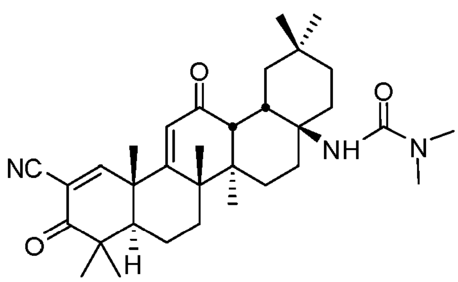
,
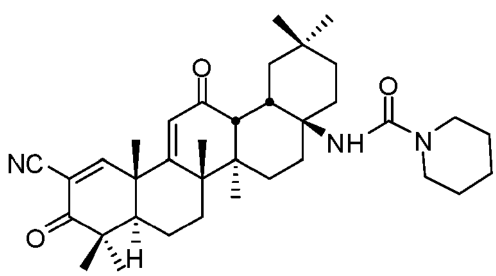
,

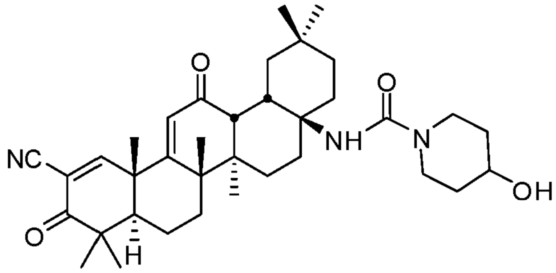
,
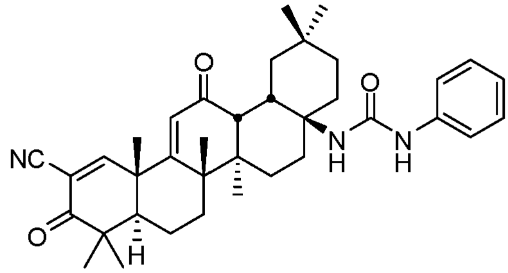
,
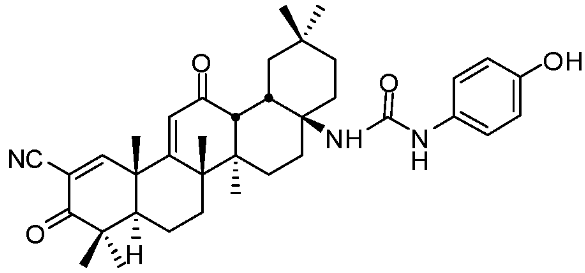
,
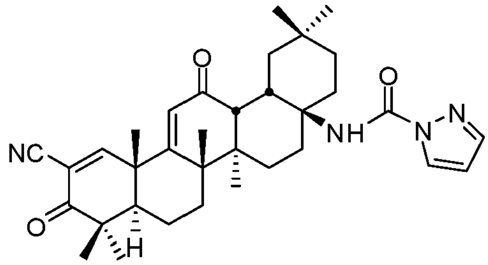
,
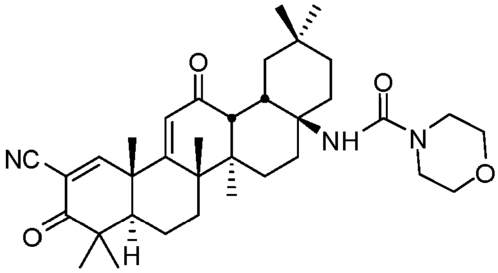
,
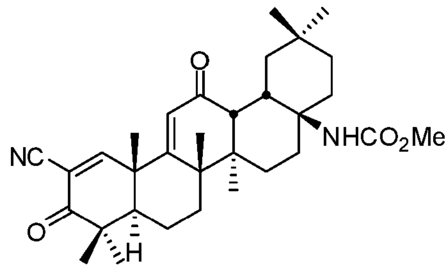
,
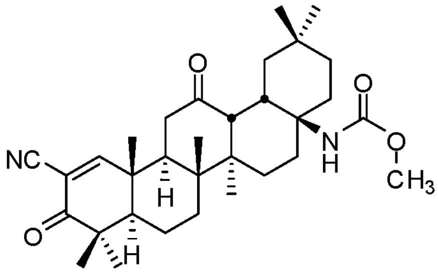
,
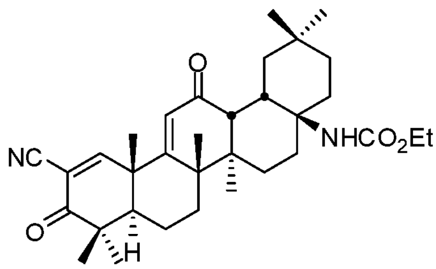
,
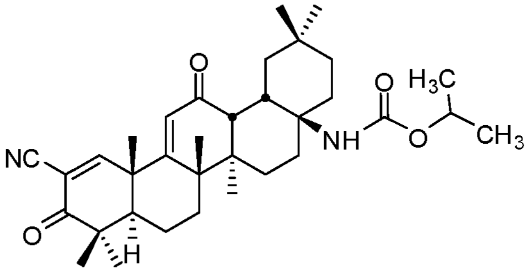
,
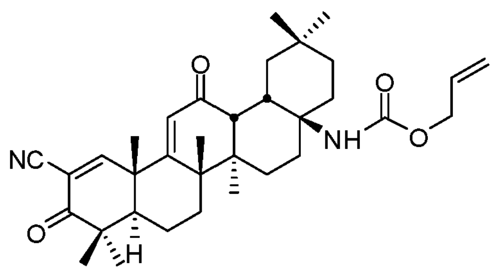
,

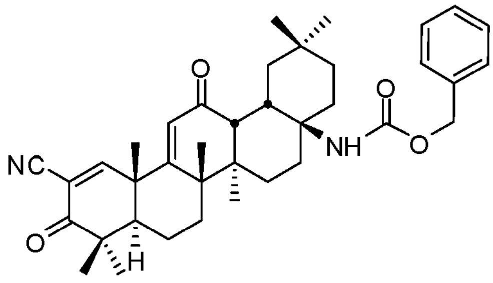,
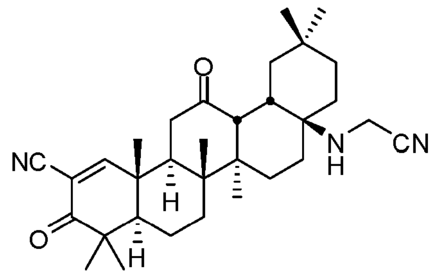,

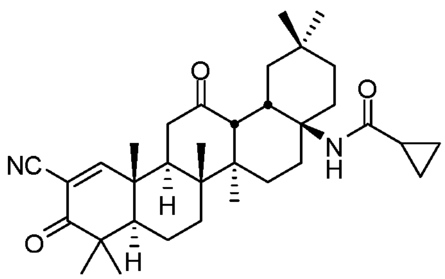,
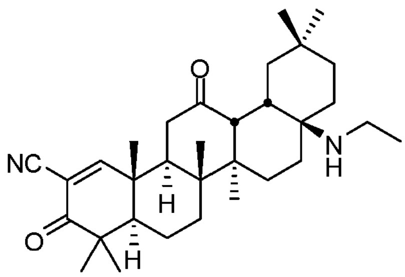,

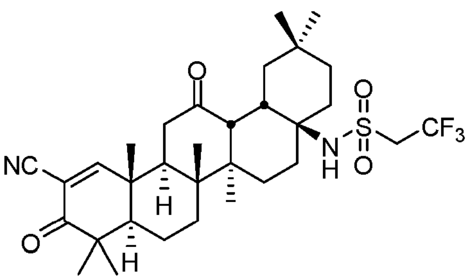,
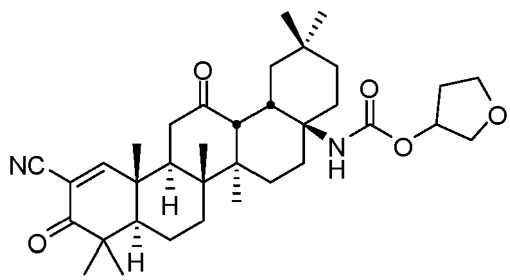,

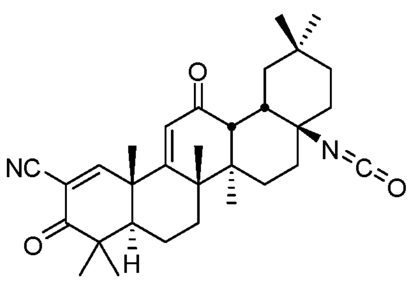,
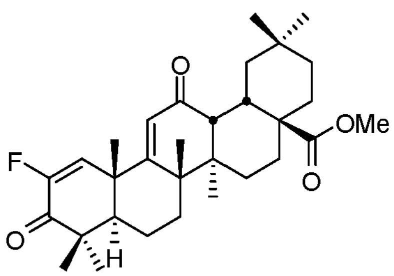,

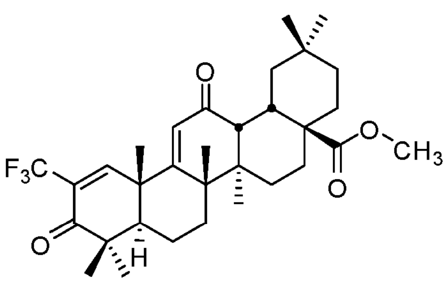,
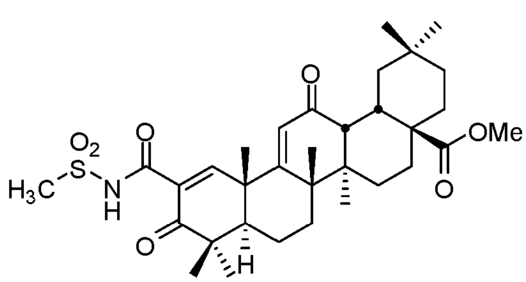,

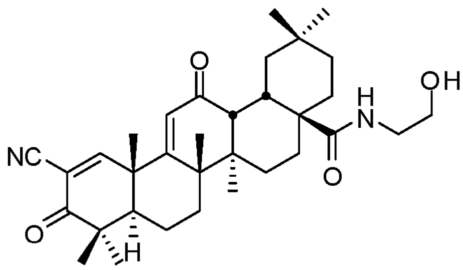

,

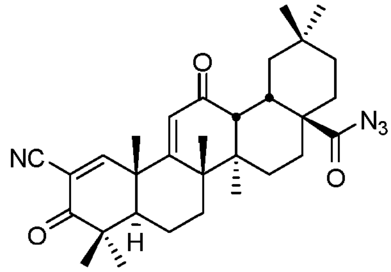

,

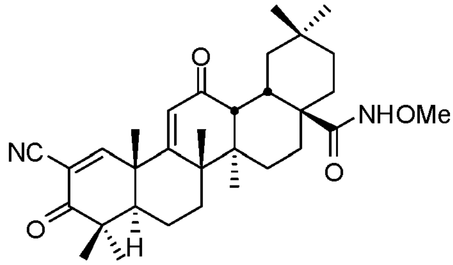

,

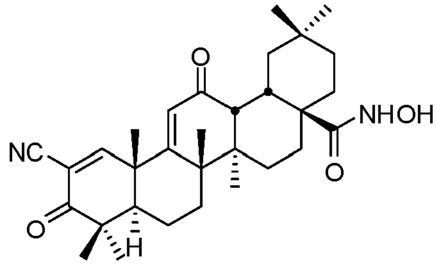

,

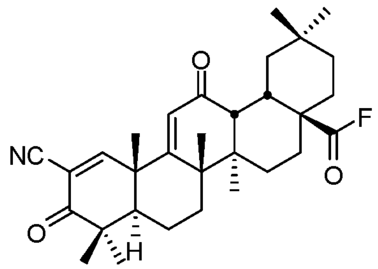

,

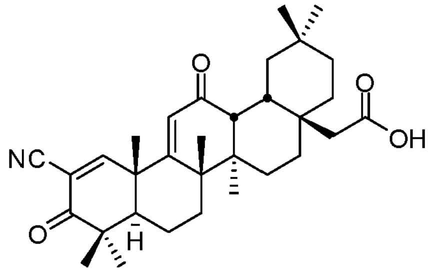

,

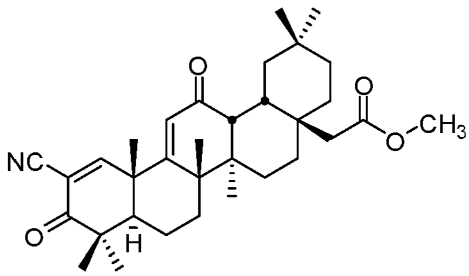

,

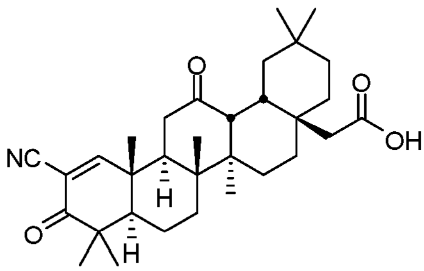

,

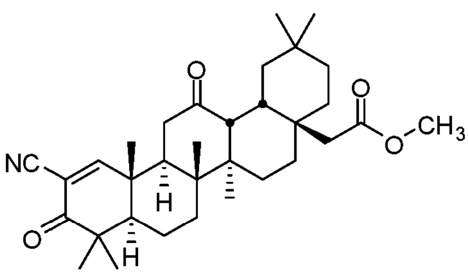

,

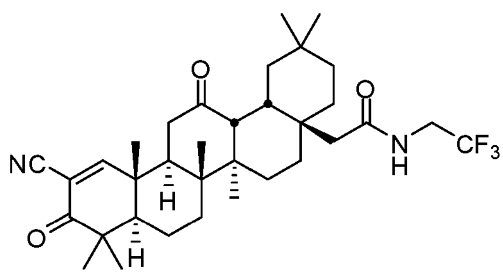

,

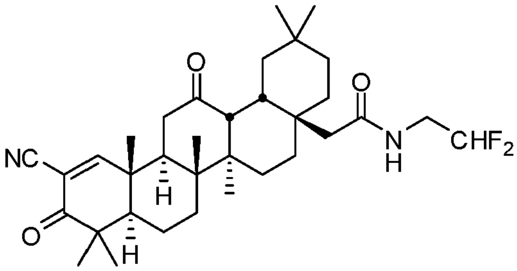, 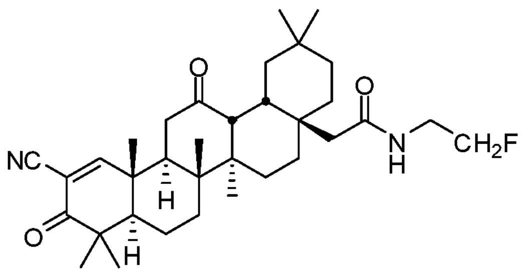,

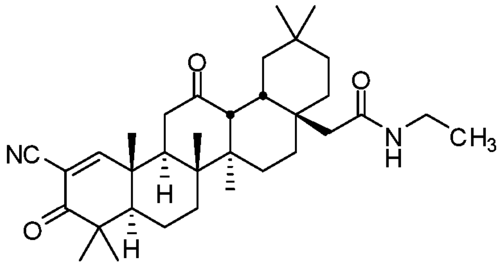, 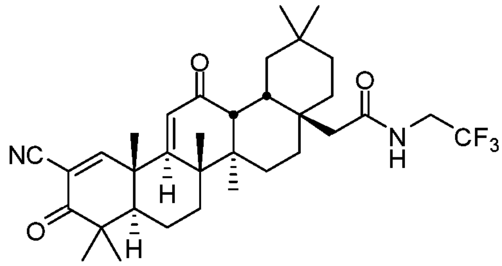,

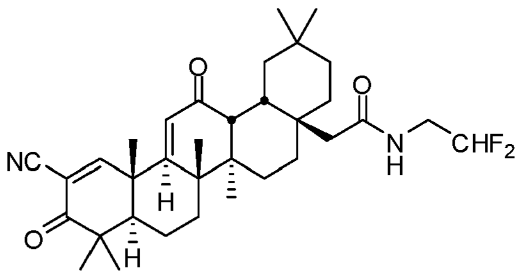, 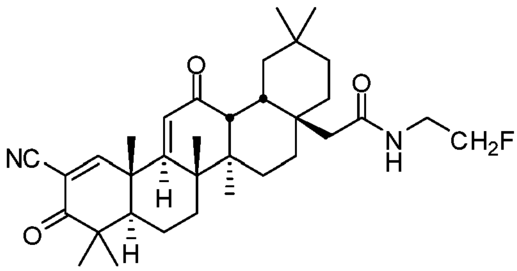,

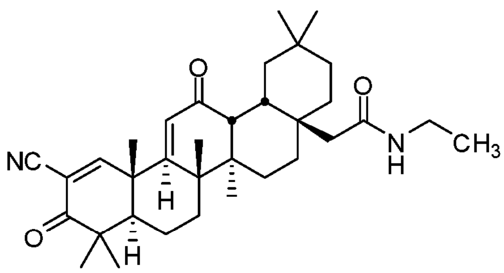, 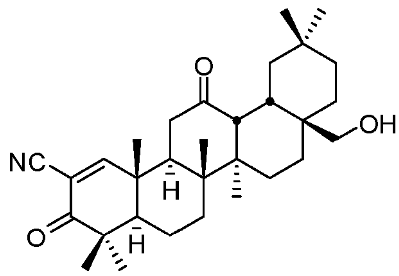,

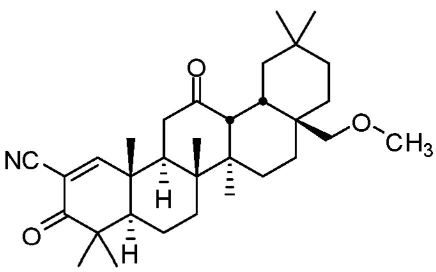, 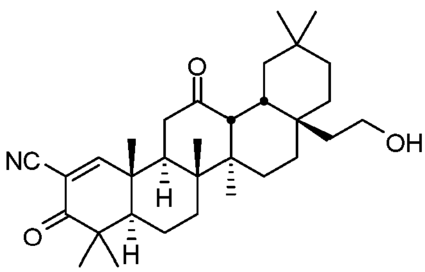,

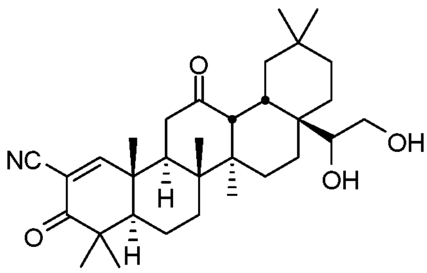 , 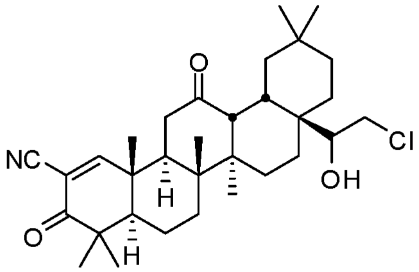 ,

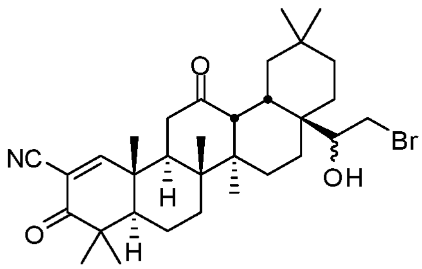 , 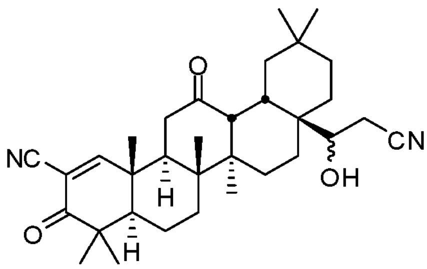 ,

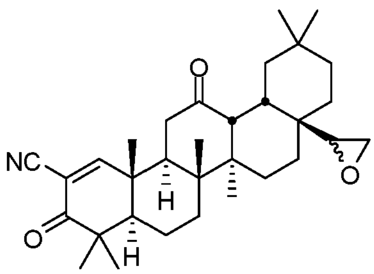 , 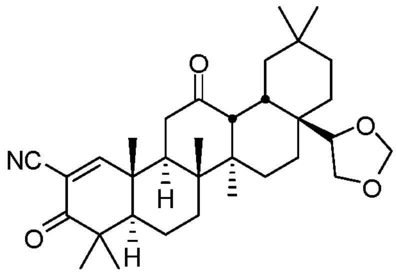 , 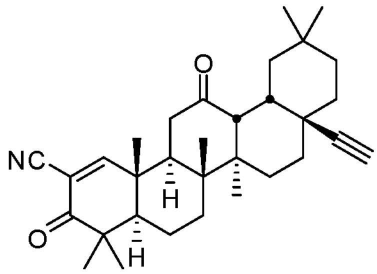 ,

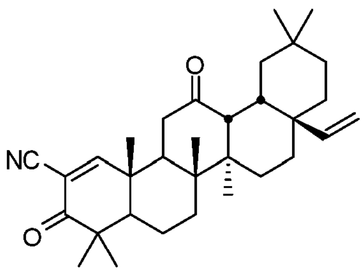 , 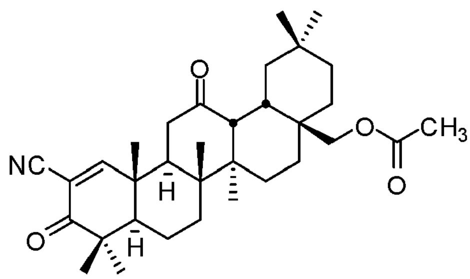 ,

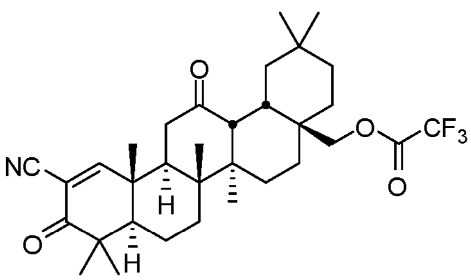 , 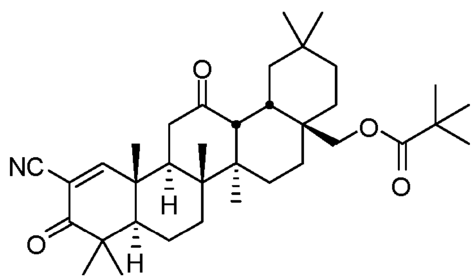 ,

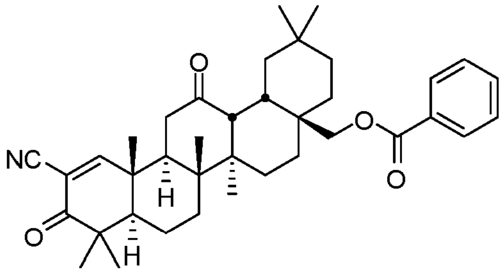
,
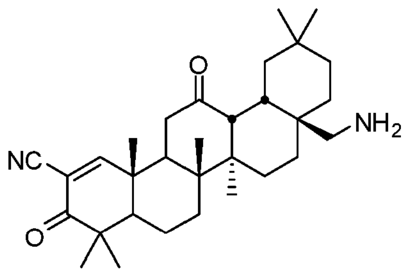
,

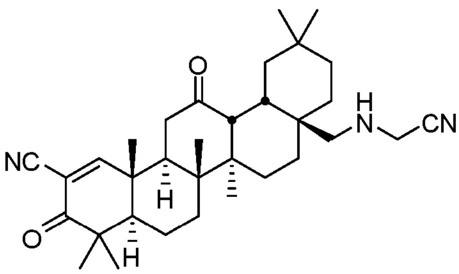
,
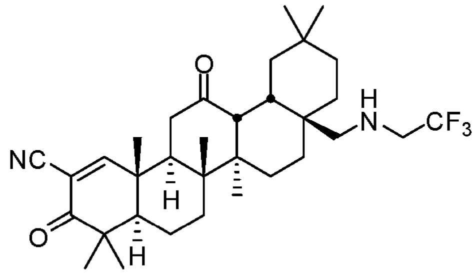
,

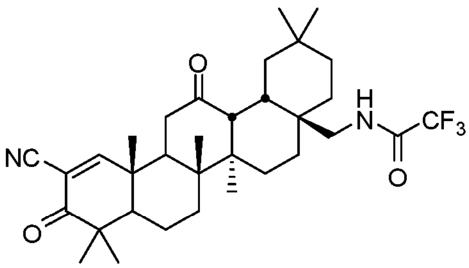
,
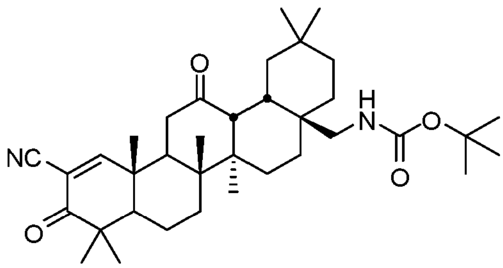
,

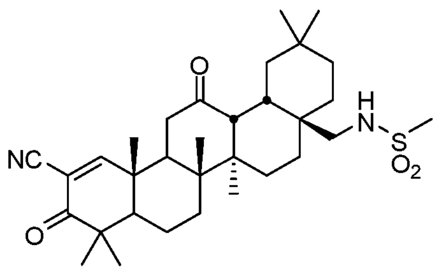
,

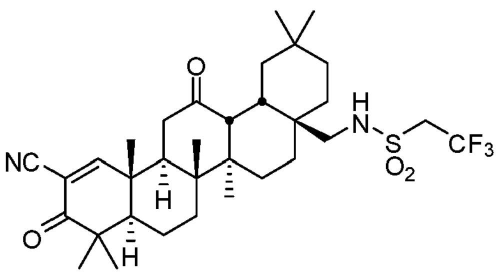
,
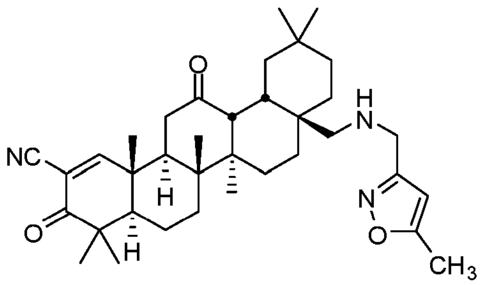
,

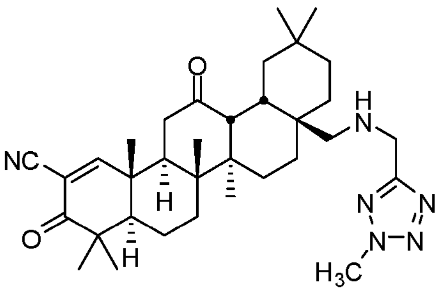,
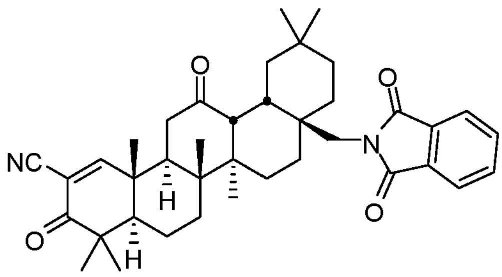,

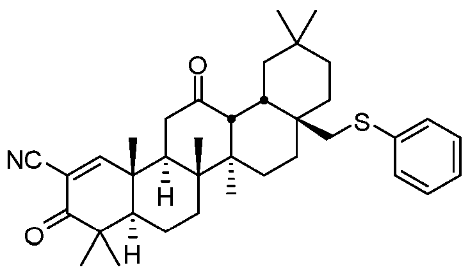,
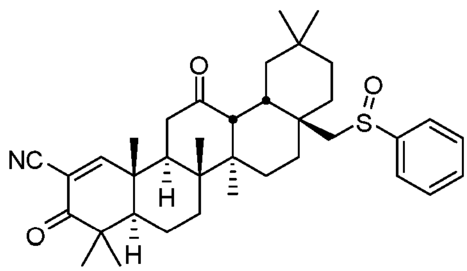,

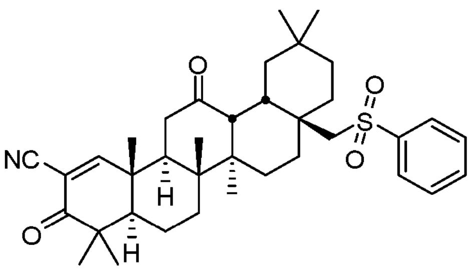,
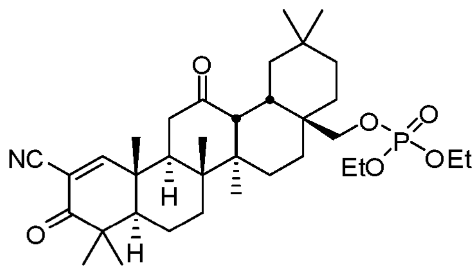,

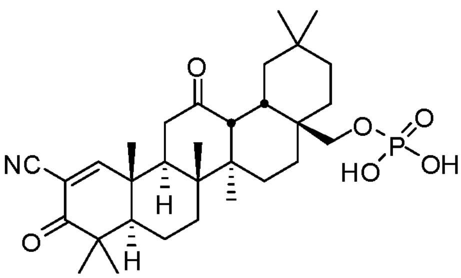,
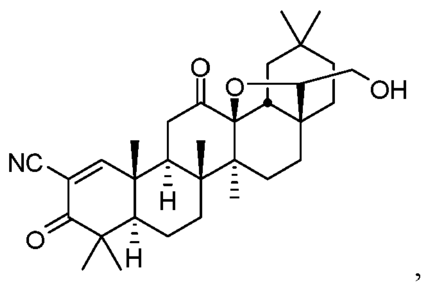,

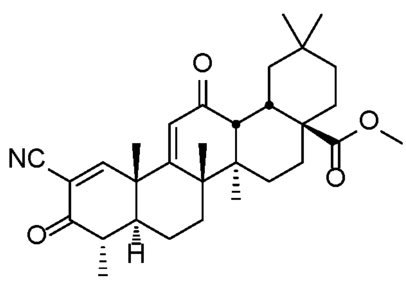,
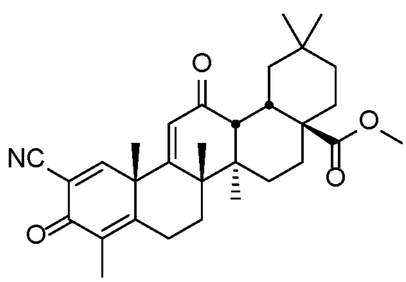,

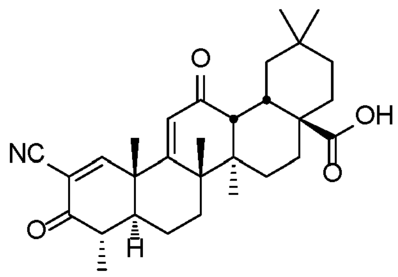,
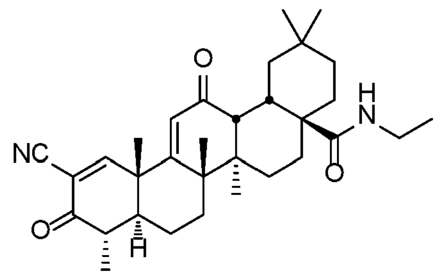,

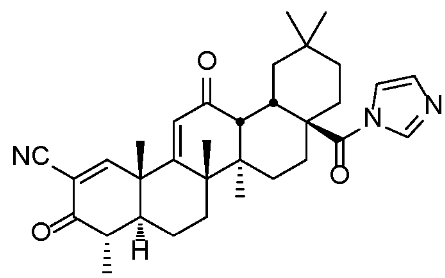,
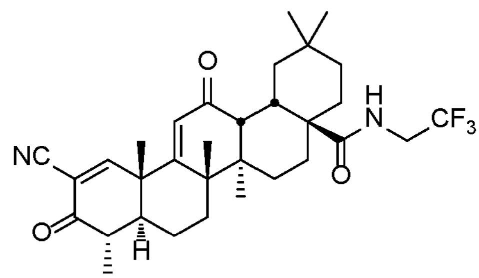,

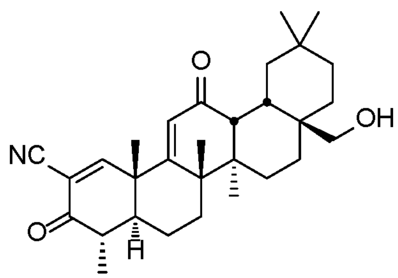,
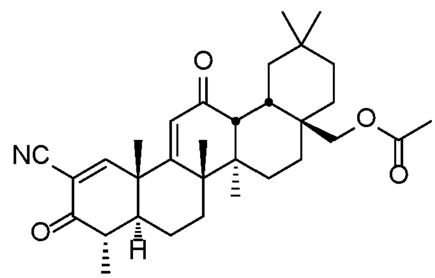,

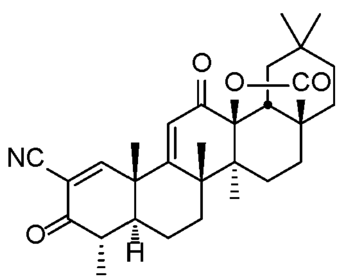,
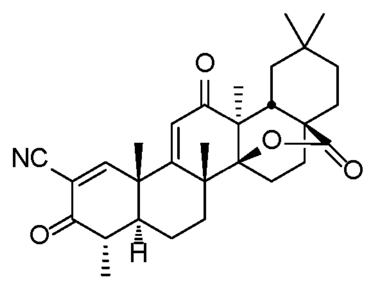,
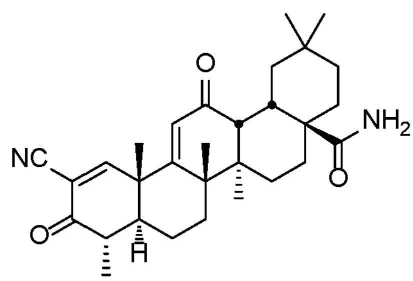,

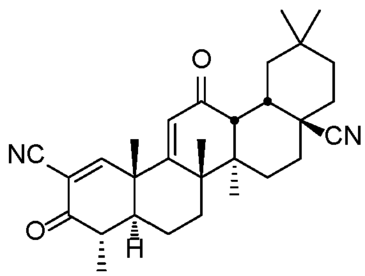,
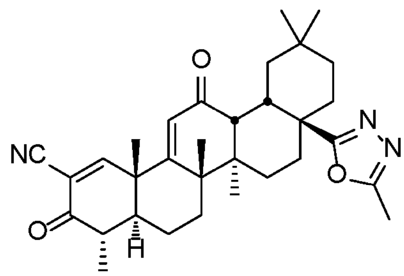,
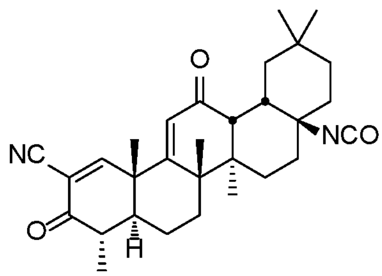,

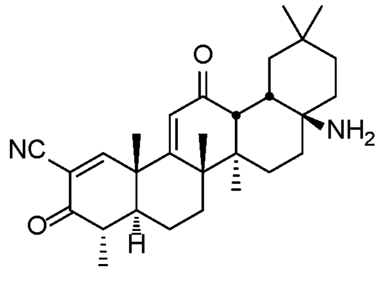
,
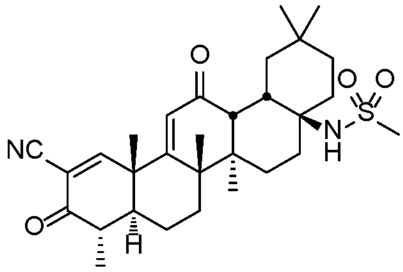
,
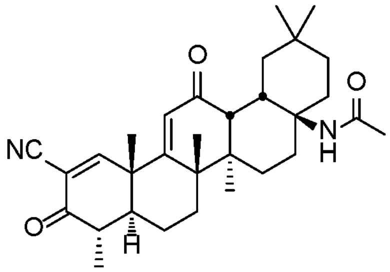
,
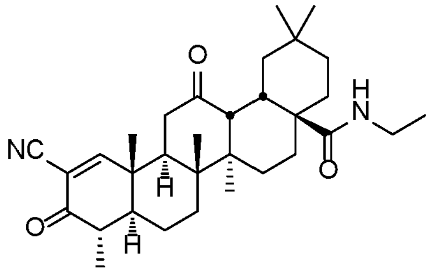
,
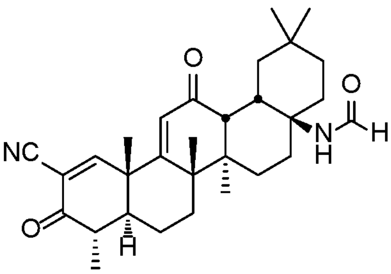
,
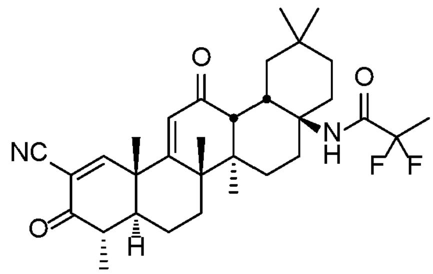
,
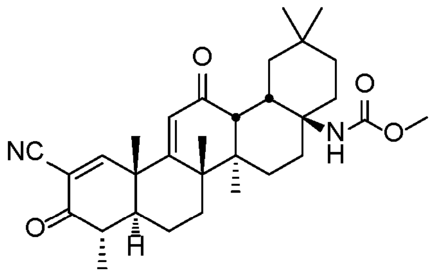
,
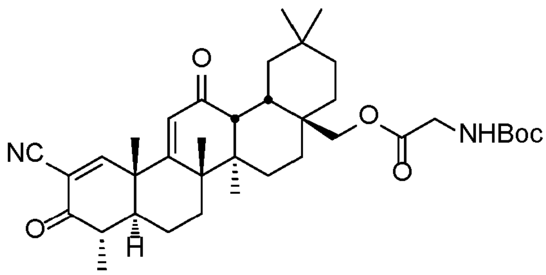
,
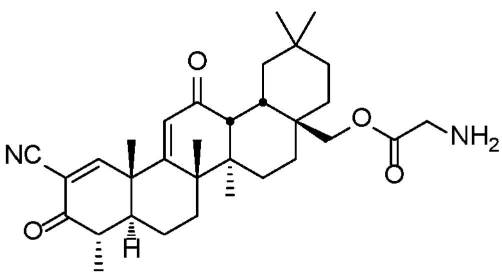
,
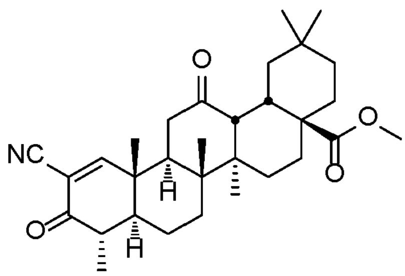
,

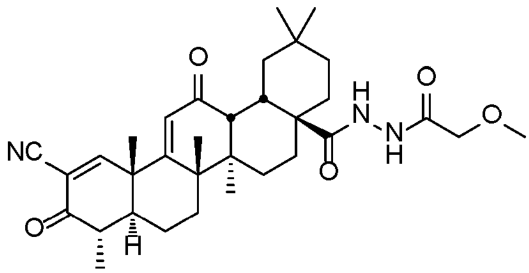,
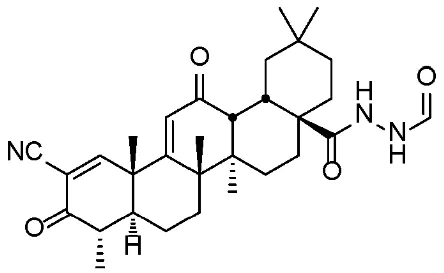,

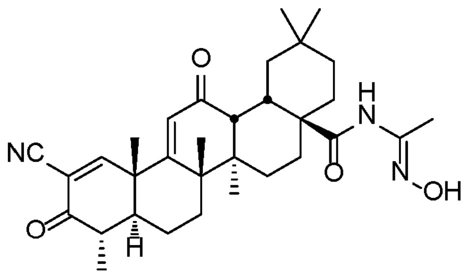,
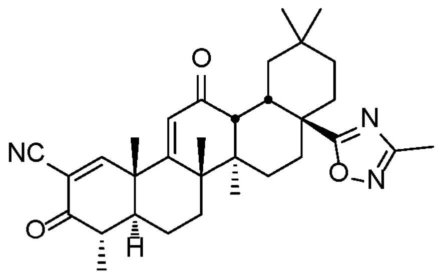,

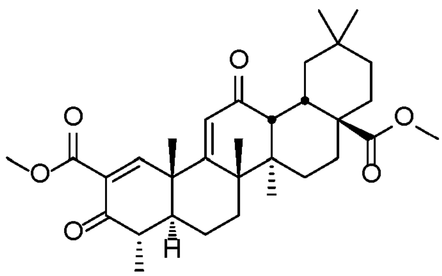,
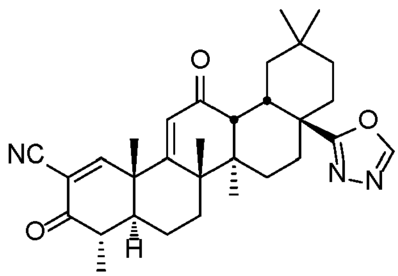,

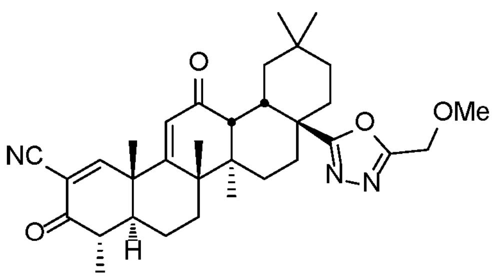,
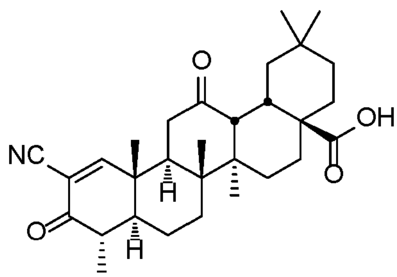,

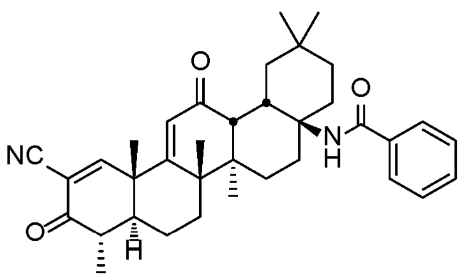,
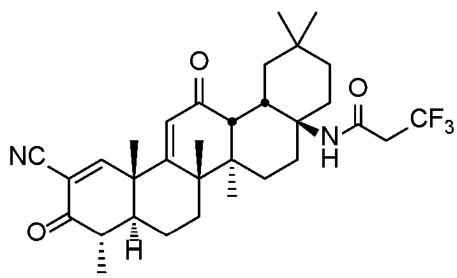,

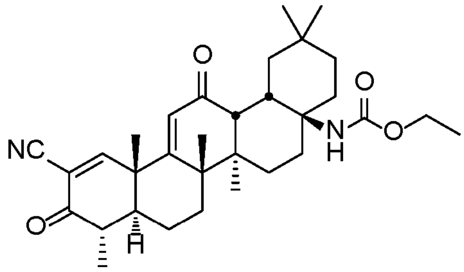,

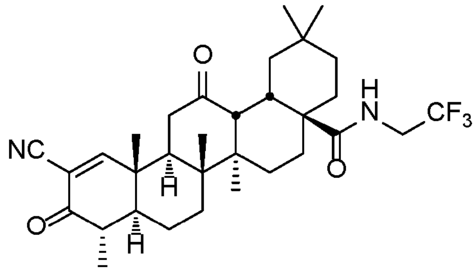,

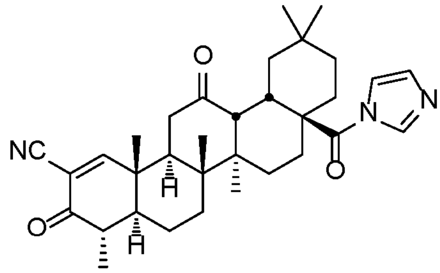,

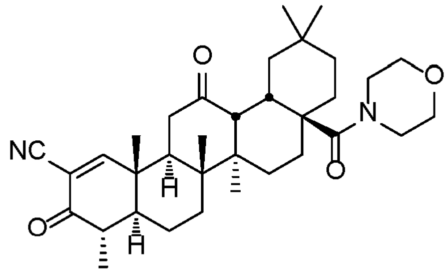,

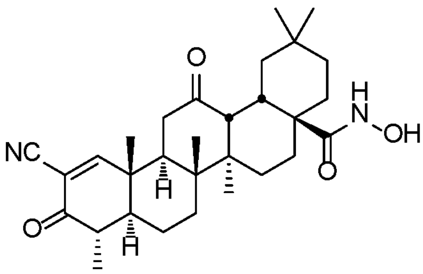,

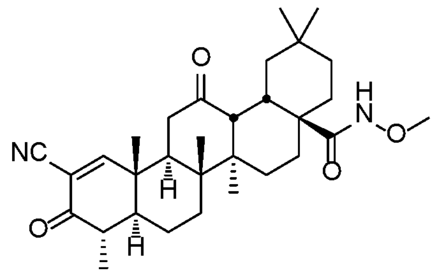,

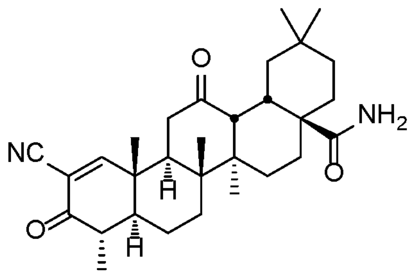,

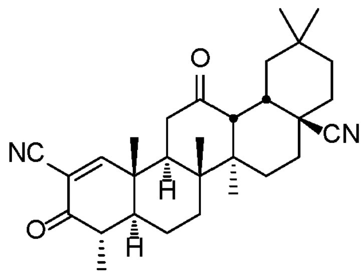,

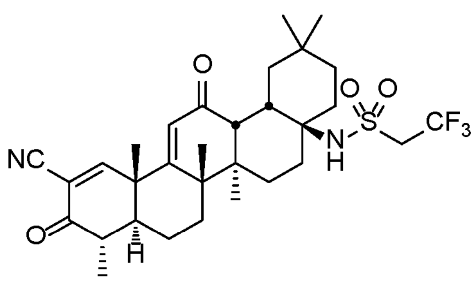,

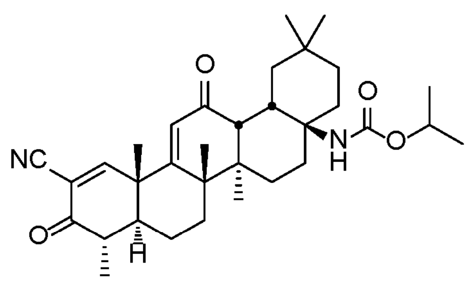,

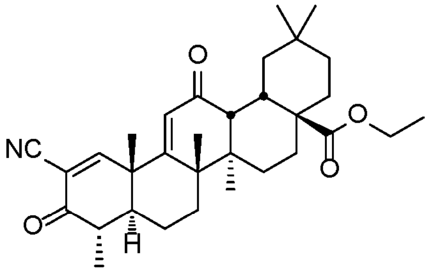
,
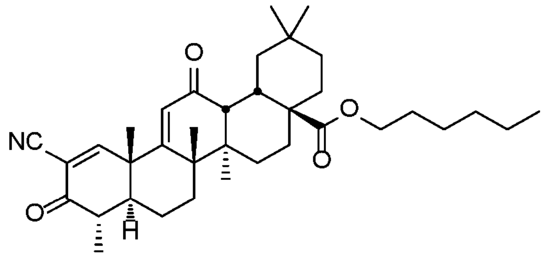
,
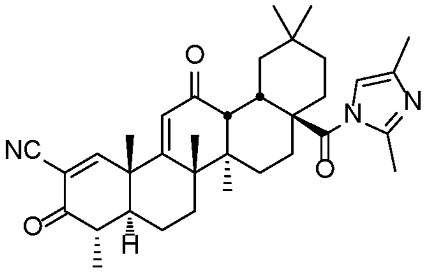
,
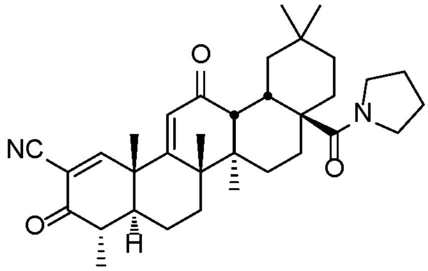
,
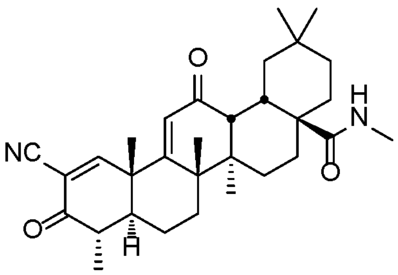
,
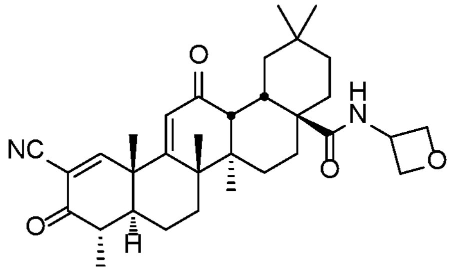
,
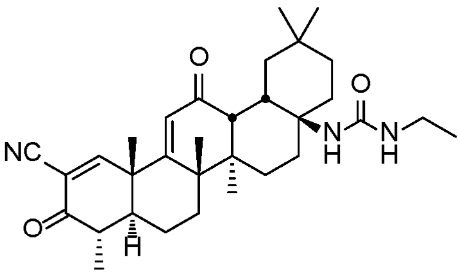
,
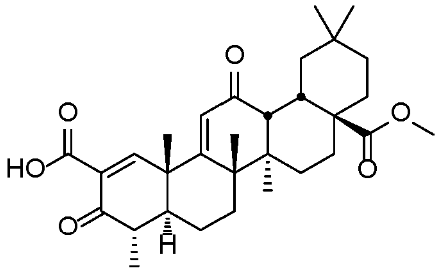
,
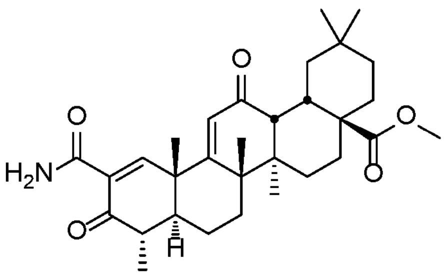
,-
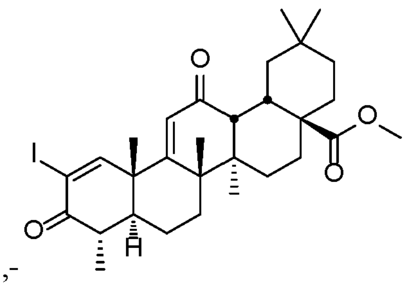
,

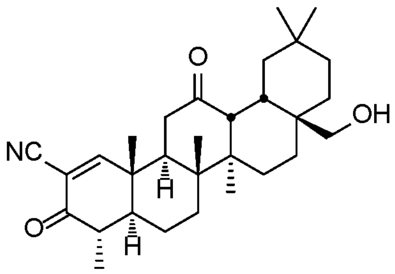,
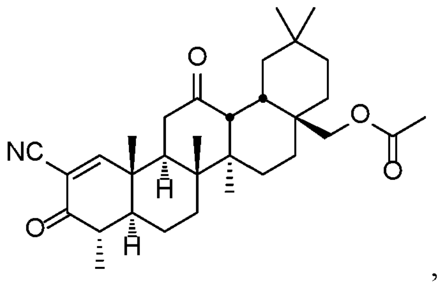,

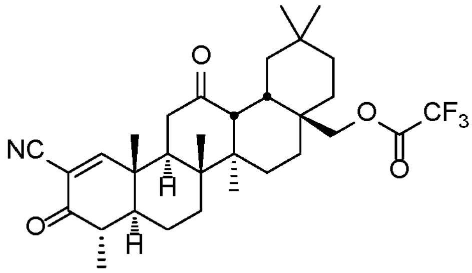,
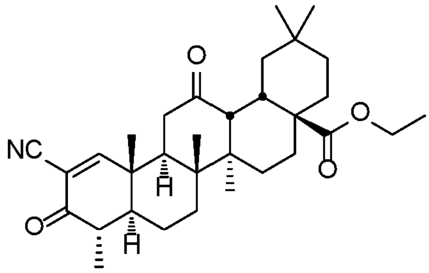,

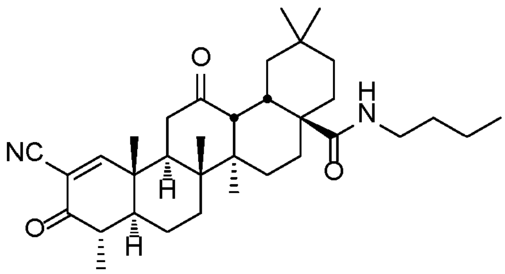,
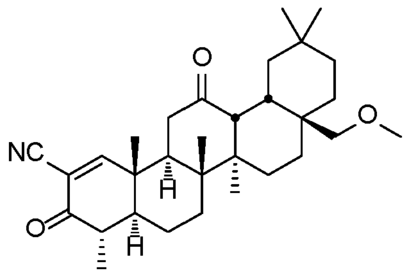,

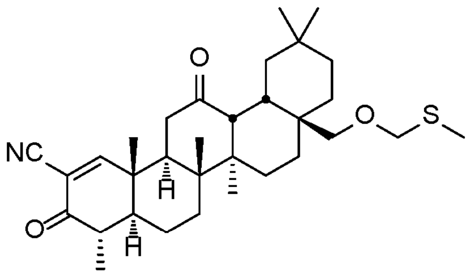,
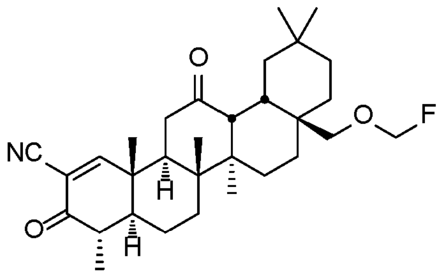,

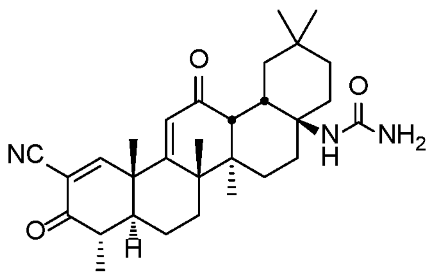,
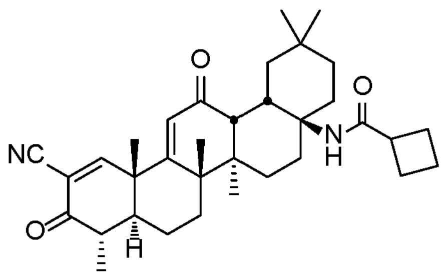,

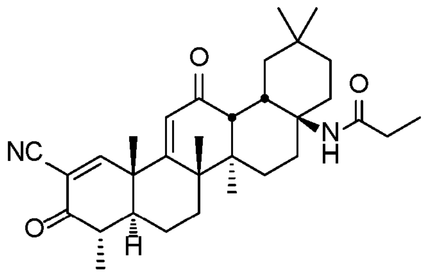
,

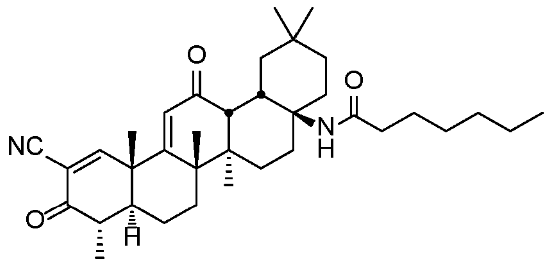
,

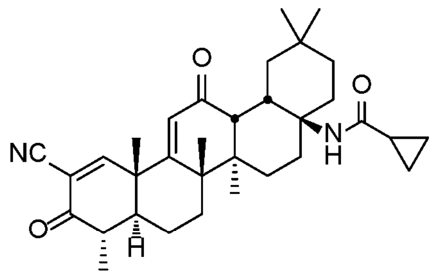
,

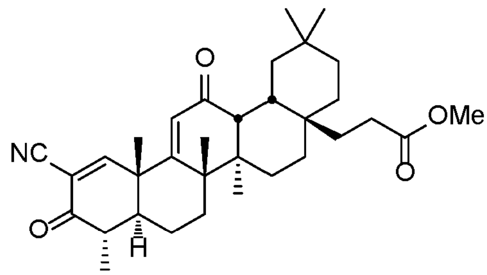
,

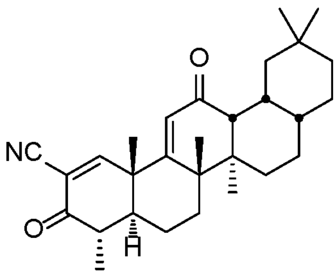
,

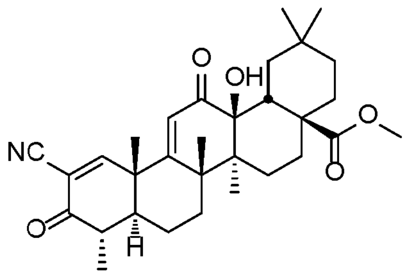
,

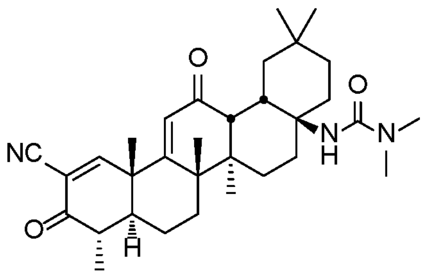
,

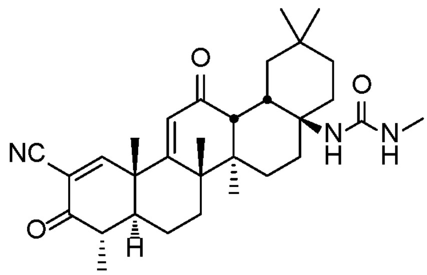
,

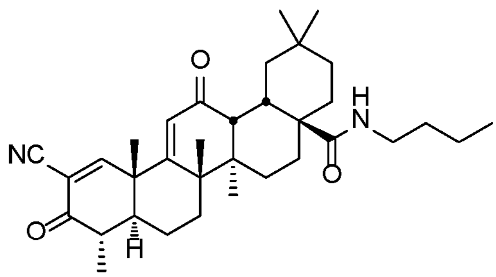
,

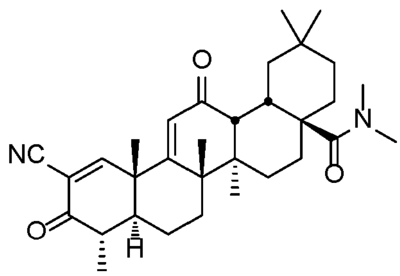
,

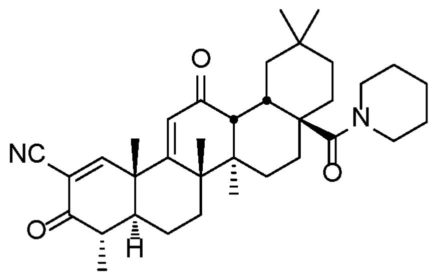
,
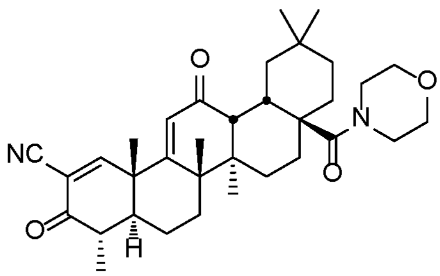
,
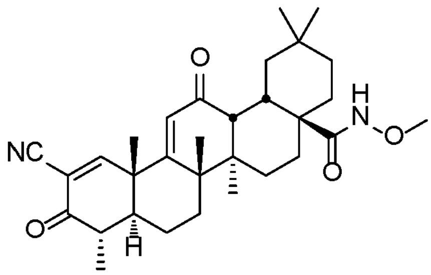
,
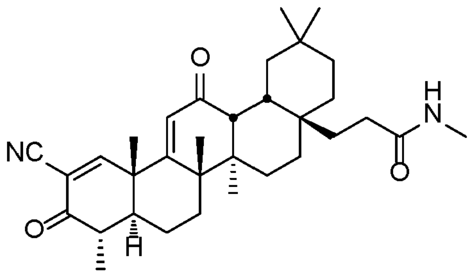
,
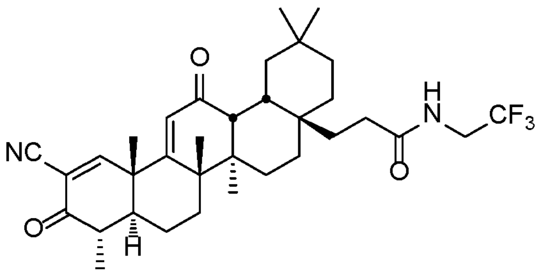
,
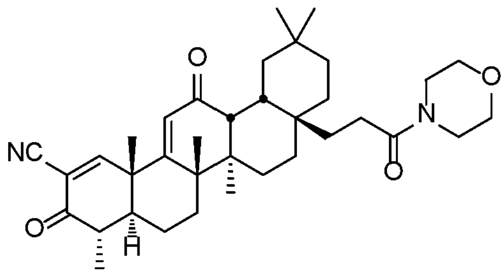
,
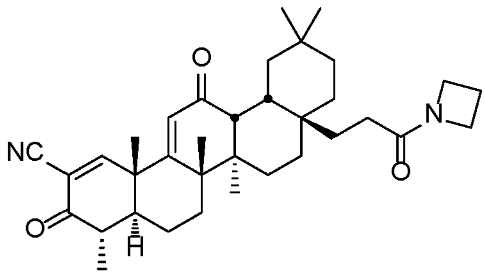
,
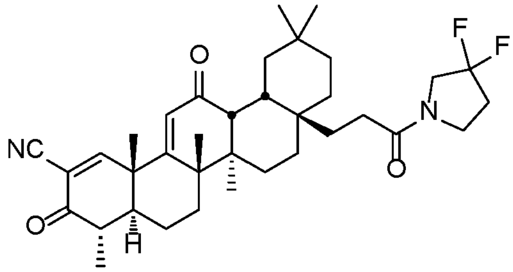
,
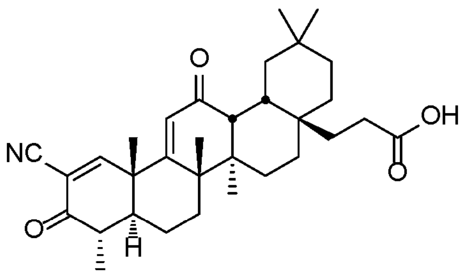
,
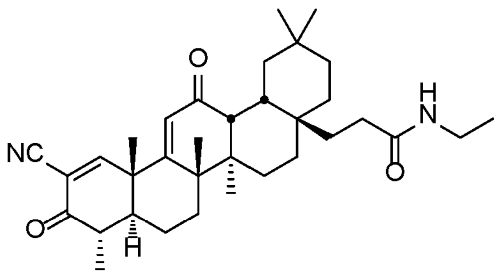
,

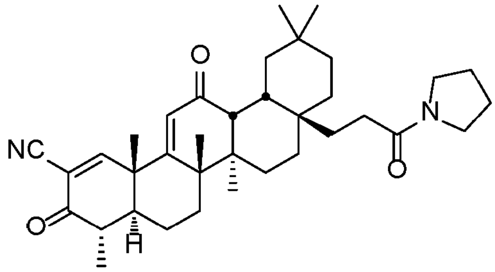
,
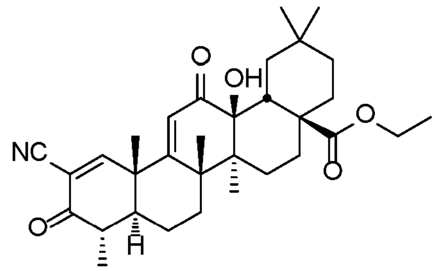
,

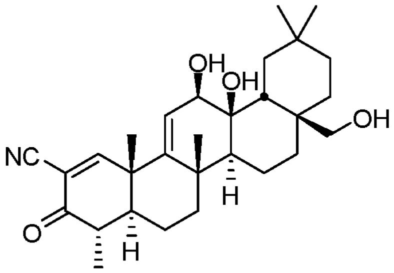
,
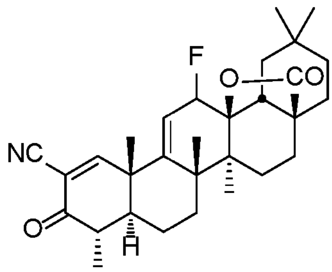
,
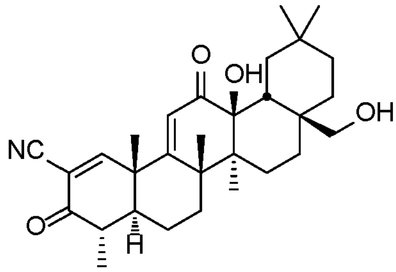
,

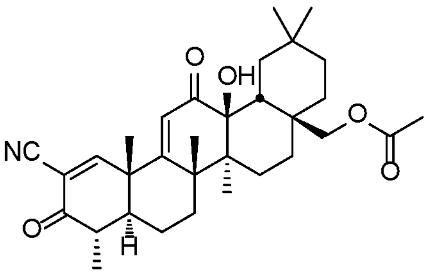
,
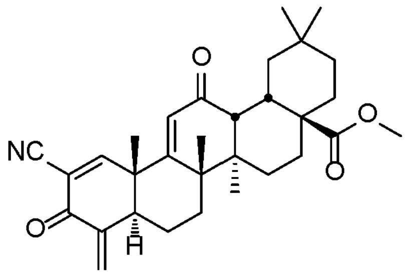
,

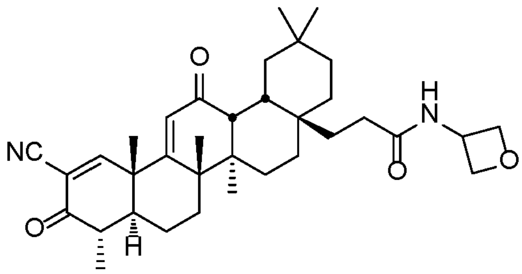
,
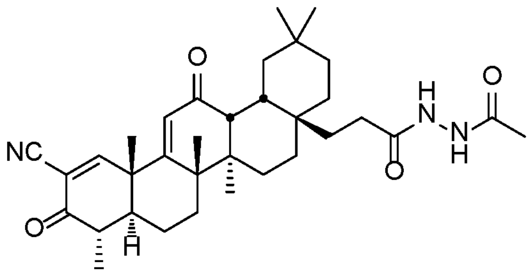
,

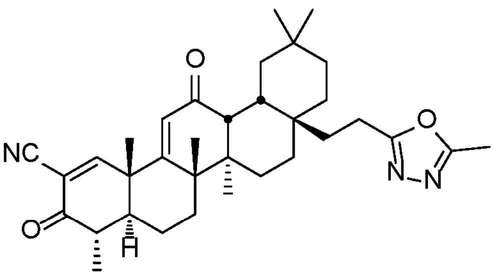
,
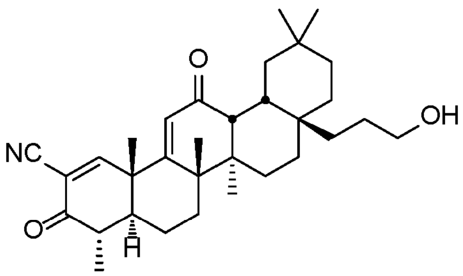
,

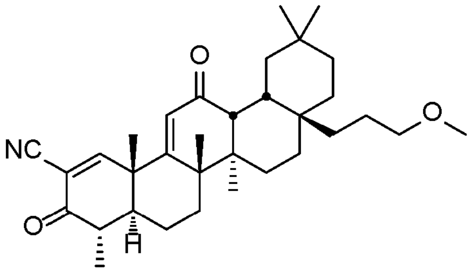

,

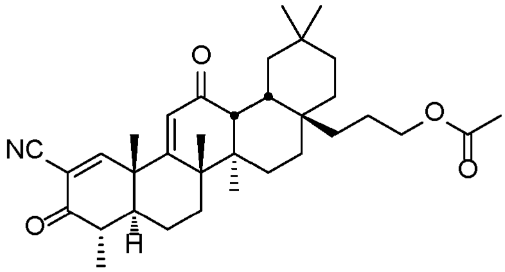

,

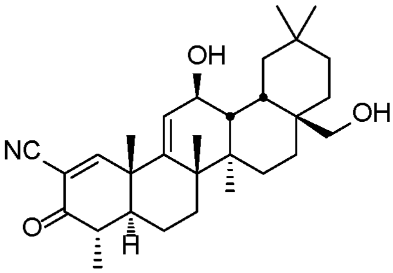

,

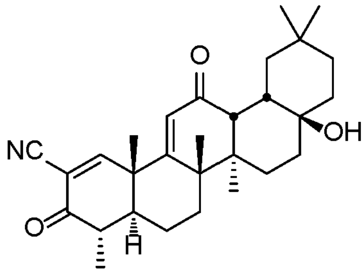

,

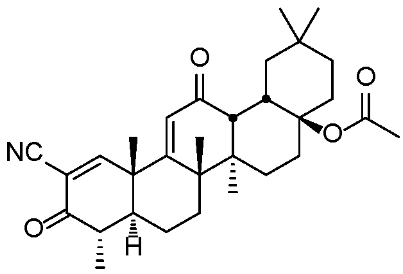

,

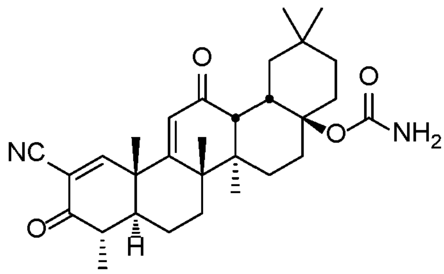

,

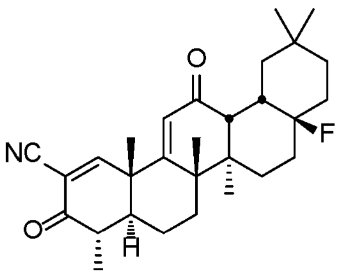

,

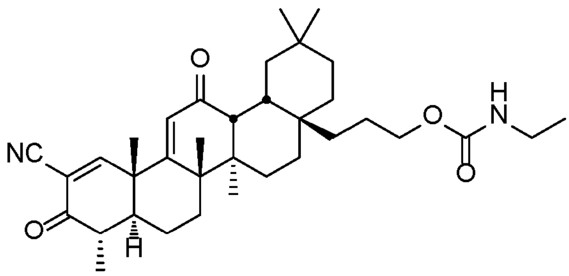

,

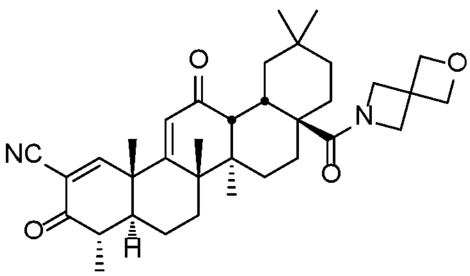

,

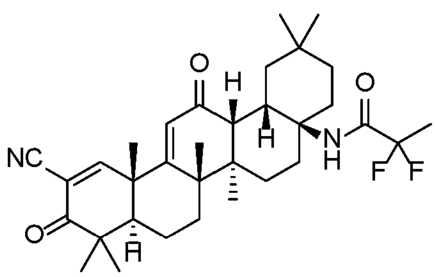

,

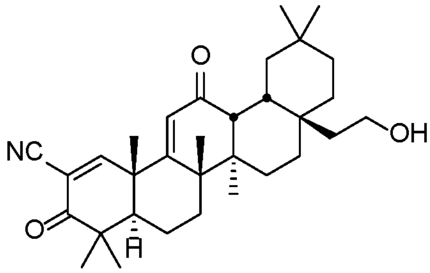 , 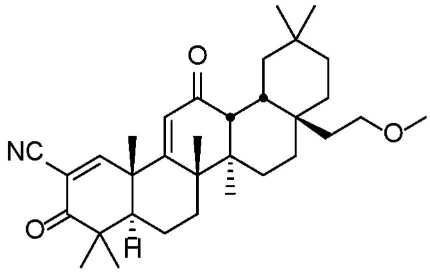 ,

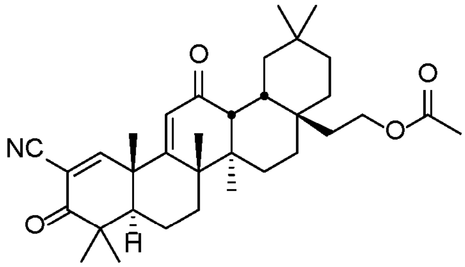 , 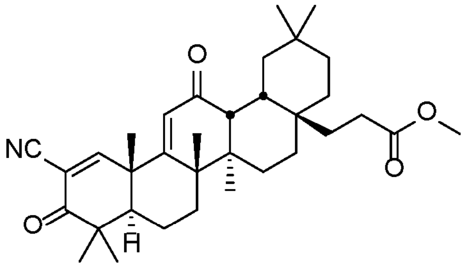 ,

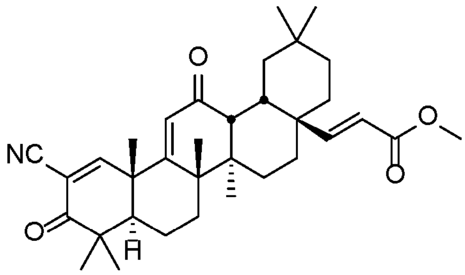 , 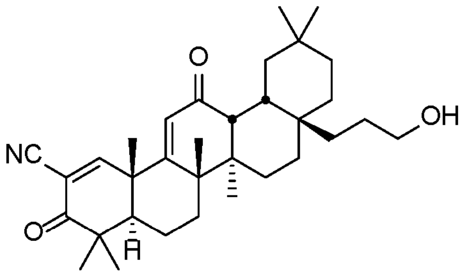 ,

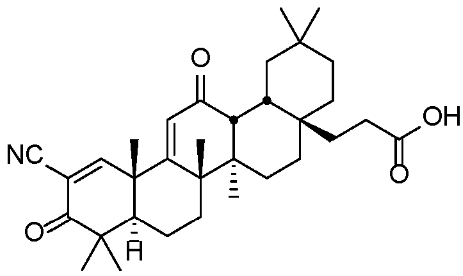 , 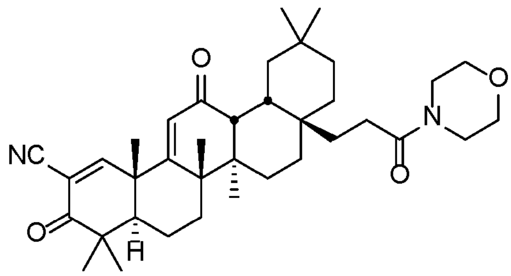 ,

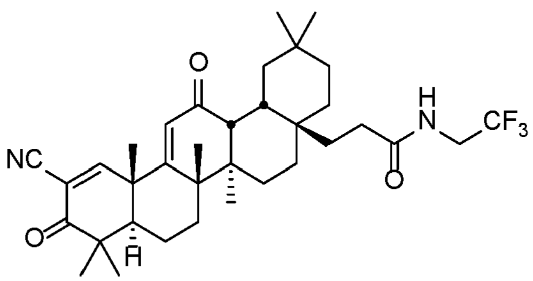 , 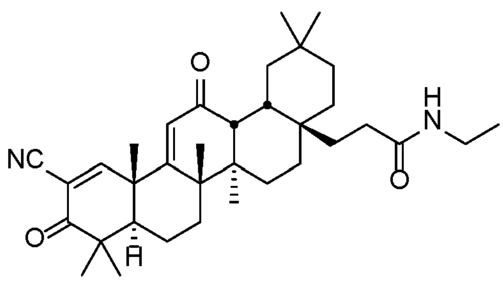 ,

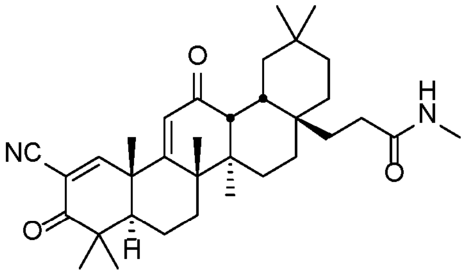, 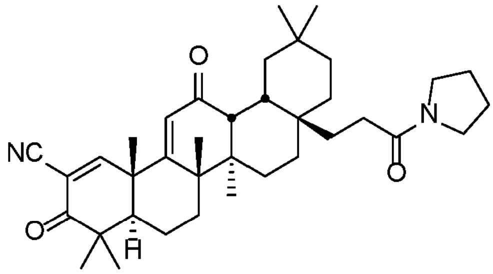,

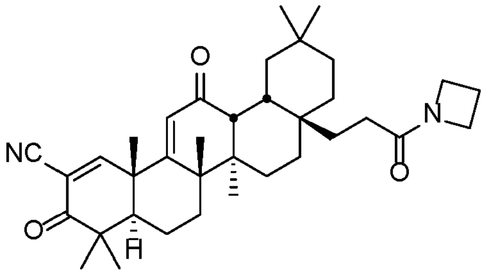, 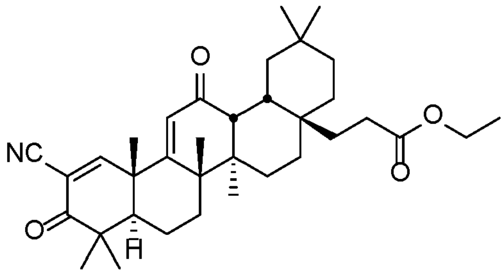,

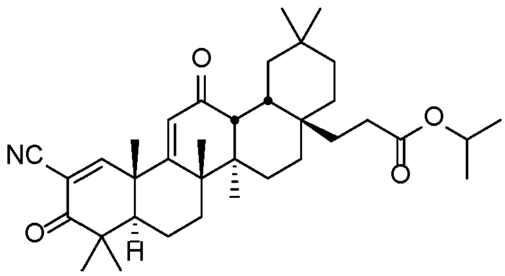, 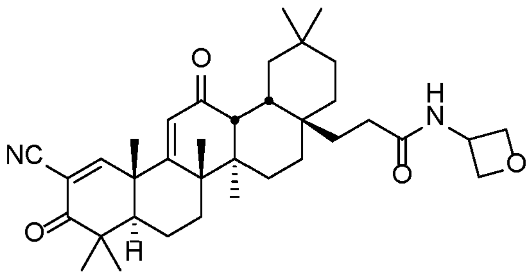,

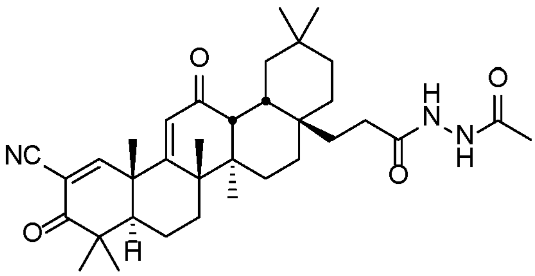, 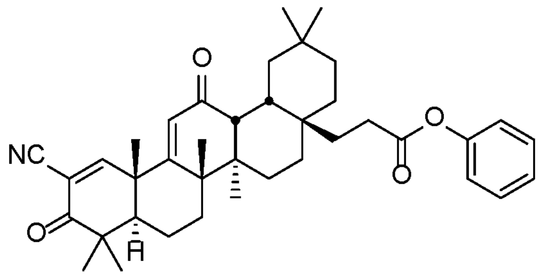,

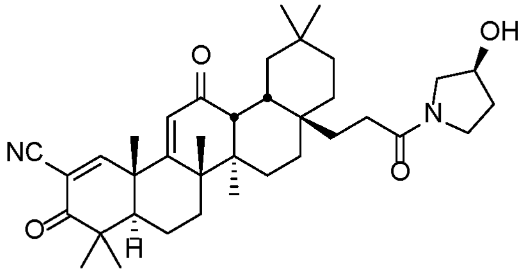, 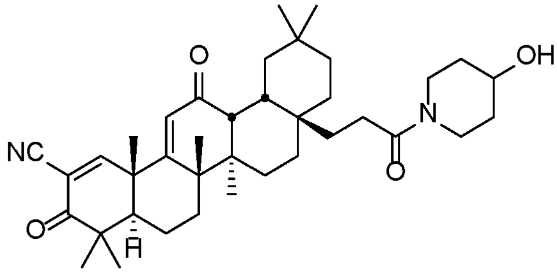,

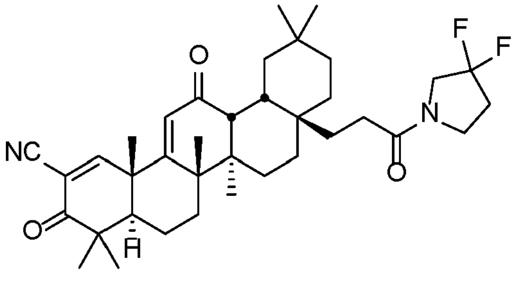,

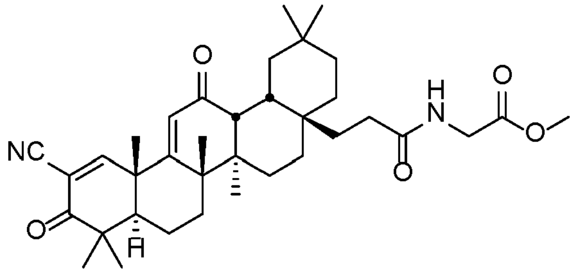,

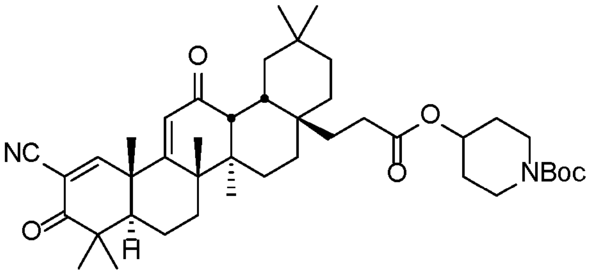

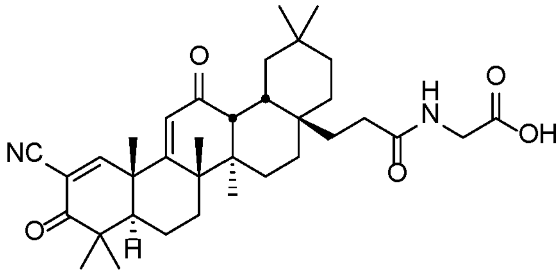,

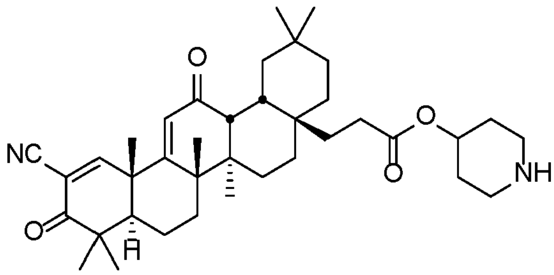,

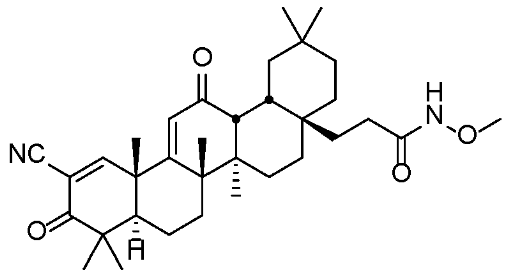,

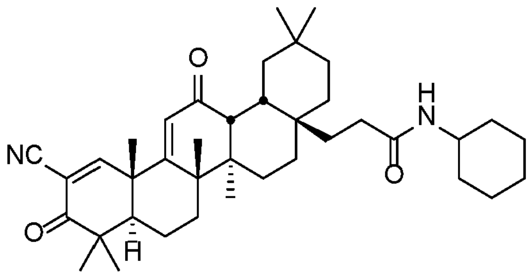,

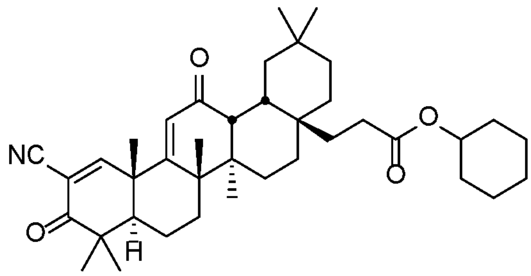,

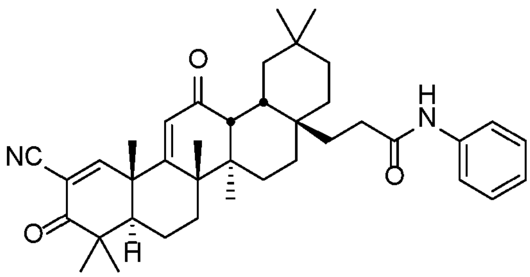,

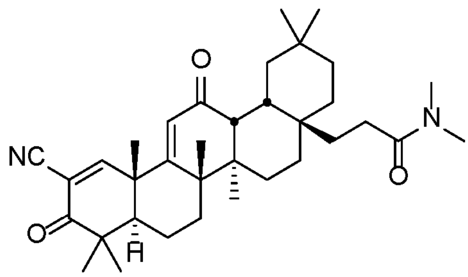,

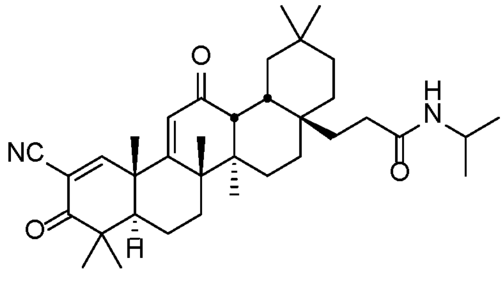
,
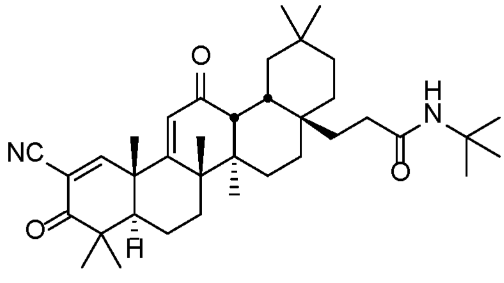
,
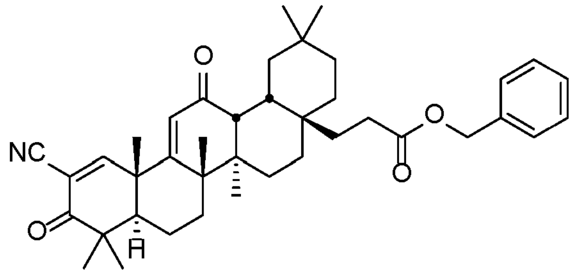
,
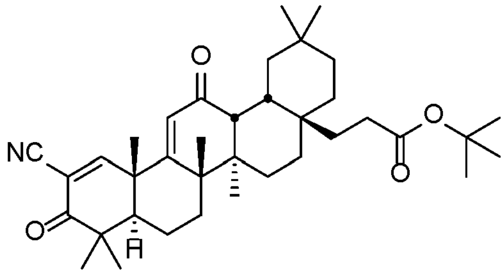
,
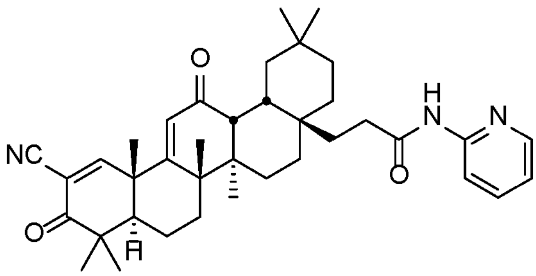
,
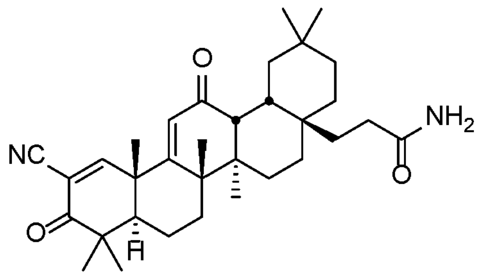
,
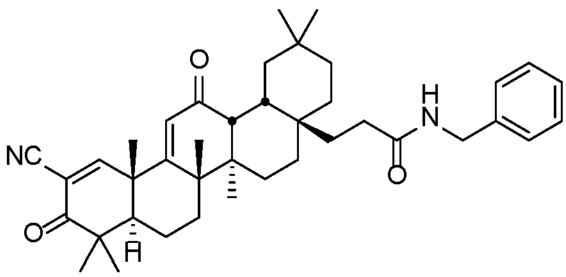
,
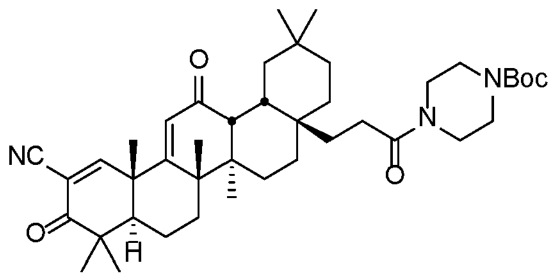
,
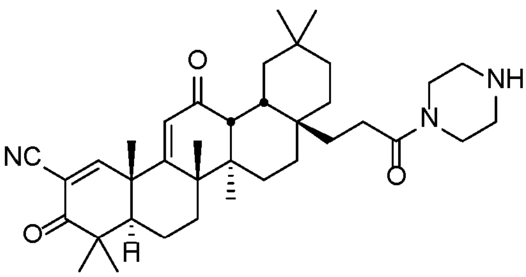
,
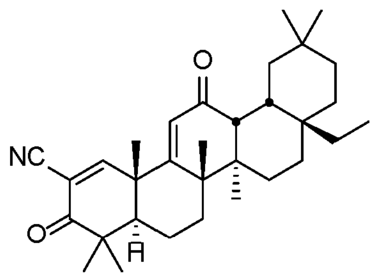
,

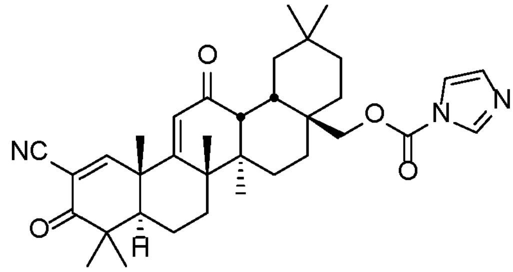
,
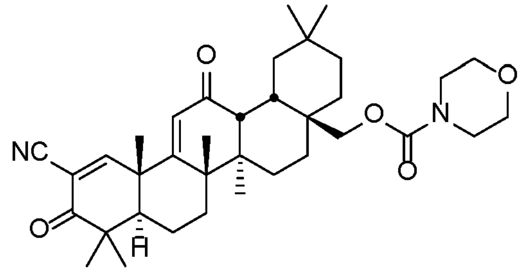
,
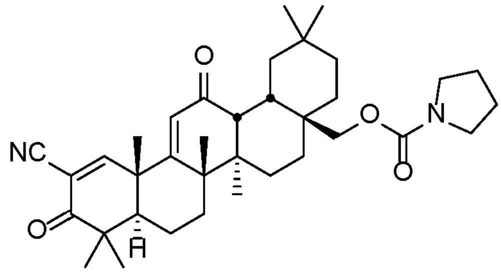
,
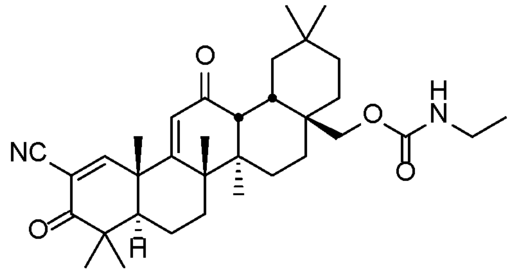
,
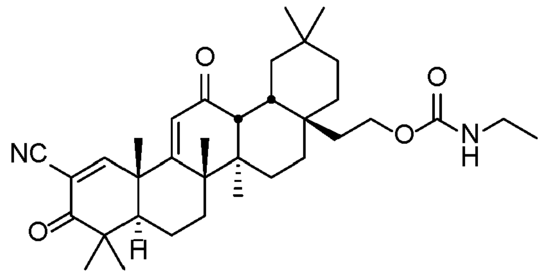
,
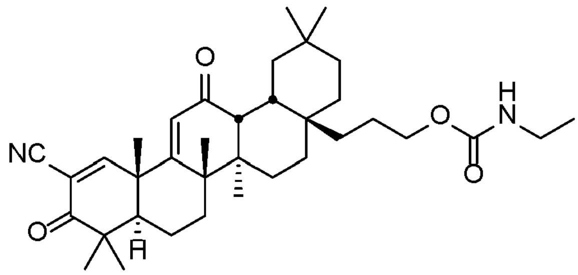
,
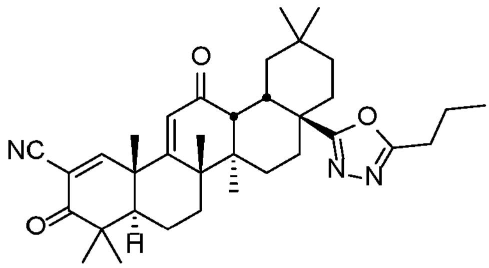
,
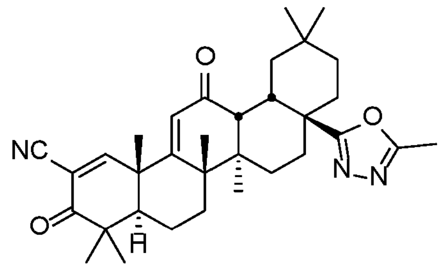
,
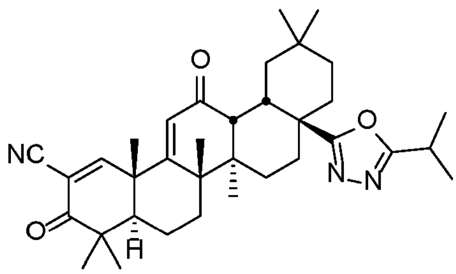
,
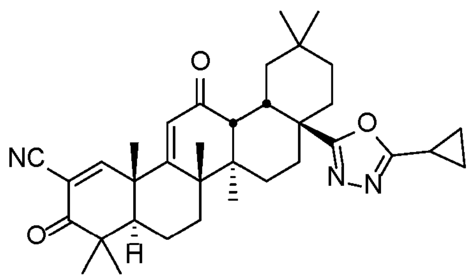
,

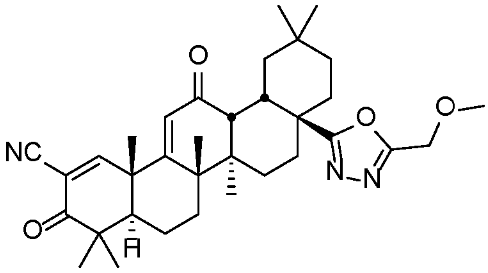
,
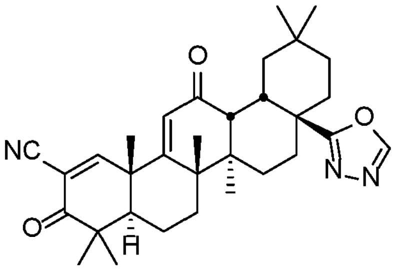
,

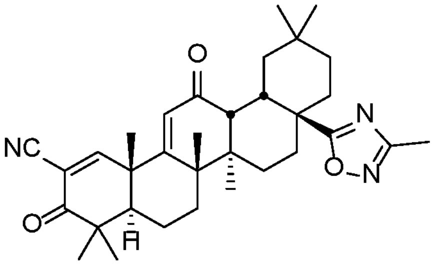
,
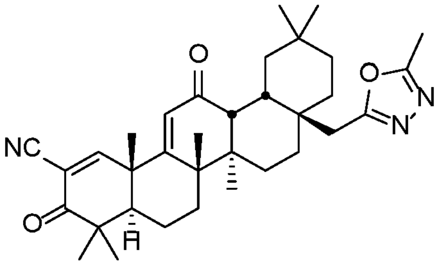
,

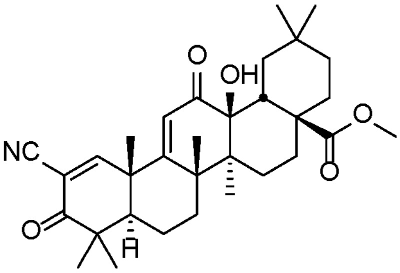
,
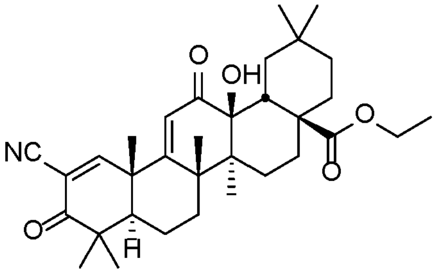
,

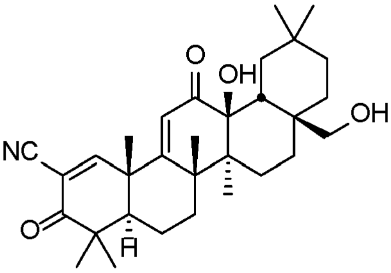
,
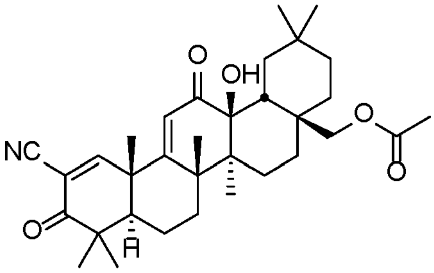
,

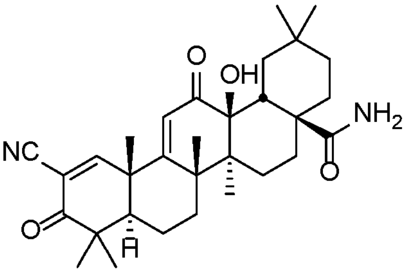
,
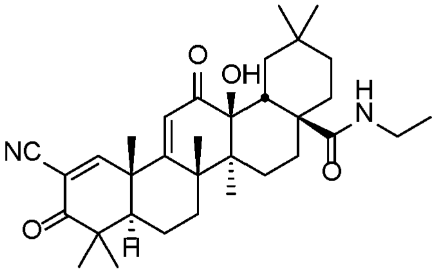
,

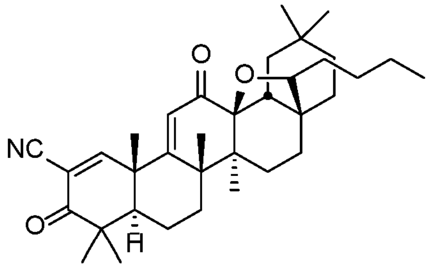
, and
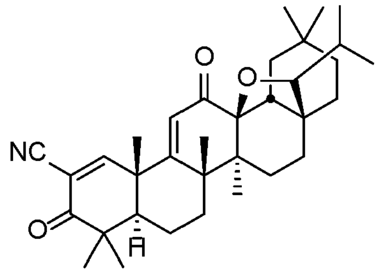
.

Table 21 summarizes *in vitro* results for several of these compounds in which RAW264.7 macrophages were pre-treated with DMSO or drugs at various concentrations (nM) for 2 hours, and then treated with 20 ng/mL IFNγ for 24 hours. NO concentration in the media was determined using a Griess reagent system; cell viability was determined using WST-1 reagent. NQO1 CD represents the concentration required to induce a two-fold increase in the expression of NQO1, an Nrf2-regulated antioxidant enzyme, in Hepa1c1c7 murine hepatoma cells (Dinkova-Kostova *et al.,* 2005). All these results are orders of magnitude more active than, for example, the parent oleanolic acid molecule. In part because induction of antioxidant pathways resulting from Nrf2 activation provides important protective effects against oxidative stress and inflammation, analogs of RTA 402 may therefore also be used to for the treatment and/or prevention of diseases, such as pulmonary arterial hypertension.

**Table 21. Suppression of IFNy-induced NO production.**

| Working ID | RAW264.7 (20 ng/ml IFNγ) | | Hepa1c1c7 cells |
|---|---|---|---|
| | NO $IC_{50}$ | WST-1 $IC_{50}$ | NQO1 CD |
| RTA 401 Reference compound | ~10 nM | > 200 **nM** | 2.3 nM |
| RTA 402 | 2.2 nM | 80 nM | 1.0 nM |
| RTA 403 Reference compound | ~0.6 nM | 100 nM | 3.3 nM |
| RTA 404 Reference compound | 5.8 nM | 100 nM | n/a |
| RTA 405 Reference compound | 6 nM | ~200 nM | n/a |
| TP-225 Reference compound | ~0.4 nM | 75 nM | 0.28 nM |

Without being bound by theory, the potency of the compound of the present invention, RTA 402, is largely derived from the addition of α,β-unsaturated carbonyl groups. In *in vitro* assays, most activity of the compounds can be abrogated by the introduction of dithiothreitol (DTT), N-acetyl cysteine (NAC), or glutathione (GSH); thiol containing moieties that interact with α,β-unsaturated carbonyl groups (Wang *et al.,* 2000; Ikeda *et al.,* 2003; 2004; Shishodia *et al.,* 2006). Biochemical assays have established that RTA 402 directly interacts with a critical cysteine residue (C179) on IKKβ (see below) and inhibits its activity (Shishodia *et al.,* 2006; Ahmad *et al.,* 2006). IKKβ controls activation of NF-κB through the "classical" pathway which involves phosphorylation-induced degradation of IκB resulting in release of NF-κB dimers to the nucleus. In macrophages, this pathway is responsible for the production of many pro-inflammatory molecules in response to TNFα and other pro-inflammatory stimuli.

RTA 402 also inhibits the JAK/STAT signaling pathway at multiple levels. JAK proteins are recruited to transmembrane receptors (*e.g.,* IL-6R) upon activation by ligands such as interferons and interleukins. JAKs then phosphorylate the intracellular portion of the receptor causing recruitment of STAT transcription factors. The STATs are then phosphorylated by JAKs, form dimers, and translocate to the nucleus where they activate transcription of several genes involved in inflammation. RTA 402 inhibits constitutive and IL-6-induced STAT3 phosphorylation and dimer formation and directly binds to cysteine residues in STAT3 (C259) and in the kinase domain of JAK1 (C1077). Biochemical assays have also established that the triterpenoids directly interact with critical cysteine residues on Keap1 (Dinkova-Kostova *et al.,* 2005). Keap1 is an actin-tethered protein that keeps the transcription factor Nrf2 sequestered in the cytoplasm under normal conditions (Kobayashi and Yamamoto, 2005). Oxidative stress results in oxidation of the regulatory cysteine residues on Keap1 and causes the release of Nrf2. Nrf2 then translocates to the nucleus and binds to antioxidant response elements (AREs) resulting in transcriptional activation of many antioxidant and anti-inflammatory genes. Another target of the Keap1/Nrf2/ARE pathway is heme oxygenase 1 (HO-1). HO-1 breaks down heme into bilirubin and carbon monoxide and plays many antioxidant and anti-inflammatory roles (Maines and Gibbs, 2005). HO-1 has recently been shown to be potently induced by the triterpenoids (Liby *et al.,* 2005), including RTA 402. RTA 402 and many structural analogs have also been shown to be potent inducers of the expression of other Phase 2 proteins (Yates *et*

*al.,* 2007). RTA 402 is a potent inhibitor of NF-κB activation. Furthermore, RTA 402 activates the Keap1/Nrf2/ARE pathway and induces expression of HO-1.

Compounds employed may be made using the methods described by Honda *et al.* (2000a); Honda *et al.* (2000b); Honda *et al.* (2002); and U.S. Patent Application Publications 2009/0326063, 2010/0056777, 2010/0048892, 2010/0048911, 2010/0041904, 2003/0232786, 2008/0261985 and 2010/0048887. These methods can be further modified and optimized using the principles and techniques of organic chemistry as applied by a person skilled in the art. Such principles and techniques are taught, for example, in March's Advanced Organic Chemistry: Reactions, Mechanisms, and Structure (2007*).*

Compounds employed in methods of the invention may contain one or more asymmetrically-substituted carbon or nitrogen atoms, and may be isolated in optically active or racemic form. Thus, all chiral, diastereomeric, racemic, epimeric, and geometric isomeric forms of a structure are intended, unless the specific stereochemistry or isomeric form is specifically indicated. Compounds may occur as racemates and racemic mixtures, single enantiomers, diastereomeric mixtures and individual diastereomers. In some embodiments, a single diastereomer is obtained. The chiral centers of the compounds of the present invention can have the S or the R configuration.

Polymorphic forms of the compounds of the present invention, e.g., Forms A and B of CDDO-Me, may be used in accordance with the methods of this inventions. Form B displays a bioavailability that is surprisingly better than that of Form A. Specifically the bioavailability of Form B was higher than that of Form A CDDO-Me in monkeys when the monkeys received equivalent dosages of the two forms orally, in gelatin capsules. See U.S. Patent Application Publication 2009/0048204.

"Form A" of CDDO-Me (RTA 402) is unsolvated (non-hydrous) and can be characterized by a distinctive crystal structure, with a space group of $P4_3\,2_12$ (no. 96) unit cell dimensions of a=14.2 Å, b=14.2 Å and c=81.6 Å, and by a packing structure, whereby three molecules are packed in helical fashion down the crystallographic b axis. In some embodiments, Form A can also be characterized by X-ray powder diffraction (XRPD) pattern (CuKα) comprising significant diffraction peaks at about 8.8, 12.9, 13.4, 14.2 and 17.4 °θ. In some variations, the X-ray powder diffraction of Form A is substantially as shown in FIG. 1A or FIG. 1B.

Unlike Form A, "Form B" of CDDO-Me is in a single phase but lacks such a defined crystal structure. Samples of Form B show no long-range molecular correlation, *i.e.,* above roughly 20 Å. Moreover, thermal analysis of Form B samples reveals a glass transition temperature ($T_g$) in a range from about 120°C to about 130°C. In contrast, a disordered nanocrystalline material does not display a $T_g$ but instead only a melting temperature ($T_m$), above which crystalline structure becomes a liquid. Form B is typified by an XRPD spectrum (FIG. 1C) differing from that of Form A (FIG. 1A or FIG. 1B). Since it does not have a defined crystal structure, Form B likewise lacks distinct XRPD peaks, such as those that typify Form A, and instead is characterized by a general "halo" XRPD pattern. In particular, the non-crystalline Form B falls into the category of "X-ray amorphous" solids because its XRPD pattern exhibits three or fewer primary diffraction halos. Within this category, Form B is a "glassy" material.

Form A and Form B of CDDO-Me are readily prepared from a variety of solutions of the compound. For example, Form B can be prepared by fast evaporation or slow evaporation in MTBE, THF, toluene, or ethyl acetate. Form A can be prepared in several ways, including *via* fast evaporation, slow evaporation, or slow cooling of a CDDO-Me solution in ethanol or methanol. Preparations of CDDO-Me in acetone can produce either Form A, using fast evaporation, or Form B, using slow evaporation.

Various means of characterization can be used together to distinguish Form A and Form B CDDO-Me from each other and from other forms of CDDO-Me. Illustrative of the techniques suitable for this purpose are solid state Nuclear Magnetic Resonance (NMR), X-ray powder diffraction (compare FIGS. 1A & B with FIG. 1C), X-ray crystallography, differential scanning calorimetry (DSC), dynamic vapor sorption/desorption (DVS), Karl Fischer analysis (KF), hot stage microscopy, modulated differential screening calorimetry, FT-IR, and Raman spectroscopy. In particular, analysis of the XRPD and DSC data can distinguish Form A, Form B, and a hemibenzenate form of CDDO-Me. See U.S. Patent Application Publication 2009/0048204.

Additional details regarding polymorphic forms of CDDO-Me are described in U.S. Patent Application Publication 2009/0048204, PCT Publication WO 2009/023232 and PCT Publication WO 2010/093944.

Non-limiting specific formulations of the compounds disclosed herein include CDDO-Me polymer dispersions. See, for example, PCT Publication WO 2010/093944. Some of the formulations reported therein exhibit higher bioavailability than either the micronized Form A or nanocrystalline Form A formulations. Additionally, the polymer dispersion based formulations demonstrate further surprising improvements in oral bioavailability relative to the micronized Form B formulations. For example, the methacrylic acid copolymer, Type C and HPMC-P formulations showed the greatest bioavailability in the subject monkeys.

Compounds employed in methods of the invention may also exist in prodrug form. Since prodrugs enhance numerous desirable qualities of pharmaceuticals, e.g., solubility, bioavailability, manufacturing, *etc.,* the compounds employed in some methods of the invention may, if desired, be delivered in prodrug form. Thus, the invention contemplates prodrugs of compounds of the present invention as well as methods of delivering prodrugs. Prodrugs of the compounds

employed in the invention may be prepared by modifying functional groups present in the compound in such a way that the modifications are cleaved, either in routine manipulation or *in vivo,* to the parent compound. Accordingly, prodrugs include, for example, compounds described herein in which a hydroxy, amino, or carboxy group is bonded to any group that, when the prodrug is administered to a subject, cleaves to form a hydroxy, amino, or carboxylic acid, respectively.

It should be recognized that the particular anion or cation forming a part of any salt of this invention is not critical, so long as the salt, as a whole, is pharmacologically acceptable. Examples of pharmaceutically acceptable salts and their methods of preparation and use are presented in Handbook of Pharmaceutical Salts: Properties, and Use (2002).

Compounds employed in methods of the invention may also have the advantage that they may be more efficacious than, be less toxic than, be longer acting than, be more potent than, produce fewer side effects than, be more easily absorbed than, have a better pharmacokinetic profile (*e.g.,* higher oral bioavailability and/or lower clearance) than, and/or have other useful pharmacological, physical, or chemical properties over compounds known in the prior art for use in the indications stated herein.

PAH is a substantially different disease than systemic hypertension. PAH is characterized by high pulmonary artery and right ventricular pressures due to increased pulmonary vascular resistance; systemic hypertension is characterized by elevated pressure in the systemic circulation. Typically patients with PAH do not have systemic hypertension.

The symptoms of PAH are well known. Moreover, animal models of these conditions may be used to optimize dosages (*see,* Bauer *et al.,* 2007). The skilled practitioner will be able to determine optimal dosages without undue experimentation.

Because a drug may be effective as a treatment for systemic hypertension does not mean that it will also be effective for treating PAH. For example, a vasodilator drug that is effective for treating systemic hypertension, such as the ACE-inhibitor Captopril, can worsen pulmonary arterial hypertension and RV failure in patients with PAH. Evidence for the potential deleterious effects of drugs used to treat systemic hypertension on PAH are given by Packer (1985). The one known exception to this limitation is that approximately 15%-20% of patients with idiopathic PAH respond to calcium channel blockers, agents that also may be used to treat systemic hypertension. To determine if a patient has so-called "reactive" PAH and may respond to therapy with a calcium channel blocker, the diagnostic evaluation of PAH includes a pulmonary artery catheterization and acute challenge with adenosine, prostacyclin, or inhaled nitric oxide. If the patient has a greater than 10 mm Hg reduction in the mean pulmonary artery pressure and the mean pulmonary artery pressure decreases to less than or equal to 40 mm Hg with one of these agents, then testing to determine if the patient will respond to a calcium channel blocker may be performed (Rich *et al.,* 1992; Badesch *et al.,* 2004). Some clinicians consider PAH reactive if there is a 20% or greater decrease in the mean pulmonary artery pressure in response to adenosine, prostacyclin, or inhaled nitric oxide. The reason that testing for acute vasoreactivity with prostacyclin, adenosine, or inhaled nitric oxide is performed prior to testing with a calcium channel blocker is that some patients given a calcium channel blocker who were not previously shown to have acute vasoreactivity have died (Badesch *et al.,* 2004). This complicated evaluation and treatment algorithm emphasizes that drugs used to treat systemic hypertension are not necessarily appropriate for patients with PAH.

### III. Pulmonary Arterial Hypertension

Pulmonary arterial hypertension is a life-threatening disease characterized by a marked and sustained elevation of pulmonary artery pressure and an increase in pulmonary vascular resistance leading to right ventricular (RV) failure and death. Current therapeutic approaches for the treatment of chronic pulmonary arterial hypertension mainly provide symptomatic relief, as well as some improvement of prognosis. Although postulated for all treatments, evidence for direct anti-proliferative effects of most approaches is missing. In addition, the use of most of the currently applied agents is hampered by either undesired side effects or inconvenient drug administration routes. Pathological changes of hypertensive pulmonary arteries include endothelial injury, proliferation and hyper-contraction of vascular smooth muscle cells (SMCs).

PAH with no apparent cause is termed primary pulmonary hypertension ("PPH"). Recently, various pathophysiological changes associated with this disorder, including vasoconstriction, vascular remodeling (*i.e.* proliferation of both media and intima of the pulmonary resistance vessels), and in situ thrombosis have been characterized (*e.g.,* D'Alonzo *et al.,* 1991; Palevsky *et al.,* 1989; Rubin, 1997; Wagenvoort and Wagenvoort, 1970; Wood, 1958). Impairment of vascular and endothelial homeostasis is evidenced from a reduced synthesis of prostacyclin ($PGI_2$), increased thromboxane production, decreased formation of nitric oxide and increased synthesis of endothelin-1 (Giaid and Saleh, 1995; Xue and Johns, 1995). The intracellular free calcium concentration of vascular smooth muscle cells of pulmonary arteries in PPH has been reported to be elevated.

Pulmonary arterial hypertension (PAH) is defined as pulmonary vascular disease affecting the pulmonary arterioles resulting in an elevation in pulmonary artery pressure and pulmonary vascular resistance but with normal or only mildly elevated left-sided filling pressures (McLaughlin and Rich, 2004). PAH is caused by a constellation of diseases

that affect the pulmonary vasculature. PAH can be caused by or associated with collagen vascular disorders, such as systemic sclerosis (scleroderma), uncorrected congenital heart disease, liver disease, portal hypertension, HIV infection, Hepatitis C, certain toxins, splenectomy, hereditary hemorrhagic telangiectasia, and primary genetic abnormalities. In particular, a mutation in the bone morphogenetic protein type 2 receptor (a TGF-b receptor) has been identified as a cause of familial primary pulmonary hypertension (PPH) (Lane *et al.,* 2000; Deng *et al.,* 2000). It is estimated that 6% of cases of PPH are familial, and that the rest are "sporadic." The incidence of PPH is estimated to be approximately 1 case per 1 million population. Secondary causes of PAH have a much higher incidence. The pathologic signature of PAH is the plexiform lesion of the lung, which consists of obliterative endothelial cell proliferation and vascular smooth muscle cell hypertrophy in small precapillary pulmonary arterioles. PAH is a progressive disease associated with a high mortality. Patients with PAH may develop right ventricular (RV) failure, the extent of which predicts outcome (McLaughlin *et al.,* 2002).

The evaluation and diagnosis of PAH is reviewed by McLaughlin and Rich (2004) and McGoon *et al.* (2004). A clinical history, such as symptoms of shortness of breath, a family history of PAH, presence of risk factors, and findings on physical examination, chest X-ray and electrocardiogram may lead to the suspicion of PAH. The next step in the evaluation will usually include an echocardiogram. The echocardiogram can be used to estimate the pulmonary artery pressure from the Doppler analysis of the tricuspid regurgitation jet. The echocardiogram can also be used to evaluate the function of the right and left ventricle, and the presence of valvular heart disease, such as mitral stenosis and aortic stenosis. The echocardiogram can also be useful in diagnosing congenital heart disease, such as an uncorrected atrial septal defect or patent ductus arteriosus. Findings on echocardiogram consistent with a diagnosis of PAH would include: 1) Doppler evidence for elevated pulmonary artery pressure; 2) right atrial enlargement; 3) right ventricular enlargement and/or hypertrophy; 4) absence of mitral stenosis, pulmonic stenosis, and aortic stenosis; 5) normal size or small left ventricle; 6) relative preservation of or normal left ventricular function. To confirm the diagnosis of PAH a cardiac catheterization to directly measure the pressures in the right side of the heart and in the pulmonary vasculature is mandatory. An accurate measurement of the pulmonary capillary wedge pressure (PCWP), which gives an accurate estimate of the left atrial and left ventricular end-diastolic pressure, is also required. If an accurate PCWP cannot be obtained, then direct measurement of LV end-diastolic pressure by left heart catheterization is advised. By definition, patients with PAH should have a low or normal PCWP. However, in the late stages of PAH, the PCWP can become somewhat elevated though usually not greater than 16 mm Hg (McLaughlin and Rich, 2004; McGoon *et al.,* 2004). The upper limit of normal for mean pulmonary artery pressure in an adult human is 19 mm Hg. A commonly used definition of mean pulmonary artery pressure is one-third the value of the systolic pulmonary artery pressure plus two-thirds of the diastolic pulmonary artery pressure. Severe PAH may be defined as a mean pulmonary artery pressure greater than or equal to 25 mm Hg with a PCWP less than or equal to 15-16 mm Hg, and a pulmonary vascular resistance (PVR) greater than or equal to 240 dynes sec/$cm^5$. Pulmonary vascular resistance is defined as the mean pulmonary artery pressure minus the PCWP divided by the cardiac output. This ratio is multiplied by 80 to express the result in dyne sec/$cm^5$. The PVR may also be expressed in millimeters Hg per liter per minute, which is referred to as Wood Units. The PVR in a normal adult is 67 $\pm$ 23 dyne sec/$cm^5$ or 1 Wood Unit (McLaughlin and Rich, 2004; McGoon *et al.,* 2004; Galie *et al.,* 2005). In clinical trials to test efficacy of drugs for PAH, patients with left sided myocardial disease or valvular heart disease are typically excluded (Galie *et al.,* 2005).

The status of pulmonary arterial hypertension can be assessed in patients according to the World Health Organization (WHO) classification (modified after the New York Association Functional Classification) as detailed below:

Class I-Patients with pulmonary hypertension but without resulting limitation of physical activity. Ordinary physical activity does not cause undue dyspnea or fatigue, chest pain or near syncope.

Class II-Patients with pulmonary hypertension resulting in slight limitation of physical activity. They are comfortable at rest. Ordinary physical activity causes undue dispend or fatigue, chest pain or near syncope.

Class III-Patients with pulmonary hypertension resulting in marked limitation of physical activity. They are comfortable at rest. Less than ordinary activity causes undue dyspnea or fatigue, chest pain or near syncope.

Class IV-Patients with pulmonary hypertension with inability to carry out any physical activity without symptoms. These patients manifest signs of right heart failure. Dyspnea and/or fatigue may even be present at rest. Discomfort is increased by any physical activity.

At one time, the only effective long-term therapy for PAH in conjunction with anticoagulant therapy was continuous intravenous administration of prostacyclin, also known as epoprostenol ($PGI_2$) (Barst *et al.,* 1996; McLaughlin *et al.,* 1998). Later, the non-selective endothelin receptor antagonist, bosentan, showed efficacy for the treatment of PAH (Rubin *et al.,* 2002). As the first orally bioavailable agent with efficacy in the treatment of PAH, bosentan represented a significant advance. However, the current leading therapeutic category for PAH is treatment with a selective endothelin type A receptor antagonist (Galie *et al.,* 2005; Langleben *et al.,* 2004). Inhibitors of phosphodiesterase type V (PDE-V), including sildenafil and tadalafil, have been approved for the treatment of PAH (Lee *et al.,* 2005; Kataoka *et al.,* 2005). PDE-V inhibition results in an increase in cyclic GMP, which leads to vasodilation of the pulmonary vasculature. Treprostinil, an

analogue of $PGI_2$, can be administered subcutaneously to appropriately selected patients with PAH (Oudiz *et al.,* 2004; Vachiery and Naeije, 2004). In addition, Iloprost, another prostacyclin analogue, can be administered in nebulized form by direct inhalation (Galie *et al.,* 2002). Riociguat, a stimulator of soluble guanylate cyclas (sGC), is also approved for the treatment of PAH. These agents are used to treat PAH of multiple etiologies, including PAH associated with or caused by familial PAH (primary pulmonary hypertension or PPH), idiopathic PAH, scleroderma, mixed connective tissue disease, systemic lupus erythematosus, HIV infection, toxins, such as phentermine/fenfluramine, congenital heart disease, Hepatitis C, liver cirrhosis, chronic thrombo-embolic pulmonary artery hypertension (distal or inoperable), hereditary hemorrhagic telangiectasia, and splenectomy. All approved agents for PAH are essentially vasodilatory in effect. Consequently, they only address a portion of the overall pathology of PAH. Without being bound by theory, the compound of the invention, in contrast, has documented anti-inflammatory and antiproliferative effects in addition to its effects on vascular tone, potentially addressing PAH pathology in a more comprehensive fashion. In addition, the methods provided herein may be used to activate Nrf2 with positive effects on mitochondrial function, and thereby address metabolic and energetic aspects of PAH (Sutendra, 2014; Hayes and Dinkova-Kostova, 2014).

### IV. Cardiovascular Disease

The compound and methods of this invention may be used for treating patients with cardiovascular disease. See U.S. Patent Application 12/352,473. Cardiovascular (CV) disease is among the most important causes of mortality worldwide, and is the leading cause of death in many developed nations. The etiology of CV disease is complex, but the majority of causes are related to inadequate or completely disrupted supply of blood to a critical organ or tissue. Frequently such a condition arises from the rupture of one or more atherosclerotic plaques, which leads to the formation of a thrombus that blocks blood flow in a critical vessel. Such thrombosis is the principal cause of heart attacks, in which one or more of the coronary arteries is blocked and blood flow to the heart itself is disrupted. The resulting ischemia is highly damaging to cardiac tissue, both from lack of oxygen during the ischemic event and from excessive formation of free radicals after blood flow is restored (a phenomenon known as ischemia-reperfusion injury). Similar damage occurs in the brain during a thrombotic stroke, when a cerebral artery or other major vessel is blocked by thrombosis. Hemorrhagic strokes, in contrast, involve rupture of a blood vessel and bleeding into the surrounding brain tissue. This creates oxidative stress in the immediate area of the hemorrhage, due to the presence of large amounts of free heme and other reactive species, and ischemia in other parts of the brain due to compromised blood flow. Subarachnoid hemorrhage, which is frequently accompanied by cerebral vasospasm, also causes ischemia/reperfusion injury in the brain.

Alternatively, atherosclerosis may be so extensive in critical blood vessels that stenosis (narrowing of the arteries) develops and blood flow to critical organs (including the heart) is chronically insufficient. Such chronic ischemia can lead to end-organ damage of many kinds, including the cardiac hypertrophy associated with congestive heart failure.

Atherosclerosis, the underlying defect leading to many forms of cardiovascular disease, occurs when a physical defect or injury to the lining (endothelium) of an artery triggers an inflammatory response involving the proliferation of vascular smooth muscle cells and the infiltration of leukocytes into the affected area. Ultimately, a complicated lesion known as an atherosclerotic plaque may form, composed of the above-mentioned cells combined with deposits of cholesterol-bearing lipoproteins and other materials (*e.g.,* Hansson *et al.,* 2006).

One study found that RTA dh404 lessened diabetes-associated atherosclerosis. In this study, an animal model was used in conjunction with RTA dh404 as a surrogate for RTA 402. Specifically, treatment with RTA dh404, in an inverse dose-dependent manner, was found to reduce plaque in the arch, thoracic, and abdominal regions of the aorta as well as attenuate lesion deposition within the aortic sinus (Tam *et al.,* 2014).

Pharmaceutical treatments for cardiovascular disease include preventive treatments, such as the use of drugs intended to lower blood pressure or circulating levels of cholesterol and lipoproteins, as well as treatments designed to reduce the adherent tendencies of platelets and other blood cells (thereby reducing the rate of plaque progression and the risk of thrombus formation). More recently, drugs such as streptokinase and tissue plasminogen activator have been introduced and are used to dissolve the thrombus and restore blood flow. Surgical treatments include coronary artery bypass grafting to create an alternative blood supply, balloon angioplasty to compress plaque tissue and increase the diameter of the arterial lumen, and carotid endarterectomy to remove plaque tissue in the carotid artery. Such treatments, especially balloon angioplasty, may be accompanied by the use of stents, expandable mesh tubes designed to support the artery walls in the affected area and keep the vessel open. Recently, the use of drug-eluting stents has become common in order to prevent post-surgical restenosis (renarrowing of the artery) in the affected area. These devices are wire stents coated with a biocompatible polymer matrix containing a drug that inhibits cell proliferation (*e.g.,* paclitaxel or rapamycin). The polymer allows a slow, localized release of the drug in the affected area with minimal exposure of non-target tissues. Despite the significant benefits offered by such treatments, mortality from cardiovascular disease remains high and significant unmet needs in the treatment of cardiovascular disease remain.

As noted above, induction of HO-1 has been shown to be beneficial in a variety of models of cardiovascular disease, and low levels of HO-1 expression have been clinically correlated with elevated risk of CV disease. The methods

of the invention, therefore, may be used for treating or preventing a variety of cardiovascular disorders including but not limited to atherosclerosis, hypertension, myocardial infarction, chronic heart failure, stroke, subarachnoid hemorrhage, and restenosis.

## V. Pharmaceutical Formulations and Routes of Administration

Administration of the compound of the present invention to a patient will follow general protocols for the administration of pharmaceuticals, taking into account the toxicity, if any, of the drug. It is expected that the treatment cycles would be repeated as necessary.

The compound of the present invention may be administered by a variety of methods, *e.g.,* orally or by injection (*e.g.* subcutaneous, intravenous, intraperitoneal, *etc*.). Depending on the route of administration, the active compound may be coated by a material to protect the compound from the action of acids and other natural conditions which may inactivate the compound. It may also be administered by continuous perfusion/infusion of a disease or wound site. Specific examples of formulations, including a polymer-based dispersion of CDDO-Me that showed improved oral bioavailability, are provided in U.S. Application No. 12/191,176. It will be recognized by those skilled in the art that other methods of manufacture may be used to produce dispersions of the present invention with equivalent properties and utility (*see,* Repka *et al.,* 2002 and references cited therein). Such alternative methods include but are not limited to solvent evaporation, extrusion, such as hot melt extrusion, and other techniques.

To administer the therapeutic compound by other than parenteral administration, it may be necessary to coat the compound with, or co-administer the compound with, a material to prevent its inactivation. For example, the therapeutic compound may be administered to a patient in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan *et al.,* 1984).

The therapeutic compound may also be administered parenterally, intraperitoneally, intraspinally, or intracerebrally. Dispersions may be prepared in, *e.g.,* glycerol, liquid polyethylene glycols, mixtures thereof, and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi. The carrier may be a solvent or dispersion medium containing, for example, water, ethanol, polyol (such as, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, sodium chloride, or polyalcohols such as mannitol and sorbitol, in the composition. Prolonged absorption of the injectable compositions can be brought about by including in the composition an agent which delays absorption, for example, aluminum monostearate or gelatin.

Sterile injectable solutions can be prepared by incorporating the therapeutic compound in the required amount in an appropriate solvent with one or a combination of ingredients enumerated above, as required, followed by filtered sterilization. Generally, dispersions are prepared by incorporating the therapeutic compound into a sterile carrier which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (*i.e.,* the therapeutic compound) plus any additional desired ingredient from a previously sterile-filtered solution thereof.

The therapeutic compound can be orally administered, for example, with an inert diluent or an assimilable edible carrier. The therapeutic compound and other ingredients may also be enclosed in a hard or soft shell gelatin capsule, compressed into tablets, or incorporated directly into the subject's diet. For oral therapeutic administration, the therapeutic compound may be incorporated with excipients and used in the form of ingestible tablets, buccal tablets, troches, capsules, elixirs, suspensions, syrups, wafers, and the like. The percentage of the therapeutic compound in the compositions and preparations may, of course, be varied. The amount of the therapeutic compound in such therapeutically useful compositions is such that a suitable dosage will be obtained.

It is especially advantageous to formulate parenteral compositions in dosage unit form for ease of administration and uniformity of dosage. Dosage unit form as used herein refers to physically discrete units suited as unitary dosages for the subjects to be treated; each unit containing a predetermined quantity of therapeutic compound calculated to produce the desired therapeutic effect in association with the required pharmaceutical carrier. The specification for the dosage unit

forms of the invention are dictated by and directly dependent on (a) the unique characteristics of the therapeutic compound and the particular therapeutic effect to be achieved, and (b) the limitations inherent in the art of compounding such a therapeutic compound for the treatment of a selected condition in a patient.

The therapeutic compound may also be administered topically to the skin, eye, or mucosa. Alternatively, if local delivery to the lungs is desired the therapeutic compound may be administered by inhalation in a dry-powder or aerosol formulation.

The therapeutic compound may be formulated in a biocompatible matrix for use in a drug-eluting stent.

The actual dosage amount of the compound of the present invention or composition comprising the compound of the present invention administered to a subject may be determined by physical and physiological factors such as age, sex, body weight, severity of condition, the type of disease being treated, previous or concurrent therapeutic interventions, idiopathy of the subject and on the route of administration. These factors may be determined by a skilled artisan. The practitioner responsible for administration will typically determine the concentration of active ingredient(s) in a composition and appropriate dose(s) for the individual subject. The dosage may be adjusted by the individual physician in the event of any complication.

In some embodiments, the pharmaceutically effective amount is a daily dose from about 0.1 mg to about 500 mg of the compound. In some variations, the daily dose is from about 1 mg to about 300 mg of the compound. In some variations, the daily dose is from about 10 mg to about 200 mg of the compound. In some variations, the daily dose is about 25 mg of the compound. **In** other variations, the daily dose is about 75 mg of the compound. **In** still other variations, the daily dose is about 150 mg of the compound. **In** further variations, the daily dose is from about 0.1 mg to about 30 mg of the compound. **In** some variations, the daily dose is from about 0.5 mg to about 20 mg of the compound. **In** some variations, the daily dose is from about 1 mg to about 15 mg of the compound. **In** some variations, the daily dose is from about 1 mg to about 10 mg of the compound. **In** some variations, the daily dose is from about 1 mg to about 5 mg of the compound.

**In** some embodiments, the pharmaceutically effective amount is a daily dose is 0.01 - 25 mg of compound per kg of body weight. **In** some variations, the daily dose is 0.05 - 20 mg of compound per kg of body weight. **In** some variations, the daily dose is 0.1 - 10 mg of compound per kg of body weight. In some variations, the daily dose is 0.1 - 5 mg of compound per kg of body weight. In some variations, the daily dose is 0.1 - 2.5 mg of compound per kg of body weight.

In some embodiments, the pharmaceutically effective amount is a daily dose is of 0.1 - 1000 mg of compound per kg of body weight. In some variations, the daily dose is 0.15 - 20 mg of compound per kg of body weight. In some variations, the daily dose is 0.20 - 10 mg of compound per kg of body weight. In some variations, the daily dose is 0.40 - 3 mg of compound per kg of body weight. In some variations, the daily dose is 0.50 - 9 mg of compound per kg of body weight. In some variations, the daily dose is 0.60 - 8 mg of compound per kg of body weight. In some variations, the daily dose is 0.70 - 7 mg of compound per kg of body weight. In some variations, the daily dose is 0.80 - 6 mg of compound per kg of body weight. In some variations, the daily dose is 0.90 - 5 mg of compound per kg of body weight. In some variations, the daily dose is from about 1 mg to about 5 mg of compound per kg of body weight.

An effective amount typically will vary from about 0.001 mg/kg to about 1,000 mg/kg, from about 0.01 mg/kg to about 750 mg/kg, from about 0.1 mg/kg to about 500 mg/kg, from about 0.2 mg/kg to about 250 mg/kg, from about 0.3 mg/kg to about 150 mg/kg, from about 0.3 mg/kg to about 100 mg/kg, from about 0.4 mg/kg to about 75 mg/kg, from about 0.5 mg/kg to about 50 mg/kg, from about 0.6 mg/kg to about 30 mg/kg, from about 0.7 mg/kg to about 25 mg/kg, from about 0.8 mg/kg to about 15 mg/kg, from about 0.9 mg/kg to about 10 mg/kg, from about 1 mg/kg to about 5 mg/kg, from about 100 mg/kg to about 500 mg/kg, from about 1.0 mg/kg to about 250 mg/kg, or from about 10.0 mg/kg to about 150 mg/kg, in one or more dose administrations daily, for one or several days (depending, of course, of the mode of administration and the factors discussed above). Other suitable dose ranges include 1 mg to 10,000 mg per day, 100 mg to 10,000 mg per day, 500 mg to 10,000 mg per day, and 500 mg to 1,000 mg per day. In some particular embodiments, the amount is less than 10,000 mg per day with a range, for example, of 750 mg to 9,000 mg per day.

The effective amount may be less than 1 mg/kg/day, less than 500 mg/kg/day, less than 250 mg/kg/day, less than 100 mg/kg/day, less than 50 mg/kg/day, less than 25 mg/kg/day, less than 10 mg/kg/day, or less than 5 mg/kg/day. It may alternatively be in the range of 1 mg/kg/day to 200 mg/kg/day. For example, regarding treatment of diabetic patients, the unit dosage may be an amount that reduces blood glucose by at least 40% as compared to an untreated subject. In another embodiment, the unit dosage is an amount that reduces blood glucose to a level that is within $\pm$10% of the blood glucose level of a non-diabetic subject.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 1 mg/kg/body weight to about 5 mg/kg/body weight, a range of about 5 mg/kg/body weight to about

100 mg/kg/body weight, about 5 microgram/kg/body weight to about 500 milligram/kg/body weight, *etc.,* can be administered, based on the numbers described above.

A pharmaceutical composition of the present invention may comprise, for example, at least about 0.1% of the compound of the present invention. The compound of the present invention may comprise between about 2% to about 75% of the weight of the unit, or between about 25% to about 60%, for example, and any range derivable therein.

Single or multiple doses of the agents are contemplated. Desired time intervals for delivery of multiple doses can be determined by one of ordinary skill in the art employing no more than routine experimentation. As an example, subjects may be administered two doses daily at approximately 12 hour intervals. The agent may be administered once a day.

The agent(s) may be administered on a routine schedule. As used herein a routine schedule refers to a predetermined designated period of time. The routine schedule may encompass periods of time which are identical or which differ in length, as long as the schedule is predetermined. For instance, the routine schedule may involve administration twice a day, every day, every two days, every three days, every four days, every five days, every six days, a weekly basis, a monthly basis or any set number of days or weeks therebetween. Alternatively, the predetermined routine schedule may involve administration on a twice daily basis for the first week, followed by a daily basis for several months, *etc.* The agent(s) may taken orally and that the timing of which is or is not dependent upon food intake. Thus, for example, the agent can be taken every morning and/or every evening, regardless of when the subject has eaten or will eat.

Non-limiting specific formulations include CDDO-Me polymer dispersions (see U.S. Application No. 12/191,176, filed August 13, 2008). Some of the formulations reported therein exhibited higher bioavailability than either the micronized Form A or nanocrystalline Form A formulations. Additionally, the polymer dispersion based formulations demonstrated further surprising improvements in oral bioavailability relative to the micronized Form B formulations. For example, the methacrylic acid copolymer, Type C and HPMC-P formulations showed the greatest bioavailability in the subject monkeys.

## VI. Combination Therapy

In addition to being used as a monotherapy, the compound of the present invention may also find use in combination therapies. Effective combination therapy may be achieved with a single composition or pharmacological formulation that includes both agents, or with two distinct compositions or formulations, administered at the same time, wherein one composition includes the compound of this invention, and the other includes the second agent(s). Alternatively, the therapy may precede or follow the other agent treatment by intervals ranging from minutes to months.

Various combinations may be employed, such as when the compound of the present invention is "A" and "B" represents a secondary agent, non-limiting examples of which are described below:

A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

It is contemplated that other anti-inflammatory agents may be used in conjunction with the treatments of the current invention. For example, other COX inhibitors may be used, including arylcarboxylic acids (salicylic acid, acetylsalicylic acid, diflunisal, choline magnesium trisalicylate, salicylate, benorylate, flufenamic acid, mefenamic acid, meclofenamic acid and triflumic acid), arylalkanoic acids (diclofenac, fenclofenac, alclofenac, fentiazac, ibuprofen, flurbiprofen, ketoprofen, naproxen, fenoprofen, fenbufen, suprofen, indoprofen, tiaprofenic acid, benoxaprofen, pirprofen, tolmetin, zomepirac, clopinac, indomethacin and sulindac) and enolic acids (phenylbutazone, oxyphenbutazone, azapropazone, feprazone, piroxicam, and isoxicam. See also U.S. Patent 6,025,395.

FDA approved treatments for pulmonary hypertension include prostanoids (epoprostenol, iloprost, and treprostinil), endothelin receptor antagonists (bosentan, ambrisentan, and macitentan), phosphodiesterase-5 inhibitors (sildenafil and tadalafil), and sGC stimulators (riociguat). The use of any of these agents in conjunction with the treatments of the current invention is contemplated. When combined with the compound of the current invention, such agents may be administered at the standard approved dose or in the standard approved range of doses, or may be administered at a lower than standard dose. Furthermore, the use of the following combination agents is contemplated: rho-kinase inhibitors, such as Y-27632, fasudil, and H-1152P; epoprostenol derivatives, such as prostacyclin, treprostinil, beraprost, and iloprost; serotonin blockers, such as sarpogrelate; endothelin receptor antagonists, such as besentan, sitaxsentan, ambrisentan, and TBC3711; PDE inhibitors, such as sildenafil, tadalafil, udenafil, and vardenafil; calcium channel blockers, such as amlodipine, bepridil, clentiazem, diltiazem, fendiline, gallopamil, mibefradil, prenylamine, semotiadil, terodiline, verapamil, aranidipine, bamidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, cinnarizine, flunarizine, lidoflazine, lomerizine, bencyclane, etafenone, and perhexiline; tyrosine kinase inhibitors, such as imatinib; inhaled nitric

oxide and nitric oxide-donating agents, such as inhaled nitrite; IκB inhibitors, such as IMD 1041; prostacyclin receptor agonists, such as selexipag; stimulators of hematopoiesis, such as TXA 127 (angiotensin (1-7)), darbepoetin alfa, erythropoetin, and epoetin alfa; anticoagulants and platelet-inhibiting agents; and diuretics.

Dietary and nutritional supplements with reported benefits for treatment or prevention of Parkinson's, Alzheimer's, multiple sclerosis, amyotrophic lateral sclerosis, rheumatoid arthritis, inflammatory bowel disease, and all other diseases whose pathogenesis is believed to involve excessive production of either nitric oxide (NO) or prostaglandins, such as acetyl-L-carnitine, octacosanol, evening primrose oil, vitamin B6, tyrosine, phenylalanine, vitamin C, L-dopa, or a combination of several antioxidants may be used in conjunction with the compound of the current invention.

Other particular secondary therapies include immunosuppressants (for transplants and autoimmune-related RKD), anti-hypertensive drugs (for high blood pressure-related RKD, *e.g.,* angiotensin-converting enzyme inhibitors and angiotensin receptor blockers), insulin (for diabetic RKD), lipid/cholesterol-lowering agents *(e.g.,* HMG-CoA reductase inhibitors such as atorvastatin or simvastatin), treatments for hyperphosphatemia or hyperparathyroidism associated with CKD (*e.g.,* sevelamer acetate, cinacalcet), dialysis, and dietary restrictions (*e.g.,* protein, salt, fluid, potassium, phosphorus).

## VII. Diagnostic Tests

### A. Measurement of B-type Natriuretic Peptide (BNP) Levels

B-type natriuretic peptide (BNP) is a 32-amino acid neurohormone that is synthesized in the ventricular myocardium and released into circulation in response to ventricular dilation and pressure overload. The functions of BNP include natriuresis, vasodilation, inhibition of the renin-angiotensin-aldosterone axis, and inhibition of sympathetic nerve activity. The plasma concentration of BNP is elevated among patients with congestive heart failure (CHF), and increases in proportion to the degree of left ventricular dysfunction and the severity of CHF symptoms.

Numerous methods and devices are well known to the skilled artisan for measuring BNP levels in patient samples, including serum and plasma. With regard to polypeptides, such as BNP, immunoassay devices and methods are often used. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule. *See, e.g.,* U.S. Patents 5,631,170 and 5,955,377. In a specific example, B-type natriuretic peptide (BNP) levels may be determined by the following method(s): protein immunoassays as described in US Patent Publication 2011/0201130. Furthermore, a number of commercially available methods exist (*e.g.,* Rawlins *et al.,* 2005).

### B. Measurement of Albumin/Creatinine Ratio (ACR)

Conventionally, proteinuria is diagnosed by a simple dipstick test. Traditionally, dipstick protein tests are quantified by measuring the total quantity of protein in a 24-hour urine collection test.

Alternatively the concentration of protein in the urine may be compared to the creatinine level in a spot urine sample. This is termed the protein/creatinine ratio (PCR). The UK Chronic Kidney Disease Guidelines (2005) states PCR is a better test than 24-hour urinary protein measurement. Proteinuria is defined as a protein/creatinine ratio greater than 45 mg/mmol (which is equivalent to albumin/creatinine ratio of greater than 30 mg/mmol or approximately 300 mg/g as defined by dipstick proteinuria of 3+) with very high levels of proteinuria being for a PCR greater than 100 mg/mmol.

Protein dipstick measurements should not be confused with the amount of protein detected on a test for microalbuminuria, which denotes values for protein for urine in mg/day versus urine protein dipstick values which denote values for protein in mg/dL. That is, there is a basal level of proteinuria that can occur below 30 mg/day which is considered non-pathological. Values between 30-300 mg/day are termed microalbuminuria which is considered pathologic. Urine protein lab values for microalbumin of >30 mg/day correspond to a detection level within the "trace" to "1+" range of a urine dipstick protein assay. Therefore, positive indication of any protein detected on a urine dipstick assay obviates any need to perform a urine microalbumin test as the upper limit for microalbuminuria has already been exceeded.

### C. Measurement of Estimated Glomerular Filtration Rate (eGFR)

A number of formulae have been devised to estimate GFR values on the basis of serum creatinine levels. A commonly used surrogate marker for estimate of creatinine clearance ($eC_{Cr}$) is the Cockcroft-Gault (CG) formula, which in turn estimates GFR in mL/min. It employs serum creatinine measurements and a patient's weight to predict the creatinine clearance. The formula, as originally published, is:

$$eCCr = \frac{(140 - Age) \times Mass(in\ kg)}{72\ \times Serum\ creatinine\ (in \frac{mg}{dL})}$$

This formula expects weight to be measured in kilograms and creatinine to be measured in mg/dL, as is standard in the USA. The resulting value is multiplied by a constant of 0.85 if the patient is female. This formula is useful because the calculations are simple and can often be performed without the aid of a calculator.

When serum creatinine is measured in μmol/L, then:

$$eCCr = \frac{(140 - Age) \times Mass\ (in\ kg) \times Constant}{Serum\ creatinine\ (in \frac{\mu mol}{L})}$$

where Constant is 1.23 for men and 1.04 for women.

One interesting feature of the Cockcroft and Gault equation is that it shows how dependent the estimation of $C_{Cr}$ is based on age. The age term is (140 - age). This means that a 20-year-old person (140-20 = 120) will have twice the creatinine clearance as an 80-year-old (140-80 = 60) for the same level of serum creatinine. The CG equation assumes that a woman will have a 15% lower creatinine clearance than a man at the same level of serum creatinine.

Alternatively, eGFR values may be calculated using the Modification of Diet in Renal Disease (MDRD) formula. The 4-variable formula is as follows:

$$eGFR = 175\ \times Standardized\ serum\ creatinine^{-1.154} \times Age^{-0.203} \times C$$

where C is 1.212 if the patient is a black male, 0.899 if the patient is a black female, and 0.742 if the patient is a non-black female. Serum creatinine values are based on the IDMS-traceable creatinine determination (see below).

Chronic kidney disease is defined as a GFR less than 60 mL/min/1.73 $m^2$ that is present for three or more months.

**D. Measurement of Pressure in the Pulmonary Artery**

There are two main methods used to measure pulmonary artery (PA) pressures: trans-thoracic echocardiogram (TTE) and right heart catheterization.

An echocardiogram is an ultrasound of the heart; trans-thoracic means that the ultrasound probe rests on the outside of the chest (or "thorax"). Nothing is inserted into the body, so this test is called "non-invasive" and can be performed on an outpatient basis. Both the right ventricle and the beginning of the pulmonary arteries can be seen on the TTE. The TTE looks at the right ventricle as it pumps blood into the pulmonary arteries. Some of the blood from the right ventricle, instead of going forward into the pulmonary arteries, naturally leaks back into the right atrium via the tricuspid valve. When PA pressures are higher than they should be, it is harder for the right ventricle to pump blood forward and more of it therefore leaks back through the tricuspid valve. The TTE can measure the amount of leakage (or regurgitation) and use that to estimate the PA pressure. In some patients, PAH is seen only with exercise. In these cases, a TTE can be done to measure PA pressure after an exercise test (such as walking on a treadmill).

A right heart catheterization is a more invasive test that requires the placement of a pressure monitor directly into the pulmonary arteries. This technique allows for direct measurement of the systolic and diastolic PA pressures, and thus often results in more accurate measurements.

**E. Measurement of Serum Creatinine Levels**

A serum creatinine test measures the level of creatinine in the blood and provides an estimate glomerular filtration rate. Serum creatinine values in the BEACON and BEAM trials were based on the isotope dilution mass spectrometry (IDMS)-traceable creatinine determinations. Other commonly used creatinine assay methodologies include (1) alkaline picrate methods (*e.g.,* Jaffe method [classic] and compensated [modified] Jaffe methods), (2) enzymatic methods, (3) high-performance liquid chromatography, (4) gas chromatography, and (5) liquid chromatography. The IDMS method is widely considered to be the most accurate assay (Peake and Whiting, 2006).

**F. Measurement of Cystatin C Levels**

Cystatin C can be measured in a random sample of serum using immunoassays such as nephelometry or

particle-enhanced turbidimetry. Reference values differ in many populations and with sex and age. Across different studies, the mean reference interval (as defined by the 5th and 95th percentile) was between 0.52 and 0.98 mg/L. For women, the average reference interval is 0.52 to 0.90 mg/L with a mean of 0.71 mg/L. For men, the average reference interval is 0.56 to 0.98 mg/L with a mean of 0.77 mg/L. The normal values decrease until the first year of life, remaining relatively stable before they increase again, especially beyond age 50. Creatinine levels increase until puberty and differ according to gender from then on, making their interpretation problematic for pediatric patients.

In a large study from the United States National Health and Nutrition Examination Survey (Kottgen *et al.,* 2008), the reference interval (as defined by the 1st and 99th percentile) was between 0.57 and 1.12 mg/L. This interval was 0.55 - 1.18 for women and 0.60 - 1.11 for men. Non-Hispanic blacks and Mexican Americans had lower normal cystatin C levels. Other studies have found that in patients with an impaired renal function, women have lower and blacks have higher cystatin C levels for the same GFR. For example, the cutoff values of cystatin C for chronic kidney disease for a 60-year-old white women would be 1.12 mg/L and 1.27 mg/L in a black man (a 13% increase). For serum creatinine values adjusted with the MDRD equation, these values would be 0.95 mg/dL to 1.46 mg/dL (a 54% increase).

**G. Measurement of Uric Acid Levels**

Serum levels of uric acid are typically determined by clinical chemistry methods, *e.g.,* spectrophotometric measurement based on the reaction of uric acid with a specified reagent to form a colored reaction product. Because uric acid determination is a standard clinical chemistry test, a number of products are commercially available for this purpose.

In human blood plasma, the reference range of uric acid is typically 3.4-7.2 mg/dL (200-430 μmol/L) for men (1 mg/dL = 59.48 μmol/L), and 2.4-6.1 mg/dL for women (140-360 μmol/L). However, blood test results should always be interpreted using the range provided by the laboratory that performed the test. Uric acid concentrations in blood plasma above and below the normal range are known, respectively, as hyperuricemia and hypouricemia. Similarly, uric acid concentrations in urine above and below normal are known as hyperuricosuria and hypouricosuria.

**H. Measurement of Circulating Endothelial Cells (CEC)**

CECs were isolated from whole blood by using CD 146 Ab (an antibody to the CD 146 antigen that is expressed on endothelial cells and leukocytes). After CEC isolation, a FITC (fluorescein isothiocyanate) conjugated CD105 Ab (a specific antibody for endothelial cells) was used to identify CECs using the CellSearch™ system. A fluorescent conjugate of CD45 Ab was added to stain the leukocytes, and these were then gated out. For a general overview of this method, see Blann *et al.* (2005). CEC samples were also assessed for the presence of iNOS by immunostaining.

**VIII. Definitions**

When used in the context of a chemical group: "hydrogen" means -H; "hydroxy" means -OH; "oxo" means =O; "carbonyl" means -C(=O)-; "carboxy" means -C(=O)OH (also written as -COOH or $-CO_2H$); "halo" means independently -F, -Cl, -Br or -I; "amino" means $-NH_2$; "hydroxyamino" means -NHOH; "nitro" means $-NO_2$; imino means =NH; "cyano" means -CN; "isocyanate" means -N=C=O; "azido" means $-N_3$; in a monovalent context "phosphate" means $-OP(O)(OH)_2$ or a deprotonated form thereof; in a divalent context "phosphate" means -OP(O)(OH)O- or a deprotonated form thereof; "mercapto" means -SH; and "thio" means =S; "sulfonyl" means $-S(O)_2-$; and "sulfinyl" means -S(O)-.

In the context of chemical formulas, the symbol "-" means a single bond, "=" means a double bond, and "≡" means triple bond. The symbol "----" represents an optional bond, which if present is either single or double. The symbol "- - - -" represents a single bond or a double bond. Thus, for example, the formula

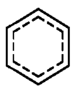

includes

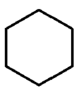, 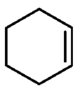, 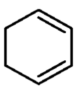, 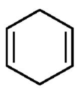

and

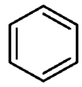.

And it is understood that no one such ring atom forms part of more than one double bond. Furthermore, it is noted that the covalent bond symbol "-", when connecting one or two stereogenic atoms, does not indicate any preferred stereochemistry. Instead, it cover all stereoisomers as well as mixtures thereof. The symbol "", when drawn perpendicularly across a bond (*e.g.,*

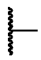

$CH_3$ for methyl) indicates a point of attachment of the group. It is noted that the point of attachment is typically only identified in this manner for larger groups in order to assist the reader in unambiguously identifying a point of attachment. The symbol "" means a single bond where the group attached to the thick end of the wedge is "out of the page." The symbol "" means a single bond where the group attached to the thick end of the wedge is "into the page". The symbol "" means a single bond where the geometry around a double bond (*e.g.,* either *E* or *Z*) is undefined. Both options, as well as combinations thereof are therefore intended. The bond orders described above are not limiting when one of the atoms connected by the bond is a metal atom (M). In such cases, it is understood that the actual bonding may comprise significant multiple bonding and/or ionic character. Therefore, unless indicated otherwise, the formulas M-C, M=C, M----C, and M----C, each refers to a bond of any and type and order between a metal atom and a carbon atom. Any undefined valency on an atom of a structure shown in this application implicitly represents a hydrogen atom bonded to that atom. A bold dot on a carbon atom indicates that the hydrogen attached to that carbon is oriented out of the plane of the paper.

When a group "R" is depicted as a "floating group" on a ring system, for example, in the formula:

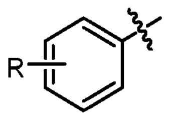

,

then R may replace any hydrogen atom attached to any of the ring atoms, including a depicted, implied, or expressly defined hydrogen, so long as a stable structure is formed. When a group "R" is depicted as a "floating group" on a fused ring system, as for example in the formula:

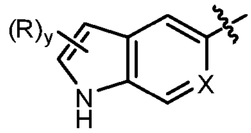

,

then R may replace any hydrogen attached to any of the ring atoms of either of the fused rings unless specified otherwise. Replaceable hydrogens include depicted hydrogens (*e.g.,* the hydrogen attached to the nitrogen in the formula above), implied hydrogens (*e.g.,* a hydrogen of the formula above that is not shown but understood to be present), expressly defined hydrogens, and optional hydrogens whose presence depends on the identity of a ring atom (*e.g.,* a hydrogen attached to group X, when X equals -CH-), so long as a stable structure is formed. In the example depicted, R may reside on either the 5-membered or the 6-membered ring of the fused ring system. In the formula above, the subscript letter "y" immediately following the group "R" enclosed in parentheses, represents a numeric variable. Unless specified otherwise, this variable can be 0, 1, 2, or any integer greater than 2, only limited by the maximum number of replaceable hydrogen atoms of the ring or ring system.

For the groups and classes below, the following parenthetical subscripts further define the group/class as follows: "(Cn)" defines the exact number (n) of carbon atoms in the group/class. "(C≤n)" defines the maximum number (n) of carbon atoms that can be in the group/class, with the minimum number as small as possible for the group in question, *e.g.,* it is understood that the minimum number of carbon atoms in the group "alkenyl$_{(C\leq8)}$" or the class "alkene$_{(C\leq8)}$" is two. For example, "alkoxy$_{(C\leq10)}$" designates those alkoxy groups having from 1 to 10 carbon atoms. (Cn-n') defines both the minimum (n) and maximum number (n') of carbon atoms in the group. Similarly, "alkyl$_{(C2-10)}$" designates those alkyl groups

having from 2 to 10 carbon atoms.

The term "saturated" as used herein means the compound or group so modified has no carbon-carbon double and no carbon-carbon triple bonds, except as noted below. In the case of substituted versions of saturated groups, one or more carbon oxygen double bond or a carbon nitrogen double bond may be present. And when such a bond is present, then carbon-carbon double bonds that may occur as part of keto-enol tautomerism or imine/enamine tautomerism are not precluded.

The term "aliphatic" when used without the "substituted" modifier signifies that the compound/group so modified is an acyclic or cyclic, but non-aromatic hydrocarbon compound or group. In aliphatic compounds/groups, the carbon atoms can be joined together in straight chains, branched chains, or non-aromatic rings (alicyclic). Aliphatic compounds/groups can be saturated, that is joined by single bonds (alkanes/alkyl), or unsaturated, with one or more double bonds (alkenes/alkenyl) or with one or more triple bonds (alkynes/alkynyl).

The term "alkyl" when used without the "substituted" modifier refers to a monovalent saturated aliphatic group with a carbon atom as the point of attachment, a linear or branched, cyclo, cyclic or acyclic structure, and no atoms other than carbon and hydrogen. Thus, as used herein cycloalkyl is a subset of alkyl, with the carbon atom that forms the point of attachment also being a member of one or more non-aromatic ring structures wherein the cycloalkyl group consists of no atoms other than carbon and hydrogen. As used herein, the term does not preclude the presence of one or more alkyl groups (carbon number limitation permitting) attached to the ring or ring system. The groups $-CH_3$ (Me), $-CH_2CH_3$ (Et), $-CH_2CH_2CH_3$ (n-Pr or propyl), $-CH(CH_3)_2$ (i-Pr, $^i$Pr or isopropyl), $-CH(CH_2)_2$ (cyclopropyl), $-CH_2CH_2CH_2CH_3$ (n-Bu), $-CH(CH_3)CH_2CH_3$ (*sec*-butyl), $-CH_2CH(CH_3)_2$ (isobutyl), $-C(CH_3)_3$ (*tert*-butyl, *t*-butyl, *t*-Bu or $^t$Bu), $-CH_2C(CH_3)_3$ (*neo*-pentyl), cyclobutyl, cyclopentyl, cyclohexyl, and cyclohexylmethyl are non-limiting examples of alkyl groups. The term "alkanediyl" when used without the "substituted" modifier refers to a divalent saturated aliphatic group, with one or two saturated carbon atom(s) as the point(s) of attachment, a linear or branched, cyclo, cyclic or acyclic structure, no carbon-carbon double or triple bonds, and no atoms other than carbon and hydrogen. The groups, $-CH_2-$(methylene), $-CH_2CH_2-$, $-CH_2C(CH_3)_2CH_2-$, $-CH_2CH_2CH_2-$, and

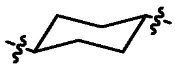,

are non-limiting examples of alkanediyl groups. The term "alkylidene" when used without the "substituted" modifier refers to the divalent group =CRR' in which R and R' are independently hydrogen, alkyl, or R and R' are taken together to represent an alkanediyl having at least two carbon atoms. Non-limiting examples of alkylidene groups include: $=CH_2$, $=CH(CH_2CH_3)$, and $=C(CH_3)_2$. An "alkane" refers to the compound H-R, wherein R is alkyl as this term is defined above. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$. The following groups are non-limiting examples of substituted alkyl groups: $-CH_2OH$, $-CH_2Cl$, $-CF_3$, $-CH_2CN$, $-CH_2C(O)OH$, $-CH_2C(O)OCH_3$, $-CH_2C(O)NH_2$, $-CH_2C(O)CH_3$, $-CH_2OCH_3$, -CHzOC(O)$CH_3$, $-CH_2NH_2$, $-CH_2N(CH_3)_2$, and $-CH_2CH_2Cl$. The term "haloalkyl" is a subset of substituted alkyl, in which one or more hydrogen atoms has been substituted with a halo group and no other atoms aside from carbon, hydrogen and halogen are present. The group, $-CH_2Cl$ is a non-limiting example of a haloalkyl. The term "fluoroalkyl" is a subset of substituted alkyl, in which one or more hydrogen has been substituted with a fluoro group and no other atoms aside from carbon, hydrogen and fluorine are present. The groups, $-CH_2F$, $-CF_3$, and $-CH_2CF_3$ are non-limiting examples of fluoroalkyl groups.

The term "alkenyl" when used without the "substituted" modifier refers to an monovalent unsaturated aliphatic group with a carbon atom as the point of attachment, a linear or branched, cyclo, cyclic or acyclic structure, at least one nonaromatic carbon-carbon double bond, no carbon-carbon triple bonds, and no atoms other than carbon and hydrogen. Non-limiting examples of alkenyl groups include: $-CH=CH_2$ (vinyl), $-CH=CHCH_3$, $-CH=CHCH_2CH_3$, $-CH_2CH=CH_2$ (allyl), $-CH_2CH=CHCH_3$, $-CH=CHCH=CH_2$, and $-CH=CH-C_6H_5$. The term "alkenediyl" when used without the "substituted" modifier refers to a divalent unsaturated aliphatic group, with two carbon atoms as points of attachment, a linear or branched, cyclo, cyclic or acyclic structure, at least one nonaromatic carbon-carbon double bond, no carbon-carbon triple bonds, and no atoms other than carbon and hydrogen. The groups, -CH=CH-, $-CH=C(CH_3)CH_2-$, $-CH=CHCH_2-$, and

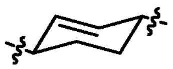,

are non-limiting examples of alkenediyl groups. It is noted that while the alkenediyl group is aliphatic, once connected at both ends, this group is not precluded from forming an aromatic structure. The terms "alkene" or "olefin" are synonymous and refer to a compound having the formula H-R, wherein R is alkenyl as this term is defined above. A "terminal alkene"

refers to an alkene having just one carbon-carbon double bond, wherein that bond forms a vinyl group at one end of the molecule. When any of these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$. The groups, -CH=CHF, -CH=CHCl and -CH=CHBr, are non-limiting examples of substituted alkenyl groups.

The term "alkynyl" when used without the "substituted" modifier refers to an monovalent unsaturated aliphatic group with a carbon atom as the point of attachment, a linear or branched, cyclo, cyclic or acyclic structure, at least one carbon-carbon triple bond, and no atoms other than carbon and hydrogen. As used herein, the term alkynyl does not preclude the presence of one or more non-aromatic carbon-carbon double bonds. The groups, -C≡CH, $-C{\equiv}CCH_3$, and $-CH_2C{\equiv}CCH_3$, are non-limiting examples of alkynyl groups. An "alkyne" refers to the compound H-R, wherein R is alkynyl. When any of these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$.

The term "aryl" when used without the "substituted" modifier refers to a monovalent unsaturated aromatic group with an aromatic carbon atom as the point of attachment, said carbon atom forming part of a one or more six-membered aromatic ring structure, wherein the ring atoms are all carbon, and wherein the group consists of no atoms other than carbon and hydrogen. If more than one ring is present, the rings may be fused or unfused. As used herein, the term does not preclude the presence of one or more alkyl or aralkyl groups (carbon number limitation permitting) attached to the first aromatic ring or any additional aromatic ring present. Non-limiting examples of aryl groups include phenyl (Ph), methylphenyl, (dimethyl)phenyl, $-C_6H_4CH_2CH_3$ (ethylphenyl), naphthyl, and a monovalent group derived from biphenyl. The term "arenediyl" when used without the "substituted" modifier refers to a divalent aromatic group with two aromatic carbon atoms as points of attachment, said carbon atoms forming part of one or more six-membered aromatic ring structure(s) wherein the ring atoms are all carbon, and wherein the monovalent group consists of no atoms other than carbon and hydrogen. As used herein, the term does not preclude the presence of one or more alkyl, aryl or aralkyl groups (carbon number limitation permitting) attached to the first aromatic ring or any additional aromatic ring present. If more than one ring is present, the rings may be fused or unfused. Unfused rings may be connected via one or more of the following: a covalent bond, alkanediyl, or alkenediyl groups (carbon number limitation permitting). Non-limiting examples of arenediyl groups include:

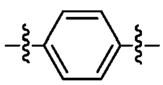, 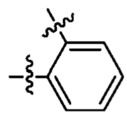, 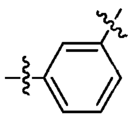, 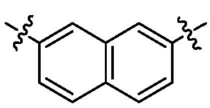 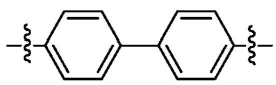,

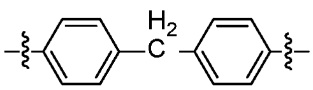, and 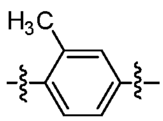.

An "arene" refers to the compound H-R, wherein R is aryl as that term is defined above. Benzene and toluene are non-limiting examples of arenes. When any of these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$.

The term "aralkyl" when used without the "substituted" modifier refers to the monovalent group -alkanediyl-aryl, in which the terms alkanediyl and aryl are each used in a manner consistent with the definitions provided above. Non-limiting examples of aralkyls are: phenylmethyl (benzyl, Bn) and 2-phenyl-ethyl. When the term aralkyl is used with the "substituted" modifier one or more hydrogen atom from the alkanediyl and/or the aryl group has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$. Non-limiting examples of substituted aralkyls are: (3-chlorophenyl)-methyl, and 2-chloro-2-phenyl-eth-1-yl.

The term "heteroaryl" when used without the "substituted" modifier refers to a monovalent aromatic group with an aromatic carbon atom or nitrogen atom as the point of attachment, said carbon atom or nitrogen atom forming part of one or more aromatic ring structures wherein at least one of the ring atoms is nitrogen, oxygen or sulfur, and wherein the heteroaryl group consists of no atoms other than carbon, hydrogen, aromatic nitrogen, aromatic oxygen and aromatic sulfur. If more than one ring is present, the rings may be fused or unfused. As used herein, the term does not preclude the presence of one or more alkyl, aryl, and/or aralkyl groups (carbon number limitation permitting) attached to the aromatic ring or aromatic ring system. Non-limiting examples of heteroaryl groups include furanyl, imidazolyl, indolyl, indazolyl (Im),

isoxazolyl, methylpyridinyl, oxazolyl, phenylpyridinyl, pyridinyl, pyrrolyl, pyrimidinyl, pyrazinyl, quinolyl, quinazolyl, quinoxalinyl, triazinyl, tetrazolyl, thiazolyl, thienyl, and triazolyl. The term "N-heteroaryl" refers to a heteroaryl group with a nitrogen atom as the point of attachment. The term "heteroarenediyl" when used without the "substituted" modifier refers to an divalent aromatic group, with two aromatic carbon atoms, two aromatic nitrogen atoms, or one aromatic carbon atom and one aromatic nitrogen atom as the two points of attachment, said atoms forming part of one or more aromatic ring structure(s) wherein at least one of the ring atoms is nitrogen, oxygen or sulfur, and wherein the divalent group consists of no atoms other than carbon, hydrogen, aromatic nitrogen, aromatic oxygen and aromatic sulfur. **If** more than one ring is present, the rings may be fused or unfused. Unfused rings may be connected via one or more of the following: a covalent bond, alkanediyl, or alkenediyl groups (carbon number limitation permitting). As used herein, the term does not preclude the presence of one or more alkyl, aryl, and/or aralkyl groups (carbon number limitation permitting) attached to the aromatic ring or aromatic ring system. Non-limiting examples of heteroarenediyl groups include:

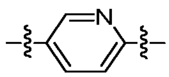 , 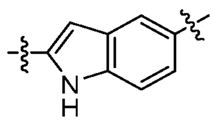 and 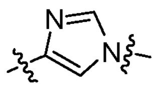 .

A "heteroarene" refers to the compound H-R, wherein R is heteroaryl. Pyridine and quinoline are non-limiting examples of heteroarenes. When these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$.

The term "heterocycloalkyl" when used without the "substituted" modifier refers to a monovalent non-aromatic group with a carbon atom or nitrogen atom as the point of attachment, said carbon atom or nitrogen atom forming part of one or more non-aromatic ring structures wherein at least one of the ring atoms is nitrogen, oxygen or sulfur, and wherein the heterocycloalkyl group consists of no atoms other than carbon, hydrogen, nitrogen, oxygen and sulfur. If more than one ring is present, the rings may be fused or unfused. As used herein, the term does not preclude the presence of one or more alkyl groups (carbon number limitation permitting) attached to the ring or ring system. Also, the term does not preclude the presence of one or more double bonds in the ring or ring system, provided that the resulting group remains non-aromatic. Non-limiting examples of heterocycloalkyl groups include aziridinyl, azetidinyl, pyrrolidinyl, piperidinyl, piperazinyl, morpholinyl, thiomorpholinyl, tetrahydrofuranyl, tetrahydrothiofuranyl, tetrahydropyranyl, pyranyl, oxiranyl, and oxetanyl. The term "N-heterocycloalkyl" refers to a heterocycloalkyl group with a nitrogen atom as the point of attachment. The term "heterocycloalkanediyl" when used without the "substituted" modifier refers to an divalent cyclic group, with two carbon atoms, two nitrogen atoms, or one carbon atom and one nitrogen atom as the two points of attachment, said atoms forming part of one or more ring structure(s) wherein at least one of the ring atoms is nitrogen, oxygen or sulfur, and wherein the divalent group consists of no atoms other than carbon, hydrogen, nitrogen, oxygen and sulfur. If more than one ring is present, the rings may be fused or unfused. Unfused rings may be connected via one or more of the following: a covalent bond, alkanediyl, or alkenediyl groups (carbon number limitation permitting). As used herein, the term does not preclude the presence of one or more alkyl groups (carbon number limitation permitting) attached to the ring or ring system. Also, the term does not preclude the presence of one or more double bonds in the ring or ring system, provided that the resulting group remains non-aromatic. Non-limiting examples of heterocycloalkanediyl groups include:

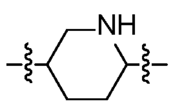 , 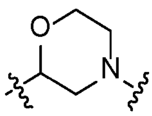 , and 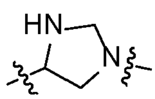 .

When these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, $-S(O)_2NH_2$, or $-C(O)OC(CH_3)_3$ (tert-butyloxycarbonyl, BOC).

The term "acyl" when used without the "substituted" modifier refers to the group -C(O)R, in which R is a hydrogen, alkyl, aryl, aralkyl or heteroaryl, as those terms are defined above. The groups, -CHO, $-C(O)CH_3$ (acetyl, Ac), $-C(O)CH_2CH_3$, $-C(O)CH_2CH_2CH_3$, $-C(O)CH(CH_3)_2$, $-C(O)CH(CH_2)_2$, $-C(O)C_6H_5$, $-C(O)C_6H_4CH_3$, $-C(O)CH_2C_6H_5$, -C(O)(imidazolyl) are non-limiting examples of acyl groups. A "thioacyl" is defined in an analogous manner, except that the oxygen atom of the group -C(O)R has been replaced with a sulfur atom, -C(S)R. The term "aldehyde" corresponds to an alkane, as defined above, wherein at least one of the hydrogen atoms has been replaced with a -CHO group. When any of these terms are used with the "substituted" modifier one or more hydrogen atom (including a hydrogen atom directly attached the carbonyl or thiocarbonyl group, if any) has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or

$-S(O)_2NH_2$. The groups, $-C(O)CH_2CF_3$, $-CO_2H$ (carboxyl), $-CO_2CH_3$ (methylcarboxyl), $-CO_2CH_2CH_3$, $-C(O)NH_2$ (carbamoyl), and $-CON(CH_3)_2$, are non-limiting examples of substituted acyl groups.

The term "alkoxy" when used without the "substituted" modifier refers to the group -OR, in which R is an alkyl, as that term is defined above. Non-limiting examples of alkoxy groups include: $-OCH_3$ (methoxy), $-OCH_2CH_3$ (ethoxy), $-OCH_2CH_2CH_3$, $-OCH(CH_3)_2$ (isopropoxy), $-O(CH_3)_3$ (*tert*-butoxy), $-OCH(CH_2)_2$, -O-cyclopentyl, and -O-cyclohexyl. The terms "alkenyloxy", "alkynyloxy", "aryloxy", "aralkoxy", "heteroaryloxy", "heterocycloalkoxy", and "acyloxy", when used without the "substituted" modifier, refers to groups, defined as -OR, in which R is alkenyl, alkynyl, aryl, aralkyl, heteroaryl, heterocycloalkyl, and acyl, respectively. The term "alkoxydiyl" refers to the divalent group -O-alkanediyl-, -O-alkanediyl-O-, or -alkanediyl-O-alkanediyl-. The term "alkylthio" and "acylthio" when used without the "substituted" modifier refers to the group -SR, in which R is an alkyl and acyl, respectively. The term "alcohol" corresponds to an alkane, as defined above, wherein at least one of the hydrogen atoms has been replaced with a hydroxy group. The term "ether" corresponds to an alkane, as defined above, wherein at least one of the hydrogen atoms has been replaced with an alkoxy group. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$.

The term "alkylamino" when used without the "substituted" modifier refers to the group -NHR, in which R is an alkyl, as that term is defined above. Non-limiting examples of alkylamino groups include: $-NHCH_3$ and $-NHCH_2CH_3$. The term "dialkylamino" when used without the "substituted" modifier refers to the group -NRR’, in which R and R’ can be the same or different alkyl groups, or R and R’ can be taken together to represent an alkanediyl. Non-limiting examples of dialkylamino groups include: $-N(CH_3)_2$, $-N(CH_3)(CH_2CH_3)$, and N-pyrrolidinyl. The terms "alkoxyamino", "alkenylamino", "alkynylamino", "arylamino", "aralkylamino", "heteroarylamino", "heterocycloalkylamino" and "alkylsulfonylamino" when used without the "substituted" modifier, refers to groups, defined as -NHR, in which R is alkoxy, alkenyl, alkynyl, aryl, aralkyl, heteroaryl, heterocycloalkyl, and alkylsulfonyl, respectively. A non-limiting example of an arylamino group is $-NHC_6H_5$. The term "amido" (acylamino), when used without the "substituted" modifier, refers to the group -NHR, in which R is acyl, as that term is defined above. A non-limiting example of an amido group is $-NHC(O)CH_3$. The term "alkylimino" when used without the "substituted" modifier refers to the divalent group =NR, in which R is an alkyl, as that term is defined above. The term "alkylaminodiyl" refers to the divalent group -NH-alkanediyl-, -NH-alkanediyl-NH-, or -alkanediyl-NH-alkanediyl-. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$. The groups $-NHC(O)OCH_3$ and $-NHC(O)NHCH_3$ are non-limiting examples of substituted amido groups.

The terms "alkylsulfonyl" and "alkylsulfinyl" when used without the "substituted" modifier refers to the groups $-S(O)_2R$ and -S(O)R, respectively, in which R is an alkyl, as that term is defined above. The terms "alkenylsulfonyl", "alkynylsulfonyl", "arylsulfonyl", "aralkylsulfonyl", "heteroarylsulfonyl", and "heterocycloalkylsulfonyl" are defined in an analogous manner. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$.

The term "alkylphosphate" when used without the "substituted" modifier refers to the group -OP(O)(OH)(OR), in which R is an alkyl, as that term is defined above. Non-limiting examples of alkylphosphate groups include: -OP(O)(OH)(OMe) and -OP(O)(OH)(OEt). The term "dialkylphosphate" when used without the "substituted" modifier refers to the group -OP(O)(OR)(OR’), in which R and R’ can be the same or different alkyl groups, or R and R’ can be taken together to represent an alkanediyl. Non-limiting examples of dialkylphosphate groups include: $-OP(O)(OMe)_2$, -OP(O)(OEt)(OMe) and $-OP(O)(OEt)_2$. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-OC(O)CH_3$, or $-S(O)_2NH_2$.

The use of the word "a" or "an," when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects.

As used herein, average molecular weight refers to the weight average molecular weight (Mw) as determined by static light scattering.

As used herein, a "chiral auxiliary" refers to a removable chiral group that is capable of influencing the stereoselectivity of a reaction. Persons of skill in the art are familiar with such compounds, and many are commercially available.

The terms "comprise," "have" and "include" are open-ended linking verbs. Any forms or tenses of one or more of these verbs, such as "comprises," "comprising," "has," "having," "includes" and "including," are also open-ended. For

example, any method that "comprises," "has" or "includes" one or more steps is not limited to possessing only those one or more steps and also covers other unlisted steps.

The term "effective," as that term is used in the specification and/or claims, means adequate to accomplish a desired, expected, or intended result. "Effective amount," "therapeutically effective amount" or "pharmaceutically effective amount" when used in the context of treating a patient or subject with a compound means that amount of the compound which, when administered to a subject or patient for treating a disease, is sufficient to effect such treatment for the disease. In the case of PAH in humans, a medical response to a therapeutically effective amount may include any one or more of the following: 1) An improvement in the six minute walk test by 5-10 meters, 10-20 meters, 20-30 meters, or greater compared to a baseline study prior to initiation of the therapy; 2) an improvement in World Health Organization functional Class from Class IV to Class III, Class IV to Class II, Class IV to Class I, Class III to Class II, Class III to Class I, or Class II to Class I with the former class being the WHO Class prior to initiation of the therapy; 3) a decrease in mean pulmonary artery pressure by 2-4 mm Hg, 4-6 mm Hg, 6-10 mm Hg or greater compared to a baseline study performed prior to initiation of the therapy; 4) an increase in the cardiac index by 0.05-0.1, 0.1-0.2, 0.2-0.4 liter/min/$m^2$ or greater compared to baseline study performed prior to initiation of the therapy; 5) an improvement in PVR (*i.e.,* a decrease) by 25-100, 100-200, 200-300 dyne sec/$cm^5$ or greater from baseline values obtained prior to initiation of the therapy; 6) a decrease in right atrial pressure by 0.1-0.2, 0.2-0.4, 0.4-1, 1-5 mm Hg or greater compared to a baseline study performed prior to initiation of the therapy; 7) a improvement in survival compared to a group of patients not given the therapy. The time between baseline study prior to initiation of therapy and time of evaluation of efficacy can vary but would typically fall in the range of 4-12 weeks, 12-24 weeks, or 24-52 weeks. Examples of therapeutic efficacy endpoints are given in references McLaughlin *et al.* (2002), Galie *et al.* (2005), Barst *et al.* (1996), McLaughlin *et al.* (1998), Rubin *et al.* (2002), Langleben *et al.* (2004), and Badesch *et al.* (2000).

The term "hydrate" when used as a modifier to a compound means that the compound has less than one (*e.g.,* hemihydrate), one (*e.g.,* monohydrate), or more than one (*e.g.,* dihydrate) water molecules associated with each compound molecule, such as in solid forms of the compound.

As used herein, the term "$IC_{50}$" refers to an inhibitory dose which is 50% of the maximum response obtained. This quantitative measure indicates how much of a particular drug or other substance (inhibitor) is needed to inhibit a given biological, biochemical or chemical process (or component of a process, i.e. an enzyme, cell, cell receptor or microorganism) by half.

An "isomer" of a first compound is a separate compound in which each molecule contains the same constituent atoms as the first compound, but where the configuration of those atoms in three dimensions differs.

As used herein, the term "patient" or "subject" refers to a living mammalian organism, such as a human, monkey, cow, sheep, goat, dog, cat, mouse, rat, guinea pig, or transgenic species thereof. In certain embodiments, the patient or subject is a primate. Non-limiting examples of human subjects are adults, juveniles, infants and fetuses.

As generally used herein "pharmaceutically acceptable" refers to those compounds, materials, compositions, and/or dosage forms which are, within the scope of sound medical judgment, suitable for use in contact with the tissues, organs, and/or bodily fluids of human beings and animals without excessive toxicity, irritation, allergic response, or other problems or complications commensurate with a reasonable benefit/risk ratio.

"Pharmaceutically acceptable salts" means salts of the compound of the present invention which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as 1,2-ethanedisulfonic acid, 2-hydroxyethanesulfonic acid, 2-naphthalenesulfonic acid, 3-phenylpropionic acid, 4,4'-methylenebis(3-hydroxy-2-ene-1-carboxylic acid), 4-methylbicyclo[2.2.2]oct-2-ene-1-carboxylic acid, acetic acid, aliphatic mono- and dicarboxylic acids, aliphatic sulfuric acids, aromatic sulfuric acids, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, laurylsulfuric acid, maleic acid, malic acid, malonic acid, mandelic acid, methanesulfonic acid, muconic acid, *o*-(4-hydroxybenzoyl)benzoic acid, oxalic acid, *p*-chlorobenzenesulfonic acid, phenyl-substituted alkanoic acids, propionic acid, *p*-toluenesulfonic acid, pyruvic acid, salicylic acid, stearic acid, succinic acid, tartaric acid, tertiarybutylacetic acid, trimethylacetic acid, and the like. Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, *N*-methylglucamine and the like. It should be recognized that the particular anion or cation forming a part of any salt of this invention is not critical, so long as the salt, as a whole, is pharmacologically acceptable. Additional examples of pharmaceutically acceptable salts and their methods of preparation and use are presented in Handbook of Pharmaceutical Salts: Properties, and Use (P. H. Stahl & C. G. Wermuth eds., Verlag Helvetica Chimica Acta, 2002).

The term "pharmaceutically acceptable carrier," as used herein means a pharmaceutically-acceptable material,

composition or vehicle, such as a liquid or solid filler, diluent, excipient, solvent or encapsulating material, involved in carrying or transporting a chemical agent.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

"Prodrug" means a compound that is convertible *in vivo* metabolically into an inhibitor according to the present invention. The prodrug itself may or may not also have activity with respect to a given target protein. For example, a compound comprising a hydroxy group may be administered as an ester that is converted by hydrolysis *in vivo* to the hydroxy compound. Suitable esters that may be converted *in vivo* into hydroxy compounds include acetates, citrates, lactates, phosphates, tartrates, malonates, oxalates, salicylates, propionates, succinates, fumarates, maleates, methylene-bis-β-hydroxynaphthoate, gentisates, isethionates, di-*p*-toluoyltartrates, methanesulfonates, ethanesulfonates, benzenesulfonates, *p*-toluenesulfonates, cyclohexylsulfamates, quinates, esters of amino acids, and the like. Similarly, a compound comprising an amine group may be administered as an amide that is converted by hydrolysis *in vivo* to the amine compound.

A "stereoisomer" or "optical isomer" is an isomer of a given compound in which the same atoms are bonded to the same other atoms, but where the configuration of those atoms in three dimensions differs. "Enantiomers" are stereoisomers of a given compound that are mirror images of each other, like left and right hands. "Diastereomers" are stereoisomers of a given compound that are not enantiomers. Chiral molecules contain a chiral center, also referred to as a stereocenter or stereogenic center, which is any point, though not necessarily an atom, in a molecule bearing groups such that an interchanging of any two groups leads to a stereoisomer. In organic compounds, the chiral center is typically a carbon, phosphorus or sulfur atom, though it is also possible for other atoms to be stereocenters in organic and inorganic compounds. A molecule can have multiple stereocenters, giving it many stereoisomers. In compounds whose stereoisomerism is due to tetrahedral stereogenic centers (*e.g.,* tetrahedral carbon), the total number of hypothetically possible stereoisomers will not exceed 2n, where n is the number of tetrahedral stereocenters. Molecules with symmetry frequently have fewer than the maximum possible number of stereoisomers. A 50:50 mixture of enantiomers is referred to as a racemic mixture. Alternatively, a mixture of enantiomers can be enantiomerically enriched so that one enantiomer is present in an amount greater than 50%. Typically, enantiomers and/or diastereomers can be resolved or separated using techniques known in the art. It is contemplated that that for any stereocenter or axis of chirality for which stereochemistry has not been defined, that stereocenter or axis of chirality can be present in its *R* form, *S* form, or as a mixture of the *R* and *S* forms, including racemic and non-racemic mixtures. As used herein, the phrase "substantially free from other stereoisomers" means that the composition contains ≤ 15%, more preferably ≤ 10%, even more preferably ≤ 5%, or most preferably ≤ 1% of another stereoisomer(s).

"Treatment" or "treating" includes (1) inhibiting a disease in a subject or patient experiencing or displaying the pathology or symptomatology of the disease (*e.g.,* arresting further development of the pathology and/or symptomatology), (2) ameliorating a disease in a subject or patient that is experiencing or displaying the pathology or symptomatology of the disease (*e.g.,* reversing the pathology and/or symptomatology), and/or (3) effecting any measurable decrease in a disease in a subject or patient that is experiencing or displaying the pathology or symptomatology of the disease.

The above definitions supersede any conflicting definition in any reference herein. The fact that certain terms are defined, however, should not be considered as indicative that any term that is undefined is indefinite. Rather, all terms used are believed to describe the invention in terms such that one of ordinary skill can appreciate the scope and practice the present invention.

## IX. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

### Example 1 - Bardoxolone methyl alleviates endothelial dysfunction

In clinical trials of bardoxolone methyl (BARD) in patients with stage 3b or 4 chronic kidney disease and type 2 diabetes, significant improvements in levels of circulating endothelial cells were noted (FIG. 2). Improvements were also noted in the numbers of CECs testing positive for inducible nitric oxide synthase (iNOS), an inflammation-promoting

enzyme. Non-clinical studies have shown that BARD and other AIMs can reduce ROS levels and increase NO bioavailability in endothelial cells (FIG. 3).

Non-clinical studies have also shown that BARD can reduce expression of endothelin-1 (ET-1) in mesangial cells (found in the kidney) and endothelial cells (Table 22). In this study BARD also reduced the expression of the vasoconstrictive $ET_A$ receptor while increasing the expression of the vasodilatory $ET_B$ receptor (Table 22). ET-1, a naturally occurring peptide, is the most potent endogenous vasoconstrictor and has been implicated in the pathogenesis of several cardiovascular diseases. ET-1 can also act as a mitogen and pro-inflammatory signaling molecule. Excessive activity of ET-1 is a source of endothelial dysfunction, in part due to the inhibition of NO signaling (Sud and Black, 2009) and in part due to pro-inflammatory effects (Pernow, 2012). Thus, inhibition of excessive ET-1 signaling is recognized as a potentially attractive therapeutic strategy for certain cardiovascular diseases. Endothelin receptor antagonists have been studied in a number of these diseases, and two are approved for the treatment of pulmonary arterial hypertension. However, as discussed above, suppression of ET-1 signaling may have adverse consequences in certain patient populations and patients in these groups should be excluded from treatment with agents that counteract ET-1 signaling.

**Table 22. Effect of Bardoxolone Methyl on Nrf2 Targets and Endothelin Gene Expression in Human Mesangial and Endothelial Cells**

| | Mesangial Cells | | Endothelial Cells | |
|---|---|---|---|---|
| | **BARD (50 nM)** | **BARD (250 nM)** | **BARD (50 nM)** | **BARD (250 nM)** |
| **NQO1** | +2.83 | +2.83 | NC | NC |
| **SRXN1** | +3.25 | +4.29 | +1.15 | +1.87 |
| **GCLC** | +2.64 | +4.92 | NC | +1.62 |
| **GCLM** | +2.14 | +4.59 | +2.14 | +3.03 |
| **ET-1** | -1.74 | -4.59 | -1.32 | -3.73 |
| **ET Receptor A** | NC | -1.62 | NC | NC |
| **ET Receptor B** | NC | NC | +2.00 | +4.92 |
| Human mesangial and endothelial cells were treated with bardoxolone methyl (50 or 250 nM) or vehicle. mRNA levels of Nrf2 target genes (NQO1, SRXN1, GCLC, and GCLM), as well as endothelin-1 and endothelin receptors A and B were quantified. Values denote fold-change vs. vehicle control; NC = no change. | | | | |

Treatment with AIMs *in vivo* also alters the expression levels of endothelin receptors. A bardoxolone methyl analog, RTA dh404, was tested in the 5/6 nephrectomy model of chronic renal failure in the rat, a widely-accepted model of hyperfiltration and pressure overload-induced renal injury and failure. Increased oxidative stress and inflammation caused by increased NF-κB and decreased Nrf2 activation in the 5/6 nephrectomy model results in approximately 30% increases in systolic and diastolic blood pressure (Kim, 2010). Moreover, the 5/6 nephrectomy model is associated with intra- and extra-renal hypertension and endothelial dysfunction.

In the 5/6 nephrectomy model, in addition to affording histological protection and suppressing expression of pro-inflammatory and pro-fibrotic mediators, the bardoxolone methyl analog RTA dh404 promoted a vasodilatory endothelin receptor phenotype, whereby the induction of the $ET_A$ receptor was completely suppressed, and reduced expression of the $ET_B$ receptor was partially reversed (FIG. 4). These data provide further evidence that bardoxolone methyl and analogs modulate the endothelin pathway to reverse endothelial dysfunction and promote vasodilation.

### Example 2 - Effect of RTA dh404 on Lung Histology in a Rat Model of Monocrotaline-Induced Pulmonary Arterial Hypertension

The effects of oral administration of the bardoxolone methyl analog RTA dh404 were evaluated in the rat model of monocrotaline (MCT)-induced pulmonary arterial hypertension (PAH). MCT is a macrolytic pyrrolizidine alkaloid and is activated to a toxic metabolite (*i.e.,* dehydromonocrotaline) in the liver by cytochrome P450 enzymes, which then induces a syndrome characterized by PAH, pulmonary mononuclear vasculitis, and right ventricular hypertrophy (Gomez-Arroyo *et al.,* 2012).

To evaluate RTA dh404, male Sprague-Dawley rats received a single injection of MCT on Day 1 and then either vehicle (sesame oil), RTA dh404 (2, 10, or 30 mg/kg/day), or the positive control sildenafil (60 mg/kg/day) for 21 days. Lung tissue was then analyzed by histopathology for arterial hypertrophy, cell infiltrates, and pulmonary edema. Vehicle lungs appeared artifactually more severe because of poor inflation during processing due, in part, to the loss of compliancy from

the injury and edema. RTA dh404 inhibited microscopic changes induced from MCT in the lung (*i.e.,* arteriolar hypertrophy, pulmonary edema, and cell and fibrin infiltrates) at all dose level with the 10 mg/kg RTA dh404 dose being as effective as the positive control sildenafil. Pulmonary edema was completely abrogated with the 10 mg/kg/day RTA dh404 treatment and with 60 mg/kg/day of sildenafil (FIG. 18).

Suppression of MCT-induced PAH by injection of RTA dh404 was also associated with the dose-dependent and significant induction of the mRNA expression of antioxidative Nrf2 target genes and the decrease in expression of pro-inflammatory NF-κB target genes (FIGS. 19 and 20).

Overall, these data suggest that RTA dh404 administration is associated with improvements in lung histopathology in the rat model of MCT-induced PAH, which is associated with the induction of antioxidative Nrf2 target genes and suppression of pro-inflammatory NF-κB target genes.

**Example 3 - A Dose-Ranging Study of the Efficacy and Safety of Bardoxolone Methyl in Patients with Pulmonary Arterial Hypertension**

This two-part phase 2 trial will study the safety, tolerability, and efficacy of bardoxolone methyl in patients with WHO Group 1 PAH. Part 1 will be a double-blind, randomized, dose-ranging, placebo-controlled treatment period and Part 2 will be an extension period. Eligible patients must have been receiving an oral, disease-specific PAH therapy consisting of an endothelin-receptor antagonist (ERA) and/or a phosphodiesterase type-5 inhibitor (PDE5i). Doses of prior therapy must have been stable for at least 90 days prior to Day 1.

Part 1: Part 1 of the study will include both dose-escalation and expansion cohorts. Dose-escalation cohorts will be enrolled one cohort at a time. Each cohort will include the next eight eligible patients randomized using a 3:1 assignment ratio to receive bardoxolone methyl or matching placebo to be administered once daily for 16 weeks. The starting dose of bardoxolone methyl will be 2.5 mg with subsequent doses of 5, 10, 20, and 30 mg.

For each dose-escalation cohort, after all eight patients complete the Week 4 visit, a Protocol Safety Review Committee (PSRC) will assess the safety and tolerability of bardoxolone methyl using all available data from this study to determine the appropriate dose for the next dose-escalation cohort. The PSRC will choose to randomize either the next higher dose, a lower dose, or another dose-escalation cohort at the current dose. Up to eight dose-escalation cohorts will be enrolled to initially evaluate safety and tolerability of bardoxolone methyl.

At each dose-escalation evaluation, the PSRC will also evaluate data for signs of pharmacodynamic activity and efficacy and may recommend adding expansion cohorts to further characterize safety and efficacy at up to two doses of bardoxolone methyl. Expansion cohorts will be enrolled one cohort at a time and each will include a minimum of 24 patients randomized using a 3:1 assignment ratio to receive bardoxolone methyl or matching placebo. Two expansion cohorts will be randomized at the doses selected by the PSRC. Expansion cohorts will only be enrolled at the subset of sites selected by the Sponsor for cardiopulmonary exercise testing (CPET) assessments, and CPET assessments will be required for all patients enrolling in the expansion cohorts. Additional near-infrared spectroscopy (NIRS) muscle tests as well as muscle biopsies are optional for dose expansion cohorts at qualified sites. The expansion cohorts may be enrolled in parallel with the dose-escalation cohorts, however, the randomizations of these cohorts will be carried out independently. The size of the expansion cohorts may be increased by up to 10 total patients across both cohorts to ensure at least 24 patients in each cohort have a fully evaluable CPET assessment at baseline for comparison with the Week 16 assessment. Thus, the two expansion cohorts combined will not exceed a total of 58 patients.

All patients in Part 1 of the study (i.e., both dose-escalation and expansion cohorts) will follow the same visit schedule. Following randomization, patients will be assessed in person during treatment at Weeks 1, 2, 4, 8, 12, and 16 and by telephone contact on Days 3, 10, and 21. Patients who do not enter Part 2 of the study (i.e. the extension period), either because they have discontinued taking study drug during Part 1 or have completed the 16-week treatment period as planned but chosen not to continue to Part 2 of the study, will complete an end-of-treatment visit as well as a follow-up visit four weeks after the date of administration of the last dose of study drug.

Part 2 (extension period): Patients who discontinue treatment prematurely in study Part 1 are not eligible to continue into study Part 2. All patients from Part 1 who complete the 16-week treatment period as planned will be eligible to continue directly into the extension period to evaluate the intermediate and long-term safety and efficacy of bardoxolone methyl. Day 1 of the extension period will be the same as the Week 16 visit for the treatment period, and patients continuing to the extension period will therefore continue taking study drug at the same dose. Patients randomized to placebo in Part 1 of the study will be assigned to receive bardoxolone methyl at their cohort-specific dose in the extension period. During the first four weeks of the extension period, all patients will be assessed using the same first four-week visit schedule as in Part 1 and will be seen every 12 weeks thereafter for the duration of the extension period as long as the patient continues to take study drug. Upon discontinuation of study drug administration during Part 2 of the study, patients will be assessed in person at a final follow-up visit occurring 4 weeks after the date of administration of the last dose of study drug. The extension period is planned to continue at least twelve weeks after the last patient enters the extension part of the study.

**A. Patient Population**

Up to 122 patients (64 in the dose-escalation cohorts and 58 in the expansion cohorts) will be enrolled in Part 1 of the study. All eligible patients from Part 1 will be included in Part 2, and no new patients will be randomized to Part 2 of the study.

The main criteria for inclusion will be:

1. Adult male and female patients ≥ 18 to ≤ 75 years of age upon study consent;
2. BMI > 18.5 kg/$m^2$;
3. Symptomatic pulmonary arterial hypertension WHO/NYHA FC class II and III;
4. One of the following subtypes of WHO Group 1 PAH:

    a. Idiopathic or heritable PAH;
    b. PAH associated with connective tissue disease;
    c. PAH associated with simple, congenital systemic-to-pulmonary shunts at least 1 year following shunt repair;
    d. PAH associated with anorexigens;
    e. PAH associated with human immunodeficiency virus (HIV);

5. Had a diagnostic right heart catheterization performed and documented within 36 months prior to Screening that confirmed a diagnosis of PAH according to all the following criteria:

    a. Mean pulmonary artery pressure ≥ 25 mm Hg (at rest);
    b. Pulmonary capillary wedge pressure (PCWP) ≤ 15 mm Hg;
    c. Pulmonary vascular resistance > 240 dyn.sec/$cm^5$ or > 3 mm Hg/Liter (L)/minute;

6. Has BNP level ≤ 200 pg/mL;
7. Has an average 6-minute walk distances (6MWDs) ≥ 150 and ≤ 450 meters on two consecutive tests performed on different days during Screenin, with both tests measuring within 15% of one another.
8. Has been receiving an oral, disease-specific PAH therapy consisting of an endothelin receptor antagonist (ERA) and/or a phosphodiesterase type-5 inhibitor (PDE5i). PAH therapy must be at a stable dose for at least 90 days prior to Day 1; (amended to include "at least one, but no more than two (2) disease-specific PAH therapies, including endothelin-receptor antagonists (ERAs), riociguat, phosphodiesterase type-5 inhibitors (PDE5i), or prostacyclins (subcutaneous, oral, or inhaled)."
9. Has maintained a stable dose for 30 days prior to Day 1 if receiving any of the following therapies that may affect PAH: vasodilators (including calcium channel blockers), digoxin, L-arginine supplementation, or oxygen supplementation;
10. If receiving prednisone, has maintained a stable dose of ≤ 20 mg/day (or equivalent dose if other corticosteroid) for at least 30 days prior to Day 1. If receiving treatment for connective tissue disease (CTD) with any other drugs, doses should remain stable for the duration of the study;
11. Had pulmonary function tests (PFTs) within 90 days prior to Day 1 with no evidence of significant parenchymal lung disease per the following criteria:

    a. Forced expiratory volume in 1 second (FEV1 ) ≥ 65% (predicted);
    b. FEV1/forced vital capacity ratio (FEV1/FVC) ≥ 65%; or
    c. Total lung capacity ≥ 65% (predicted), must be measured in patients with connective tissue disease;

12. Had a ventilation-perfusion (V/Q) lung scan, spiral/helical/electron beam computed tomography (CT) or pulmonary angiogram prior to Screening that shows no evidence of thromboembolic disease (i.e. should note normal or low probability for pulmonary embolism). If V/Q scan was abnormal (i.e. results other than normal or low probability), then a confirmatory CT or selective pulmonary angiography must exclude chronic thromboembolic disease;
13. Has adequate kidney function defined as an estimated glomerular filtration rate (eGFR) ≥ 60 mL/min/1.73 $m^2$ using the Modification of Diet in Renal Disease (MDRD) 4-variable formula (note: this was subsequently lowered to ≥ 45 mL/min/1.73 $m^2$);
14. Willing and able to comply with scheduled visits, treatment plan, laboratory tests, and other study procedures;
15. Evidence of a personally signed and dated informed consent document indicating that the patient (or a legally acceptable representative) has been informed of all pertinent aspects of the study prior to initiation of any patient-mandated procedures.

The main criteria for exclusion will be:

1. Participation in other investigational clinical studies involving pharmaceutical products being tested or used in a way different from the approved form or when used for an unapproved indication within 30 days prior to Day 1;
2. Participation in an intensive exercise training program for pulmonary rehabilitation within 90 days prior to Screening;
3. Receiving chronic treatment with a prostacyclin/prostacyclin analogue within 60 days prior to Day 1. Use of prostacyclin for acute vasodilator testing during right heart catheterization is allowed;
4. Requirement for receipt of intravenous inotropes within 30 days prior to Day 1;
5. Has uncontrolled systemic hypertension as evidenced by sitting systolic blood pressure (BP) > 160 mm Hg or sitting diastolic blood pressure > 100 mm Hg during Screening after a period of rest;
6. Has systolic BP < 90 mm Hg during Screening after a period of rest;
7. Has a history of clinically significant left-sided heart disease and/or clinically significant cardiac disease, including but not limited to any of the following:

    a. Congenital or acquired valvular disease if clinically significant apart from tricuspid valvular insufficiency due to pulmonary hypertension;
    b. Pericardial constriction;
    c. Restrictive or congestive cardiomyopathy;
    d. Left ventricular ejection fraction < 40% at the Screen A echocardiogram (ECHO);
    e. Any current or prior history of symptomatic coronary disease (prior myocardial infarction, percutaneous coronary intervention, coronary artery bypass graft surgery, or anginal chest pain);

8. Acutely decompensated heart failure within 30 days prior to Day 1, as per Investigator assessment;
9. Has more than two of the following clinical risk factors for left ventricular diastolic dysfunction:

    a. Age > 65 years;
    b. BMI $\geq$ 30 kg/m$^2$;
    c. History of systemic hypertension;
    d. History of type 2 diabetes;
    e. History of atrial fibrillation;

10. History of atrial septostomy within 180 days prior to Day 1;
11. History of obstructive sleep apnea that is untreated;
12. For patients with HIV-associated PAH, any of the following:

    a. Concomitant active opportunistic infections within 180 days prior to Screening;
    b. Detectable viral load within 90 days prior to Screening;
    c. Cluster designation (CD+) T-cell count < 200 mm$^3$ within 90 days prior to Screening;
    d. Changes in antiretroviral regimen within 90 days prior to Screening;
    e. Using inhaled pentamidine;

13. Has a history of portal hypertension or chronic liver disease, including hepatitis B and/or hepatitis C (with evidence of recent infection and/or active virus replication) defined as mild to severe hepatic impairment (Child-Pugh Class A-C);
14. Serum aminotransferase (ALT or AST) levels > the upper limit of normal (ULN) at Screening;
15. Hemoglobin (Hgb) concentration < 8.5 g/dL at Screening;
16. Diagnosis of Down syndrome;
17. History of malignancy within 5 years prior to screening, with the exception of localized skin or cervical carcinomas;
18. Active bacterial, fungal, or viral infection, incompatible with the study;
19. Known or suspected active drug or alcohol abuse;
20. Major surgery within 30 days prior to Screening or planned to occur during the course of the study;
21. Unwilling to practice methods of birth control (both males who have partners of childbearing potential and females of childbearing potential) during screening, while taking study drug and for at least 30 days after the last dose of study drug is ingested;
22. Women who are pregnant or breastfeeding;
23. Any disability or impairment that would prohibit performance of the 6MWT;
24. Any abnormal laboratory level that, in the opinion of the investigator, would put the patient at risk by trial enrollment;
25. Patient is, in the opinion of the investigator, unable to comply with the requirements of the study protocol or is

unsuitable for the study for any reason;
26. Known hypersensitivity to any component of the study drug;
27. Unable to communicate or cooperate with the investigator due to language problems, poor mental development, or impaired cerebral function.

**B. Procedures**

During Part 1 of the study, bardoxolone methyl (2.5, 5, 10, 20, or 30 mg) or placebo will be administered orally once daily in the morning for 16 weeks. During Part 2 of the study, bardoxolone methyl (2.5, 5, 10, 20, or 30 mg) will be administered orally once daily in the morning for the duration of the extension period.
A sample size of 8 patients randomized at a 3:1 (bardoxolone methyl:placebo) assignment ratio in each dose-escalation cohort includes 6 patients treated with bardoxolone methyl for identification of gross safety signals. A small number of patients at each dose is not expected to fully characterize safety, therefore issues of concern identified in only 1 of 6 patients (16%) treated with bardoxolone methyl may suggest the need to collect additional information before escalating the dose, by either adding another cohort at the current dose level or at a lower dose as determined by the PSRC. At each of the two doses selected for expansion, combined cohort sizes of 32 patients (dose-escalation cohort N=8; expansion cohort N=24) at the 3:1 assignment ratio provides at a minimum 24 patients treated with bardoxolone methyl to characterize safety, tolerability, and efficacy.

**C. Outcomes**

The objectives of the present study will be to determine the recommended dose range for further study of bardoxolone methyl, to assess the change from baseline in 6-minute walk distance (6MWD) in those patients treated with bardoxolone methyl versus patients given placebo for 16 weeks, and to assess the safety and tolerability of 16 weeks of treatment with bardoxolone methyl versus 16 weeks of administration of placebo
The follow criteria will be evaluated:

Efficacy: Changes from baseline in 6-minute walk distance (6MWD) at Week 16; N-terminal pro-B-type natriuretic peptide (NT-Pro BNP); Borg dyspnea index; WHO/NYHA pulmonary arterial hypertension (PAH) functional class (FC); parameters collected during Doppler echocardiography (ECHO), cardiopulmonary exercise testing (CPET), optional cardiac magnetic resonance imaging (MRI), optional near-infrared spectroscopy (NIRS) muscle tests, and optional muscle biopsy; and clinical worsening.
Safety: Frequency, intensity, and relationship to study drug of adverse events and serious adverse events, concomitant medications, and change from baseline in the following assessments: physical examinations, vital sign measurements, 24-hour ambulatory blood pressure monitoring (ABPM), 12-lead electrocardiograms (ECGs), clinical laboratory measurements, and weight.
Pharmacokinetics: Bardoxolone methyl plasma concentration-time data, metabolite concentration-time data, and estimated pharmacokinetic parameters for each analyte.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the scope of the invention as defined by the appended claims.

**X. References**

The following references, to the extent that they provide exemplary procedural or other details supplementary to those set forth herein, are identified.

U.S. Pat. No. 5,480,792
U.S. Pat. No. 5,525,524
U.S. Pat. No. 5,631,170
U.S. Pat. No. 5,679,526
U.S. Pat. No. 5,824,799

U.S. Pat. No. 5,851,776
U.S. Pat. No. 5,885,527
U.S. Pat. No. 5,922,615
U.S. Pat. No. 5,939,272
U.S. Pat. No. 5,947,124
U.S. Pat. No. 5,955,377
U.S. Pat. No. 5,985,579
U.S. Pat. No. 6,019,944
U.S. Pat. No. 6,025,395
U.S. Pat. No. 6,113,855
U.S. Pat. No. 6,143,576
U.S. Pat. Appl. Ser. No. 12/352,473
U.S. Pat. Pub. 2003/0232786
U.S. Pat. Pub. 2008/0261985
U.S. Pat. Pub. 2009/0048204
U.S. Pat. Pub. 2009/0326063
U.S. Pat. Pub. 2010/0041904
U.S. Pat. Pub. 2010/0048887
U.S. Pat. Pub. 2010/0048892
U.S. Pat. Pub. 2010/0048911
U.S. Pat. Pub. 2010/0056777
U.S. Pat. Pub. 2011/0201130
PCT Pub. WO 2009/023232
PCT Pub. WO 2010/093944
Badesch et al., Continuous intravenous epoprostenol for pulmonary hypertension due to the scleroderma spectrum of disease. A randomized, controlled trial. Ann. Intern. Med., 132:425-434, 2000.
Badesch et al., Medical therapy for pulmonary arterial hypertension: ACCP evidence-based clinical practice guidelines. Chest, 126:35S-62S, 2004.
Barst et al., A comparison of continuous intravenous epoprostenol (prostacyclin) with conventional therapy for primary pulmonary hypertension. N. Engl. J. Med., 334:296-301, 1996.
Blann et al., Thromb. Haemost., 93:228-235, 2005.
Bolignano et al., Pulmonary Hypertension in CKD, Am. J. Kidney Dis., 61:612-622, 2013.
Joint Specialty Committee on Renal Medicine of the Royal College of Physicians and the Renal Association, and the Royal College of General Practitioners. Chronic kidney disease in adults: UK guidelines for identification, management and referral. London: Royal College of Physicians, 2006.
Deng et al., Familial primary pulmonary hypertension (gene PPH1) is caused by mutations in the bone morphogenetic protein receptor-II gene. Am. J. Hum. Genet., 67:737-44, 2000.
Dhaun et al., Urinary endothelin-1 in chronic kidney disease and as a marker of disease activity in lupus nephritis. American Journal of Physiology - Renal Physiology, 296:F1477-F1483, 2009.
Dumitrascu et al., Oxid. Med. Cell. Longev., 2013:234631, 2013.
Forstermann, Biol. Chem., 387:1521-1533, 2006.
Galie et al., Ambrisentan therapy for pulmonary arterial hypertension. J. Am. Coll. Cardiol., 46:529-535, 2005.
Gologanu et al., Rom J Intern Med., 50:259-68, 2012.
Gomez-Arroyo et al., The monocrotaline model of pulmonary hypertension in perspective. Am. J. Physiol. Lung Cell. Mol. Physiol., 302:L363-L369, 2012.
Guazzi and Galie, Eur. Respir. Rev., 21:338-346, 2012.
Hansson et al., Annu. Rev. Pathol. Mech. Dis., 1:297-329, 2006.
Handbook of Pharmaceutical Salts: Properties, and Use, Stahl and Wermuth Eds.), Verlag Helvetica Chimica Acta, 2002.
Hayes and Dinkova-Kostova, The Nrf2 regulatory network provides an interface between redox and intermediary metabolism. Trends in Biochem Sci., 39:199-218, 2014.
Honda et al., J. Med. Chem., 43:1866-1877, 2000a.
Honda et al., J. Med. Chem., 43:4233-4246, 2000b.
Honda et al. Bioorg. Med. Chem. Lett., 12:1027-1030, 2002.
Hosokawa, Cardiovascular Res., 99:35-43, 2013.
Humbert, New Engl. J. Med., 351:1425-1436, 2004.
Jones et al., Activation of thromboxane and prostacyclin receptors elicits opposing effects on vascular smooth muscle cell growth and mitogen-activated protein kinase signaling cascades. Mol. Pharm., 48:890-89, 1995.

Kataoka et al., Oral sildenafil improves primary pulmonary hypertension refractory to epoprostenol. Circ. J., 69:461-465, 2005.

Kim and Vaziri, Contribution of impaired Nrf2-Keap1 pathway to oxidative stress and inflammation in chronic renal failure. American Journal of Physiology - Renal Physiology, 298:F662-F671, 2010.

Kosmadakis et al., Ren. Fail., 35:514-20, 2013.

Kottgen et al., Serum cystatin C in the United States: the Third National Health and Nutrition Examination Survey, Am. J. Kidney Dis., 51:385-394, 2008.

Langleben et al., STRIDE 1: Effects of the Selective ETA Receptor Antagonist, Sitaxsentan Sodium, in a Patient Population with Pulmonary Arterial Hypertension that meets Traditional Inclusion Criteria of Previous Pulmonary Arterial Hypertension Trials. J. Cardiovasc. Pharmacol., 44:S80-S84.9, 2004.

Lee et al., Sildenafil for pulmonary hypertension. Ann. Pharmacother., 39:869-884, 2005. Mann et al., Avosentan for overt diabetic nephropathy. Journal of the American Society of Nephrology, 21,:527-535, 2010.

March's Advanced Organic Chemistry: Reactions, Mechanisms, and Structure, 2007.

McGoon et al., Screening, early detection, and diagnosis of pulmonary arterial hypertension: ACCP evidence-based clinical practice guidelines. Chest, 126:14S-34S, 2004.

McLaughlin et al., Reduction in pulmonary vascular resistance with long-term epoprostenol (prostacyclin) therapy in primary pulmonary hypertension. N. Engl. J. Med., 338:273-277, 1998.

McLaughlin et al., Survival in primary pulmonary hypertension: the impact of epoprostenol therapy. Circulation, 106:1477-1482, 2002.

McLaughlin and Rich, Pulmonary hypertension. Curr. Probl. Cardiol., 29:575-634, 2004.

Oudiz et al., Treprostinil, a prostacyclin analogue, in pulmonary arterial hypertension associated with connective tissue disease. Chest, 126:420-427, 2004.

Packer, Vasodilator therapy for primary pulmonary hypertension. Limitations and hazards. Ann. Intern. Med., 103:258-270, 1985.

Paulin et al., STAT3 signaling in pulmonary arterial hypertension, JAK-STAT, 1:223-233, 2012.

Peake and Whiting, Clin. Biochem. Rev., 27:173-184, 2006.

Pergola et al., Bardoxolone methyl and kidney function in CKD with type 2 diabetes., New Engl. J. Med., 365, 327-336, 2011.

Rawlins et al., Am. J. Clin. Pathol., 123:439-445, 2005.

Rich et al., The effects of high doses of calcium-channel blockers on survival in primary pulmonary hypertension. N. Engl. J. Med., 327:76-81, 1992.

Rubin et al., Bosentan therapy for pulmonary arterial hypertension. N. Engl. J. Med., 346:896-903, 2002.

Schneider et al., Contrasting actions of endothelin ETA and ETB receptors in cardiovascular disease. Annual Review of Pharmacology and Toxicology, 47:731-759, 2007.

Sussan et al., Targeting Nrf2 with the triterpenoid CDDO-imidazolide attenuate cigarette smoke-induced emphysema and cardiac dysfunction in mice. Proc. Natl. Acad. Sci. U.S.A., 106:250-255, 2009.

Sutendra and Michelakis, The metabolic basis of pulmonary arterial hypertension. Cell Metabolism, 19:558-573, 2014.

Tam et al., A derivative of Bardoxolone methyl, dh404, in an inverse dose-dependent manner, lessens diabetes-associated atherosclerosis and improves diabetic kidney disease. Diabetes, doi:10.2337/db13-1743, 2014.

The International PPH Consortium, Lane et al., Heterozygous germline mutations in BMPR2, encoding a TGF-b receptor, cause familial primary pulmonary hypertension. Nature Genetics, 26:81-84, 2000.

Thenappan et al., A USA-based registry for pulmonary arterial hypertension: 1982-2006. European Respiratory Journal, 30:1103-1110, 2007.

Vachiéry and Davenport, The endothelin system in pulmonary and renal vasculopathy: les liaisons dangereuses. European Respiratory Review, 18:260-271, 2009.

Vachiery and Naeije, Treprostinil for pulmonary hypertension. Expert Rev. Cardiovasc. Ther., 2:183-191, 2004.

Vasan et al., Congestive heart failure in subjects with normal versus reduced left ventricular ejection fraction: Prevalence and mortality in a population-based cohort. Journal of the American College of Cardiology, 33: 1948-1955, 1999.

## Claims

**1.** A compound of the formula

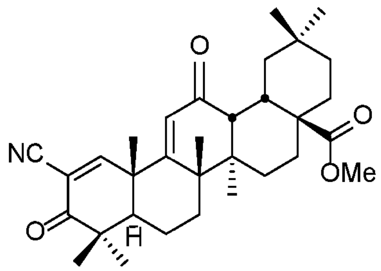

for use in treating or preventing a cardiovascular disease in a patient in need thereof, wherein the patient has been identified as not having at least one of the following characteristics:

(a) a history of left-sided myocardial disease;
(b) an elevated B-type natriuretic peptide (BNP) level;
(c) an elevated albumin/creatinine ratio (ACR); and
(d) chronic kidney disease (CKD).

**2.** The compound for use according to claim 1, wherein the patient does not have chronic kidney disease (CKD).

**3.** The compound for use according to either claim 1 or 2, wherein the cardiovascular disease is atherosclerosis, restenosis, pulmonary hypertension, pulmonary arterial hypertension, or thrombosis.

**4.** The compound for use according to any one of claims 1 to 3, wherein:

(i) the patient has been identified as not having any of said four characteristics;
(ii) the pressure in the pulmonary artery of the patient has been measured or will be measured;
(iii) the patient does not have a history of left-sided myocardial disease;
(iv) the patient's BNP level is greater than 200 pg/mL;
(v) the patient has an ACR greater than 300 mg/g;
(vi) the patient's estimated glomerular filtration rate (eGFR) is greater than or equal to 45 mL/min/1.73 $m^2$;
(vii) the patient has been identified as not having COPD;
(viii) the patient is not a smoker;
(ix) the patient has been identified as not having cancer; and/or
(x) the patient has been identified as not having type 2 diabetes.

**5.** The compound for use according to claim 1, wherein the patient has chronic kidney disease (CKD).

**6.** The compound for use according to any one of claims 1-5, wherein the patient is a human patient.

**7.** The compound for use according to any one of claims 1-6, wherein the compound is formulated as a solid dispersion comprising (i) the compound and (ii) an excipient; optionally wherein the excipient is a methacrylic acid - ethyl acrylate copolymer.

**8.** The compound for use according to any of claims 1-7, wherein the compound is administered to the patient either concomitantly or sequentially with a second therapy comprising a pharmaceutically effective amount of a second drug.

**9.** The compound for use according to claim 8, wherein the second drug is a Rho kinase inhibitor, a tyrosine kinase inhibitor, an epoprostenol derivative, a serotonin receptor antagonist, an endothelin receptor antagonist, a phosphodiesterase (PDE) inhibitor, a calcium channel blocker, a soluble guanylate cyclase stimulator, or nitric oxide; optionally wherein the Rho kinase inhibitor is HA-1077, HA-1152P, or Y-27632; optionally wherein the tyrosine kinase inhibitor is Gleevec; optionally wherein the epoprostenol derivative is treprostinil, beraprost, or iloprost; optionally wherein the serotonin receptor antagonist is sarpogrelate; optionally wherein the endothelin receptor antagonist is bosentan, sitaxsentan, ambrisentan, TBC3711, or macitentan; optionally wherein the PDE inhibitor is amrinone, sildenafil, tadalafil, vardenafil, enoximone, udenafil, or milrinone; optionally wherein the calcium channel blocker is amlodipine, diltiazem, isradipine, nicardipine, nifedipine, nimodipine, nisoldipine, nitrendipine, and verapamil; or optionally wherein the soluble guanylate cyclase stimulator is riociguat.

**10.** The compound for use according to any one of claims 1 to 9, wherein the cardiovascular disease is pulmonary hypertension.

**11.** The compound for use according to claim 10, wherein the pulmonary hypertension is pulmonary arterial hypertension.

**12.** The compound for use according to claim 11, wherein the pulmonary arterial hypertension is associated with connective tissue disease, or wherein the pulmonary arterial hypertension is idiopathic.

**13.** The compound for use according to any one of claims 1 to 12, wherein:

(i) at least a portion of the compound is present as a crystalline form having an X-ray diffraction pattern (CuK$\alpha$) comprising significant diffraction peaks at about 8.8, 12.9, 13.4, 14.2 and 17.4 °2$\theta$; or
(ii) at least a portion of the compound is present as an amorphous form having an X-ray diffraction pattern (CuK$\alpha$) with a halo peak at approximately 13.5 °2$\theta$, substantially as shown in FIG. 1C, and a transition glass temperature ($T_g$) in the range of about 120°C to about 135 °C.

## Patentansprüche

**1.** Verbindung der Formel

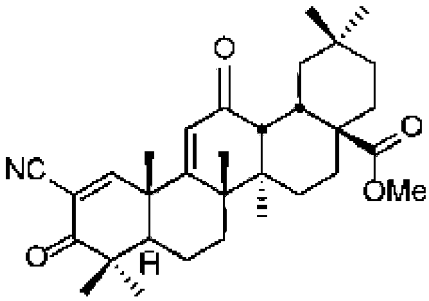

zur Verwendung bei der Behandlung oder Vorbeugung einer kardiovaskulären Krankheit bei einem Patienten, der diese benötigt, wobei der Patient mindestens eine der folgenden Eigenschaften nicht aufweist:

(a) eine linksseitige myokardiale Krankheit in der Vorgeschichte;
(b) einen erhöhten Spiegel an natriuretischem Peptid vom B-Typ (BNP);
(c) ein erhöhtes Albumin-/Kreatinin-Verhältnis (ACR); und
(d) chronische Nierenkrankheit (CKD).

**2.** Verbindung zur Verwendung nach Anspruch 1, wobei der Patient keine chronische Nierenkrankheit (CKD) aufweist.

**3.** Verbindung zur Verwendung nach einem der Ansprüche 1 oder 2, wobei die kardiovaskuläre Krankheit Atherosklerose, Restenose, pulmonale Hypertonie, pulmonale arterielle Hypertonie oder Thrombose ist.

**4.** Verbindung zur Verwendung nach einem der Ansprüche 1 bis 3, wobei:

(i) bei dem Patienten festgestellt wurde, dass er keine der vier Eigenschaften aufweist;
(ii) der Druck in der Lungenarterie des Patienten gemessen wurde oder gemessen werden wird;
(iii) der Patient keine linksseitige myokardiale Krankheit in der Vorgeschichte aufweist;
(iv) der BNP-Spiegel des Patienten größer als 200 pg/mL ist;
(v) der Patient eine ACR von mehr als 300 mg/g aufweist;
(vi) die geschätzte glomeruläre Filtrationsrate (eGFR) des Patienten größer oder gleich 45 mL/min/1,73 $m^2$ ist;
(vii) bei dem Patienten festgestellt worden ist, dass er keine COPD aufweist;
(viii) der Patient Nichtraucher ist;
(ix) bei dem Patienten festgestellt wurde, dass er keinen Krebs aufweist; und/oder
(x) bei dem Patienten festgestellt wurde, dass er keinen Typ-2-Diabetes aufweist.

5. Verbindung zur Verwendung nach Anspruch 1, wobei der Patient eine chronische Nierenkrankheit (CKD) aufweist.

6. Verbindung zur Verwendung nach einem der Ansprüche 1 bis 5, wobei der Patient ein menschlicher Patient ist.

7. Verbindung zur Verwendung nach einem der Ansprüche 1 bis 6, wobei die Verbindung als eine feste Dispersion formuliert ist, die (i) die Verbindung und (ii) einen Hilfsstoff umfasst, wobei der Hilfsstoff optional ein Methacrylsäure-Ethylacrylat-Copolymer ist.

8. Verbindung zur Verwendung nach einem der Ansprüche 1 bis 7, wobei die Verbindung dem Patienten entweder gleichzeitig oder aufeinanderfolgend mit einer zweiten Therapie verabreicht wird, die eine pharmazeutisch wirksame Menge eines zweiten Medikaments umfasst.

9. Verbindung zur Verwendung nach Anspruch 8, wobei das zweite Medikament ein Rho-Kinase-Inhibitor, ein Tyrosinkinase-Inhibitor, ein Epoprostenol-Derivat, ein Serotonin-Rezeptor-Antagonist, ein Endothelin-Rezeptor-Antagonist, ein Phosphodiesterase (PDE)-Inhibitor, ein Kalziumkanalblocker, ein löslicher Guanylatcyclase-Stimulator oder Stickstoffmonoxid ist;
optional, wobei der Rho-Kinase-Inhibitor HA-1077, HA-1152P oder Y-27632 ist; optional, wobei der Tyrosinkinase-Inhibitor Glivec ist; optional, wobei das Epoprostenol-Derivat Treprostinil, Beraprost oder Iloprost ist; optional, wobei der Serotonin-Rezeptor-Antagonist Sarpogrelat ist; optional, wobei der Endothelin-Rezeptor-Antagonist Bosentan, Sitaxsentan, Ambrisentan, TBC3711 oder Macitentan ist; optional, wobei der PDE-Inhibitor Amrinon, Sildenafil, Tadalafil, Vardenafil, Enoximon, Udenafil oder Milrinon ist; optional, wobei der Kalziumkanalblocker Amlodipin, Diltiazem, Isradipin, Nicardipin, Nifedipin, Nimodipin, Nisoldipin, Nitrendipin und Verapamil ist; oder optional, wobei der lösliche Guanylatzyklase-Stimulator Riociguat ist.

10. Verbindung zur Verwendung nach einem der Ansprüche 1 bis 9, wobei die kardiovaskuläre Krankheit pulmonale Hypertonie ist.

11. Verbindung zur Verwendung nach Anspruch 10, wobei die pulmonale Hypertonie eine pulmonale arterielle Hypertonie ist.

12. Verbindung zur Verwendung nach Anspruch 11, wobei die pulmonale arterielle Hypertonie mit einer Bindegewebskrankheit assoziiert ist, oder wobei die pulmonale arterielle Hypertonie idiopathisch ist.

13. Verbindung zur Verwendung nach einem der Ansprüche 1 bis 12, wobei:

(i) mindestens ein Abschnitt der Verbindung in kristalliner Form vorliegt und ein Röntgenbeugungsmuster (CuK$\alpha$) aufweist, umfassend signifikante Beugungsspitzen bei etwa 8,8, 12,9, 13,4, 14,2 und 17,4 °2$\theta$; oder
(ii) mindestens ein Abschnitt der Verbindung als amorphe Form vorliegt, die ein Röntgenbeugungsmuster (CuK$\alpha$) mit einer Beugungsspitze bei etwa 13,5 °2$\theta$, im wesentlichen wie in FIG. 1C gezeigt, und eine Glasübergangstemperatur (Tg) im Bereich von etwa 120 °C bis etwa 135 °C aufweist.

## Revendications

1. Composé de formule

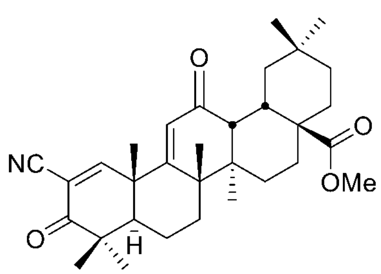

destiné à être utilisé dans le traitement ou la prévention d'une maladie cardiovasculaire chez un patient qui en a besoin, dans lequel le patient ayant été identifié comme ne présentant pas au moins une des caractéristiques

suivantes :

(a) des antécédents de maladie myocardique du côté gauche ;
(b) un taux élevé de peptide natriurétique de type B (BNP) ;
(c) un rapport albumine/créatinine (RAC) élevé ; et
(d) une insuffisance rénale chronique (IRC).

**2.** Composé destiné à être utilisé selon la revendication 1, dans lequel le patient ne souffre pas d'insuffisance rénale chronique (IRC).

**3.** Composé destiné à être utilisé selon la revendication 1 ou 2, dans lequel la maladie cardiovasculaire est l'athérosclérose, la resténose, l'hypertension pulmonaire, l'hypertension artérielle pulmonaire ou la thrombose.

**4.** Composé destiné à être utilisé selon l'une quelconque des revendications 1 à 3, dans lequel :

(i) le patient a été identifié comme ne présentant aucune desdites quatre caractéristiques ;
(ii) la pression dans l'artère pulmonaire du patient a été mesurée ou sera mesurée ;
(iii) le patient n'a pas d'antécédents de maladie myocardique du côté gauche ;
(iv) le taux de BNP du patient est supérieur à 200 pg/mL ;
(v) le patient présente un RAC supérieur à 300 mg/g ;
(vi) le taux de filtration glomérulaire estimé (DFGe) du patient est supérieur ou égal à 45 mL/min/1,73 $m^2$ ;
(vii) le patient a été identifié comme ne souffrant pas de BPCO ;
(viii) le patient n'est pas fumeur ;
(ix) le patient a été identifié comme n'ayant pas de cancer ; et/ou
(x) le patient a été identifié comme n'ayant pas de diabète de type 2.

**5.** Composé destiné à être utilisé selon la revendication 1, dans lequel le patient souffre d'une insuffisance rénale chronique (IRC).

**6.** Composé destiné à être utilisé selon l'une quelconque des revendications 1 à 5, dans lequel le patient est un patient humain.

**7.** Composé destiné à être utilisé selon l'une quelconque des revendications 1 à 6, dans lequel le composé est formulé sous forme d'une dispersion solide comprenant (i) le composé et (ii) un excipient ; éventuellement dans lequel l'excipient est un copolymère d'acide méthacrylique et d'acrylate d'éthyle.

**8.** Composé destiné à être utilisé selon l'une quelconque des revendications 1 à 7, dans lequel le composé est administré au patient soit concomitamment, soit séquentiellement avec une seconde thérapie comprenant une quantité pharmaceutiquement efficace d'un second médicament.

**9.** Composé destiné à être utilisé selon la revendication 8, dans lequel le second médicament est un inhibiteur de la Rho kinase, un inhibiteur de la tyrosine kinase, un dérivé de l'époprosténol, un antagoniste des récepteurs de la sérotonine, un antagoniste des récepteurs de l'endothéline, un inhibiteur de la phosphodiestérase (PDE), un bloqueur des canaux calciques, un stimulateur de la guanylate cyclase soluble ou de l'oxyde nitrique ;
éventuellement, dans lequel l'inhibiteur de la Rho kinase est HA-1077, HA-1152P ou Y-27632 ; éventuellement, dans lequel l'inhibiteur de la tyrosine kinase est Gleevec ; éventuellement, dans lequel le dérivé d'époprosténol est le tréprostinil, le béraprost ou l'iloprost ; éventuellement, dans lequel l'antagoniste des récepteurs de la sérotonine est le sarpogrelate ; éventuellement, dans lequel l'antagoniste des récepteurs de l'endothéline est le bosentan, le sitaxsentan, l'ambrisentan, le TBC3711 ou le macitentan ; éventuellement, dans lequel l'inhibiteur de la PDE est l'amrinone, le sildénafil, le tadalafil, le vardénafil, l'énoximone, l'udénafil ou le milrinone ; éventuellement, dans lequel l'inhibiteur des canaux calciques est l'amlodipine, le diltiazem, l'isradipine, la nicardipine, la nifédipine, la nimodipine, la nisoldipine, la nitrendipine ou le vérapamil ; ou, éventuellement, dans lequel le stimulateur de la guanylate cyclase soluble est le riociguat.

**10.** Composé destiné à être utilisé selon l'une quelconque des revendications 1 à 9, dans lequel la maladie cardiovasculaire est l'hypertension pulmonaire.

**11.** Composé destiné à être utilisé selon la revendication 10, dans lequel l'hypertension pulmonaire est une hypertension

artérielle pulmonaire.

**12.** Composé destiné à être utilisé selon la revendication 11, dans lequel l'hypertension artérielle pulmonaire est associée à une maladie du tissu conjonctif, ou dans lequel l'hypertension artérielle pulmonaire est idiopathique.

**13.** Composé destiné à être utilisé selon l'une quelconque des revendications 1 à 12, dans lequel :

(i) au moins une partie du composé est présente sous forme cristalline présentant un diagramme de diffraction des rayons X (K$\alpha$ du Cu) comprenant des pics de diffraction significatifs à environ 8,8, 12,9, 13,4, 14,2 et 17,4 °2$\theta$ ; ou
(ii) au moins une partie du composé est présente sous une forme amorphe ayant un diagramme de diffraction des rayons X (K$\alpha$ du Cu) avec un pic halogéno à environ 13,5 °2$\theta$, sensiblement comme indiqué sur la figure 1C, et une température de transition vitreuse ($T_g$) dans la plage d'environ 120 °C à environ 135 °C.

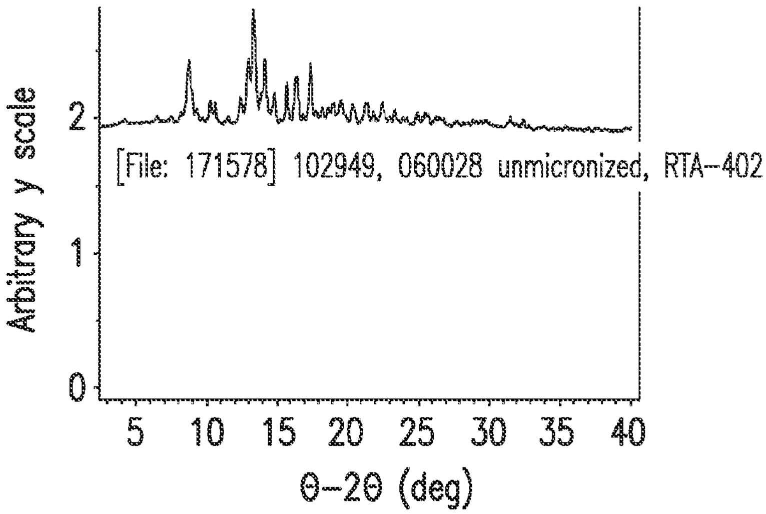


FIG.1A

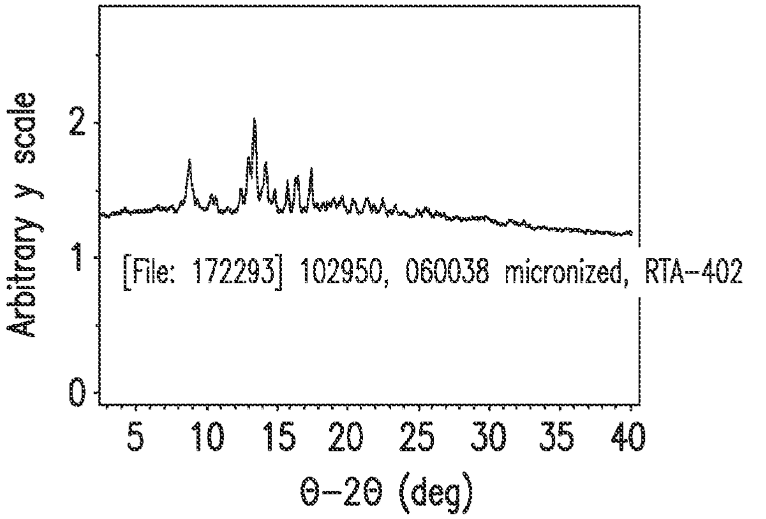


FIG.1B

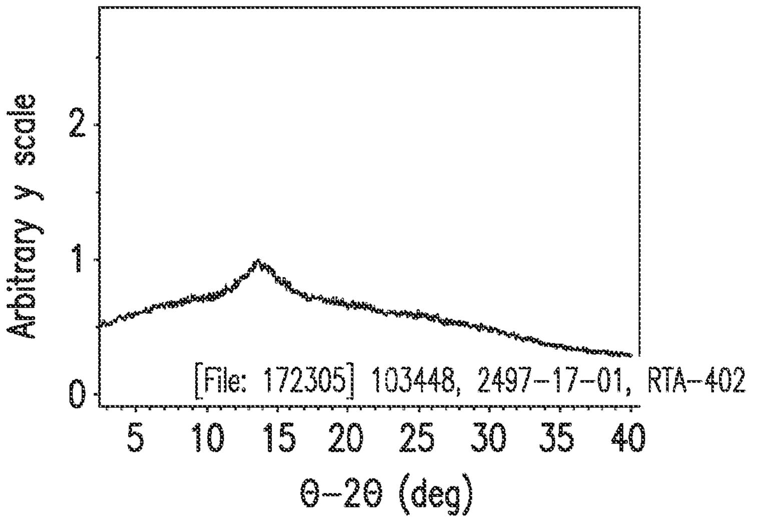


FIG.1C

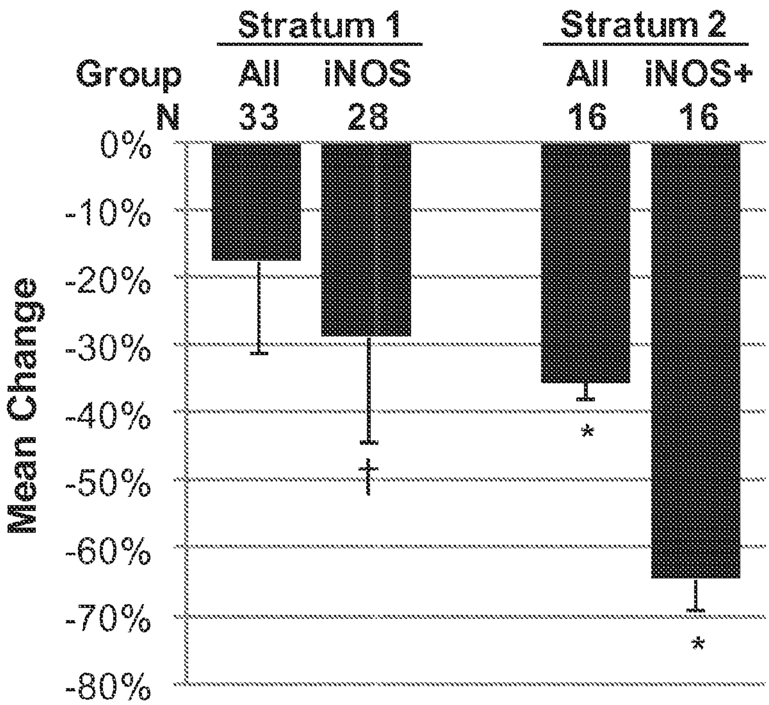
Stratum 1
Stratum 2
Group
All
iNOS
All
iNOS+
N
33
28
16
16
0%
-10%
-20%
-30%
-40%
-50%
-60%
-70%
-80%
Mean Change
†
*
*

FIG. 2

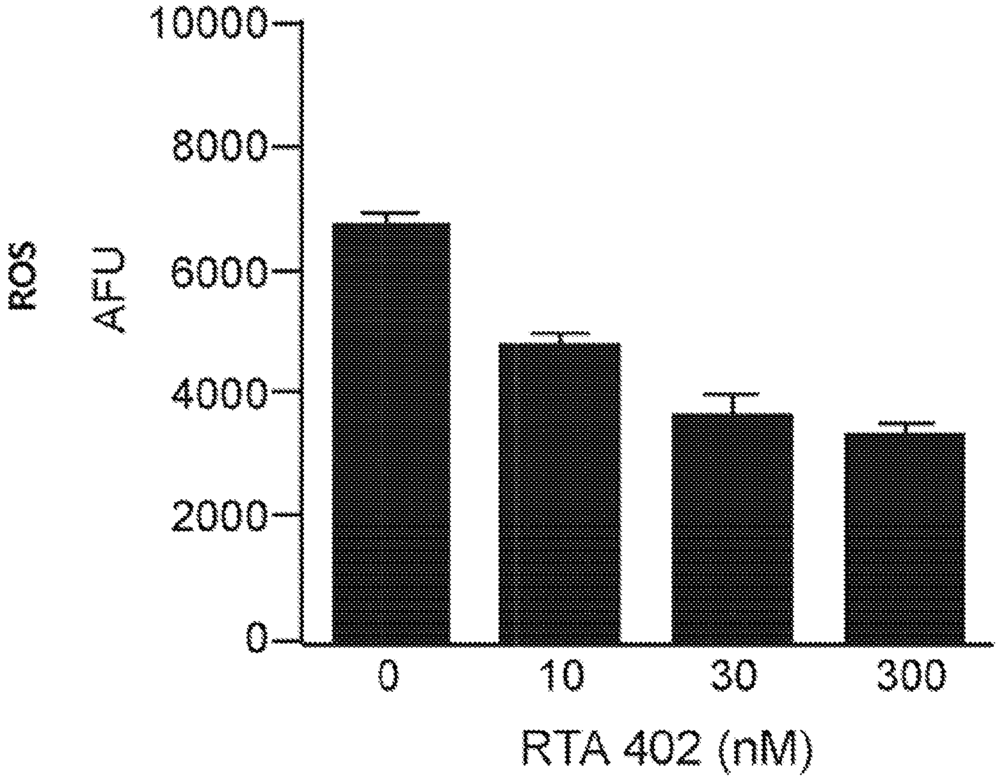


FIG. 3A

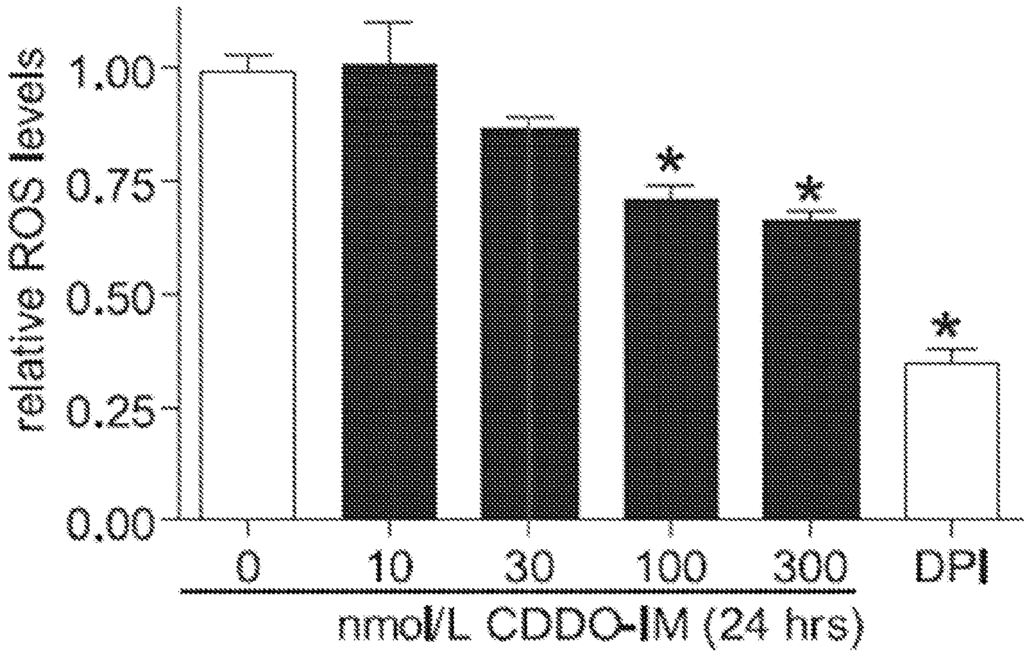
relative ROS levels
1.00
0.75
0.50
0.25
0.00
*
*
*
0
10
30
100
300
DPI
nmol/L CDDO-IM (24 hrs)

FIG. 3B

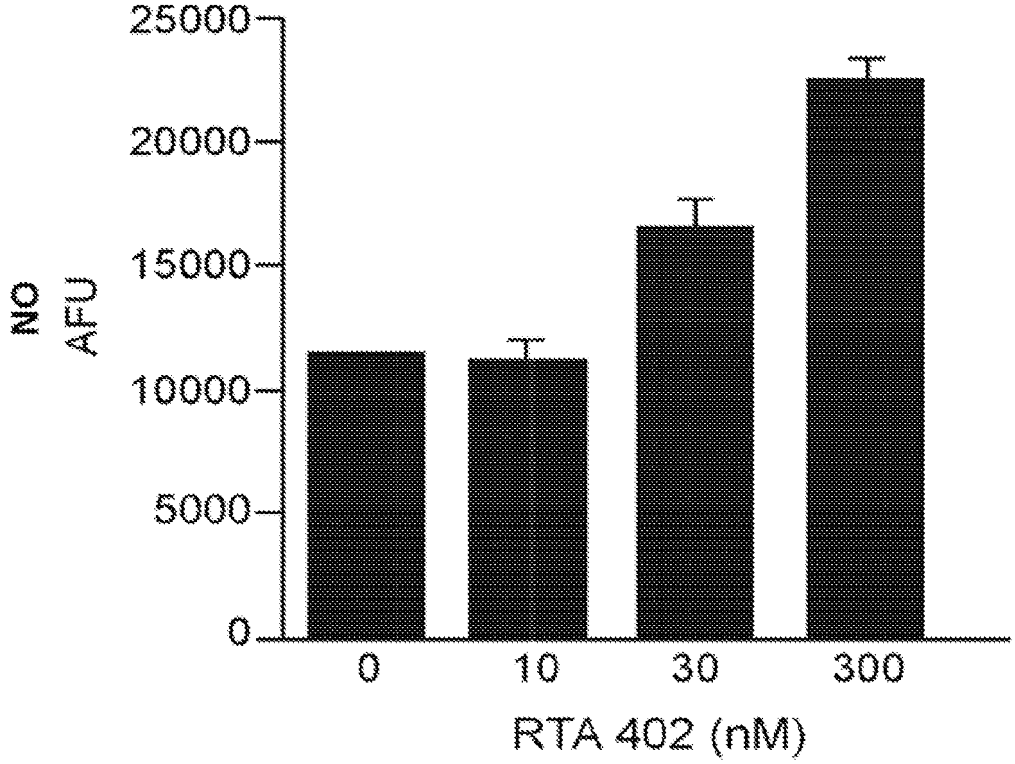


FIG. 3C

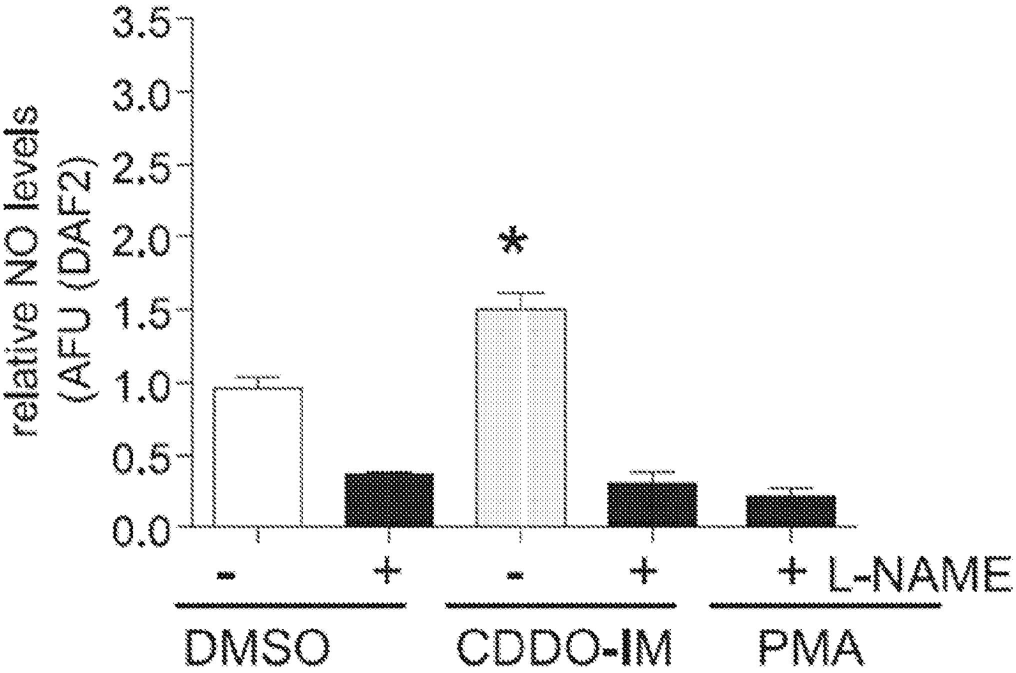
relative NO levels
(AFU (DAF2)
3.5
3.0
2.5
2.0
1.5
1.0
0.5
0.0
*
- + - + + L-NAME
DMSO
CDDO-IM
PMA

FIG. 3D

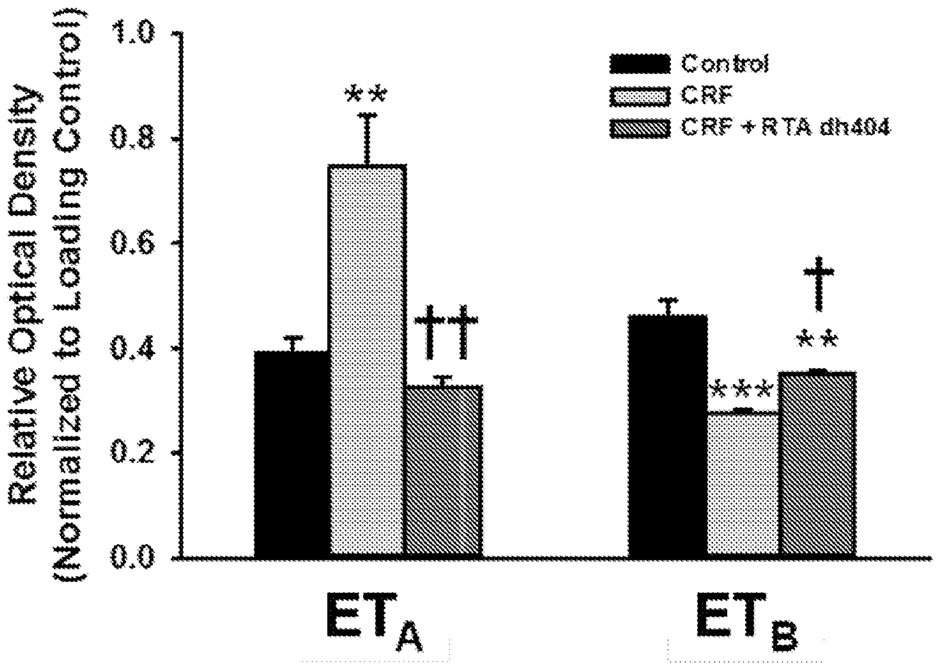
Relative Optical Density
(Normalized to Loading Control)
1.0
0.8
0.6
0.4
0.2
0.0
Control
CRF
CRF + RTA dh404
**
††
***
**
†
$ET_A$
$ET_B$

FIG. 4

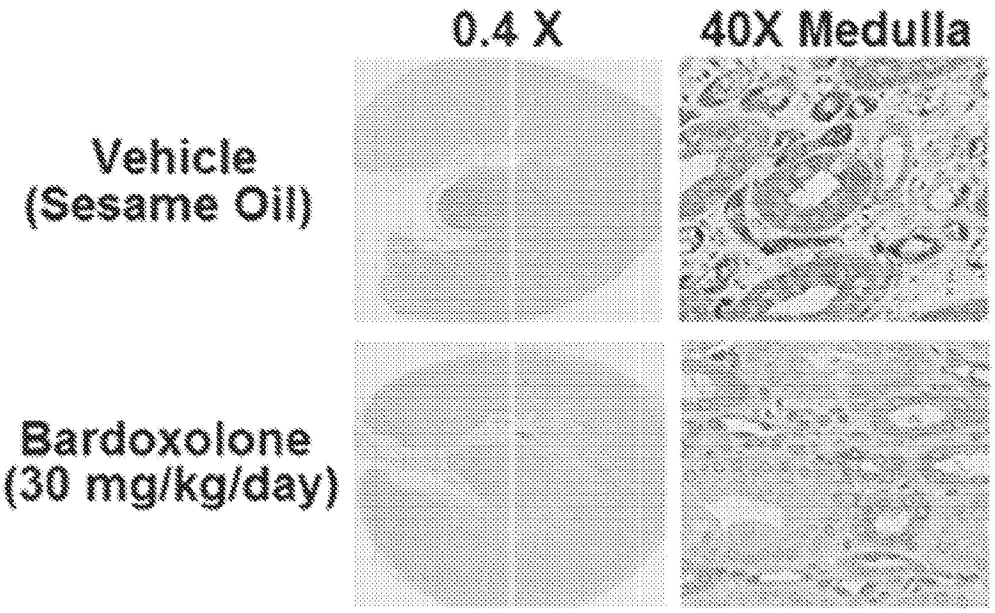


FIG. 5A

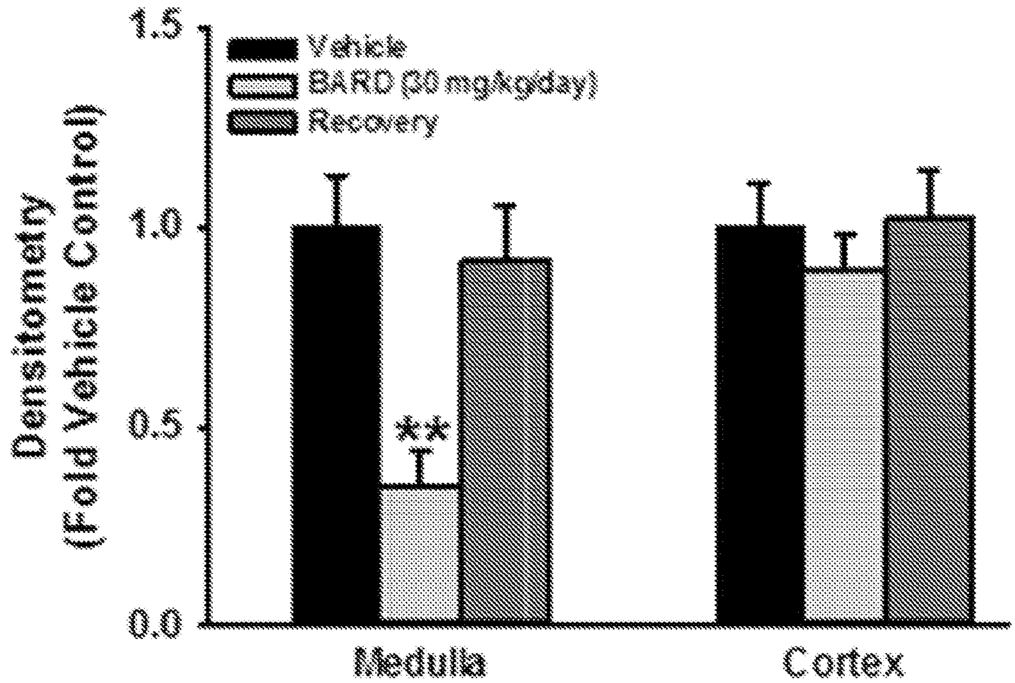
1.5
1.0
0.5
0.0
Densitometry
(Fold Vehicle Control)
Vehicle
BARD (30 mg/kg/day)
Recovery
**
Medulla
Cortex

FIG. 5B

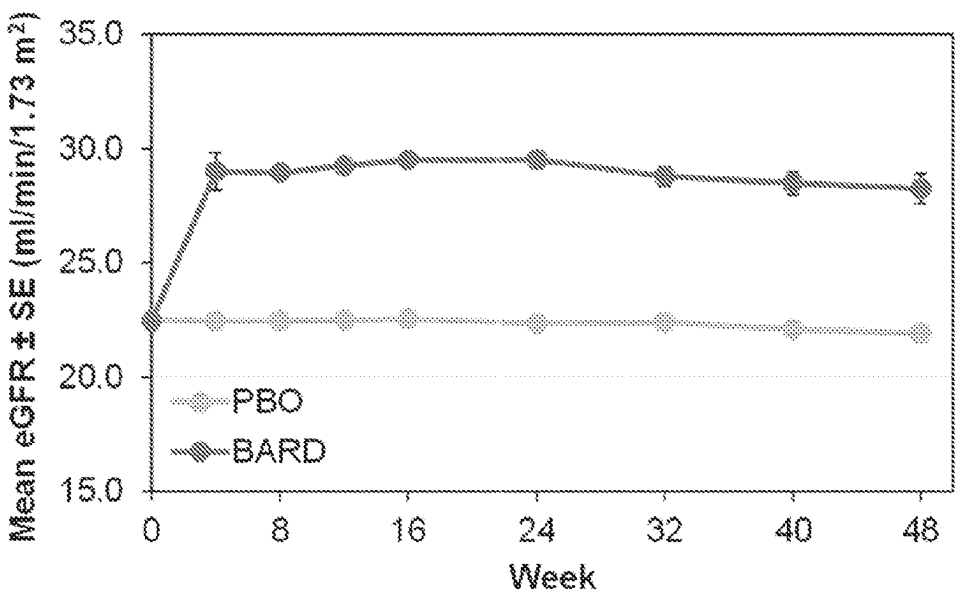
Mean eGFR ± SE (ml/min/1.73 m²)
35.0
30.0
25.0
20.0
15.0
PBO
BARD
0
8
16
24
32
40
48
Week

FIG. 6

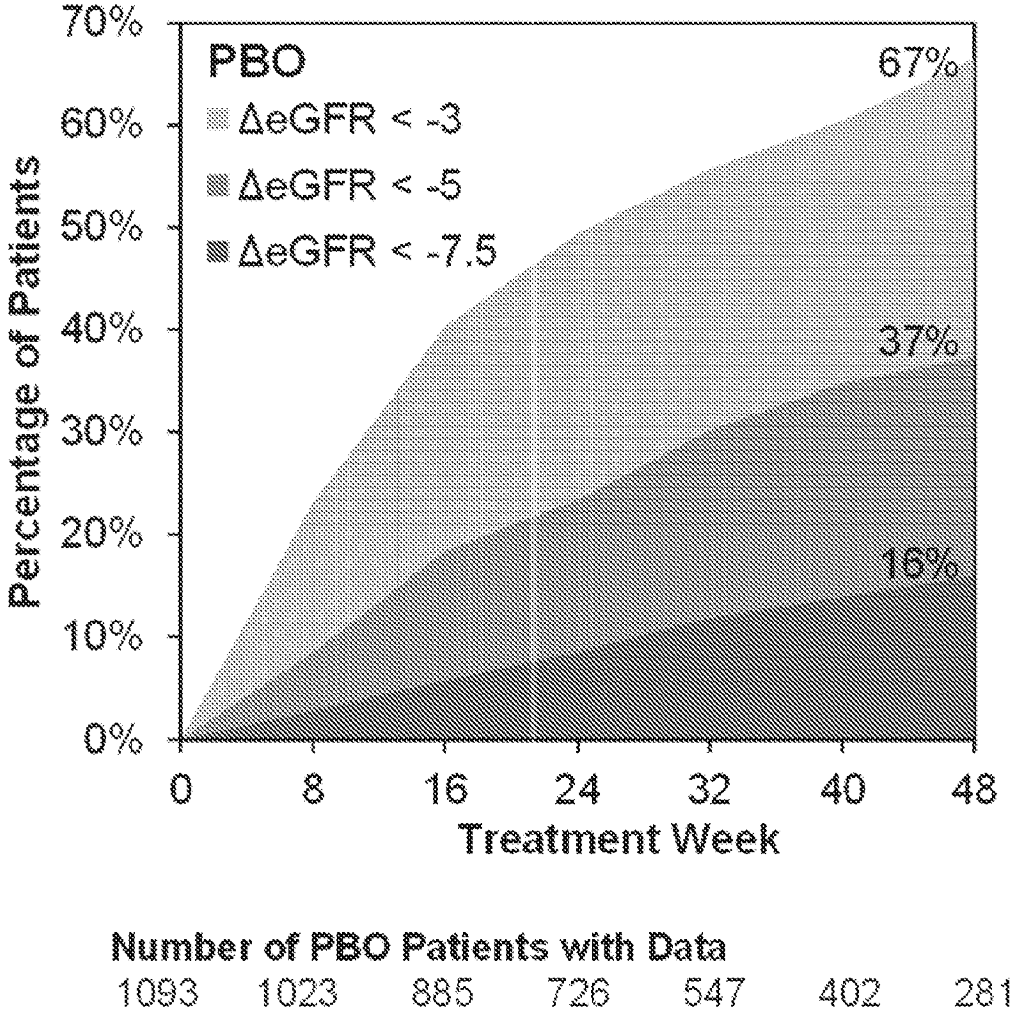
70%
60%
50%
40%
30%
20%
10%
0%
Percentage of Patients
PBO
ΔeGFR < -3
ΔeGFR < -5
ΔeGFR < -7.5
67%
37%
16%
0
8
16
24
32
40
48
Treatment Week
Number of PBO Patients with Data
1093 1023 885 726 547 402 281

FIG. 7A

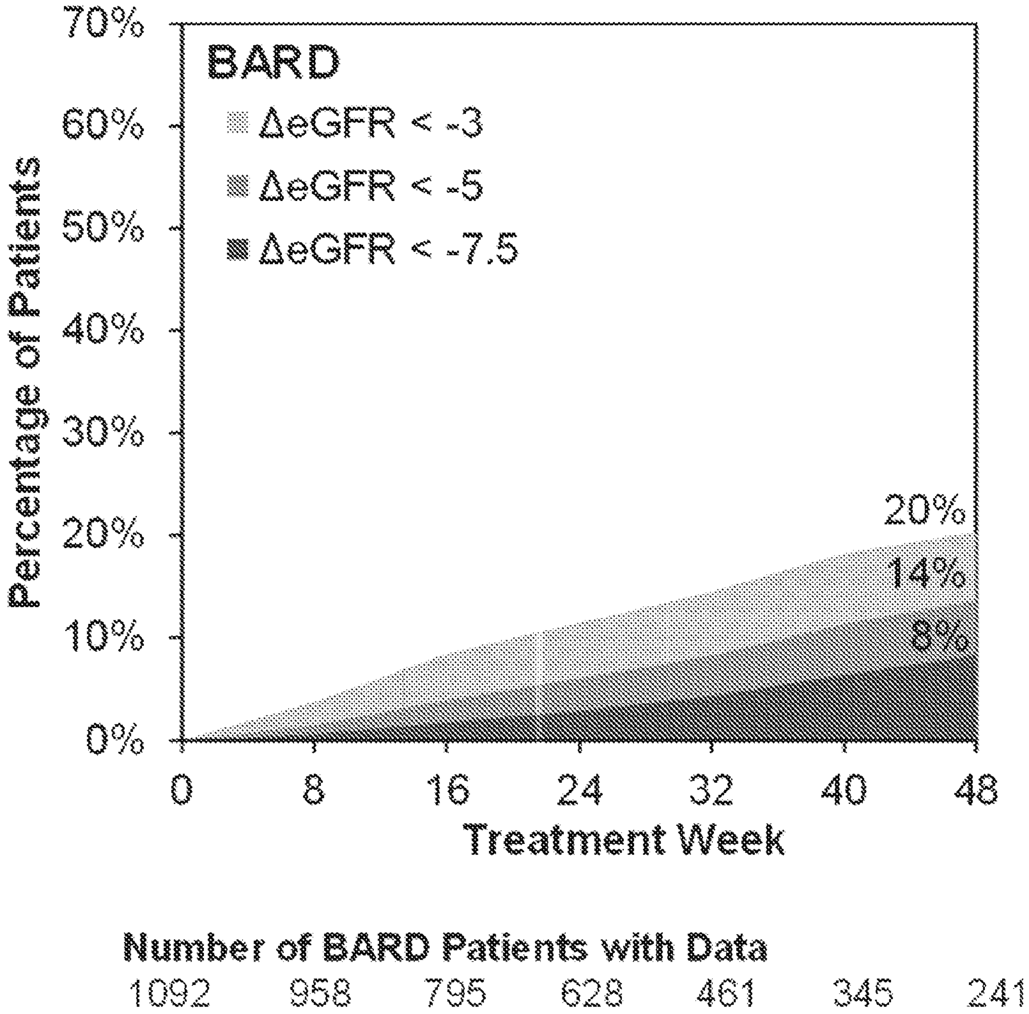
70%
60%
50%
40%
30%
20%
10%
0%
Percentage of Patients
BARD
ΔeGFR < -3
ΔeGFR < -5
ΔeGFR < -7.5
20%
14%
8%
0
8
16
24
32
40
48
Treatment Week
Number of BARD Patients with Data
1092
958
795
628
461
345
241

FIG. 7B

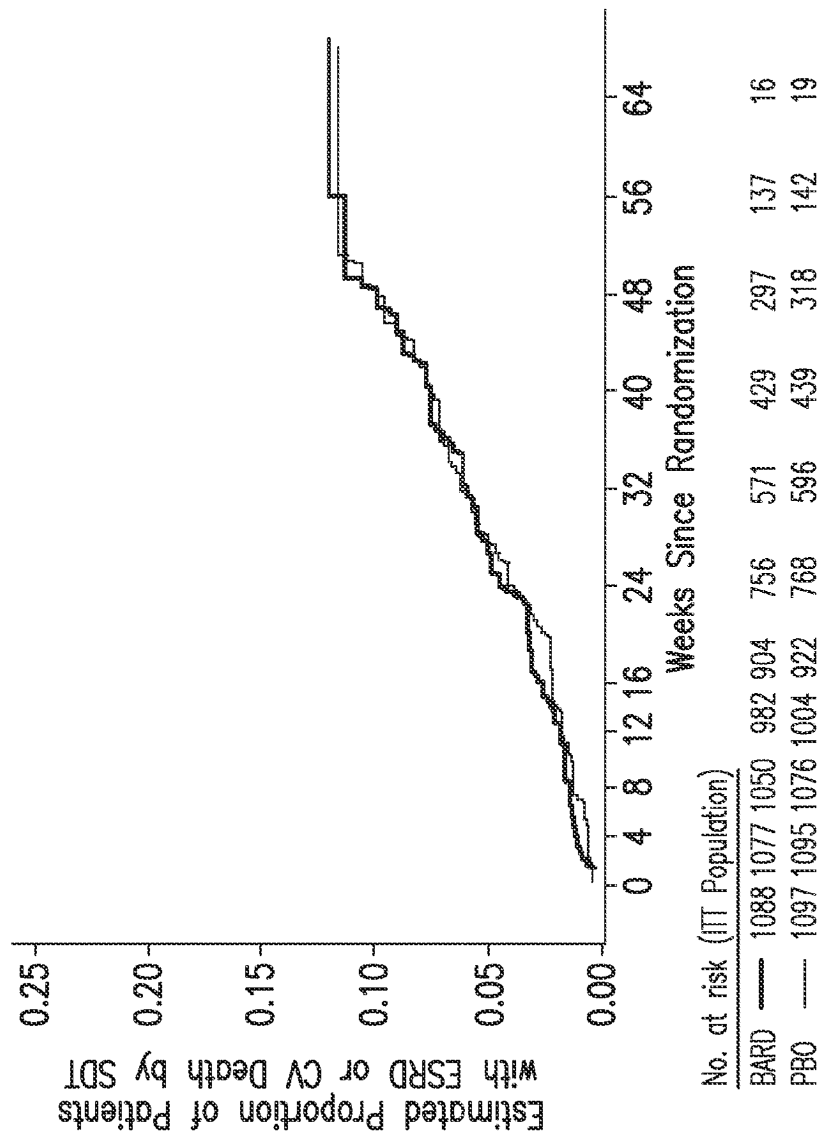
Estimated Proportion of Patients with ESRD or CV Death by SDT
0.00
0.05
0.10
0.15
0.20
0.25
0 4 8 12 16 24 32 40 48 56 64
Weeks Since Randomization
No. at risk (ITT Population)
BARD 1088 1077 1050 982 904 756 571 429 297 137 16
PBO 1097 1095 1076 1004 922 768 596 439 318 142 19

FIG.8

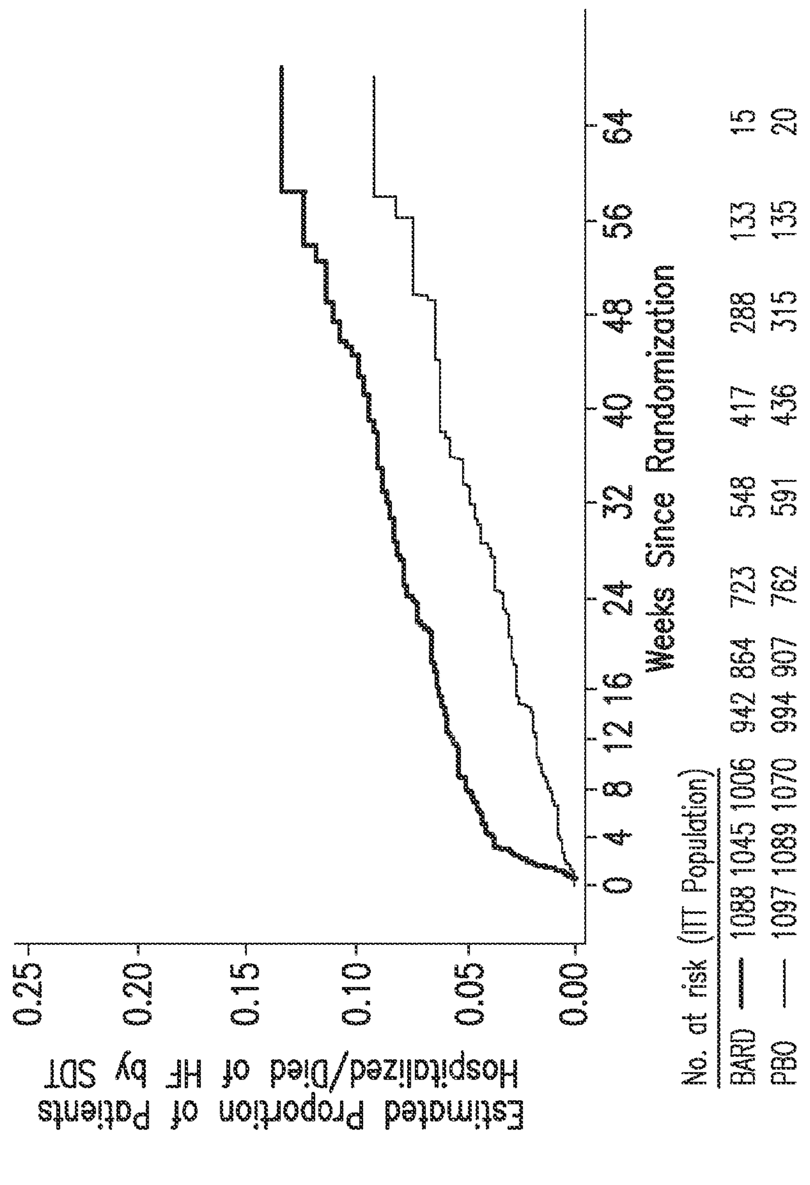
Estimated Proportion of Patients Hospitalized/Died of HF by SDT
0.00
0.05
0.10
0.15
0.20
0.25
0 4 8 12 16 24 32 40 48 56 64
Weeks Since Randomization
No. at risk (ITT Population)
BARD 1088 1045 1006 942 864 723 548 417 288 133 15
PBO 1097 1089 1070 994 907 762 591 436 315 135 20

FIG.9

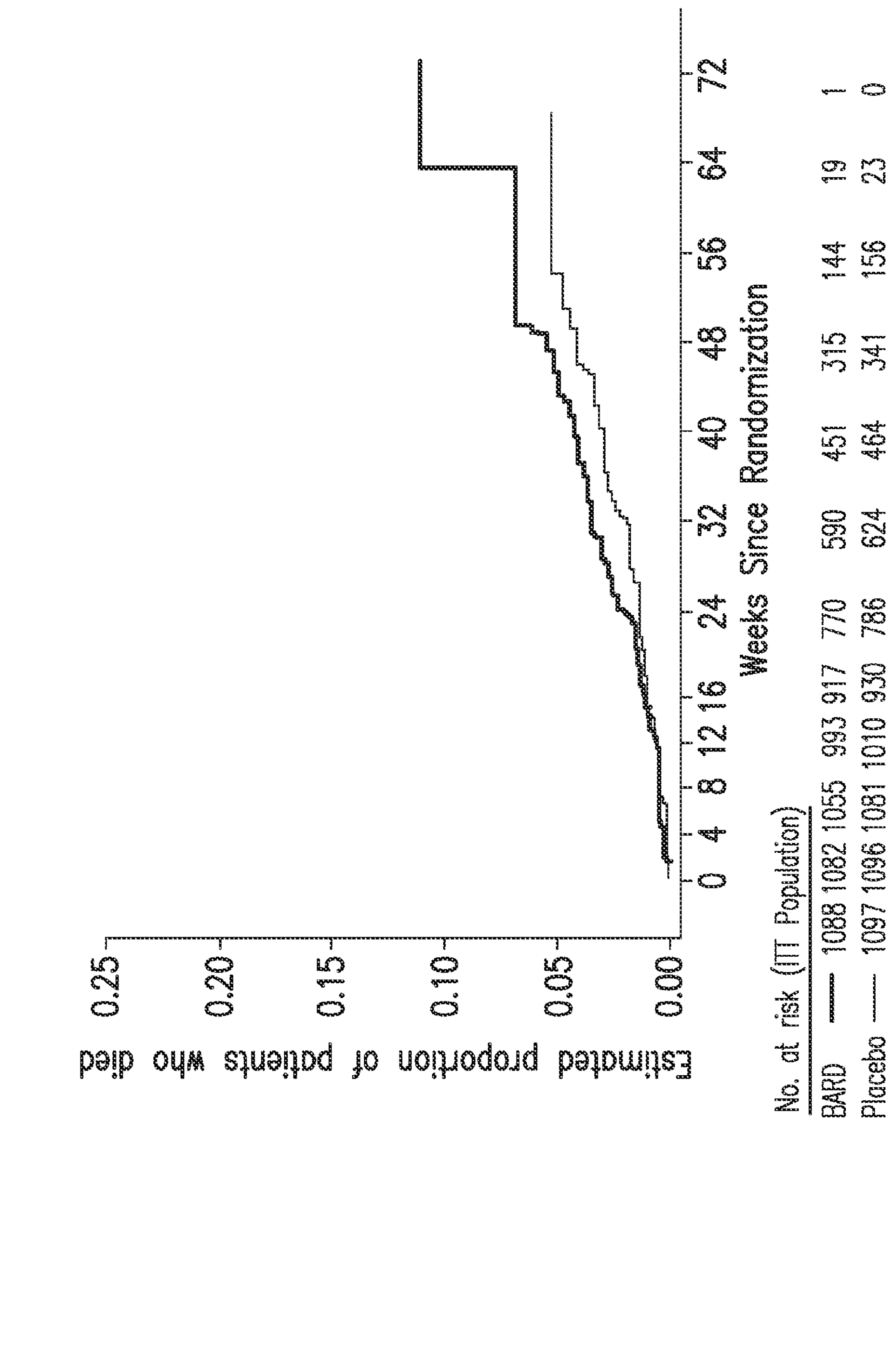
Estimated proportion of patients who died
0.00
0.05
0.10
0.15
0.20
0.25
Weeks Since Randomization
0
4
8
12
16
24
32
40
48
56
64
72
No. at risk (ITT Population)
BARD 1088 1082 1055 993 917 770 590 451 315 144 19 1
Placebo 1097 1096 1081 1010 930 786 624 464 341 156 23 0

FIG. 10

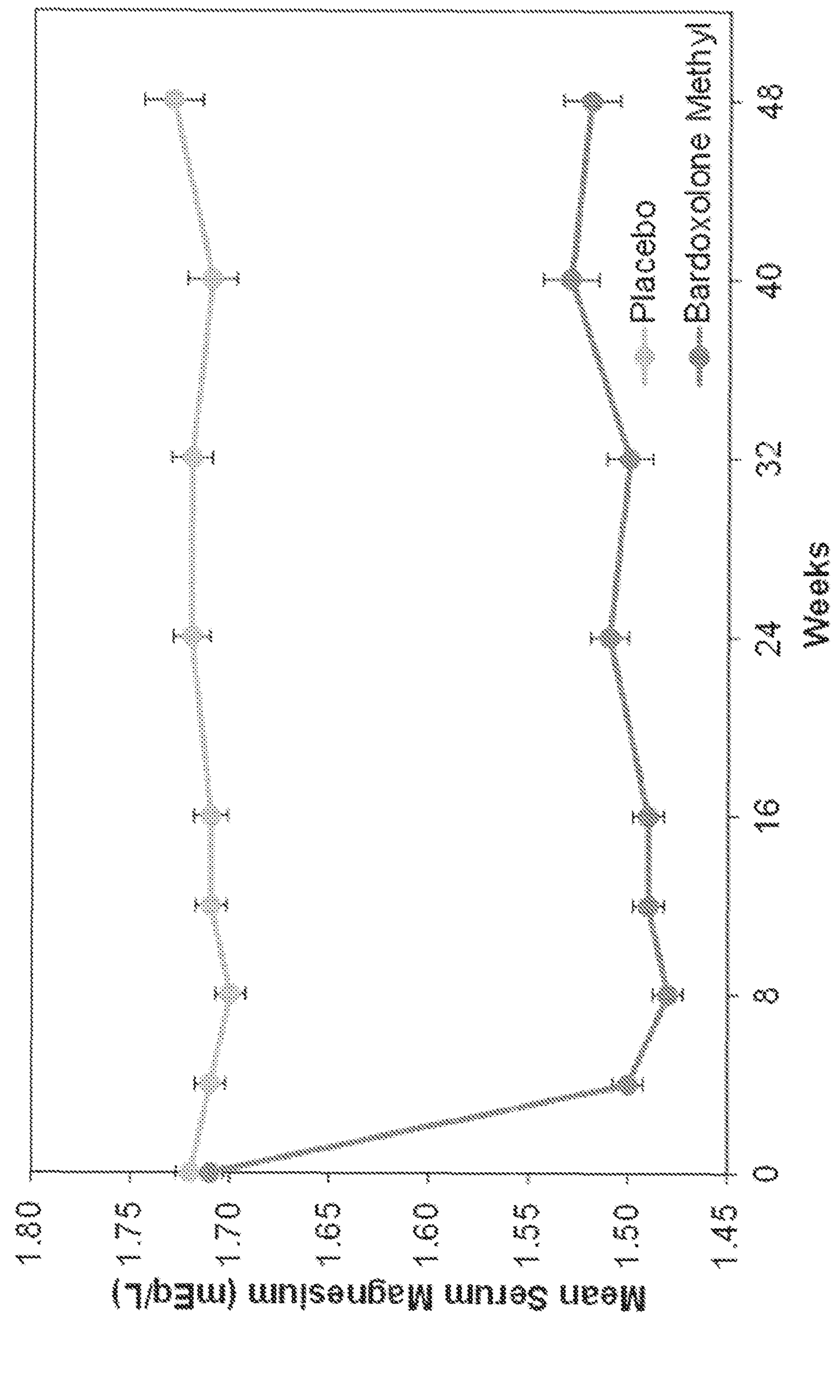
Mean Serum Magnesium (mEq/L)
1.80
1.75
1.70
1.65
1.60
1.55
1.50
1.45
0
8
16
24
32
40
48
Weeks
Placebo
Bardoxolone Methyl

FIG. 11

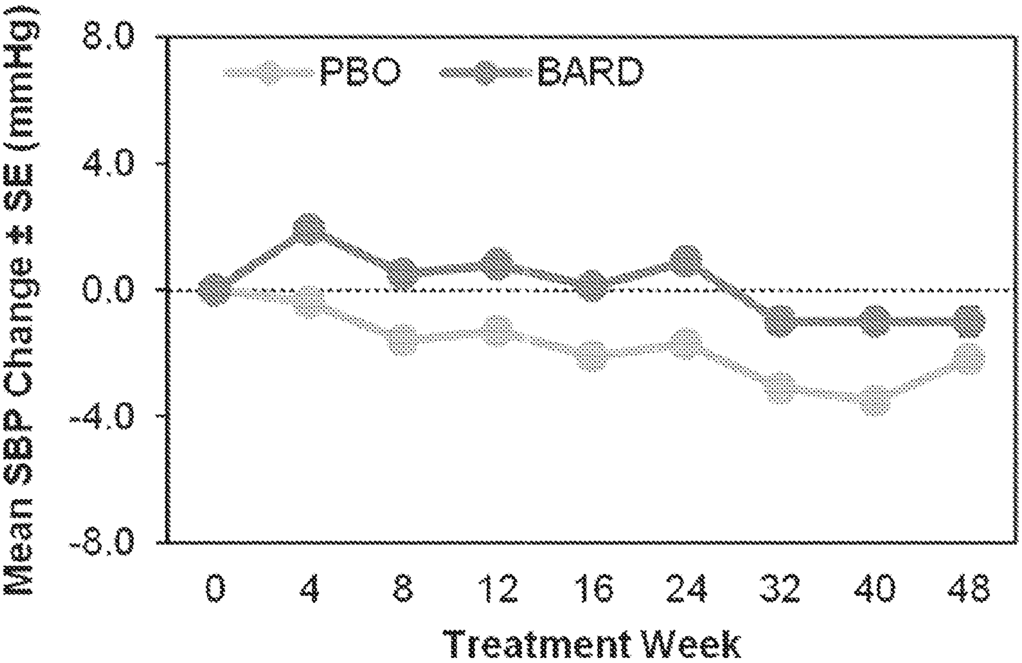
PBO
BARD
Mean SBP Change ± SE (mmHg)
8.0
4.0
0.0
-4.0
-8.0
0
4
8
12
16
24
32
40
48
Treatment Week

FIG. 12A

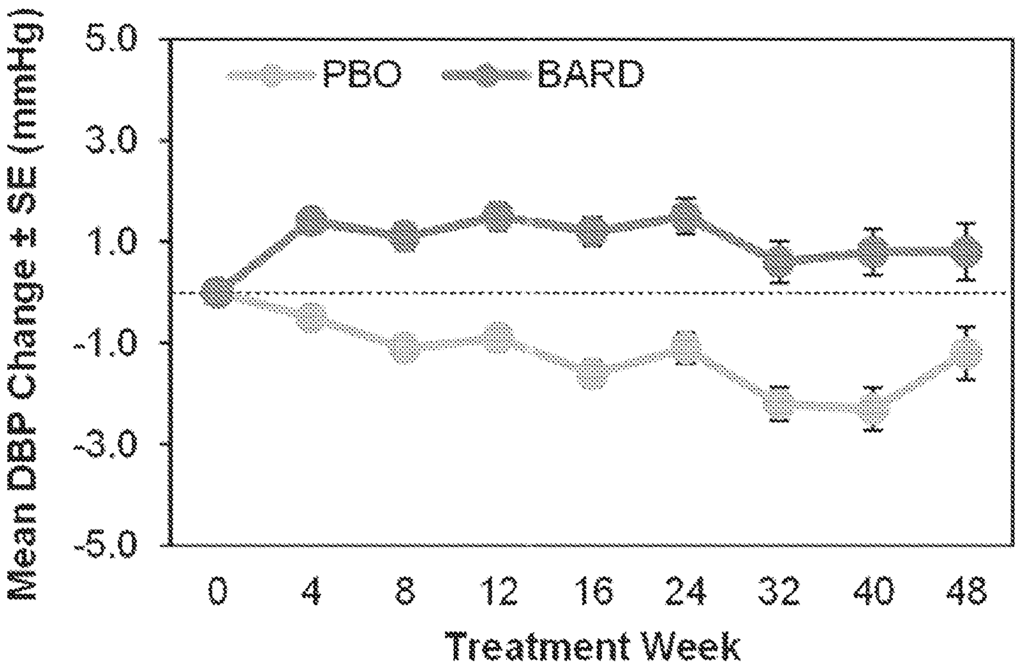
Mean DBP Change ± SE (mmHg)
5.0
3.0
1.0
-1.0
-3.0
-5.0
PBO
BARD
0
4
8
12
16
24
32
40
48
Treatment Week

FIG. 12B

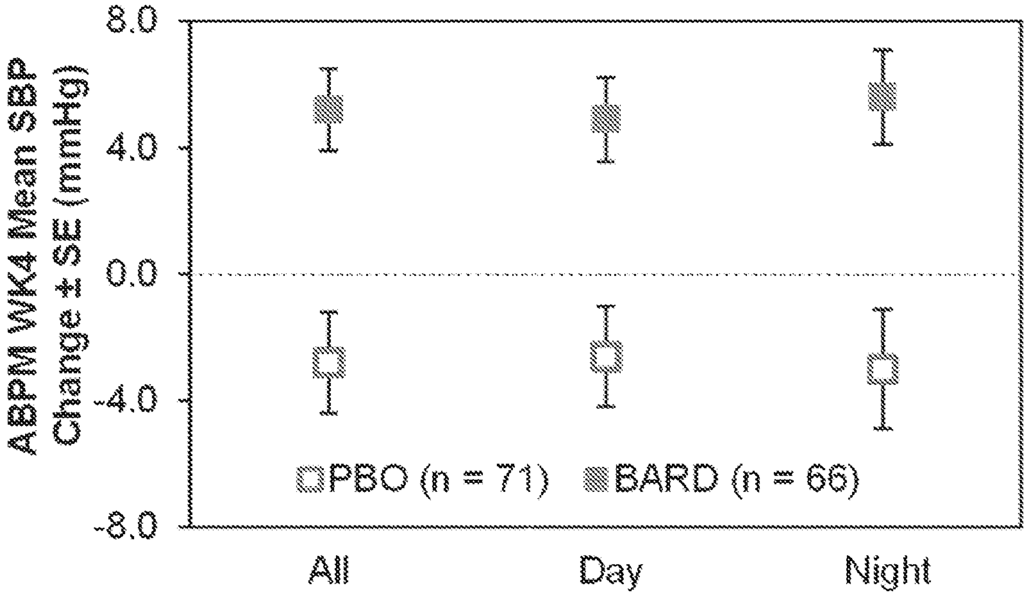
ABPM WK4 Mean SBP
Change ± SE (mmHg)
8.0
4.0
0.0
-4.0
-8.0
PBO (n = 71)
BARD (n = 66)
All
Day
Night

FIG. 13A

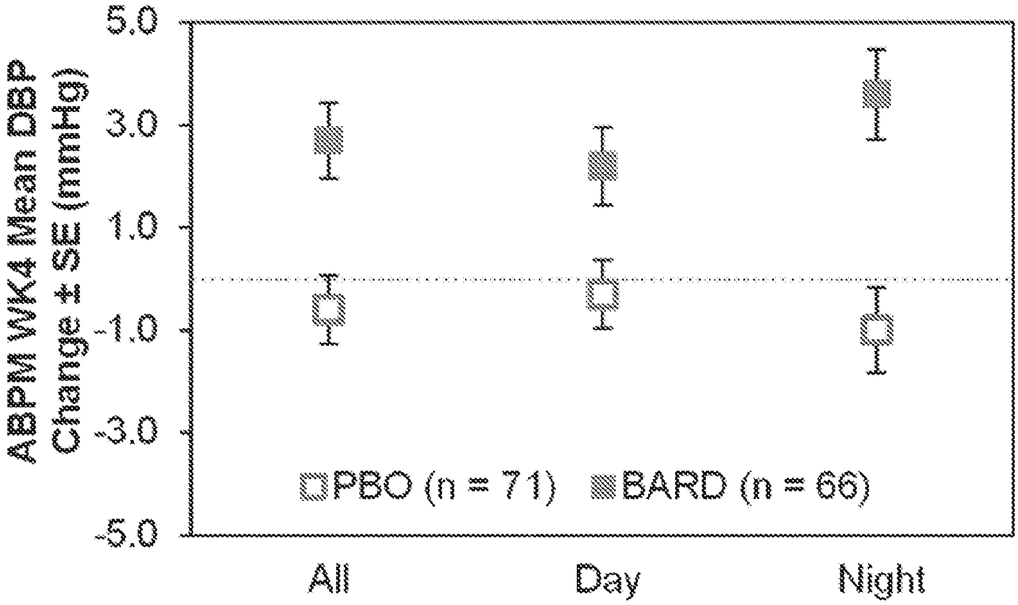
ABPM WK4 Mean DBP Change ± SE (mmHg)
5.0
3.0
1.0
-1.0
-3.0
-5.0
PBO (n = 71)
BARD (n = 66)
All
Day
Night

FIG. 13B

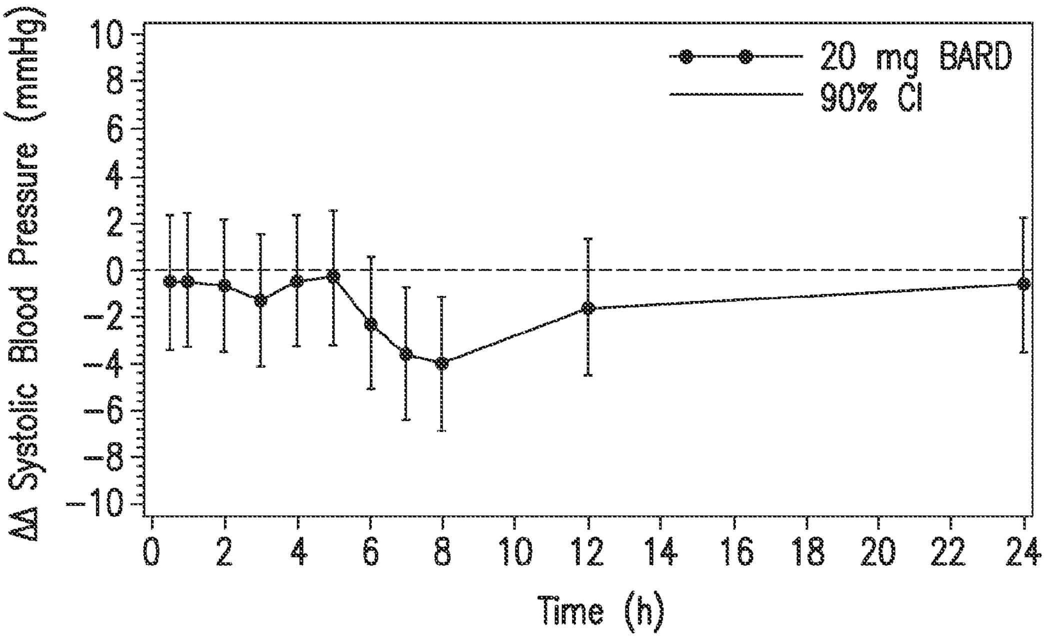
ΔΔ Systolic Blood Pressure (mmHg)
10
8
6
4
2
0
-2
-4
-6
-8
-10
0
2
4
6
8
10
12
14
16
18
20
22
24
Time (h)
20 mg BARD
90% CI

FIG.14A

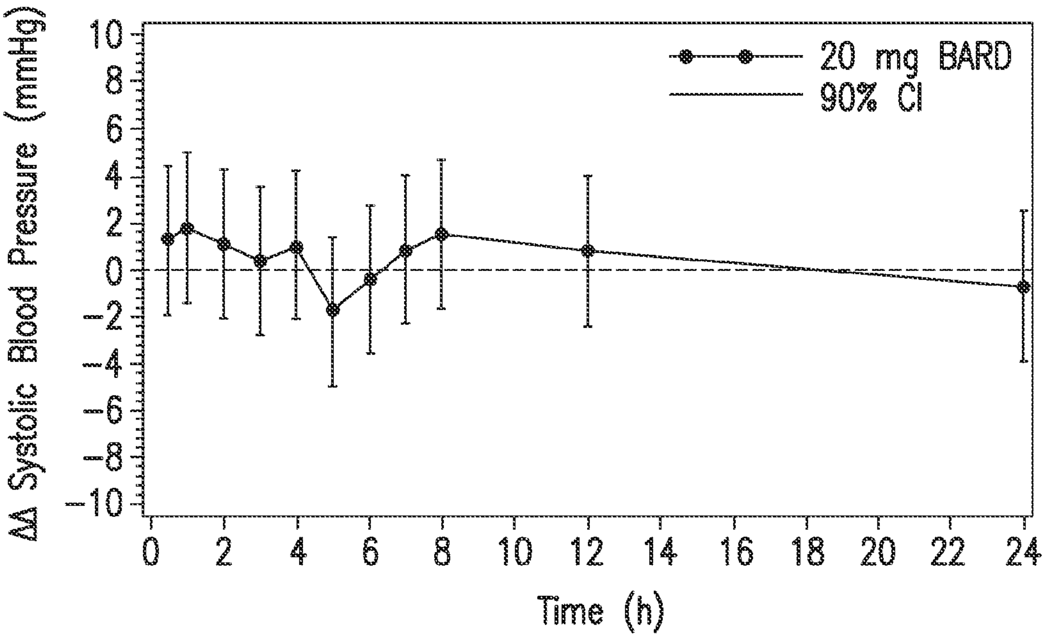
ΔΔ Systolic Blood Pressure (mmHg)
10
8
6
4
2
0
−2
−4
−6
−8
−10
0 2 4 6 8 10 12 14 16 18 20 22 24
Time (h)
20 mg BARD
90% CI

FIG.14B

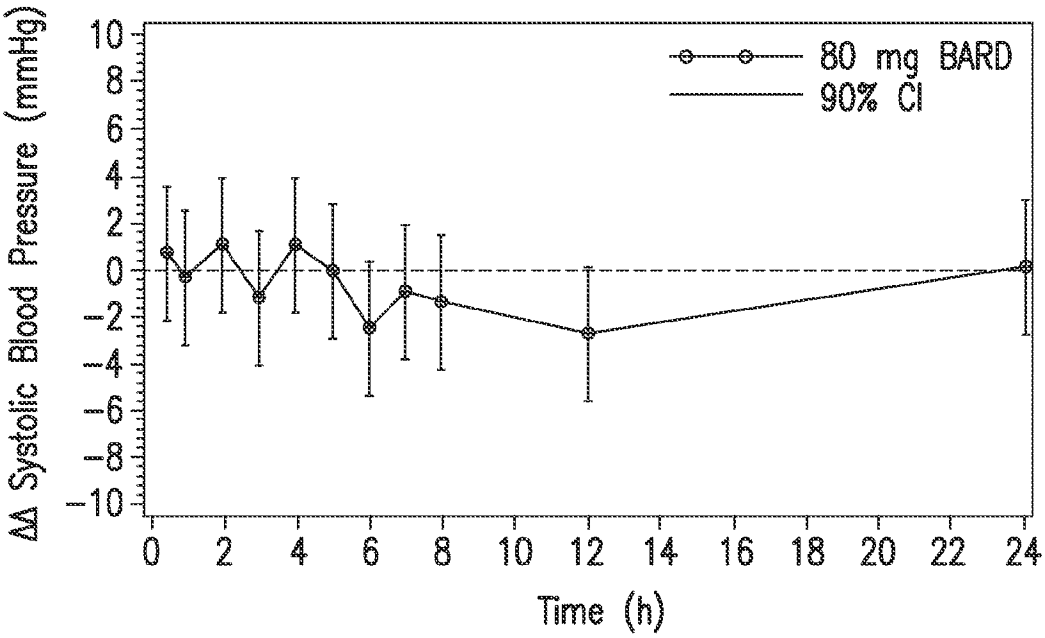
80 mg BARD
90% CI
ΔΔ Systolic Blood Pressure (mmHg)
10
8
6
4
2
0
−2
−4
−6
−8
−10
0
2
4
6
8
10
12
14
16
18
20
22
24
Time (h)

FIG.14C

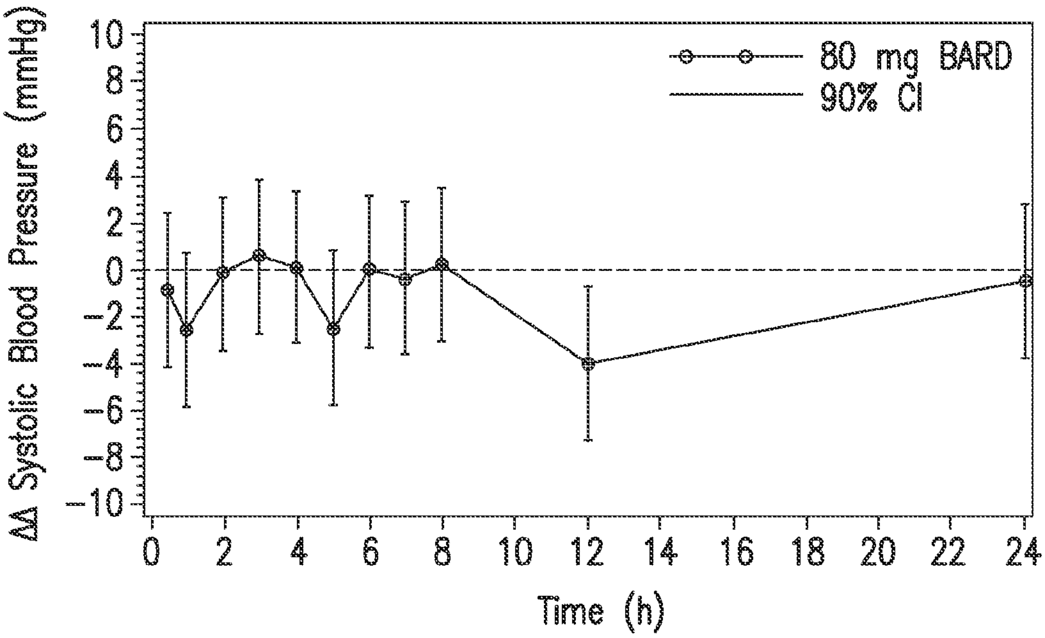
80 mg BARD
90% CI
ΔΔ Systolic Blood Pressure (mmHg)
10
8
6
4
2
0
-2
-4
-6
-8
-10
0 2 4 6 8 10 12 14 16 18 20 22 24
Time (h)

FIG.14D

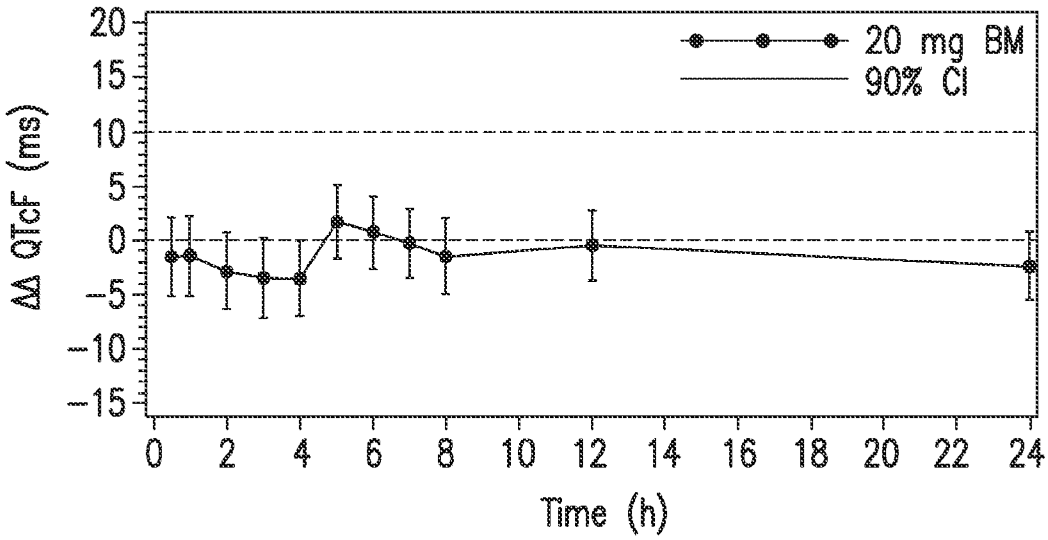
20 mg BM
90% CI
ΔΔ QTcF (ms)
20
15
10
5
0
−5
−10
−15
0
2
4
6
8
10
12
14
16
18
20
22
24
Time (h)

FIG.15A

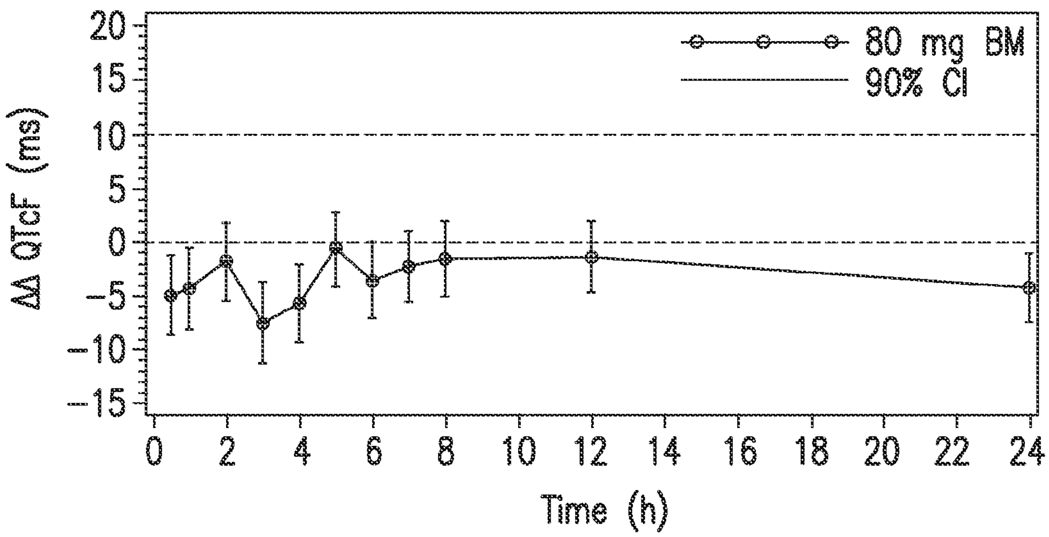
80 mg BM
90% CI
ΔΔ QTcF (ms)
20
15
10
5
0
-5
-10
-15
0
2
4
6
8
10
12
14
16
18
20
22
24
Time (h)

FIG.15B

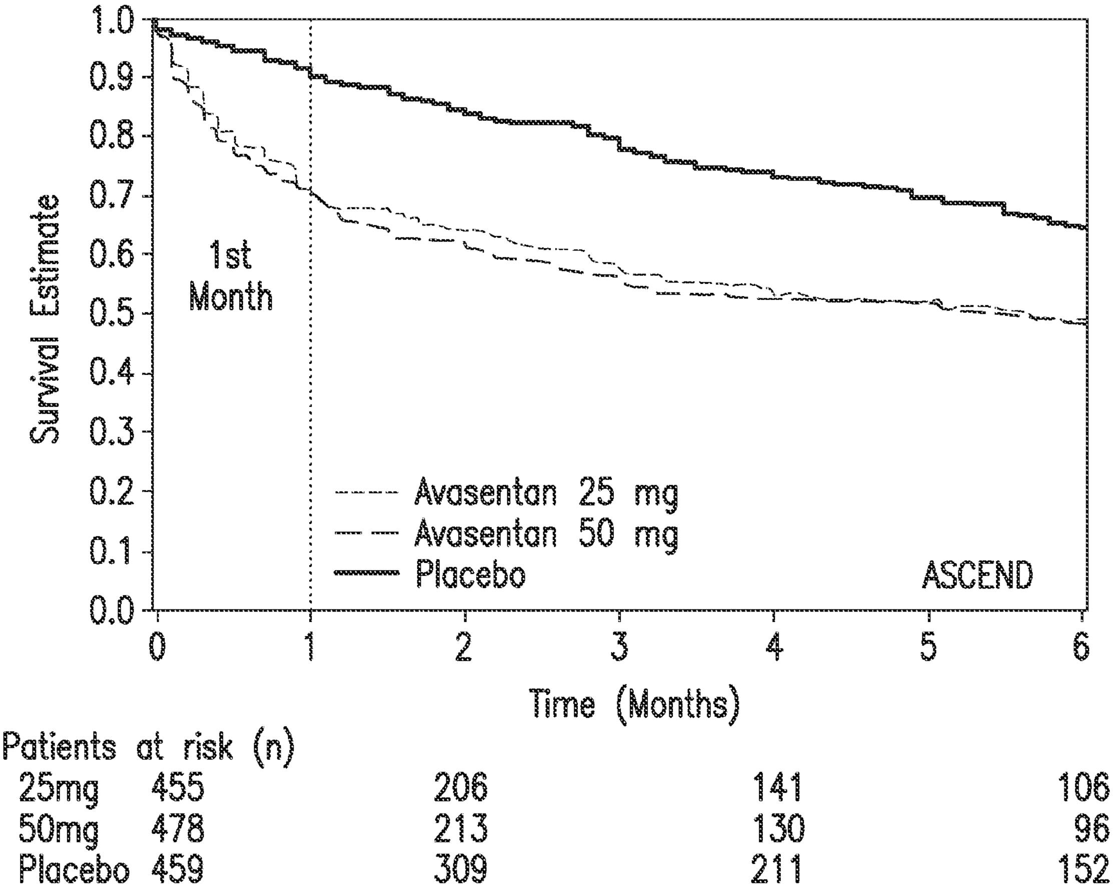


Patients at risk (n)

| | | | | |
|---|---|---|---|---|
| 25mg | 455 | 206 | 141 | 106 |
| 50mg | 478 | 213 | 130 | 96 |
| Placebo | 459 | 309 | 211 | 152 |

FIG.16A

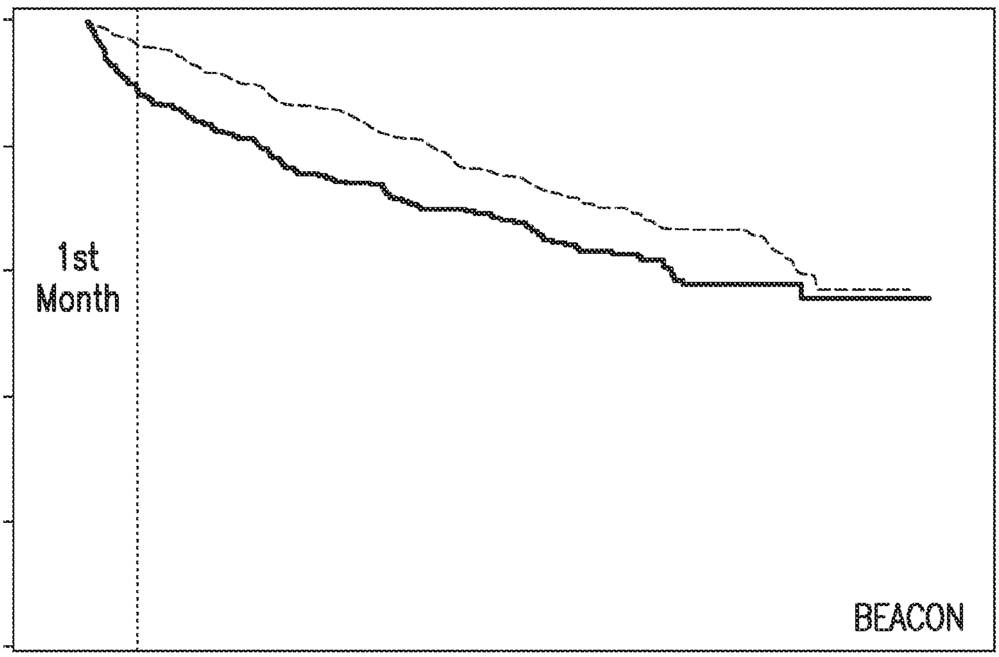


FIG.16B

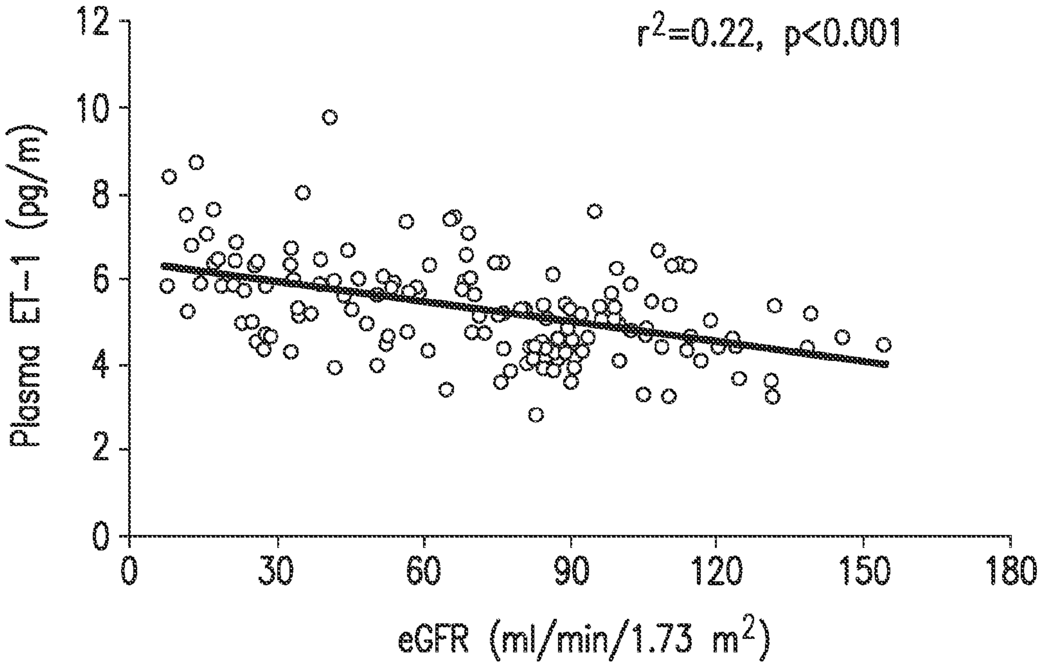
r2=0.22, p<0.001
Plasma ET-1 (pg/m)
12
10
8
6
4
2
0
0
30
60
90
120
150
180
eGFR (ml/min/1.73 m2)

FIG.17A

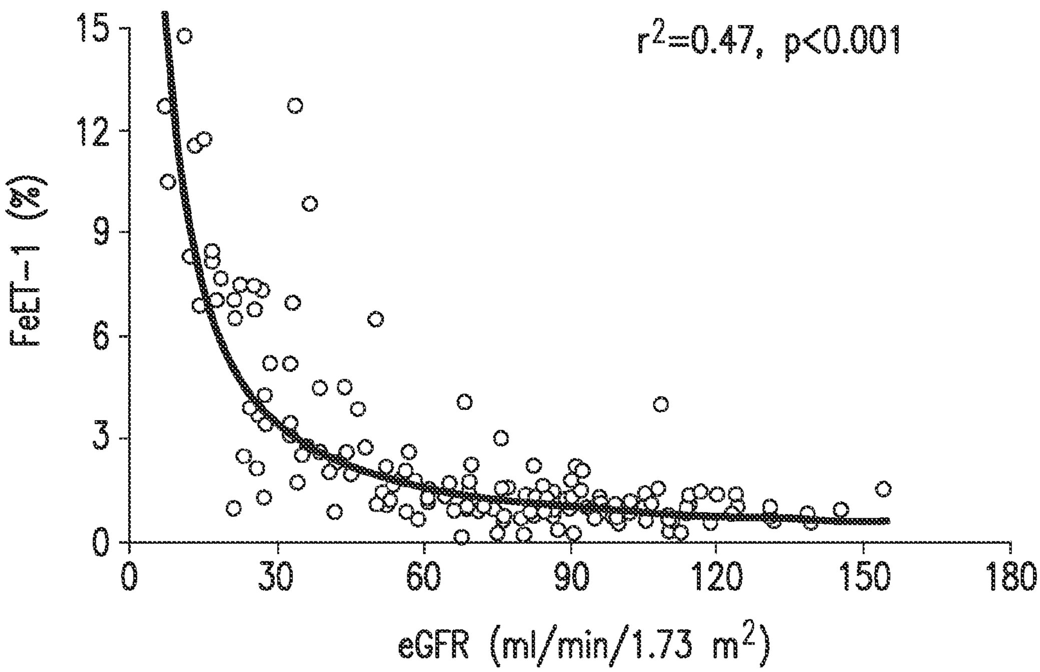
r2=0.47, p<0.001
FeET-1 (%)
eGFR (ml/min/1.73 m2)
0
3
6
9
12
15
0
30
60
90
120
150
180

FIG.17B

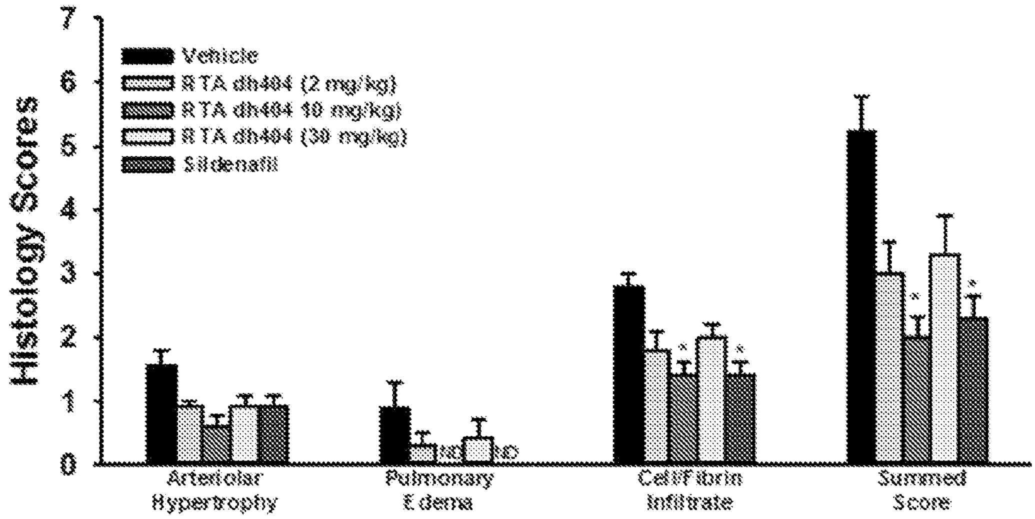
Histology Scores
7
6
5
4
3
2
1
0
Vehicle
RTA dh404 (2 mg/kg)
RTA dh404 10 mg/kg)
RTA dh404 (30 mg/kg)
Sildenafil
ND
ND
*
*
*
*
Arteriolar Hypertrophy
Pulmonary Edema
Cell/Fibrin Infiltrate
Summed Score

FIG. 18

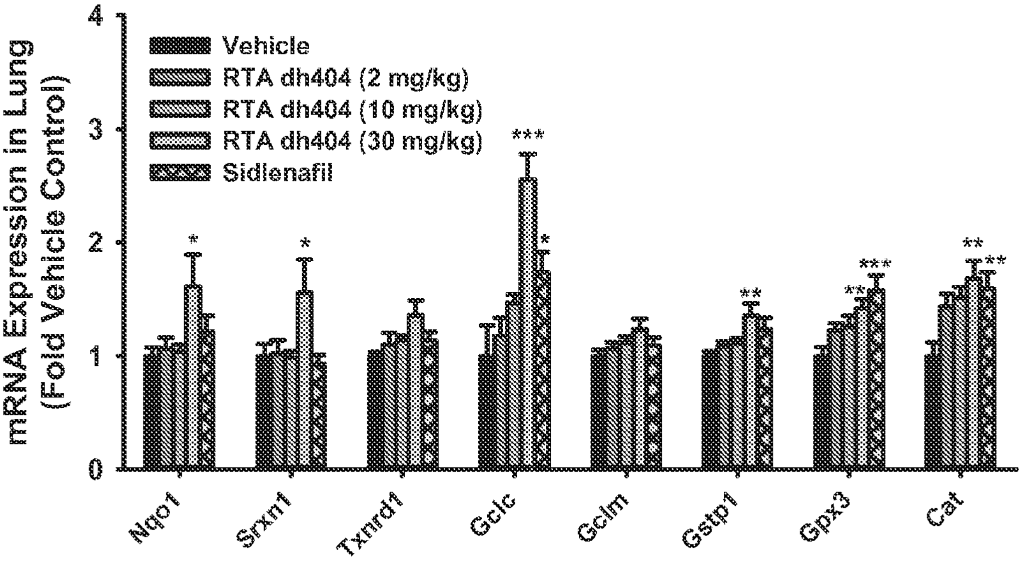
mRNA Expression in Lung
(Fold Vehicle Control)
4
3
2
1
0
Vehicle
RTA dh404 (2 mg/kg)
RTA dh404 (10 mg/kg)
RTA dh404 (30 mg/kg)
Sidlenafil
*
*
***
*
**
**
***
**
**
Nqo1
Srxn1
Txnrd1
Gclc
Gclm
Gstp1
Gpx3
Cat

FIG. 19

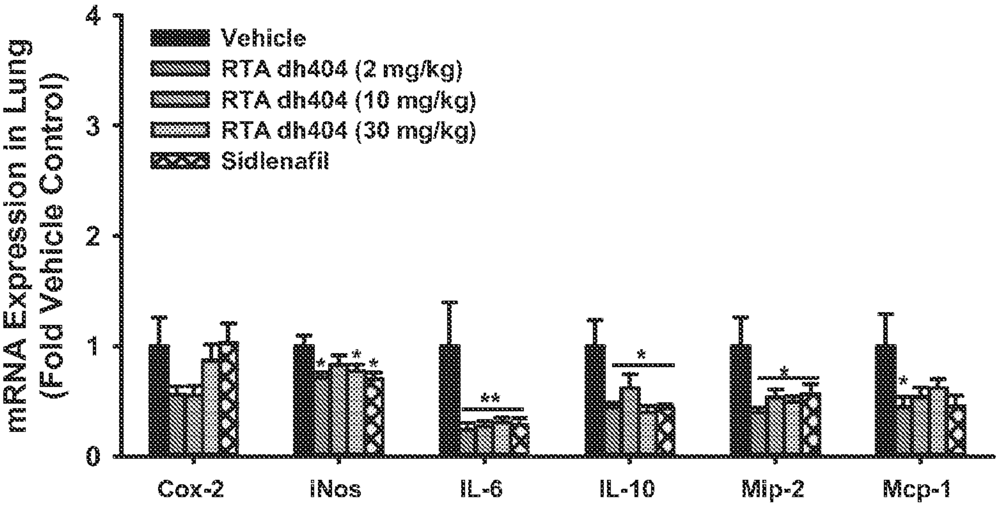
mRNA Expression in Lung
(Fold Vehicle Control)
Vehicle
RTA dh404 (2 mg/kg)
RTA dh404 (10 mg/kg)
RTA dh404 (30 mg/kg)
Sidlenafil
0
1
2
3
4
Cox-2
iNos
IL-6
IL-10
Mip-2
Mcp-1

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61869527
- US 20090326063
- US 20100056777 A
- US 20100048892 A
- US 20100048911 A
- US 20100041904 A
- US 20030232786 A
- US 20080261985 A
- US 20100048887 A
- US 20090048204
- WO 2009023232 A
- WO 2010093944 A
- US 352473
- US 191176
- US 19117608
- US 6025395 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631170 A
- US 5955377 A
- US 20110201130 A
- US 20090048204 A
- US 20090326063 A


### Non-patent literature cited in the description


- **MCCULLOUGH et al.** Cardiac and renal function in patients with type 2 diabetes who have chronic kidney disease; potential effects or bardoxolone methyl.. *DRUG DESIGN DEVELOPMENT AND THERAPY*, 2012, vol. 6, 141-149
- *March's Advanced Organic Chemistry: Reactions, Mechanisms, and Structure*, 2007
- *Handbook of Pharmaceutical Salts: Properties, and Use*, 2002
- Handbook of Pharmaceutical Salts: Properties, and Use. Verlag Helvetica Chimica Acta, 2002
- **BADESCH et al.** Continuous intravenous epoprostenol for pulmonary hypertension due to the scleroderma spectrum of disease. A randomized, controlled trial.. *Ann. Intern. Med.*, 2000, vol. 132, 425-434
- **BADESCH et al.** Medical therapy for pulmonary arterial hypertension: ACCP evidence-based clinical practice guidelines.. *Chest*, 2004, vol. 126, 35S-62S
- **BARST et al.** A comparison of continuous intravenous epoprostenol (prostacyclin) with conventional therapy for primary pulmonary hypertension.. *N. Engl. J. Med.*, 1996, vol. 334, 296-301
- **BLANN et al.** *Thromb. Haemost.*, 2005, vol. 93, 228-235
- **BOLIGNANO et al.** Pulmonary Hypertension in CKD. *Am. J. Kidney Dis.*, 2013, vol. 61, 612-622
- Joint Specialty Committee on Renal Medicine of the Royal College of Physicians and the Renal Association, and the Royal College of General Practitioners.. *Chronic kidney disease in adults: UK guidelines for identification, management and referral. London: Royal College of Physicians*, 2006
- **DENG et al.** Familial primary pulmonary hypertension (gene PPH1) is caused by mutations in the bone morphogenetic protein receptor-II gene.. *Am. J. Hum. Genet.*, 2000, vol. 67, 737-44
- **DHAUN et al.** Urinary endothelin-1 in chronic kidney disease and as a marker of disease activity in lupus nephritis.. *American Journal of Physiology - Renal Physiology*, 2009, vol. 296, F1477-F1483
- **DUMITRASCU et al.** *Oxid. Med. Cell. Longev.*, 2013, vol. 2013, 234631
- **FORSTERMANN**. *Biol. Chem.*, 2006, vol. 387, 1521-1533
- **GALIE et al.** Ambrisentan therapy for pulmonary arterial hypertension.. *J. Am. Coll. Cardiol.*, 2005, vol. 46, 529-535
- **GOLOGANU et al.** *Rom J Intern Med.*, 2012, vol. 50, 259-68

- **GOMEZ-ARROYO et al.** The monocrotaline model of pulmonary hypertension in perspective.. *Am. J. Physiol. Lung Cell. Mol. Physiol.*, 2012, vol. 302, L363-L369
- **GUAZZI** ; **GALIE**. *Eur. Respir. Rev.*, 2012, vol. 21, 338-346
- **HANSSON et al.** *Annu. Rev. Pathol. Mech. Dis.*, 2006, vol. 1, 297-329
- **HAYES** ; **DINKOVA-KOSTOVA**. The Nrf2 regulatory network provides an interface between redox and intermediary metabolism.. *Trends in Biochem Sci.*, 2014, vol. 39, 199-218
- **HONDA et al.** *J. Med. Chem.*, 2000, vol. 43, 1866-1877
- **HONDA et al.** *J. Med. Chem.*, 2000, vol. 43, 4233-4246
- **HONDA et al.** *Bioorg. Med. Chem. Lett.*, 2002, vol. 12, 1027-1030
- **HOSOKAWA**. *Cardiovascular Res.*, 2013, vol. 99, 35-43
- **HUMBERT**. *New Engl. J. Med.*, 2004, vol. 351, 1425-1436
- **JONES et al.** Activation of thromboxane and prostacyclin receptors elicits opposing effects on vascular smooth muscle cell growth and mitogen-activated protein kinase signaling cascades.. *Mol. Pharm.*, 1995, vol. 48, 890-89
- **KATAOKA et al.** Oral sildenafil improves primary pulmonary hypertension refractory to epoprostenol.. *Circ. J.*, 2005, vol. 69, 461-465
- **KIM** ; **VAZIRI**. Contribution of impaired Nrf2-Keap1 pathway to oxidative stress and inflammation in chronic renal failure.. *American Journal of Physiology - Renal Physiology*, 2010, vol. 298, F662-F671
- **KOSMADAKIS et al.** *Ren. Fail.*, 2013, vol. 35, 514-20
- **KOTTGEN et al.** Serum cystatin C in the United States: the Third National Health and Nutrition Examination Survey. *Am. J. Kidney Dis.*, 2008, vol. 51, 385-394
- **LANGLEBEN et al.** STRIDE 1: Effects of the Selective ETA Receptor Antagonist, Sitaxsentan Sodium, in a Patient Population with Pulmonary Arterial Hypertension that meets Traditional Inclusion Criteria of Previous Pulmonary Arterial Hypertension Trials.. *J. Cardiovasc. Pharmacol.*, 2004, vol. 44, S80-S84
- **LEE et al.** Sildenafil for pulmonary hypertension.. *Ann. Pharmacother.*, 2005, vol. 39, 869-884
- **MANN et al.** Avosentan for overt diabetic nephropathy.. *Journal of the American Society of Nephrology*, 2010, vol. 21, 527-535
- **MCGOON et al.** Screening, early detection, and diagnosis of pulmonary arterial hypertension: ACCP evidence-based clinical practice guidelines.. *Chest*, 2004, vol. 126, 14S-34S
- **MCLAUGHLIN et al.** Reduction in pulmonary vascular resistance with long-term epoprostenol (prostacyclin) therapy in primary pulmonary hypertension.. *N. Engl. J. Med.*, 1998, vol. 338, 273-277
- **MCLAUGHLIN et al.** Survival in primary pulmonary hypertension: the impact of epoprostenol therapy.. *Circulation*, 2002, vol. 106, 1477-1482
- **MCLAUGHLIN** ; **RICH**. Pulmonary hypertension.. *Curr. Probl. Cardiol.*, 2004, vol. 29, 575-634
- **OUDIZ et al.** Treprostinil, a prostacyclin analogue, in pulmonary arterial hypertension associated with connective tissue disease.. *Chest*, 2004, vol. 126, 420-427
- **PACKER**. Vasodilator therapy for primary pulmonary hypertension. Limitations and hazards.. *Ann. Intern. Med.*, 1985, vol. 103, 258-270
- **PAULIN et al.** STAT3 signaling in pulmonary arterial hypertension. *JAK-STAT*, 2012, vol. 1, 223-233
- **PEAKE** ; **WHITING**. *Clin. Biochem. Rev.*, 2006, vol. 27, 173-184
- **PERGOLA et al.** Bardoxolone methyl and kidney function in CKD with type 2 diabetes.. *New Engl. J. Med.*, 2011, vol. 365, 327-336
- **RAWLINS et al.** *Am. J. Clin. Pathol.*, 2005, vol. 123, 439-445
- **RICH et al.** The effects of high doses of calcium-channel blockers on survival in primary pulmonary hypertension.. *N. Engl. J. Med.*, 1992, vol. 327, 76-81
- **RUBIN et al.** Bosentan therapy for pulmonary arterial hypertension.. *N. Engl. J. Med.*, 2002, vol. 346, 896-903
- **SCHNEIDER et al.** Contrasting actions of endothelin ETA and ETB receptors in cardiovascular disease.. *Annual Review of Pharmacology and Toxicology*, 2007, vol. 47, 731-759
- **SUSSAN et al.** Targeting Nrf2 with the triterpenoid CDDO-imidazolide attenuate cigarette smoke-induced emphysema and cardiac dysfunction in mice.. *Proc. Natl. Acad. Sci. U.S.A.*, 2009, vol. 106, 250-255
- **SUTENDRA** ; **MICHELAKIS**. The metabolic basis of pulmonary arterial hypertension.. *Cell Metabolism*, 2014, vol. 19, 558-573
- **TAM et al.** A derivative of Bardoxolone methyl, dh404, in an inverse dose-dependent manner, lessens diabetes-associated atherosclerosis and improves diabetic kidney disease.. *Diabetes*, 2014
- **LANE et al.** Heterozygous germline mutations in BMPR2, encoding a TGF-b receptor, cause familial primary pulmonary hypertension.. *Nature Genetics*, 2000, vol. 26, 81-84
- **THENAPPAN et al.** A USA-based registry for pulmonary arterial hypertension: 1982-2006.. *European Respiratory Journal*, 2007, vol. 30, 1103-1110

- **VACHIÉRY** ; **DAVENPORT**. The endothelin system in pulmonary and renal vasculopathy: les liaisons dangereuses.. *European Respiratory Review*, 2009, vol. 18, 260-271
- **VACHIERY** ; **NAEIJE**. Treprostinil for pulmonary hypertension.. *Expert Rev. Cardiovasc. Ther.*, 2004, vol. 2, 183-191
- **VASAN et al.** Congestive heart failure in subjects with normal versus reduced left ventricular ejection fraction: Prevalence and mortality in a population-based cohort.. *Journal of the American College of Cardiology*, 1999, vol. 33, 1948-1955